# EUROPEAN PATENT APPLICATION

(11) **EP 4 840 508 A2**
(43) Date of publication of application: **18.11.2026**
(21) Application number: 26208402.3
(22) Date of filing: 18.03.2024
(51) Int. Cl.: A61P 9/12

(54) **SPRAY DRIED TREPROSTINIL FORMULATION**

(30) Priority: 17.03.2023 US 202363452985 P
(62) Document number(s) of the earlier application(s) in accordance with Art. 76 EPC: 24775547.3 / 4 680 223
(71) Applicant: Insmed Incorporated, Bridgewater, NJ 08807 (US)
(72) Inventor: CIPOLLA, David, New Jersey, 08807 (US); LI, Zhili, New Jersey, 08807-1704 (US); PALWAI, Naveen, New Jersey, 08807-1704 (US); AMIN, Harshh, New Jersey, 08807-1704 (US); SULLIVAN, Eugene, New Jersey, 08807-1704 (US); FERNANDEZ, Carlos, New Jersey, 08807-1704 (US)
(74) Representative: Cooley (UK) LLP

(57) **Abstract**

The present disclosure provides aerosol properties of a powder composition administered via a dry powder inhaler, as well as spray drying processes. Also provided is a method for treating pulmonary hypertension (PH) in a patient comprising administering once daily to the lungs of a patient an effective amount of the powder composition via inhalation with the dry powder inhaler. A system for treating PH is further provided using the powder composition and the dry powder inhaler.

## Description

### CROSS-REFERENCE TO RELATED APPLICATIONS

This application claims priority from U.S. Provisional Application No. 63/452,985, filed March 17, 2023, the disclosure of which is incorporated by reference herein in its entirety.

### BACKGROUND OF THE DISCLOSURE

Pulmonary hypertension is characterized by an abnormally high blood pressure in the lung vasculature. It is a progressive, lethal disease that leads to heart failure and can occur in the pulmonary artery, pulmonary vein, or pulmonary capillaries. Symptomatic patients experience shortness of breath, dizziness, fainting, and other symptoms, all of which are made worse by exertion. There are multiple causes, and can be of unknown origin, idiopathic, and can lead to hypertension in other systems, for example, portopulmonary hypertension in which patients have both portal and pulmonary hypertension.

Pulmonary hypertension (PH) has been classified into five groups by the World Health Organization (WHO). Group 1 PH is called pulmonary arterial hypertension (PAH), and includes PAH that has no known cause (idiopathic), inherited PAH (*i.e.*, familial PAH or FPAH), PAH that is caused by drugs or toxins, and PAH caused by conditions such as connective tissue diseases, HIV infection, liver disease, and congenital heart disease. Group 2 PH is characterized as pulmonary hypertension associated with left heart disease. Group 3 PH is characterized as PH associated with lung diseases, such as chronic obstructive pulmonary disease and interstitial lung diseases, as well as PH associated with sleep-related breathing disorders (*e.g*., sleep apnea). Group 4 PH is PH due to chronic thrombotic and/or embolic disease, *e.g.*, PH caused by blood clots in the lungs or blood clotting disorders. Group 5 PH includes PH caused by other disorders or conditions, *e.g*., blood disorders (*e.g*., polycythemia vera, essential thrombocythemia), systemic disorders (*e.g*., sarcoidosis, vasculitis), and metabolic disorders (*e.g*., thyroid disease, glycogen storage disease).

Pulmonary arterial hypertension (PAH) afflicts approximately 200,000 people globally with approximately 30,000-40,000 of those patients in the United States. PAH patients experience constriction of pulmonary arteries which leads to high pulmonary arterial pressures, making it difficult for the heart to pump blood to the lungs. Patients suffer from shortness of breath and fatigue which often severely limits the ability to perform physical activity.

The New York Heart Association (NYHA) has categorized PAH patients into four functional classes to rate the severity of the disease. Class I PAH patients as categorized by the NYHA do not have a limitation of physical activity, as ordinary physical activity does not cause undue dyspnoea or fatigue, chest pain, or near syncope. Class II PAH patients as categorized by the NYHA have a slight limitation on physical activity. These patients are comfortable at rest, but ordinary physical activity causes undue dyspnoea or fatigue, chest pain or near syncope. Class III PAH patients as categorized by the NYHA have a marked limitation of physical activity. Although comfortable at rest, class III PAH patients experience undue dyspnoea or fatigue, chest pain or near syncope as a result of less than ordinary physical activity. Class IV PAH patients as categorized by the NYHA are unable to carry out any physical activity without symptoms. Class IV PAH patients might experience dyspnoea and/or fatigue at rest, and discomfort is increased by any physical activity. Signs of right heart failure are often manifested by class IV PAH patients.

Patients with PAH are treated with an endothelin receptor antagonist (ERA), phosphodiesterase type 5 (PDE-5) inhibitor, a guanylate cyclase stimulator, a prostanoid (*e.g.*, prostacyclin), or a combination thereof. ERAs include ambrisentan (Letairis^{®}), sitaxentan, bosentan (Tracleer^{®}), and macitentan (Opsumit^{®}). PDE-5 inhibitors indicated for the treatment of PAH include sildenafil (Revatio^{®}) and tadalafil (Adcirca^{®}). Prostanoids indicated for the treatment of PAH include iloprost (Ventavis^{®}), epoprosentol (Flolan^{®}, Veletri^{®}) and treprostinil (Remodulin^{®}, Tyvaso^{®}). The one approved guanylate cyclase stimulator is riociguat (Adempas^{®}). Additionally, patients are often treated with combinations of the aforementioned compounds.

Dry powder inhalers (DPIs) may be used to administered drugs to patients via inhalation. Typically, powder formulations are prepared using a blending process to reduce aggregation and provide sufficient flowability, and thus achieve therapeutic benefit. Unfortunately, the fine particle fraction (FPF) - the amount of drug-containing particles that are small enough (< 5 µm) to enter the lungs and provide a clinical effect - is typically low, often less than 50%.

There is a need for developing powder compositions (e.g., treprostinil palmitil inhalation powder) with improved formulation properties, which are administered via a dry powder inhaler for the treatment of patients with PH and PAH.

### SUMMARY OF THE DISCLOSURE

The present disclosure addresses the need for novel treatment options for pulmonary hypertension (PH) (including pulmonary arterial hypertension (PAH) and PH associated with interstitial lung disease), portopulmonary hypertension (PPH), and pulmonary fibrosis by providing improved powder compositions of treprostinil prodrugs administered via a dry powder inhaler, and methods for administering the same to patients in need of treatment.

In one aspect, the present disclosure relates to a powder composition comprising (a) from about 0.5 wt% to about 5 wt% of a compound of Formula (I): 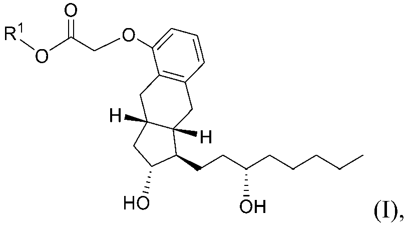 a stereoisomer thereof, or a pharmaceutically acceptable salt thereof, wherein R¹ is tetradecyl, pentadecyl, hexadecyl, heptadecyl, or octadecyl; (b) from about 10 wt% to about 61 wt% of leucine, and the balance being (c) a sugar selected from the group consisting of trehalose and mannitol. The entirety of (a), (b), and (c), is 100 wt%. When the powder composition is administered via a dry powder inhaler, the dry powder inhaler provides an aerosol comprising a fine particle fraction (FPF) greater than or equal to about 68%, when measured using a Next Generation Impactor (NGI), with an inspiratory flow rate of 60 liter per minute (L/min), inspiratory volume of 4 L, and inhalation acceleration rate of 0.5-20 L/sec². The powder composition, in one embodiment, is a spray-dried powder composition.

In some embodiments, the composition includes from about 29 wt% to about 61 wt% of leucine. In even a further embodiment, the composition comprises 0.5 wt% to about 4 wt% of the compound of Formula (I), a stereoisomer thereof, or a pharmaceutically acceptable salt thereof.

In some embodiments, the FPF ranges from about 68% to about 73%. In some embodiments, the FPF ranges from about 68% to about 80%. In some embodiments, the FPF ranges from about 78% to about 80%.

In some embodiments, the aerosol comprises a fine particle dose (FPD) greater than or equal to 50% of a loaded dose of the powder composition. In some embodiments, the FPD ranging from about 53% to about 77% or from about 60% to about 65%.

In some embodiments, the aerosol comprises an emitted dose (ED) greater than or equal to 72%. In some embodiments, the ED ranges from about 79% to about 89%.

In some embodiments, the aerosol comprises a mass mean aerodynamic diameter (MMAD) less than 5 µm. In some embodiments, the MMAD ranges from about 0.75 µm to about 3.0 µm or from about 0.8 µm to about 2.0 µm.

In some embodiments, the powder composition comprises particles, at least a portion of the particles comprising: (i) a compound of Formula (I) or stereoisomer or a pharmaceutically acceptable salt, (ii) leucine, and (iii) trehalose, mannitol or a combination thereof, wherein the particles have an average diameter ranging from about 1.0 µm to about 2.0 µm. In some embodiments, the particles have a D10 of about 0.3 µm. In some embodiments, the particles have a D50 of about 1.7 µm. In some embodiments, the particles have a D90 of about 3.8 µm. The powder composition, in one embodiment, is a spray-dried powder composition.

The stereoisomer, in one embodiment, is a diastereomer of a compound of Formula (I), or a pharmaceutically acceptable salt thereof. In a further embodiment, the stereoisomer is a diastereomer of a compound of Formula (I). In another embodiment, the stereoisomer is a diastereomer of a pharmaceutically acceptable salt of a compound of Formula (I).

In one embodiment, R¹ is tetradecyl. In a further embodiment, R¹ is linear tetradecyl.

In one embodiment, R¹ is pentadecyl. In a further embodiment, R¹ is linear pentadecyl.

In one embodiment, R¹ is heptadecyl. In a further embodiment, R¹ is linear heptadecyl.

In one embodiment, R¹ is octadecyl. In a further embodiment, R¹ is linear octadecyl.

In one embodiment, R¹ is hexadecyl. In a further embodiment, R¹ is linear hexadecyl.

In one embodiment, the compound of Formula (I), a stereoisomer thereof, or a pharmaceutically acceptable salt thereof, is present at from about 0.5 wt% to about 4 wt% of the total weight of the powder composition. In a further embodiment, R¹ is hexadecyl. In even a further embodiment, R¹ is linear hexadecyl. In even a further embodiment, the compound of Formula (I), or a pharmaceutically acceptable salt thereof is present at from about 2 wt% to about 4 wt% of the total weight of the powder composition. The powder composition, in one embodiment, is a spray-dried powder composition.

In one embodiment, the compound of Formula (I), a stereoisomer thereof, or a pharmaceutically acceptable salt thereof, is present at from about 1 wt% to about 4 wt% of the total weight of the powder composition. In a further embodiment, R¹ is hexadecyl. In even a further embodiment, R¹ is linear hexadecyl.

In one embodiment, the compound of Formula (I), a stereoisomer thereof, or a pharmaceutically acceptable salt thereof, is present at from about 1 wt% to about 3.5 wt% of the total weight of the powder composition. In a further embodiment, R¹ is hexadecyl. In even a further embodiment, R¹ is linear hexadecyl.

In one embodiment, the compound of Formula (I), a stereoisomer thereof, or a pharmaceutically acceptable salt thereof, is present at from about 1 wt% to about 3 wt% of the total weight of the powder composition. In a further embodiment, R¹ is hexadecyl. In even a further embodiment, R¹ is linear hexadecyl.

In one embodiment, the compound of Formula (I), a stereoisomer thereof, or a pharmaceutically acceptable salt thereof, is present at from about 1.5 wt% to about 4 wt% of the total weight of the powder composition. In a further embodiment, R¹ is hexadecyl. In even a further embodiment, R¹ is linear hexadecyl.

In one embodiment, the compound of Formula (I), a stereoisomer thereof, or a pharmaceutically acceptable salt thereof, is present at from about 0.8 wt% to about 4 wt% of the total weight of the powder composition. In a further embodiment, R¹ is hexadecyl. In even a further embodiment, R¹ is linear hexadecyl.

In one embodiment, the compound of Formula (I), a stereoisomer thereof, or a pharmaceutically acceptable salt thereof, is present at from about 0.8 wt% to about 3.3 wt% of the total weight of the powder composition. In a further embodiment, R¹ is hexadecyl. In even a further embodiment, R¹ is linear hexadecyl.

In one embodiment, the compound of Formula (I), a stereoisomer thereof, or a pharmaceutically acceptable salt thereof is present at from about 1 wt% to about 2 wt% of the total weight of the powder composition. In a further embodiment, R¹ is hexadecyl. In even a further embodiment, R¹ is linear hexadecyl.

In one embodiment, the compound of Formula (I), a stereoisomer thereof, or a pharmaceutically acceptable salt thereof is present at from about 1 wt% to about 1.5 wt% of the total weight of the powder composition. In a further embodiment, R¹ is hexadecyl. In even a further embodiment, R¹ is linear hexadecyl.

In one embodiment, the compound of Formula (I), a stereoisomer thereof, or a pharmaceutically acceptable salt thereof is present at about 1 wt% of the total weight of the powder composition. In a further embodiment, R¹ is hexadecyl. In even a further embodiment, R¹ is linear hexadecyl.

In one embodiment, the compound of Formula (I), a stereoisomer thereof, or a pharmaceutically acceptable salt thereof is present at about 2 wt% of the total weight of the powder composition. In a further embodiment, R¹ is hexadecyl. In even a further embodiment, R¹ is linear hexadecyl.

In one embodiment, the compound of Formula (I), a stereoisomer thereof, or a pharmaceutically acceptable salt thereof is present at about 3 wt% of the total weight of the powder composition. In a further embodiment, R¹ is hexadecyl. In even a further embodiment, R¹ is linear hexadecyl.

In one embodiment, the compound of Formula (I), a stereoisomer thereof, or a pharmaceutically acceptable salt thereof is present at about 4 wt% of the total weight of the powder composition. In a further embodiment, R¹ is hexadecyl. In even a further embodiment, R¹ is linear hexadecyl.

In one embodiment, the leucine is present at from about 20 wt% to about 40 wt% of the total weight of the powder composition. In a further embodiment, R¹ is hexadecyl. In even a further embodiment, R¹ is linear hexadecyl. In even a further embodiment, the compound of Formula (I), a stereoisomer thereof, or a pharmaceutically acceptable salt thereof, is present at from about 0.5 wt% to about 4 wt% of the total weight of the powder composition.

In another embodiment, the leucine is present at from about 29 wt% to about 61 wt% of the total weight of the powder composition. In a further embodiment, R¹ is hexadecyl. In even a further embodiment, R¹ is linear hexadecyl. In even a further embodiment, the compound of Formula (I), a stereoisomer thereof, or a pharmaceutically acceptable salt thereof, is present at from about 0.5 wt% to about 4 wt% of the total weight of the powder composition.

In another embodiment, the leucine is present at from about 25 wt% to about 35 wt% of the total weight of the powder composition. In a further embodiment, R¹ is hexadecyl. In even a further embodiment, R¹ is linear hexadecyl. In even a further embodiment, the compound of Formula (I), a stereoisomer thereof, or a pharmaceutically acceptable salt thereof, is present at from about 0.5 wt% to about 4 wt% of the total weight of the powder composition.

In another embodiment, the leucine is present at about 40 wt% to 61 wt% of the total weight of the powder composition. In a further embodiment, R¹ is hexadecyl. In even a further embodiment, R¹ is linear hexadecyl. In even a further embodiment, the compound of Formula (I), a stereoisomer thereof, or a pharmaceutically acceptable salt thereof, is present at from about 0.5 wt% to about 4 wt% of the total weight of the powder composition. In a further embodiment, the leucine is present at about 45 wt% to 61 wt% of the total weight of the powder composition. In even a further embodiment, the leucine is present at about 55 wt% to 61 wt% of the total weight of the powder composition.

In another embodiment, the leucine is present at from about 28 wt% to about 33 wt% of the total weight of the powder composition. In a further embodiment, R¹ is hexadecyl. In even a further embodiment, R¹ is linear hexadecyl. In a further embodiment, the compound of Formula (I), or a pharmaceutically acceptable salt thereof, is present at from about 0.5 wt% to about 4 wt% of the total weight of the powder composition.

In another embodiment, the leucine is present at from about 25 wt% to about 33 wt% of the total weight of the powder composition, for example, at from about 27 wt% to about 33 wt%, from about 27 wt% to about 31 wt%, from about 27 wt% to about 30 wt%, from about 28 wt% to about 30 wt%, or at about 30 wt% of the total weight of the powder composition. In a further embodiment, R¹ is hexadecyl. In even a further embodiment, R¹ is linear hexadecyl.

In one embodiment, the powder composition provided herein has a leucine-to-mannitol weight ratio of about 0.40-to-1 (leucine-to-mannitol) to about 0.50-to-1 (leucine-to-mannitol). In another embodiment, the powder composition provided herein has a leucine-to-mannitol weight ratio of about 0.75-to-1 (leucine-to-mannitol) to about 0.90-to-1 (leucine-to-mannitol). In yet another embodiment, the powder composition provided herein has a leucine-to-mannitol weight ratio of about 0 about 1.5-to-1 (leucine-to-mannitol) to about 1.7-to-1 (leucine-to-mannitol).

In one embodiment, the sugar is mannitol. In a further embodiment, R¹ is hexadecyl. In a further embodiment, R¹ is linear hexadecyl.

In one embodiment, the powder composition includes (a) about 1 wt% of the compound of Formula (I), a stereoisomer thereof, or a pharmaceutically acceptable salt thereof, (b) about 29.3 wt% or about 29.6 wt% of the leucine, and the balance being (c) mannitol. In a further embodiment, R¹ is hexadecyl. In a further embodiment, R¹ is linear hexadecyl.

In one embodiment, the powder composition includes (a) about 3 wt% of the compound of Formula (I), a stereoisomer thereof, or a pharmaceutically acceptable salt thereof, (b) about 29.3 wt% or about 29.6 wt% of the leucine, and the balance being (c) mannitol. In a further embodiment, R¹ is hexadecyl. In a further embodiment, R¹ is linear hexadecyl.

The present disclosure provides that a powder composition comprises from about 160 µg to about 320 µg of a compound of Formula (I), wherein R1 is linear hexadecyl, and upon once daily inhalation administration via a dry powder inhaler, the dry powder inhaler provides an aerosol comprising a fine particle fraction (FPF) greater than or equal to about 68%, as measured using a Next Generation Impactor (NGI). In some embodiments, the dry powder inhaler provides an aerosol comprising the FPF that ranges from 78% to about 80%. In some embodiments, the dry powder inhaler provides the dry powder inhaler provides an aerosol comprising the FPF that ranges from 68% to about 73%. In some embodiments, the dry powder inhaler provides an aerosol comprising a fine particle dose (FPD) greater than or equal to 50% of a loaded dose of the powder composition. In some embodiments, the dry powder inhaler provides an aerosol comprising the FPD that ranges from 60% to about 65%. In some embodiments, the dry powder inhaler provides an aerosol comprising the FPF that ranges from 53% to about 65%. In some embodiments, the dry powder inhaler provides an aerosol comprising an emitted dose (ED) greater than or equal to 79%. In some embodiments, the dry powder inhaler provides an aerosol comprising the ED that ranges from about 79% to about 89%. In some embodiments, the dry powder inhaler provides an aerosol comprising a mass mean aerodynamic diameter (MMAD) less than 5 µm. In some embodiments, the dry powder inhaler provides an aerosol comprising the MMAD that ranges from about 1.0 µm to about 1.3 µm. In some embodiments, the dry powder inhaler provides an aerosol comprising the MMAD that ranges from about 1.3 µm to about 1.7 µm.

In a further embodiments, the composition comprises a dose selected from the group consisting of 80 µg, 160 µg, 240 µg, 320 µg, 400 µg, 480 µg, 560 µg, 640 µg, 675 µg, 720 µg, 800 µg, 880 µg, 960 µg, 1040 µg , 1120 µg, 1200 µg, and 1280 µg of a compound of Formula (I). In yet a further embodiment, the composition comprises a dose selected from the group consisting of 80 µg, 160 µg, 240 µg, 320 µg, 400 µg, 480 µg and 640 µg of a compound of Formula (I). The dose can be present, e.g., in one dry powder capsule, or multiple capsules.

In another aspect of the disclosure, a method for treating pulmonary hypertension (PH) in a patient in need thereof is provided. The method includes administering an effective amount of the powder composition disclosed herein to the lungs of the patient by inhalation via a dry powder inhaler. In embodiments, the effective amount comprises 80 µg-1280 µg of a compound of Formula (I). In embodiments, the effective amount comprises 80 µg, 160 µg, 240 µg, 320 µg, 400 µg, 480 µg, 640 µg, 675 µg, 720 µg, 800 µg, 880 µg, 960 µg, 1040 µg , 1120 µg, 1200 µg, of 1280 µg of a compound of Formula (I).

In one embodiment, the PH is group 1 PH, as characterized by the World Health Organization (WHO).

The pulmonary hypertension, in one embodiment, is pulmonary arterial hypertension (PAH). The PAH, in one embodiment, is class I PAH, as characterized by the New York Heart Association (NYHA). In another embodiment, the PAH is class II PAH, as characterized by NYHA. In another embodiment, the PAH is class III PAH, as characterized by NYHA. In another embodiment, the PAH is class IV PAH, as characterized by NYHA.

In another embodiment, the PH is group 2 PH, as characterized by the WHO. In another embodiment, the PH is group 3 PH, as characterized by the WHO. In a further embodiment, the group 3 PH is PH associated with interstitial lung disease (ILD). In another embodiment, the PH is group 4 PH, as characterized by the WHO. In another embodiment, the PH is group 5 PH, as characterized by the WHO.

In one embodiment of the treatment methods described herein, the administering is conducted in a once-a-day or twice-a-day.

In still another aspect, the present disclosure relates to a system for treating PH. The system includes one of the powder compositions disclosed herein and a dry powder inhaler (DPI), which may be single dose or a multidose inhaler. In another embodiment, the DPI is pre-metered or device-metered.

In some embodiments, the powder composition described herein and used in the methods described herein comprises from about 1 wt% to about 5 wt% of a compound of Formula (I), or a pharmaceutically acceptable salt thereof, with the balance being one or more pharmaceutically acceptable excipients which are suitable for use in a dry powder inhaler.

### BRIEF DESCRIPTION OF THE FIGURES

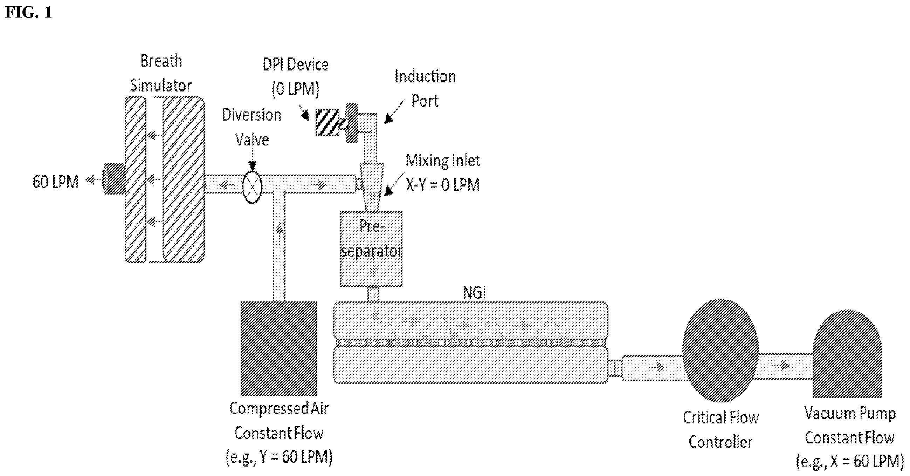

**FIG. 1** is a schematic diagram showing system setup with breath simulator and Next Generation Impactor (NGI) before simulation started.

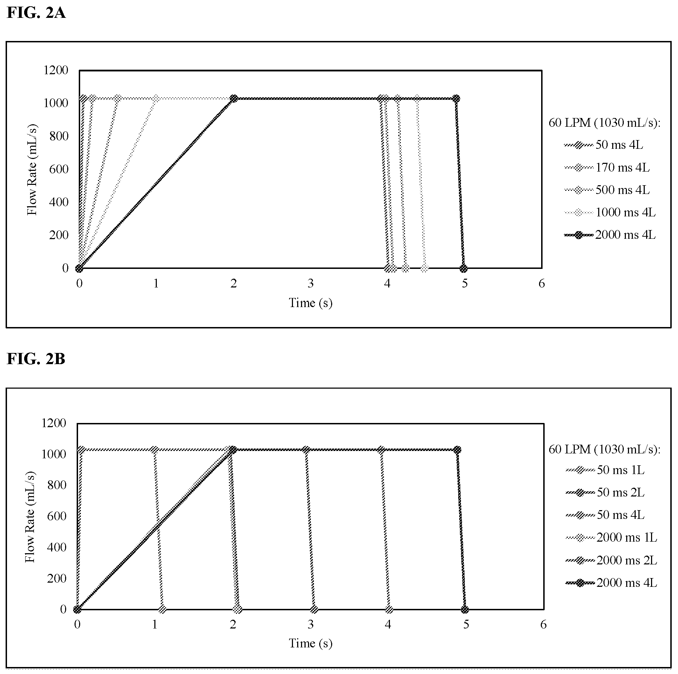

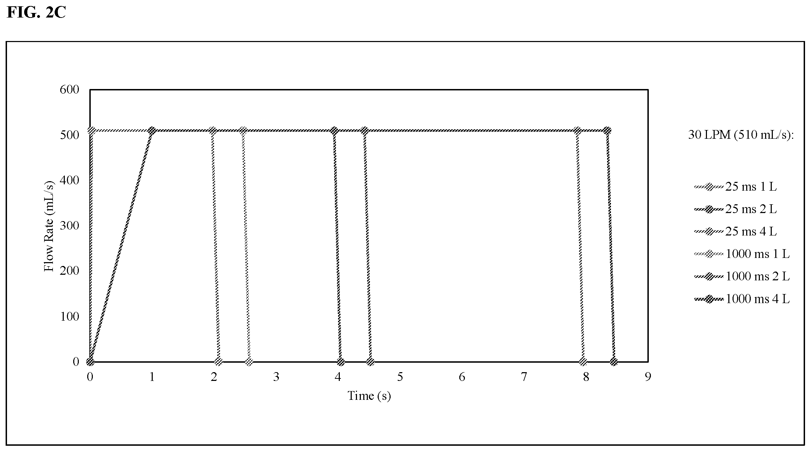

**FIG. 2A** is a graph showing various simulated inhalation profiles at different acceleration rates with a target inspiratory flow rate of 60 LPM (1030 mL/s) and total inhalation volume of 4 Liters. **FIG. 2B** is a graph showing various simulated inhalation profiles at two acceleration rates (50 ms vs 2000 ms) with a target inspiratory flow rate of 60 LPM (1030 mL/s) and total inhalation volume of 1L, 2L, or 4L. **FIG. 2C** is a graph showing various simulated inhalation profiles at two acceleration rates (25 ms vs 1000 ms) with a target inspiratory flow rate of 30 LPM (510 mL/s) and total inhalation volume of 1L, 2L, or 4L.

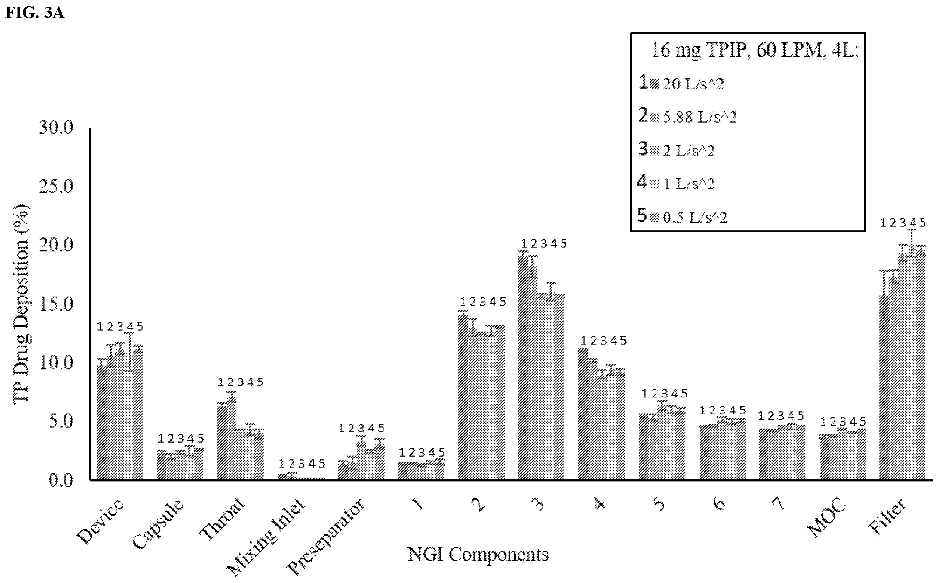

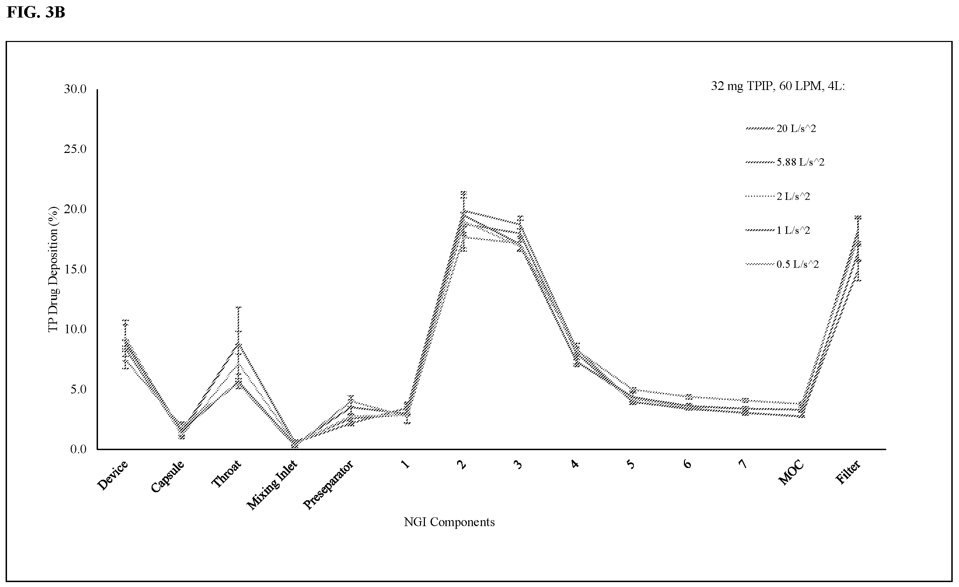

**FIGs. 3A-3B** are graphs showing TP aerosol deposition profiles in the NGI as a function of acceleration rate. The profiles show the percentage of TP recovered (mean + SD) from each component of the NGI at each inhalation acceleration rate to reach the 60 LPM peak inspiratory flow rate with a 4 L inhalation volume. **FIG. 3A**:16 mg TPIP capsules. **FIG. 3B**:32 mg TPIP capsules. Acceleration rates are 20 L/s² (blue), 5.88 L/s² (orange), 2 L/s² (gray), 1 L/s² (yellow), and 0.5 L/s² (light blue).

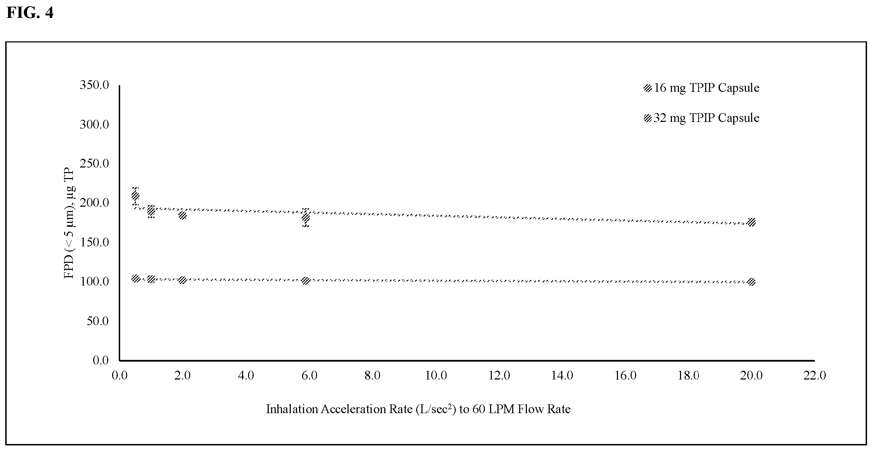

**FIG. 4** is a graph showing TP fine particle dose (FPD) as a function of inhalation acceleration rate to reach the 60 LPM peak inspiratory flow rate for a 4 L inhalation volume. The dotted lines show the change in FPD values for the 16 mg TPIP (blue) and 32 mg TPIP (orange) capsule as the acceleration rate increases from 0.5 L/sec² (2000 ms ramp) to 20 L/sec² (50 ms ramp). *p-value > 0.05 overall inhalation acceleration rate comparison for the 16 mg TPIP capsule, #p-value < 0.05 overall inhalation acceleration rate comparison for the 32 mg capsule associated with data from Tables 1 and 2.

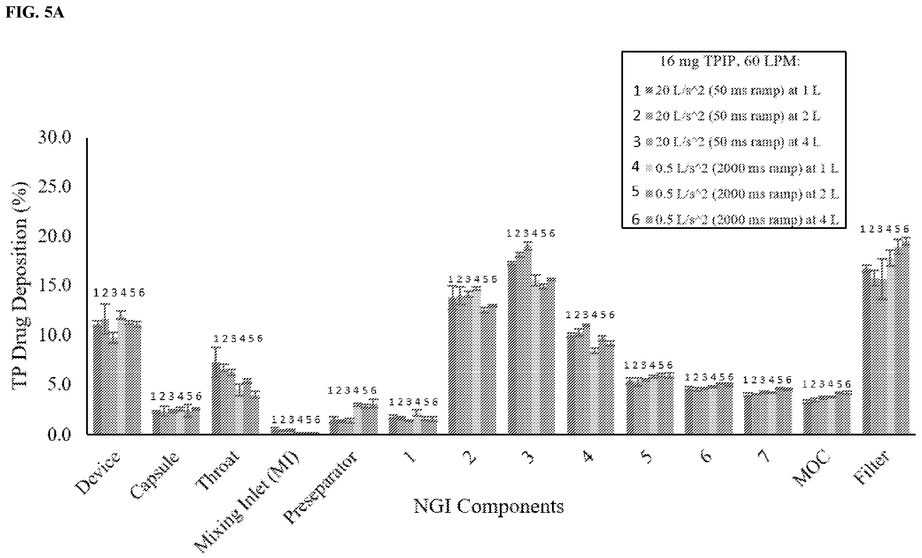

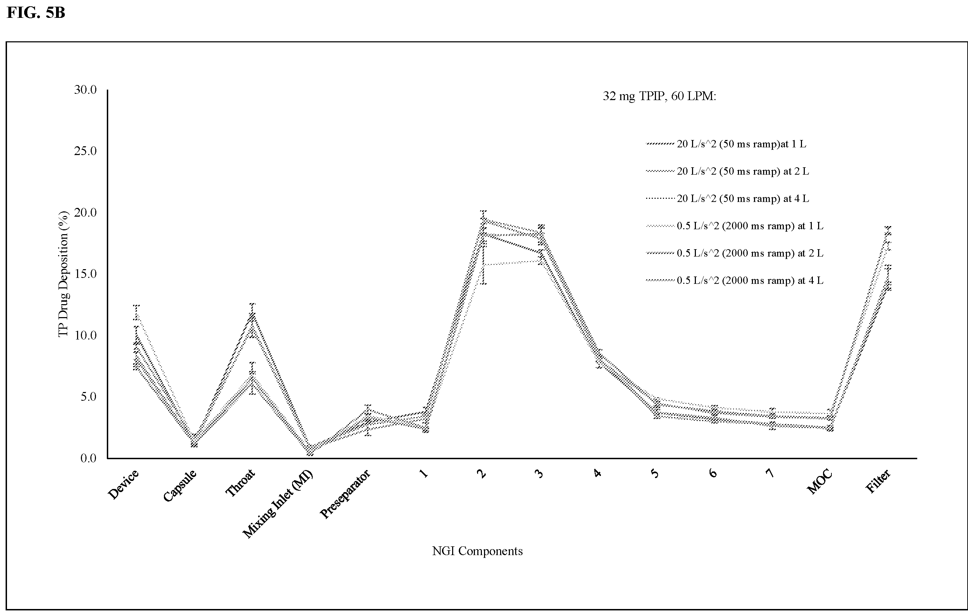

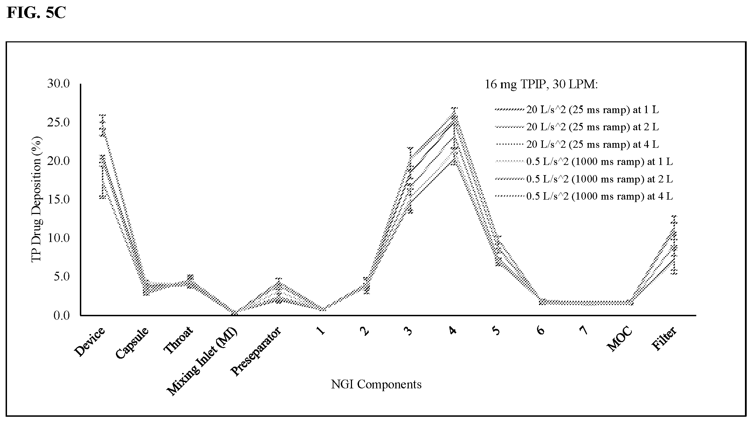

**FIGs. 5A-5C** are graphs showing TP aerosol deposition profiles in the NGI as a function of inhaled volume. The profiles show the percentage of TP recovered (mean + SD) from each component of the NGI for each inhalation volume with 20 and 0.5 L/s² acceleration rates to reach the 60 and 30 LPM peak inspiratory flow rates. **FIG 5A**:16 mg TPIP capsules at a 50 ms ramp time for the 1 L (blue), 2 L (orange), and 4 L (gray) volumes, and 2000 ms ramp time for the 1 L (yellow), 2 L (light blue), and 4 L (green) volumes to reach the 60 LPM peak flow rate. **FIG 5B**:32 mg TPIP capsules at a 50 ms ramp time for the 1 L (blue), 2 L (orange), and 4 L (gray) volumes, and 2000 ms ramp time for the 1 L (yellow), 2 L (light blue), and 4 L (green) volumes to reach the 60 LPM peak flow rate. **FIG 5C**:16 mg TPIP capsules at a 25 ms ramp time for the 1 L (blue), 2 L (orange), and 4 L (gray) volumes, and 1000 ms ramp time for the 1 L (yellow), 2 L (light blue), and 4 L (green) volumes to reach the 30 LPM peak flow rate.

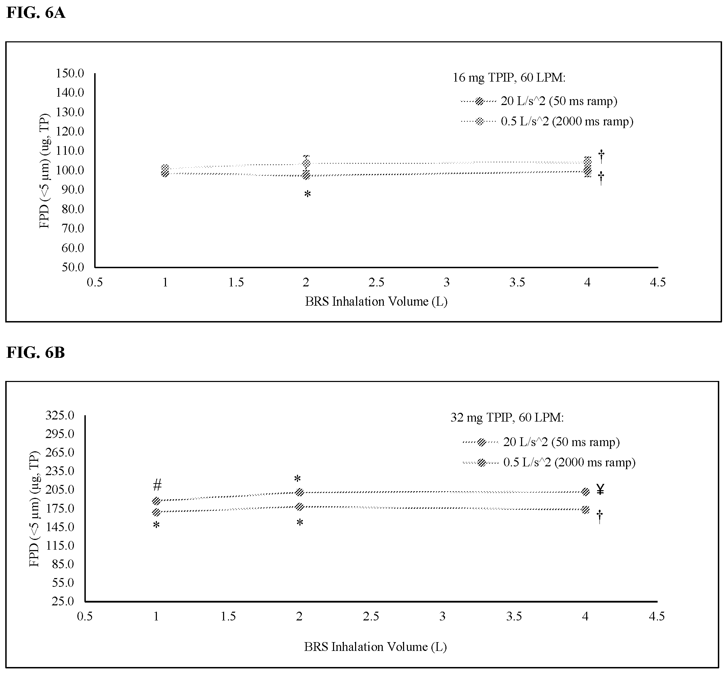

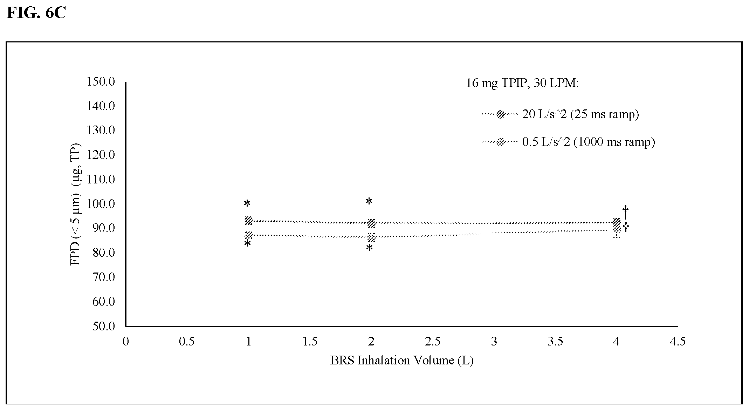

**FIGs. 6A-6C** are graphs showing TP fine particle dose (FPD) trend lines as a function of the total inhalation volume. The dotted lines show the change in FPD values as inhalation volume increases for the 20 L/s² (blue) and 0.5 L/s² (orange) acceleration rates at the 30 and 60 LPM peak inspiratory flow rates. **FIG. 6A**:16 mg TPIP capsules at the 60 LPM flow rate. **FIG. 6B**:32 mg TPIP capsules at the 60 LPM flow rate. **FIG. 6C**:16 mg TPIP capsules at the 30 LPM flow rate. *p-value > 0.05 for the 1 and 2 L inhalation volumes compared to the 4 L inhalation volume, #p-value < 0.05 for the 1 L inhalation volume compared to the 4 L inhalation volume, †p-value > 0.05 for the overall comparison of inhalation volumes for the 20 L/s² acceleration rate (16 mg and 32 mg) and 0.5 L/s² acceleration rate (16 mg), and ¥p-value <0.05 overall comparison of inhalation volume for the 0.5 L/s² acceleration rate (32 mg) associated with data from Tables 3 and 4.

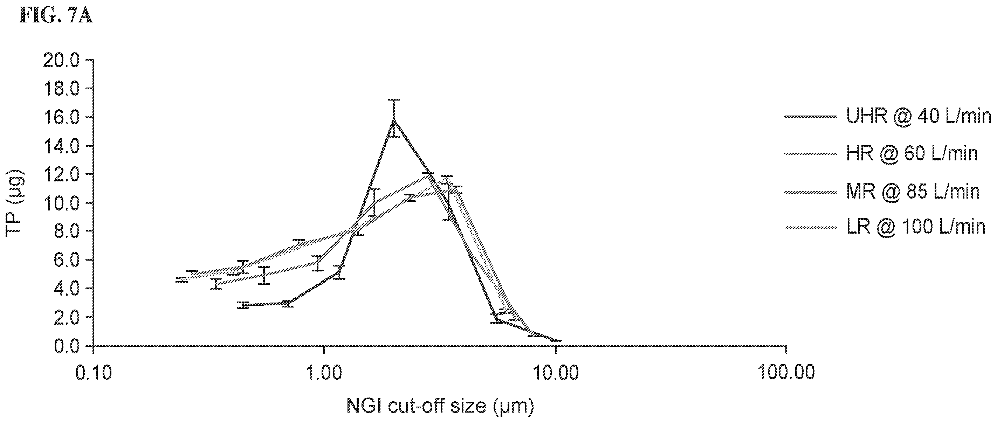

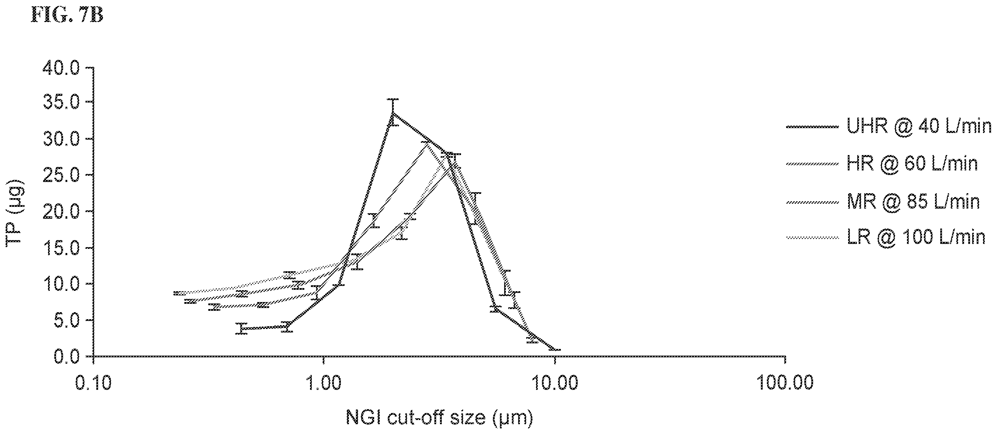

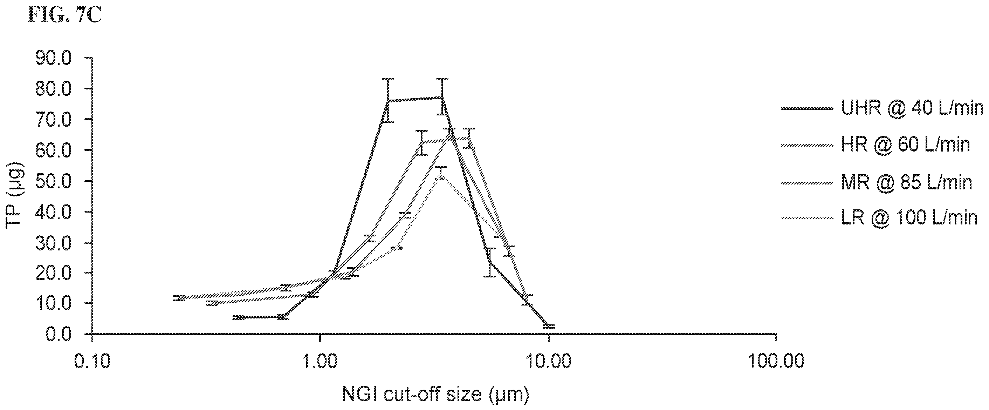

**FIGs. 7A-7C** are graphs showing TP aerosol particle size distribution profiles from the Next Generation Impactor (NGI). The profiles show the recovery of TP (mean ± SD) from each stage of the NGI with respect to its stage cut-off diameter for the ultra-high resistance (UHR, blue), high resistance (HR, orange), medium resistance (MR, gray) and low resistance (LR, yellow) devices. **FIG. 7A**: 8 mg TPIP capsules. **FIG. 7B**: 16 mg TPIP capsules. **FIG. 7C**: 32 mg TPIP capsules.

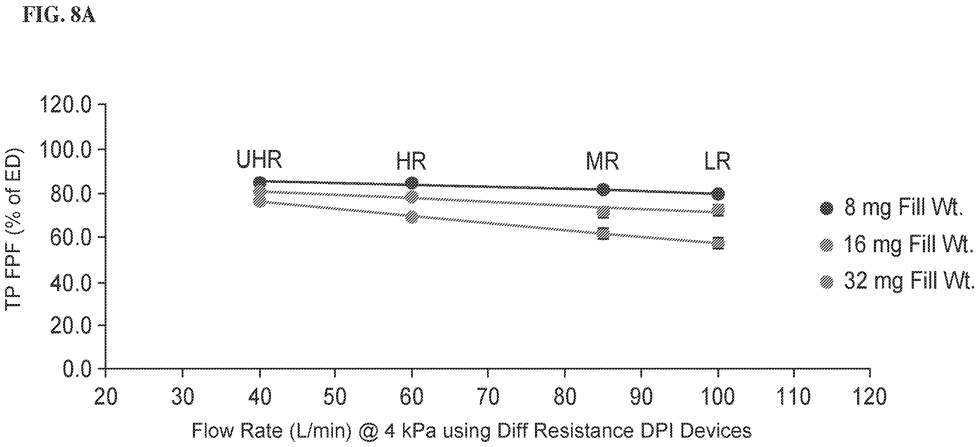

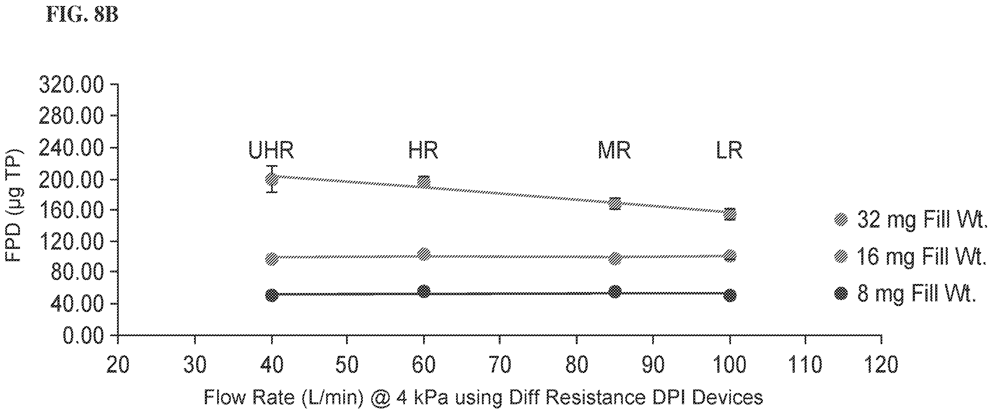

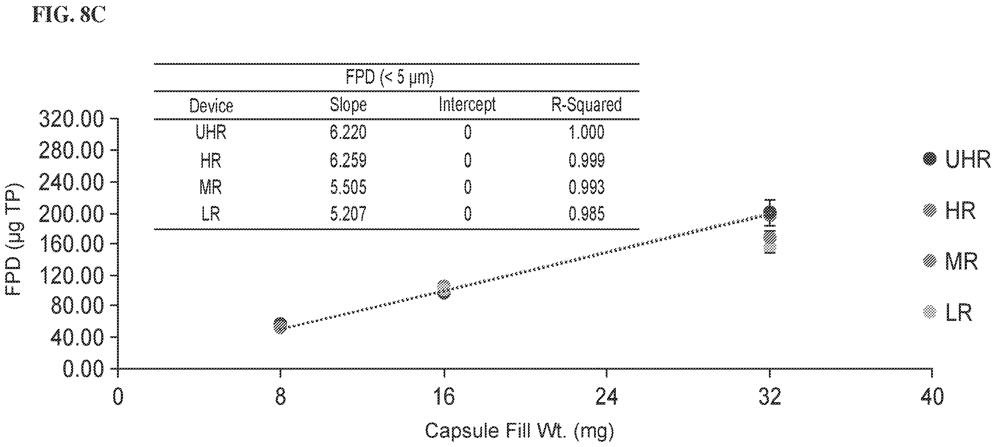

**FIGs. 8A-8C** are graphs showing TP Fine Particle Fraction (FPF) (**FIG. 8A**); Fine Particle Dose (FPD) as a Function of Device Resistance for the 8 mg (blue), 16 mg (orange), and 32 mg (gray) Capsule Dose (**FIG. 8B**); and TP FPD as a Function of Capsule Dose for Each Device (**FIG. 8C**). Trend lines are only shown for the UHR and HR DPIs in **FIG. 8C**. The ultra-high resistance (UHR), the high resistance (HR), the medium resistance (MR) and the low resistance (LR) devices were tested at 40, 60, 85 and 100 LPM, respectively.

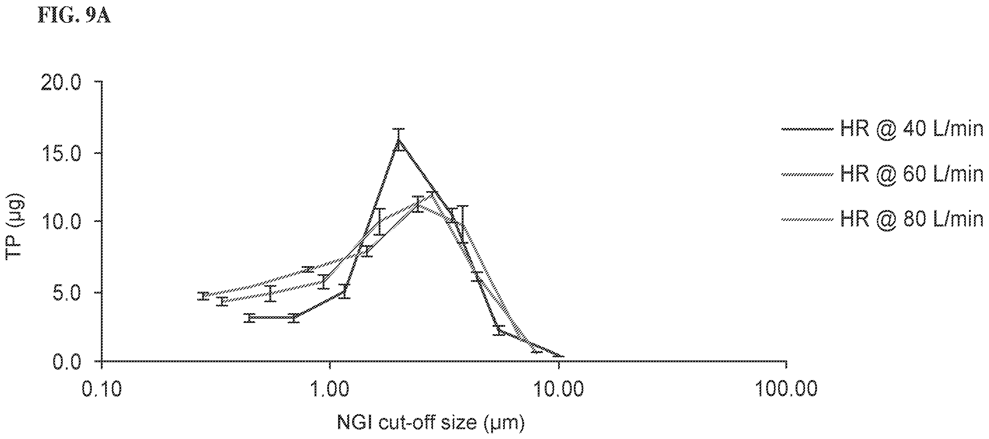

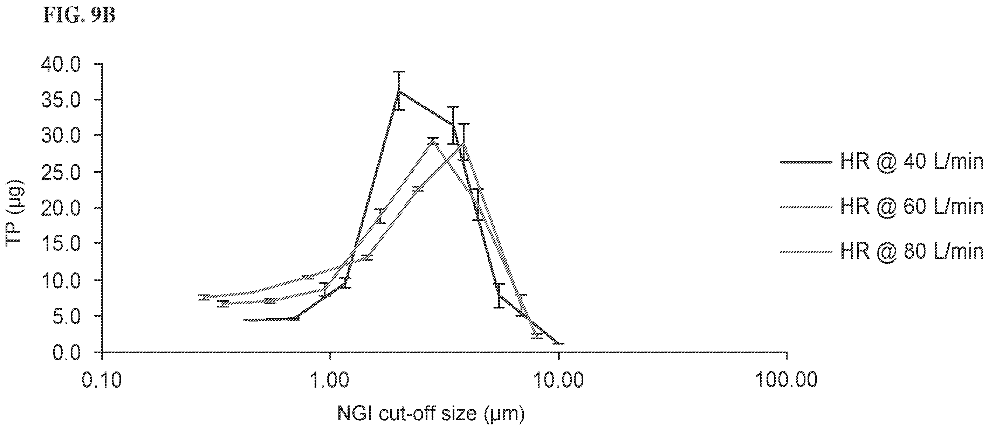

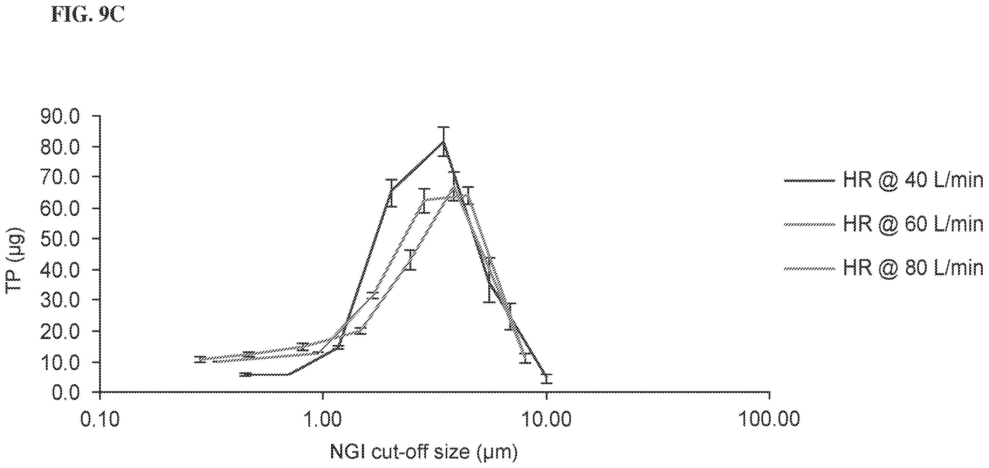

**FIGs. 9A-9C** are graphs showing TP aerosol particle size distribution profiles from the Next Generation Impactor (NGI). The profiles show the recovery of TP (mean ± SD) from each stage of the NGI with respect to its stage cut-off diameter for high resistance (HR) device operated at 40 LPM (blue), 60 LPM (orange), and 80 LPM (gray). **FIG. 9A**: 8 mg TPIP capsules. **FIG. 9B**: 16 mg TPIP capsules. **FIG. 9C**: 32 mg TPIP capsules.

### DETAILED DESCRIPTION OF THE DISCLOSURE

### Definitions

Throughout the present disclosure, the term "about" may be used in conjunction with numerical values and/or ranges. The term "about" is understood to mean those values near to a recited value. For example, "about 40 [units]" may mean within ± 25% of 40 (e.g., from 30 to 50), within ± 20%, ± 15%, ± 10%, ± 9%, ± 8%, ± 7%, ± 6%, ± 5%, ± 4%, ± 3%, ± 2%, ± 1 %, less than ± 1%, or any other value or range of values therein or there below.

The term "pharmaceutically acceptable salt" refers to salts prepared from pharmaceutically acceptable non-toxic bases or acids including inorganic or organic bases and inorganic or organic acids. The nature of the salt is not critical, provided that it is pharmaceutically acceptable. Suitable pharmaceutically acceptable acid addition salts may be prepared from an inorganic acid or from an organic acid. Exemplary pharmaceutical salts are disclosed in Stahl, P.H., Wermuth, C.G., Eds. Handbook of Pharmaceutical Salts: Properties, Selection and Use; Verlag Helvetica Chimica Acta/Wiley-VCH: Zurich, 2002, the contents of which are hereby incorporated by reference in their entirety. Specific non-limiting examples of inorganic acids are hydrochloric, hydrobromic, hydroiodic, nitric, carbonic, sulfuric and phosphoric acid. Appropriate organic acids include, without limitation, aliphatic, cycloaliphatic, aromatic, arylaliphatic, and heterocyclyl containing carboxylic acids and sulfonic acids, for example formic, acetic, propionic, succinic, glycolic, gluconic, lactic, malic, tartaric, citric, ascorbic, glucuronic, maleic, fumaric, pyruvic, aspartic, glutamic, benzoic, anthranilic, mesylic, stearic, salicylic, *p*-hydroxybenzoic, phenylacetic, mandelic, embonic (pamoic), methanesulfonic, ethanesulfonic, benzenesulfonic, pantothenic, toluenesulfonic, 2-hydroxyethanesulfonic, sulfanilic, cyclohexylaminosulfonic, algenic, 3-hydroxybutyric, galactaric or galacturonic acid. Suitable pharmaceutically acceptable salts of free acid-containing compounds disclosed herein include, without limitation, metallic salts and organic salts. Exemplary metallic salts include, but are not limited to, appropriate alkali metal (group Ia) salts, alkaline earth metal (group IIa) salts, and other physiological acceptable metals. Such salts can be made from aluminum, calcium, lithium, magnesium, potassium, sodium and zinc. Exemplary organic salts can be made from primary amines, secondary amines, tertiary amines and quaternary ammonium salts, for example, tromethamine, diethylamine, tetra-*N-*methylammonium, *N*,*N'*-dibenzylethylenediamine, chloroprocaine, choline, diethanolamine, ethylenediamine, meglumine (*N*-methylglucamine) and procaine.

The term "stereoisomer" as used herein refers to two molecules having the same molecular formula and sequence of bonded atoms, but differ in three-dimensional orientations of their atoms in space. One preferred stereoisomer according to the present disclosure is a diastereomer. The stereoisomer, in one embodiment, is a diastereomer of a compound of Formula (I), or a pharmaceutically acceptable salt thereof. In a further embodiment, the stereoisomer is a diastereomer of a compound of Formula (I). In another embodiment, the stereoisomer is a diastereomer of a pharmaceutically acceptable salt of a compound of Formula (I). In yet another embodiment, the stereoisomer is a diastereomer of a compound of Formula (II). In even another embodiment, the stereoisomer is a diastereomer of a pharmaceutically acceptable salt of a compound of Formula (II).

Throughout the present specification, numerical ranges are provided for certain quantities. It is to be understood that these ranges comprise all subranges therein. Thus, the range "50-80" includes all possible ranges therein (e.g., 51-79, 52-78, 53-77, 54-76, 55-75, 60-70, etc.). Furthermore, all values within a given range may be an endpoint for the range encompassed thereby (e.g., the range 50-80 includes the ranges with endpoints such as 55-80, 50-75, etc.).

Throughout the present specification, numerical ranges are described as encompassing "about 80% to about 125%" or "about 80-125%" of a range of values. It is to be understood that these comprise 80% of the lowest endpoint of the range up to 125% of the highest endpoint of the range, and all values therein.

The term "Cₘₐₓ" means the maximum (or peak) treprostinil serum concentration measured after a compound of Formula (I) or (II), a stereoisomer thereof, or a pharmaceutically acceptable salt thereof is administered to the lungs of a subject via a powder composition described herein. In addition, Cₘₐₓ may be measured after a single administration of a compound of Formula (I) or (II), a stereoisomer thereof, or a pharmaceutically acceptable salt thereof, described herein, or treprostinil Cₘₐₓ may be measured at steady state. Unless stated otherwise, Cₘₐₓ refers to the average treprostinil Cₘₐₓ measured after a single administration among a population of subjects (e.g., a population of PH patients).

The term "AUC" means the area under the plasma concentration time curve for treprostinil, measured from time 0 to a certain time post-administration to the lungs of a subject, calculated by a combination of linear and logarithmic trapezoidal methods (Linear up/log down method). In some embodiments, AUC may be measured from time 0 to 24 hours post-administration ("AUC₀₋₂₄") or AUC may be measured form from time 0 to extrapolated to infinity ("AUC_{0-inf}"). In addition, treprostinil AUC may be measured after a single administration or at steady state values. Unless stated otherwise, AUC refers to the average AUC measured after a single administration among a population of subjects (e.g., a population of PH patients).

The term "plasma trough concentration" refers to the treprostinil plasma concentration before administering a subsequent dose of the compounds of Formula (I) or (II), a stereoisomer thereof, or a pharmaceutically acceptable salt thereof. For example, treprostinil plasma trough concentration may be measured within 2 hours, 1 hour, or 30 minutes of administering a subsequent dose. Plasma trough concentrations may be measured after a single administration or may be measured at steady state. Unless stated otherwise, plasma trough levels refer to the average treprostinil trough level measured among a population of subjects (e.g., a population of PH patients).

The term "adult" refers to a human subject, e.g., a human patient that is at least 18 years of age or older. In some embodiments, the adult is 18-100 years of age, e.g., 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, including all values and ranges in between.

The term "cascade impaction" refers to a method of measuring performance properties of a dry powder inhaler. Next Generation Impactor (NGI) is used for cascade impaction measurements discussed herein. In some embodiments, the NGI has an inspiratory (also referred to interchangeably as gas) flow rate of 30-60 (e.g., 30, 40, 50, or 60) liter per minute (L/min), an inspiratory volume of 1-4 L (e.g., 1, 2, 3, or 4 L), and an inhalation acceleration rate of 0.5-20 L/sec². In a specific embodiments, the NGI has an inspiratory flow of 60 L/min, an inspiratory volume of 4L, and inhalation accelerate rate of 0.5-20 L/sec². In embodiments, the inspiratory flow rate corresponds to a 4 kPa air pressure drop for the dry powder inhaler.

Any reference herein to a compound of Formula I includes a pharmaceutically acceptable salt thereof.

The term "geometric standard deviation" or "GSD" is a measure of the spread of an aerodynamic particle size distribution.

Dry powder inhalers (DPIs) are devices that can be used to deliver drugs to the respiratory tract. Upon inhalation, the drugs are transported to the lungs. In order to inhale the drugs via the DPIs, compacted drug powder must be broken up through a process called deagglomeration.

The composition described herein is referred to as a "powder." Such powders administered using a DPI are commonly referred to as a dry powder. Thus, all references to a powder, a powder composition, or a powder formulation used herein encompass a dry powder, a dry powder composition or a dry powder formulation.

Powders used in DPIs are typically formulated by blending the active agent and excipients to prevent aggregation. That is, DPIs typically use a micronized drug blended with carrier particles such as lactose that avoid aggregation and provide sufficient flowability. To enhance the aerosolization of the drug particles, fine lactose particles are often added to the carrier lactose to saturate high-energy binding sites on the carrier lactose and form easily detachable aggregates with the drug particles.

When the powder is emitted from the inhaler, the drug particles are detached from the carrier particles and delivered into the lungs, while the larger carrier particles end up in the upper respiratory tract. In the present disclosure, it was surprisingly discovered that spray drying treprostinil palmitil (e.g., a compound of Formula (I) and (II)) with certain excipients produces free flowing drug particles of appropriate size and particle size distribution such that more drug particles are delivered into the lungs, resulting in improved therapeutic effects (e.g., higher FPF) compared to blended formulations. More specifically, the FPF of blended formulations were typically less than 45% (for example, about 11% to about 43%), while the spray dried powder compositions of the present disclosure have FPF of greater than or equal to about 68%, measured with a inspiratory flow rate of 60 liter per minute (L/min), inspiratory volume of 4 L, and inhalation acceleration rate of 0.5-20 L/sec².

Treprostinil palmitil inhalation powder (TPIP) provided herein is generated by a spray drying process with mannitol and leucine as excipients and is administered via a dry powder inhaler (DPI), such as a high resistance (HR) DPI, e.g., RS01 Berry Global (formerly Plastiape) device. TPIP, in one embodiment, is administered to a patient in need of treatment of pulmonary arterial hypertension (PAH) or pulmonary hypertension due to interstitial lung disease (PH-ILD).

In one aspect of the present disclosure, a powder composition of a treprostinil prodrug is provided. The powder composition comprises:
(a) a compound of Formula (I) or a pharmaceutically acceptable salt thereof, present at from about 0.5 wt% to about 5 wt% of the total weight of the powder composition: 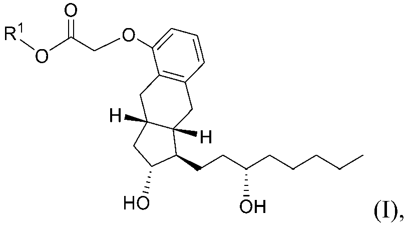 wherein R¹ is tetradecyl, pentadecyl, hexadecyl, heptadecyl, or octadecyl;
(b) from about 10 wt% to about 61 wt% of leucine, and the balance being
(c) a sugar selected from the group consisting of trehalose and mannitol. The entirety of (a), (b), and (c) is 100 wt%. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides one or more of the following: (i) an aerosol comprising a fine particle fraction (FPF) greater than or equal to about 68% (e.g., about 68% to about 80%, about 68% to about 73%, or about about 78% to about 80%), (ii) a fine particle dose (FPD) greater than or equal to 50% of a loaded dose of the powder composition (e.g. about 53% to about 77% or from about 60% to about 65%), (iii) an aerosol comprising an emitted dose (ED) greater than or equal to 72%, e.g., about 79% to about 89%, or (iv) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In a further embodiment, the powder composition comprises from about 25 wt% to about 61 wt% of leucine. In even a further embodiment, the powder composition comprises from about 25 wt% to about 45 wt% of leucine. In another embodiment, the powder composition comprises from about 45 wt% to about 61 wt% of leucine.

In some embodiments, the compound of Formula (I), a stereoisomer thereof, or a pharmaceutically acceptable salt thereof is present at about 0.4 wt% about 0.5 wt%, about 1 wt%, abot 1.1 wt%, about 1.2 wt%, about 1.3 wt%, about 1.5 wt%, about 1.7 wt%, about 2.0 wt%, about 2.3 wt%, about 2.5 wt%, about 2.6 wt%, about 2.7 wt%, about 2.8 wt%, about 2.9 wt%, about 3 wt%, about 3.1 wt%, about 3.2 wt%, about 3.3 wt%, about 3.4 wt%, about 3.5 wt%, about 4 wt%, about 3.5 wt%, or about 5 wt% of the total weight of the powder composition. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

The compound of Formula (I) and pharmaceutically acceptable salts thereof are treprostinil prodrugs as disclosed in International Application Publication WO 2015/061720, the disclosure of which is incorporated herein by reference in its entirety. In some embodiments, the leucine is present at about 25 wt%, about 30 wt%, about 35 wt%, about 40 wt%, about 45 wt%, about 50 wt%, about 55 wt%, about 58 wt%, or about 60 wt% of the total weight of the powder composition. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the leucine is present at from about 25 wt% to about 30 wt%, about 28 wt% to about 30 wt%, about 25 wt% to about 33 wt%, about 27 wt% to about 33 wt%, about 27 wt% to about 31 wt%, from about 27 wt% to about 30 wt%, or from about 28 wt% to about 30 wt% of the total weight of the powder composition. In some embodiments, the leucine is present at from about 40 wt% to about 45 wt%, about 44 wt% to about 51 wt%, or about 43 wt% to about 48 wt% of the total weight of the powder composition.

In one embodiment of the compound of Formula (I), a stereoisomer thereof, or a pharmaceutically acceptable salt thereof, R¹ is tetradecyl. In a further embodiment, R¹ is linear tetradecyl.

In another embodiment of the compound of Formula (I), a stereoisomer thereof, or a pharmaceutically acceptable salt thereof, R¹ is pentadecyl. In a further embodiment, R¹ is linear pentadecyl.

In another embodiment of the compound of Formula (I), a stereoisomer thereof, or a pharmaceutically acceptable salt thereof, R¹ is heptadecyl. In a further embodiment, R¹ is linear heptadecyl.

In another embodiment of the compound of Formula (I), a stereoisomer thereof, or a pharmaceutically acceptable salt thereof, R¹ is octadecyl. In a further embodiment, R¹ is linear octadecyl.

In another embodiment of the compound of Formula (I), a stereoisomer thereof, or a pharmaceutically acceptable salt thereof, R¹ is hexadecyl. In a further embodiment, R¹ is linear hexadecyl, i.e., the compound of Formula (I), a stereoisomer thereof, or a pharmaceutically acceptable salt thereof, is a compound of Formula (II): 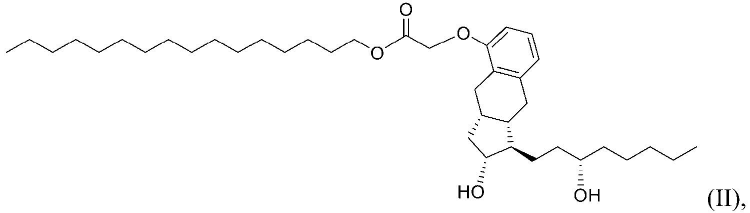 a stereoisomer thereof, or a pharmaceutically acceptable salt thereof. In a further embodiment, the compound of Formula (I) is a compound of Formula (II). The compound of Formula (I) where R¹ is linear hexadecyl is also referred to herein as C16TR or its international nonproprietary name, treprostinil palmitil. In the present application, C16TR and treprostinil palmitil are used interchangeably. Similarly, a compound of Formula (II) is equivalent to a compound of Formula (I), wherein R¹ is linear hexadecyl.

In a preferred embodiment of the compound of Formula (I), a stereoisomer thereof, or a pharmaceutically acceptable salt thereof, the compound of Formula (I) is treprostinil palmitil (i.e., R¹ is linear hexadecyl). In a further embodiment, the treprostinil palmitil is in a crystalline form. Crystalline forms of treprostinil palmitil are described in U.S. Patent No. 10,781,160, which is incorporated herein by reference in its entirety.

In one embodiment, the treprostinil palmitil is a crystal Form I having an X-ray powder diffraction (XRPD) pattern exhibiting peaks at two, three, four, five or all of the following 2θ reflection angles: 3.3±0.2°, 6.6±0.2°, 14.2±0.2°, 18.9±0.2°, 21.3±0.2°, and 22.5±0.2°. In another embodiment, the XRPD pattern of treprostinil palmitil crystal Form I further comprises peaks at one, two, three, four, five, six, seven, eight or all of the following 2θ reflection angles: 13.8±0.2°, 15.3±0.2°, 16.9±0.2°, 17.8±0.2°, 19.8±0.2°, 20.6±0.2°, 20.9±0.20, 24.4±0.2°, and 24.8±0.2°. In one embodiment, the treprostinil palmitil is a crystal Form I having an XRPD pattern exhibiting peaks at the 2θ reflection angles provided in Table A.

| **Table A.** XRPD Data for Treprostinil Palmitil Crystal Form I. | | |
|---|---|---|
| 2Θ angle (°) | D value (Å) | Relative intensity (%) |
| 3.3 | 23.6 | 100 |
| 6.6 | 13.4 | 40.1 |
| 9.8 | 9.0 | 4.5 |
| 13.1 | 6.8 | 2.8 |
| 13.8 | 6.4 | 7.9 |
| 14.2 | 6.2 | 46.0 |
| 15.3 | 5.8 | 11.3 |
| 16.3 | 5.4 | 6.7 |
| 16.9 | 5.2 | 19.7 |
| 17.8 | 5.0 | 16.0 |
| 18.9 | 4.7 | 92.8 |
| 19.8 | 4.5 | 14.5 |
| 20.6 | 4.3 | 8.2 |
| 20.9 | 4.2 | 20.2 |
| 21.3 | 4.2 | 32.2 |
| 22.5 | 4.0 | 27.8 |
| 24.4 | 3.6 | 27.7 |
| 24.8 | 3.6 | 19.4 |
| 26.2 | 3.4 | 4.5 |
| 27.3 | 3.3 | 2.3 |
| 27.8 | 3.2 | 2.1 |
| 28.8 | 3.1 | 1.5 |
| 29.9 | 3.0 | 1.6 |
| 31.2 | 2.9 | 1.2 |
| 32.5 | 2.8 | 1.5 |
| 34.0 | 2.6 | 2.5 |
| 36.2 | 2.5 | 1.8 |
| 36.8 | 2.4 | 3.4 |
| 39.7 | 2.3 | 2.7 |

In one embodiment, the treprostinil palmitil crystal Form I has a differential scanning calorimetry (DSC) thermogram pattern comprising an endothermic peak with a peak onset temperature of approximately 52.2±1° C. and a peak maximum of approximately 54.5±1° C.

In another embodiment, the treprostinil palmitil is a crystal Form II having an XRPD pattern exhibiting peaks at two, three, four, five or all of the following 2θ reflection angles: 3.4±0.2°, 6.1±0.2°, 9.4±0.2°, 20.3±0.2°, 21.6±0.2°, and 23.4±0.2°. In another embodiment, the XRPD pattern of treprostinil palmitil crystal Form II further comprises peaks at one, two, three, four, five, six, seven, eight or all the following 2θ reflection angles: 7.0±0.2°, 9.0±0.2°, 12.2±0.2°, 12.7±0.2°, 17.5±0.2°, 18.0±0.2°, 18.5±0.2°, 19.1±0.2°, and 19.4±0.2°. In one embodiment, the treprostinil palmitil is a crystal Form II having an XRPD pattern exhibiting peaks at the 2θ reflection angles provided in Table B.

| **Table B.** XRPD Data for Treprostinil Palmitil Crystal Form II. | | |
|---|---|---|
| 2Θ angle (°) | D value (Å) | Relative intensity (%) |
| 3.4 | 26.0 | 100.0 |
| 4.5 | 19.6 | 2.2 |
| 6.1 | 14.5 | 45.1 |
| 7.0 | 12.6 | 22.0 |
| 7.7 | 11.5 | 3.9 |
| 9.0 | 9.8 | 27.8 |
| 9.4 | 9.4 | 85.5 |
| 10.0 | 8.8 | 3.6 |
| 10.6 | 8.3 | 13.9 |
| 17.2 | 7.7 | 22.6 |
| 12.7 | 7.0 | 29.9 |
| 13.9 | 6.4 | 3.8 |
| 14.2 | 6.2 | 3.9 |
| 14.9 | 5.9 | 5.2 |
| 15.5 | 5.7 | 17.2 |
| 16.1 | 5.5 | 18.1 |
| 16.7 | 5.3 | 6.2 |
| 17.5 | 5.1 | 25.4 |
| 18.0 | 4.9 | 30.8 |
| 18.5 | 4.8 | 79.3 |
| 19.1 | 4.6 | 33.8 |
| 19.4 | 4.6 | 30.3 |
| 20.3 | 4.4 | 39.9 |
| 20.9 | 4.2 | 19.8 |
| 71.6 | 4.1 | 40.6 |
| 22.6 | 3.9 | 9.4 |
| 23.4 | 3,8 | 81.9 |
| 25.4 | 3.5 | 9.3 |
| 27.7 | 3.2 | 3.6 |
| 28.8 | 3.1 | 3.1 |
| 30.9 | 2.9 | 2.3 |
| 37.3 | 2.4 | 2.1 |
| 40.0 | 2.3 | 2.8 |
| 43.3 | 2.1 | 2.1 |

In one embodiment the treprostinil palmitil crystal Form II has a DSC thermogram pattern comprising an endothermic peak with a peak onset temperature of approximately 54.6±1° C. and a peak maximum of approximately 56.9±1° C.

In one embodiment, (a) is a compound of Formula (I) or a pharmaceutically acceptable salt thereof. In a further embodiment, (a) is a compound of Formula (II) or a pharmaceutically acceptable salt thereof. In a further embodiment, (a) is a compound of Formula (II).

In one embodiment, the compound of Formula (I) or (II), or a pharmaceutically acceptable salt thereof, is present at from about 1 wt% to about 5 wt% of the total weight of the powder composition. In some embodiments, the compound of Formula (I) or (II), or a pharmaceutically acceptable salt thereof, is present at from about 1 wt% to about 4.5 wt% of the total weight of the powder composition. In some embodiments, the compound of Formula (I) or (II) is present at from about 1 wt% to about 4 wt% of the total weight of the powder composition.

In one embodiment, the compound of Formula (I) or (II), or a pharmaceutically acceptable salt thereof, is present at from about 1 wt% to about 3.5 wt% of the total weight of the powder composition. In another embodiment, the compound of Formula (I) or (II), or a pharmaceutically acceptable salt thereof, is present at from about 1 wt% to about 3 wt% of the total weight of the powder composition.

In one embodiment, the compound of Formula (I) or (II), or a pharmaceutically acceptable salt thereof, is present at from about 1 wt% to about 5 wt%, from about 1 wt% to about 4.5 wt%, from about 1 wt% to about 4 wt%, at about 2 wt%, at about 3 wt%, at about 4 wt%, or at about 5 wt%, of the total weight of the powder composition. In some embodiments, the compound of Formula (I) or (II), or a pharmaceutically acceptable salt thereof, is present at from about 1 wt% to about 5 wt%, from about 1 wt% to about 4.5 wt%, from about 1 wt% to about 4 wt%, from about 1 wt% to about 2 wt%, about 2 wt%, or about 4 wt%, of the total weight of the powder composition.

In one embodiment, the compound of Formula (I) or (II), or a pharmaceutically acceptable salt thereof is present at from about 0.8 wt% to about 3.3 wt%, or from about 1 wt% to about 3 wt%, or from about 1 wt% to about 2 wt%, or from about 1 wt% to about 1.5% of the total weight of the powder composition.

In one embodiment, the compound of Formula (I) or (II), or a pharmaceutically acceptable salt thereof is present at about 1 wt% of the total weight of the powder composition. In another embodiment, the compound of Formula (I) or (II), or a pharmaceutically acceptable salt thereof is present at about 1.5 wt% of the total weight of the powder composition.

In one embodiment, the compound of Formula (I) or (II), or a pharmaceutically acceptable salt thereof, is present from about 0.8 wt% to about 1.5 wt% of the total weight of the powder composition. In another embodiment, the compound of Formula (I) or (II), or a pharmaceutically acceptable salt thereof is present from about 2.7 wt% to about 4 wt% of the total weight of the powder composition. In one embodiment, the compound of Formula (I) or (II), or a pharmaceutically acceptable salt thereof is present from about 2.7 wt% to about 3.5 wt%, for example, from about 2.8 wt% to about 3.2 wt%, or from about 2.9 wt% to about 3.1 wt% of the total weight of the powder composition.

In one embodiment, the leucine is present at from about 25 wt% to about 61 wt% of the total weight of the powder composition. In a further embodiment, the leucine is present at from about 25 wt% to about 50 wt% of the total weight of the powder composition. In a further embodiment, the leucine is present at from about 25 wt% to about 40 wt% of the total weight of the powder composition. In a further embodiment, the leucine is present at from about 20 wt% to about 33 wt%, e.g., about 20 wt%, about 25 wt%, about 26 wt%, about 27 wt%, about 28 wt%, about 29 wt%, about 30 wt%, about 31 wt%, about 32 wt%, or about 33 wt% of the total weight of the powder composition. In a further embodiment, the leucine is present at from about 25 wt% to about 33 wt% of the total weight of the powder composition. In a further embodiment, the leucine is present at from about 27 wt% to about 33 wt% of the total weight of the powder composition. In a further embodiment, the leucine is present at from about 27 wt% to about 31 wt% of the total weight of the powder composition. In a further embodiment, the leucine is present at from about 27 wt% to about 30 wt% of the total weight of the powder composition. In a further embodiment, the leucine is present at from about 28 wt% to about 30 wt% of the total weight of the powder composition.

In another embodiment, the leucine is present at about 30 wt% of the total weight of the powder composition.

In yet another embodiment, the leucine is present at from about 45 wt% to about 61 wt% of the total weight of the powder composition, for example at from about 45 wt% to about 55 wt%, or from about 50 wt% to about 55 wt%. In a further embodiment, the compound of Formula (I), or a pharmaceutically acceptable salt thereof, is present at about 3 wt% to about 4 wt% of the total weight of the powder composition. In even a further embodiment, R¹ is hexadecyl, e.g., linear hexadecyl.

In some embodiments, the sugar in the powder composition is trehalose. In another embodiment, the sugar in the powder composition is mannitol.

In one embodiment, the composition has the weight percentages set forth in Table C, below. In another embodiment, the composition has the weight percentages set forth in Table C, below, ± 5% for each component. In yet another embodiment, the composition has a leucine-to-mannitol weight ratio ("leucine : mannitol" or "leucine-to-mannitol") set forth in Table C.

| **Table C**. Exemplary TPIP compositions. | | | | |
|---|---|---|---|---|
| | **TP (%w)** | **Leucine (%w)** | **Mannitol (%w)** | **Leucine-to-Mannitol Weight Ratio** |
| **1** | 0.5 | 60.0 | 39.5 | 1.52-to-1 |
| **2** | 2.0 | 61.2 | 36.8 | 1.66-to-1 |
| **3** | 3.0 | 60.7 | 36.3 | 1.67-to-1 |
| **4** | 4.0 | 60.0 | 36.0 | 1.67-to-1 |
| **5** | 0.4 | 45.0 | 54.6 | 0.82-to-1 |
| **6** | 1.5 | 44.4 | 54.1 | 0.82-to-1 |
| **7** | 2.0 | 45.0 | 53.0 | 0.85-to-1 |
| **8** | 3.0 | 44.5 | 52.5 | 0.85-to-1 |
| **9** | 4.0 | 45.0 | 51.0 | 0.88-to-1 |
| **10** | 0.5 | 30.0 | 69.5 | 0.43-to-1 |
| **11** | 1.0 | 29.3 | 69.7 | 0.42-to-1 |
| **12** | 1.5 | 29.6 | 68.9 | 0.43-to-1 |
| **13** | 1.5 | 29.3 | 69.2 | 0.42-to-1 |
| **14** | 2.0 | 28.8 | 69.2 | 0.42-to-1 |
| **15** | 3.0 | 28.6 | 68.4 | 0.42-to-1 |
| **16** | 4.0 | 30.0 | 66.0 | 0.45-to-1 |

In one embodiment, the powder composition has the components and weight percentages set forth in Table D.

| **Table D.** Exemplary TPIP composition. | |
|---|---|
| | **Quantity (%w)** |
| **TP (drug substance)** | 1.0 |
| **Leucine (dispersion enhancer)** | 29.3 |
| **Mannitol (bulking agent)** | 69.7 |
| **Filled Capsule Weight** | 100 |

The leucine-to-sugar (i.e., mannitol or trehalose) weight ratio in a composition provided herein, in one embodiment, is from about 0.4-to-1 (leucine-to-mannitol or -trehalose) to about 1.7-to-1 (leucine-to-mannitol -trehalose). In a further embodiment, the composition comprises a compound of Formula (I), or a pharmaceutically acceptable salt thereof, at from about 0.5 wt% to about 4 wt% of the total weight of the powder composition. In a further embodiment, the leucine-to-sugar weight ratio is from about 0.4:1 (leucine-to-mannitol or - trehalose) to 0.9:1 (leucine-to-mannitol or -trehalose). In even a further embodiment, the leucine-to-sugar weight ratio is from about 0.4:1 (leucine-to-mannitol or -trehalose) to 0.5:1 (leucine-to-mannitol or -trehalose). In a further embodiment, the sugar is mannitol. The leucine, in one embodiment, is L-leucine.

In another embodiment, the sugar is mannitol and the leucine-to-mannitol weight ratio is from about 0.75-to-1 (leucine-to-mannitol) to 0.9-to-1 (leucine-to-mannitol). In a further embodiment, the composition comprises a compound of Formula (I), or a pharmaceutically acceptable salt thereof, at from about 0.5 wt% to about 4 wt% of the total weight of the powder composition. In a further embodiment, the leucine-to-mannitol weight ratio is from about 0.8:1 (leucine-to-mannitol) to 0.9:1 (leucine-to-mannitol). In another embodiment, the sugar is trehalose and the leucine-to-trehalose weight ratio is from about 0.75:1 (leucine-to-trehalose) to 0.9:1 (leucine-to-trehalose). In a further embodiment, the composition comprises a compound of Formula (I), (II), or a pharmaceutically acceptable salt thereof, at from about 1 wt% to about 4 wt% of the total weight of the powder composition. In a further embodiment, the leucine-to- trehalose weight ratio is from about 0.8:1 (leucine-to-trehalose) to 0.9:1 (leucine-to-trehalose). The leucine, in one embodiment, is L-leucine.

In yet another embodiment, the sugar is mannitol and the leucine-to-mannitol weight ratio is from about 1.5:1 (leucine-to-mannitol) to 1.7:1 (leucine-to-mannitol). In a further embodiment, the composition comprises a compound of Formula (I), (II), or a pharmaceutically acceptable salt thereof, at from about 0.5 wt% to about 4 wt% of the total weight of the powder composition. In a further embodiment, the leucine-to-mannitol weight ratio is from about 1.6:1 (leucine-to-mannitol) to 1.7:1 (leucine-to-mannitol). In yet another embodiment, the sugar is trehalose and the leucine-to-trehalose weight ratio is from about 1.5:1 (leucine-to-trehalose) to 1.7:1 (leucine-to-trehalose). In a further embodiment, the composition comprises a compound of Formula (I), (II), or a pharmaceutically acceptable salt thereof, at from about 0.5 wt% to about 4 wt% of the total weight of the powder composition. In a further embodiment, the composition comprises a compound of Formula (I), (II), or a pharmaceutically acceptable salt thereof, at from about 0.5 wt% to about 3 wt% of the total weight of the powder composition. In a further embodiment, the composition comprises a compound of Formula (I), (II), or a pharmaceutically acceptable salt thereof, at from about 0.5 wt% to about 2 wt% of the total weight of the powder composition. In a further embodiment, the composition comprises a compound of Formula (I), (II), or a pharmaceutically acceptable salt thereof, at from about 0.5 wt% to about 1.5 wt% of the total weight of the powder composition. In a further embodiment, the leucine-to-mannitol weight ratio is from about 1.6:1 (leucine-to-trehalose) to 1.7:1 (leucine-to-trehalose).

In another embodiment, the powder composition includes (a) about 1-2 wt% of the compound of Formula (I) or (II), a stereoisomer thereof, or a pharmaceutically acceptable salt thereof, (b) about 29 wt% of the leucine, and the balance being (c) mannitol. In a further embodiment, (a) in the powder composition is about 1 wt% of the compound of Formula (I) or (II), or a pharmaceutically acceptable salt thereof.

In another embodiment, (a) in the powder composition is at about 1 wt% to about 1.5 wt% of the compound of Formula (I) or (II), or a pharmaceutically acceptable salt thereof. In another embodiment, (a) in the powder composition is at about 2 wt% to about 4 wt% of the compound of Formula (I) or (II), or a pharmaceutically acceptable salt thereof. In another embodiment, (a) in the powder composition is at about 3 wt% to about 4 wt% of the compound of Formula (I) or (II), or a pharmaceutically acceptable salt thereof. In another embodiment, (a) in the powder composition is at about 2 wt% of the compound of Formula (I) or (II), or a pharmaceutically acceptable salt thereof.

In another embodiment, the powder composition includes (a) about 1 wt% of the compound of Formula (I) or (II), a stereoisomer thereof, or a pharmaceutically acceptable salt thereof, (b) about 29.6 wt% of the leucine, and the balance being (c) mannitol. In a further embodiment, R¹ is linear hexadecyl in the compound of Formula (I).

In another embodiment, the powder composition includes (a) about 1.5 wt% of the compound of Formula (I) or (II), or a pharmaceutically acceptable salt thereof, (b) about 29.6 wt% of the leucine, and the balance being (c) mannitol. In a further embodiment, R¹ is linear hexadecyl in the compound of Formula (I).

In another embodiment, the powder composition includes (a) about 1 wt% of the compound of Formula (I) or (II), a stereoisomer thereof, or a pharmaceutically acceptable salt thereof, (b) about 29 wt% to about 30 wt% of the leucine, and the balance being (c) mannitol. In a further embodiment, R¹ is linear hexadecyl in the compound of Formula (I).

In another embodiment, the powder composition includes (a) about 1.5 wt% of the compound of Formula (I) or (II), a stereoisomer thereof, or a pharmaceutically acceptable salt thereof, (b) about 29 wt% to about 30 wt% of the leucine, and the balance being (c) mannitol. In a further embodiment, R¹ is linear hexadecyl in the compound of Formula (I).

In another embodiment, the powder composition includes (a) about 2 wt% of the compound of Formula (I) or (II), a stereoisomer thereof, or a pharmaceutically acceptable salt thereof, (b) about 29 wt% to about 30 wt% of the leucine, and the balance being (c) mannitol. In a further embodiment, R¹ is linear hexadecyl in the compound of Formula (I).

In another embodiment, the powder composition includes (a) about 3 wt% of the compound of Formula (I) or (II), a stereoisomer thereof, or a pharmaceutically acceptable salt thereof, (b) about 29 wt% to about 30 wt% of the leucine, and the balance being (c) mannitol. In a further embodiment, R¹ is linear hexadecyl in the compound of Formula (I).

In another embodiment, the powder composition includes (a) about 4 wt% of the compound of Formula (I) or (II), a stereoisomer thereof, or a pharmaceutically acceptable salt thereof, (b) about 29 wt% to about 30 wt% of leucine, and the balance being (c) mannitol. In a further embodiment, R¹ is linear hexadecyl in the compound of Formula (I).

In one embodiment, the powder composition includes (a) about 0.5 wt% of the compound of Formula (I) or (II), wherein R1 is linear hexadecyl, (b) about 60 wt% to about 61 wt% leucine, and the balance being (c) mannitol. In one embodiment, the powder composition includes (a) about 1 wt% of the compound of Formula (I) or (II), wherein R1 is linear hexadecyl, (b) about 60 wt% to about 61 wt% leucine, and the balance being (c) mannitol. In another embodiment, the powder composition includes (a) about 1.5 wt% of the compound of Formula (I) or (II), wherein R1 is linear hexadecyl, (b) about 60 wt% to about 61 wt% leucine, and the balance being (c) mannitol. In one embodiment, the powder composition includes (a) about 2 wt% of the compound of Formula (I) or (II), wherein R1 is linear hexadecyl, (b) about 60 wt% to about 61 wt% leucine, and the balance being (c) mannitol. In one embodiment, the powder composition includes (a) about 3 wt% of the compound of Formula (I) or (II), wherein R1 is linear hexadecyl, (b) about 60 wt% to about 61 wt% leucine, and the balance being (c) mannitol. In one embodiment, the powder composition includes (a) about 4 wt% of the compound of Formula (I) or (II), wherein R1 is linear hexadecyl, (b) about 60 wt% to about 61 wt% leucine, and the balance being (c) mannitol.

In another embodiment, the powder composition includes (a) about 0.5 wt% of the compound of Formula (I) or (II), wherein R1 is linear hexadecyl, (b) about 43 wt% to about 45 wt% leucine, and the balance being (c) mannitol. In another embodiment, the powder composition includes (a) about 1 wt% of the compound of Formula (I) or (II), wherein R1 is linear hexadecyl, (b) about 43 wt% to about 45 wt% leucine, and the balance being (c) mannitol. In another embodiment, the powder composition includes (a) about 1.5 wt% of the compound of Formula (I) or (II), wherein R1 is linear hexadecyl, (b) about 43 wt% to about 45 wt% leucine, and the balance being (c) mannitol. In another embodiment, the powder composition includes (a) about 2 wt% of the compound of Formula (I) or (II), wherein R1 is linear hexadecyl, (b) about 43 wt% to about 45 wt% leucine, and the balance being (c) mannitol. In another embodiment, the powder composition includes (a) about 3 wt% of the compound of Formula (I) or (II), wherein R1 is linear hexadecyl, (b) about 43 wt% to about 45 wt% leucine, and the balance being (c) mannitol. In another embodiment, the powder composition includes (a) about 4 wt% of the compound of Formula (I) or (II), wherein R1 is linear hexadecyl, (b) about 43 wt% to about 45 wt% leucine, and the balance being (c) mannitol.

In some embodiments, the powder composition does not include distearoyl phosphoethanoamine-polyethylene glycol 2000 (DPSE-PEG2000).

In one embodiment, the powder composition comprises from about 80 µg to about 700 µg of a compound of Formula (I) or (II), for example, about 80 µg, about 100 µg, about 110 µg, about 112.5 µg, about 120 µg, about 130 µg, about 140 µg, about 150 µg, about 160 µg, about 170 µg, about 180 µg, about 190 µg, about 200 µg, about 210 µg, about 220 µg, about 225 µg, about 230 µg, about 240 µg, about 250 µg, about 260 µg, about 270 µg, about 280 µg, about 290 µg, about 300 µg, about 310 µg, about 320 µg, about 330 µg, about 340 µg, about 350 µg, about 360 µg, about 370 µg, about 380 µg, about 390 µg, about 400 µg, about 410 µg, about 420 µg, about 430 µg, about 440 µg, about 450 µg, about 460 µg, about 470 µg, about 480 µg, about 490 µg, about 500 µg, about 510 µg, about 520 µg, about 530 µg, about 540 µg, about 550 µg, about 560 µg, about 570 µg, about 580 µg, about 590 µg, about 600 µg, about 610 µg, about 620 µg, about 630 µg, about 640 µg, about 650 µg, about 660 µg, about 670 µg, about 675 µg, about 680 µg, about 690 µg, or about 700 µg of a compound of Formula (I), (II), a stereoisomer thereof, or a pharmaceutically acceptable salt thereof, including all values and ranges therein. In one embodiment, the powder composition comprises from about 80 µg to about 1280 µg of a compound of Formula (I) or (II), for example, about 80 µg, about 100 µg, about 110 µg, about 112.5 µg, about 120 µg, about 130 µg, about 140 µg, about 150 µg, about 160 µg, about 170 µg, about 180 µg, about 190 µg, about 200 µg, about 210 µg, about 220 µg, about 225 µg, about 230 µg, about 240 µg, about 250 µg, about 260 µg, about 270 µg, about 280 µg, about 290 µg, about 300 µg, about 310 µg, about 320 µg, about 330 µg, about 340 µg, about 350 µg, about 360 µg, about 370 µg, about 380 µg, about 390 µg, about 400 µg, about 410 µg, about 420 µg, about 430 µg, about 440 µg, about 450 µg, about 460 µg, about 470 µg, about 480 µg, about 490 µg, about 500 µg, about 510 µg, about 520 µg, about 530 µg, about 540 µg, about 550 µg, about 560 µg, about 570 µg, about 580 µg, about 590 µg, about 600 µg, about 610 µg, about 620 µg, about 630 µg, about 640 µg, about 650 µg, about 660 µg, about 670 µg, about 675 µg, about 680 µg, about 690 µg, about 700 µg, about 720 µg, about 740 µg, about 760 µg, about 780 µg, about 800 µg, about 820 µg, about 840 µg, about 860 µg, about, about 880 µg, about 900 µg, about 920 µg, about 940 µg, about 960 µg, about 980 µg, about 1000 µg, about 1020 µg, about 1040 µg, about 1060 µg, about 1080 µg, about 1100 µg, about 1120 µg, about 1140 µg, about 1160 µg, about 1180 µg, about 1200 µg, about 1240 µg, about 1260 µg, or about 1280 µg of a compound of Formula (I), (II), a stereoisomer thereof, or a pharmaceutically acceptable salt thereof, including all values and ranges therein. In one embodiment, the powder composition comprises from about 80 µg to about 700 µg of a compound of Formula (I) or (II). In one embodiment, the powder composition comprises from about 80 µg to about 1280 µg of a compound of Formula (I) or (II). In one embodiment, the powder composition comprises from about 80 µg to about 640 µg of a compound of Formula (I) or (II). In one embodiment, the powder composition comprises from about 80 µg to about 320 µg of a compound of Formula (I) or (II). In one embodiment, the composition comprises about 80 µg, about 160 µg, about 240 µg, about 320 µg, about 400 µg, about 480 µg or about 640 µg of a compound of Formula (I) or (II). In one embodiment, the composition comprises about 80 µg, about 160 µg, about 240 µg, about 320 µg, about 400 µg, about 480 µg, 560 µg, 640 µg, 675 µg, 720 µg, 800 µg, 880 µg, 960 µg, 1040 µg , 1120 µg, 1200 µg, and 1280 µg of a compound of Formula (I) or (II). The composition may be present, in one embodiment, in one powder capsule or a plurality (two or more) powder capsules. When present in multiple capsules, one of the aforementioned doses of the compound of Formula (I) or (II) is divided amongst the capsules. The capsule, in one embodiment, is a size #3 HPMC capsule.

Embodiments of a TPIP composition at different unit strengths are provided in Table E, below. It should be understood that the unit strengths of the components provided herein can be calculated based on the weight percentages of the component and the desired dosage. For example, for an 80 µg dose of TP, each component is multiplied by 80 to obtain the unit strength of each component.

| **Table E**. TPIP filled capsule embodiments. | | | | |
|---|---|---|---|---|
| | | **Unit Strength (µg / capsule)** | | |
| | | 80 | 160 | 320 |
| **Component** | **Quantity (%w)** | | | |
| TP (drug substance) | 1.0 | 80 | 160 | 320 |
| Leucine (dispersion enhancer) | 29.3 | 2344 | 4688 | 9376 |
| Mannitol (bulking agent) | 69.7 | 5576 | 11152 | 22304 |
| | | | | |
| Filled Capsule Weight | 100 | 8 mg | 16 mg | 32 mg |

In one embodiment, the powder composition comprises about 80 µg of the compound of Formula (I), (II), or a pharmaceutically acceptable salt thereof. In a further embodiment of a compound of Formula (I), R¹ is hexadecyl. In even a further embodiment of a compound of Formula (I), R¹ is linear hexadecyl. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides one or more of the following: (i) an aerosol comprising a fine particle fraction (FPF) greater than or equal to about 68% (e.g., about 68% to about 80%, about 68% to about 73%, or about about 78% to about 80%), (ii) a fine particle dose (FPD) greater than or equal to 50% of a loaded dose of the powder composition (e.g. about 53% to about 77% or from about 60% to about 65%), (iii) an aerosol comprising an emitted dose (ED) greater than or equal to 72%, e.g., about 79% to about 89%, or (iv) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In one embodiment, the powder composition comprises about 160 µg of the compound of Formula (I), (II), or a pharmaceutically acceptable salt thereof. In a further embodiment of a compound of Formula (I), R¹ is hexadecyl. In even a further embodiment of a compound of Formula (I), R¹ is linear hexadecyl. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In another embodiment, the powder composition comprises about 240 µg of the compound of Formula (I), (II), or a pharmaceutically acceptable salt thereof. In a further embodiment of a compound of Formula (I), R¹ is hexadecyl. In even a further embodiment of a compound of Formula (I), R¹ is linear hexadecyl. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In one embodiment, the powder composition comprises about 320 µg of the compound of Formula (I), (II), or a pharmaceutically acceptable salt thereof. In a further embodiment of a compound of Formula (I), R¹ is hexadecyl. In even a further embodiment of a compound of Formula (I), R¹ is linear hexadecyl. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In another embodiment, the powder composition comprises about 400 µg of the compound of Formula (I), (II), or a pharmaceutically acceptable salt thereof. In a further embodiment of a compound of Formula (I), R¹ is hexadecyl. In even a further embodiment of a compound of Formula (I), R¹ is linear hexadecyl. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In another embodiment, the powder composition comprises about 480 µg of the compound of Formula (I), (II), or a pharmaceutically acceptable salt thereof. In a further embodiment of a compound of Formula (I), R¹ is hexadecyl. In even a further embodiment of a compound of Formula (I), R¹ is linear hexadecyl. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In one embodiment, the powder composition comprises about 640 µg of the compound of Formula (I), (II), or a pharmaceutically acceptable salt thereof. In a further embodiment of a compound of Formula (I), R¹ is hexadecyl. In even a further embodiment of a compound of Formula (I), R¹ is linear hexadecyl. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In one embodiment, the powder composition comprises about 720 µg of the compound of Formula (I), (II), or a pharmaceutically acceptable salt thereof. In a further embodiment of a compound of Formula (I), R¹ is hexadecyl. In even a further embodiment of a compound of Formula (I), R¹ is linear hexadecyl. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In one embodiment, the powder composition comprises about 800 µg of the compound of Formula (I), (II), or a pharmaceutically acceptable salt thereof. In a further embodiment of a compound of Formula (I), R¹ is hexadecyl. In even a further embodiment of a compound of Formula (I), R¹ is linear hexadecyl. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In one embodiment, the powder composition comprises about 880 µg of the compound of Formula (I), (II), or a pharmaceutically acceptable salt thereof. In a further embodiment of a compound of Formula (I), R¹ is hexadecyl. In even a further embodiment of a compound of Formula (I), R¹ is linear hexadecyl. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In one embodiment, the powder composition comprises about 960 µg of the compound of Formula (I), (II), or a pharmaceutically acceptable salt thereof. In a further embodiment of a compound of Formula (I), R¹ is hexadecyl. In even a further embodiment of a compound of Formula (I), R¹ is linear hexadecyl. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In one embodiment, the powder composition comprises about 1040 µg of the compound of Formula (I), (II), or a pharmaceutically acceptable salt thereof. In a further embodiment of a compound of Formula (I), R¹ is hexadecyl. In even a further embodiment of a compound of Formula (I), R¹ is linear hexadecyl. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In one embodiment, the powder composition comprises about 1120 µg of the compound of Formula (I), (II), or a pharmaceutically acceptable salt thereof. In a further embodiment of a compound of Formula (I), R¹ is hexadecyl. In even a further embodiment of a compound of Formula (I), R¹ is linear hexadecyl. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In one embodiment, the powder composition comprises about 1200 µg of the compound of Formula (I), (II), or a pharmaceutically acceptable salt thereof. In a further embodiment of a compound of Formula (I), R¹ is hexadecyl. In even a further embodiment of a compound of Formula (I), R¹ is linear hexadecyl. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In one embodiment, the powder composition comprises about 1280 µg of the compound of Formula (I), (II), or a pharmaceutically acceptable salt thereof. In a further embodiment of a compound of Formula (I), R¹ is hexadecyl. In even a further embodiment of a compound of Formula (I), R¹ is linear hexadecyl. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In a preferred embodiment of the powder composition provided herein, the leucine is L-leucine. The powder composition, in one embodiment, is a spray-dried powder composition. In some embodiments, the spray-dried powder composition comprises particles. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In another aspect, the present disclosure provides a powder composition comprising a compound of Formula (I) or (II), or a pharmaceutically acceptable salt thereof, which provides a particular pharmacokinetic profile following once daily administration. Advantageously, pharmacokinetic profile has a lower Cₘₐₓ and longer half-life compared to the current treprostinil inhaled solution, Tyvaso^{®}.

In one embodiment, the powder composition exhibiting one of the pharmacokinetic profiles described herein is a composition described in U.S. Patent Application Publication No. 2020/0338005 and WO 2022/094100, incorporated by reference herein in their entirety for all purposes.

In another embodiment, the powder composition exhibiting one of the pharmacokinetic profiles described herein comprises (a) a compound of Formula (I) or (II) at from about 1 wt% to about 5 wt% of the total weight of the powder composition; (b) from about 25 wt% to about 61 wt% of leucine, and the balance being (c) a sugar selected from trehalose and mannitol. The entirety of (a), (b), and (c) is 100 wt%. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

Example 5 of WO 2022/094100 presents that the pharmacokinetic (PK) profile measured for the compound of Formula (I) or (II), a stereoisomer thereof, or a pharmaceutically acceptable salt thereof, was linear over the dose range of 112.5 µg to 675 µg. Based on this data, the skilled artisan can determine the pharmacokinetic parameters of doses outside of the range, or doses inside this range that were not specifically tested in the Example 5 of WO 2022/094100. For example, in order to find a pharmacokinetic parameter at a dose, Cₘₐₓ and AUC associated with specific doses (112.5 µg, 225 µg, 450 µg, and/or 675 µg) may be plotted. The scatter plot may be fit to a straight line, y= mx + b, where m is the slope of the line, b is the y intercept, and the value of an unknown pharmacokinetic parameter (y) may be calculated by plugging in the dose for x. In addition, the dose range of 112.5 µg to 675 µg was based on the molecular weight of the compound of Formula (I) when R¹ is hexadecyl (i.e., the compound of Formula (II)). Equivalent doses for other treprostinil prodrugs (when R¹ is tetradecyl, pentadecyl, heptadecyl, or octadecyl) can be calculated using the molecular weight of the treprostinil prodrug of interest. For example, the dose of the compound of Formula (I) when R¹ is tetradecyl that is equivalent to 112.5 µg of the compound of Formula II (R¹ is hexadecyl) can be calculated by multiplying 112.5 µg by the ratio of the molecular weight of the compound of Formula (II) (614.95 µg/mol) to the molecular weight of the compound of Formula (I) when R¹ is tetradecyl (586.9 µg/mol).

In embodiments, the powder composition of the disclosure is formulated to administer once daily to the lungs of a subject by inhalation a dose ranging from about 80 µg to about 1280 µg of the compound of Formula (I) or (II), a stereoisomer thereof, or a pharmaceutically acceptable salt thereof, and provide at least one of the following characteristics:
(a) a treprostinil maximum plasma concentration (Cₘₐₓ) ranging from about 14 pg/mL to about 3000 pg/mL; or
(b) a treprostinil area under the plasma concentration curve (AUC) ranging from about from about 500 pg*h/mL to about 20000 pg*h/mL.

In a further embodiment, the powder composition comprises about 80 µg, about 112.5 µg, about 160 µg, about 225 µg, about 240 µg, about 320 µg, about 400 µg, about 450 µg, about 480 µg, about 640 µg, about 675 µg, about 720 µg, about 800 µg, about 960 µg, about 1040 µg, about 1120 µg, about 1200 µg, or about 1280 µg of a compound of Formula (I). In a further embodiment, R¹ is hexadecyl, e.g., linear hexadecyl. The powder composition may be present, in one embodiment, in one powder capsule or a plurality (two or more) powder capsules. When present in multiple capsules, one of the aforementioned doses of the compound of Formula (I) is divided amongst the capsules. This multiple capsules may be used to administer a composition comprising one of the aforementioned doses (e.g., about 480 µg, about 560 µg, about 640 µg, about 675 µg, about 720 µg, about 800 µg, about 960 µg, about 1040 µg, about 1120 µg, about 1200 µg, or about 1280 µg). The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In embodiments, the powder composition of the disclosure is formulated to administer once daily to the lungs of a subject by inhalation a dose ranging from about 80 µg to about 675 µg of the compound of Formula (I) or (II), a stereoisomer thereof, or a pharmaceutically acceptable salt thereof, and provide at least one of the following characteristics:
(a) a treprostinil maximum plasma concentration (Cₘₐₓ) ranging from about 14 pg/mL to about 1430 pg/mL; or
(b) a treprostinil area under the plasma concentration curve (AUC) ranging from about from about 500 pg*h/mL to about 10000 pg*h/mL.

In a further embodiment, the powder composition comprises about 80 µg, about 112.5 µg, about 160 µg, about 225 µg, about 240 µg, about 320 µg, about 400 µg, about 450 µg, about 480 µg, about 640 µg, or about 675 µg of a compound of Formula (I) or (II), a stereoisomer thereof, or a pharmaceutically acceptable salt thereof. In a further embodiment, the composition comprises about 80 µg, about 160 µg, about 240 µg, about 320 µg, about 400 µg, about 480 µg, or about 640 µg of a compound of Formula (I) or (II), a stereoisomer thereof, or a pharmaceutically acceptable salt thereof. In a further embodiment of a compound of Formula (I), R¹ is hexadecyl, e.g., linear hexadecyl. The powder composition may be present, in one embodiment, in one powder capsule or a plurality (two or more) powder capsules. When present in multiple capsules, one of the aforementioned doses of the compound of Formula (I) or (II), a stereoisomer thereof or a pharmaceutically acceptable salt thereof, is divided amongst the capsules. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In embodiments, the powder composition of the disclosure is formulated to administer once daily to the lungs of the subject (e.g., patient) by inhalation a dose ranging from about 80 µg to about 1280 µg of the compound of Formula (I) or (II), a stereoisomer thereof, or a pharmaceutically acceptable salt thereof, and provide at least one of the following characteristics:
(a) a treprostinil maximum plasma concentration (Cₘₐₓ) ranging from about 14 pg/mL to about 3000 pg/mL; or
(b) a treprostinil area under the plasma concentration curve (AUC) ranging from about from about 380 pg*h/mL to about 20000 pg*h/mL.

In a further embodiment, the powder composition comprises about 80 µg, about 112.5 µg, about 160 µg, about 225 µg, about 240 µg, about 320 µg, about 400 µg, about 450 µg, about 480 µg, about 640 µg, about 675 µg, about 720 µg, about 800 µg, about 960 µg, about 1040 µg, about 1120 µg, about 1200 µg, or about 1280 µg of a compound of Formula (I) or (II), a stereoisomer thereof or a pharmaceutically acceptable salt thereof. The composition may be present, in one embodiment, in one powder capsule or a plurality (two or more) powder capsules. When present in multiple capsules, one of the aforementioned doses of the compound of Formula (I) or (II), or a stereoisomer thereof or a pharmaceutically acceptable salt thereof, is divided amongst the capsules. Thus, multiple capsules may be administered to achieve one of the aforementioned doses.

In embodiments, the powder composition of the disclosure is formulated to administer once daily to the lungs of the subject (e.g., patient) by inhalation a dose ranging from about 80 µg to about 640 µg of the compound of Formula (I) or (II), a stereoisomer thereof, or a pharmaceutically acceptable salt thereof, and provide at least one of the following characteristics:
(a) a treprostinil maximum plasma concentration (Cₘₐₓ) ranging from about 14 pg/mL to about 1430 pg/mL; or
(b) a treprostinil area under the plasma concentration curve (AUC) ranging from about from about 380 pg*h/mL to about 10000 pg*h/mL.

In a further embodiment, the composition comprises about 80 µg, about 160 µg, about 240 µg, about 320 µg, about 400 µg, about 480 µg or about 640 µg of a compound of Formula (I) or (II), a stereoisomer thereof or a pharmaceutically acceptable salt thereof. The composition may be present, in one embodiment, in one powder capsule or a plurality (two or more) powder capsules. When present in multiple capsules, one of the aforementioned doses of the compound of Formula (I) or (II), a stereoisomer thereof or a pharmaceutically acceptable salt thereof, is divided amongst the capsules.

In one embodiment, the powder composition is formulated to administer once daily to the lungs of the subject (e.g., patient) by inhalation a dose ranging from about 112.5 µg to about 675 µg of the compound of Formula (I) or (II), a stereoisomer thereof, or a pharmaceutically acceptable salt thereof, and provide at least one of the following characteristics:
(a) a treprostinil maximum plasma concentration (Cₘₐₓ) ranging from about 17 pg/mL to about 1370 pg/mL; or
(b) a treprostinil area under the plasma concentration curve (AUC) ranging from about from about 700 pg*h/mL to about 7800 pg*h/mL.

In a further embodiment, the composition comprises about 80 µg, about 160 µg, about 240 µg, about 320 µg, about 400 µg, about 480 µg or about 640 µg of a compound of Formula (I) or (II), a stereoisomer thereof or a pharmaceutically acceptable salt thereof. The composition may be present, in one embodiment, in one powder capsule or a plurality (two or more) powder capsules. When present in multiple capsules, one of the aforementioned doses of the compound of Formula (I) or (II), a stereoisomer thereof or a pharmaceutically acceptable salt thereof, is divided amongst the capsules.

In embodiments, the powder composition is formulated to administer once daily to the lungs of the subject (e.g., patient) by inhalation about 80 µg to about 1280 µg of a compound of Formula (I) or (II), a stereoisomer thereof, or a pharmaceutically acceptable salt thereof, for example from about 80 µg to about 1200 µg or from about 320 µg to about 1280 µg, and provide a pharmacokinetic profile described herein. In one embodiment, the powder composition having one of the pK profiles described herein comprises about 80 µg, about 100 µg, about 110 µg, about 112.5 µg, about 120 µg, about 130 µg, about 140 µg, about 150 µg, about 160 µg, about 170 µg, about 180 µg, about 190 µg, about 200 µg, about 210 µg, about 220 µg, about 225 µg, about 230 µg, about 240 µg, about 250 µg, about 260 µg, about 270 µg, about 280 µg, about 290 µg, about 300 µg, about 310 µg, about 320 µg, about 330 µg, about 340 µg, about 350 µg, about 360 µg, about 370 µg, about 380 µg, about 390 µg, about 400 µg, about 410 µg, about 420 µg, about 430 µg, about 440 µg, about 450 µg, about 460 µg, about 470 µg, about 480 µg, about 490 µg, about 500 µg, about 510 µg, about 520 µg, about 530 µg, about 540 µg, about 550 µg, about 560 µg, about 570 µg, about 580 µg, about 590 µg, about 600 µg, about 610 µg, about 620 µg, about 630 µg, about 640 µg, about 650 µg, about 660 µg, about 670 µg, about 675 µg, about 680 µg, about 690 µg, about 700 µg, about 720 µg, about 740 µg, about 760 µg, about 780 µg, about 800 µg, about 820 µg, about 840 µg, about 860 µg, about, about 880 µg, about 900 µg, about 920 µg, about 940 µg, about 960 µg, about 980 µg, about 1000 µg, about 1020 µg, about 1040 µg, about 1060 µg, about 1080 µg, about 1100 µg, about 1120 µg, about 1140 µg, about 1160 µg, about 1180 µg, about 1200 µg, about 1240 µg, about 1260 µg, or about 1280 µg of a compound of Formula (I) or (II), a stereoisomer thereof, or a pharmaceutically acceptable salt thereof, including all values and ranges therein. In a further embodiment, R¹ is hexadecyl, e.g., linear hexadecyl. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In embodiments, the powder composition is formulated to administer once daily to the lungs of the subject (e.g., patient) by inhalation about 80 µg to about 675 µg of a compound of Formula (I) or (II), or a stereoisomer or pharmaceutically acceptable salt thereof, for example from about 80 µg to about 640 µg or from about 112.5 µg to about 675 µg, and provide a pharmacokinetic profile described herein. In one embodiment, the powder composition having one of the pK profiles described herein comprises about 80 µg, about 100 µg, about 110 µg, about 112.5 µg, about 120 µg, about 130 µg, about 140 µg, about 150 µg, about 160 µg, about 170 µg, about 180 µg, about 190 µg, about 200 µg, about 210 µg, about 220 µg, about 225 µg, about 230 µg, about 240 µg, about 250 µg, about 260 µg, about 270 µg, about 280 µg, about 290 µg, about 300 µg, about 310 µg, about 320 µg, about 330 µg, about 340 µg, about 350 µg, about 360 µg, about 370 µg, about 380 µg, about 390 µg, about 400 µg, about 410 µg, about 420 µg, about 430 µg, about 440 µg, about 450 µg, about 460 µg, about 470 µg, about 480 µg, about 490 µg, about 500 µg, about 510 µg, about 520 µg, about 530 µg, about 540 µg, about 550 µg, about 560 µg, about 570 µg, about 580 µg, about 590 µg, about 600 µg, about 610 µg, about 620 µg, about 630 µg, about 640 µg, about 650 µg, about 660 µg, about 670 µg, about 675 µg, about 680 µg, about 690 µg, or about 700 µg, of a compound of Formula (I), or a pharmaceutically acceptable salt thereof, including all values and ranges therein. In a further embodiment, R¹ is hexadecyl, e.g., linear hexadecyl.

In some embodiments, following once daily administration of a dry powder composition comprising from about 80 µg to about 1280 µg of the compound of Formula (I) or (II), a stereoisomer thereof, or a pharmaceutically acceptable salt thereof, the powder composition or method of use thereof provides a maximum treprostinil plasma concentration (Cₘₐₓ) ranging from about 10 pg/mL to about 3000 pg/mL, for example, about 10 pg/mL, about 15 pg/mL, about 20 pg/mL, about 25 pg/mL about 30 pg/mL, about 35 pg/mL, about 40 pg/mL, about 45 pg/mL, about 50 pg/mL, about 55 pg/mL, about 60 pg/mL, about 65 pg/mL, about 70 pg/mL, about 75 pg/mL, about 80 pg/mL, about 85 pg/mL, about 90 pg/mL, about 95 pg/mL, about 100 pg/mL, about 110 pg/mL, about 120 pg/mL, about 130 pg/mL, about 140 pg/mL, about 150 pg/mL, about 160 pg/mL, about 170 pg/mL, about 180 pg/mL, about 190 pg/mL, about 200 pg/mL, about 210 pg/mL, about 220 pg/mL, about 230 pg/mL, about 240 pg/mL, about 250 pg/mL, about 260 pg/mL, about 270 pg/mL, about 280 pg/mL, about 290 pg/mL, about 300 pg/mL, about 310 pg/mL, about 320 pg/mL, about 330 pg/mL, about 340 pg/mL, about 350 pg/mL, about 360 pg/mL, about 370 pg/mL, about 380 pg/mL, about 390 pg/mL, about 400 pg/mL, about 410 pg/mL, about 420 pg/mL, about 430 pg/mL, about 440 pg/mL, about 450 pg/mL, about 460 pg/mL, about 470 pg/mL, about 480 pg/mL, about 490 pg/mL, about 500 pg/mL, about 510 pg/mL, about 520 pg/mL, about 530 pg/mL, about 540 pg/mL, about 550 pg/mL, about 560 pg/mL, about 570 pg/mL, about 580 pg/mL, about 590 pg/mL, about 600 pg/mL, about 610 pg/mL, about 620 pg/mL, about 630 pg/mL, about 640 pg/mL, about 650 pg/mL, about 660 pg/mL, about 670 pg/mL, about 680 pg/mL, about 690 pg/mL, about 700 pg/mL, about 750 pg/mL, about 800 pg/mL, about 850 pg/mL, about 900 pg/mL, about 950 pg/mL, about 1000 pg/mL, about 1050 pg/mL, about 1100 pg/mL, about 1150 pg/mL, about 1200 pg/mL, about 1250 pg/mL, about 1300 pg/mL, about 1350 pg/mL, about 1400 pg/mL, about 1450 pg/mL, about 1500 pg/mL, about 1550 pg/mL, about 1600 pg/mL, about 1650 pg/mL, about 1700 pg/mL, about 1750 pg/mL, about 1800 pg/mL, about 1850 pg/mL, about 1900 pg/mL, about 2000 pg/mL, about 2050 pg/mL, about 2100 pg/mL, about 2150 pg/mL, about 2200 pg/mL, about 2250 pg/mL, about 2300 pg/mL, about 2350 pg/mL, about 2400 pg/mL, about 2450 pg/mL, about 2500 pg/mL, about 2550 pg/mL, about 2600 pg/mL, about 2650 pg/mL, about 2700 pg/mL, about 2750 pg/mL, about 2800 pg/mL, about 2850 pg/mL, about 2900 pg/mL, or about 3000 pg/mL including all values and ranges therein. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, following once daily administration of a powder composition comprising from about 80 µg to about 675 µg (e.g., about 80 µg to about 640 µg, or about 112.5 µg to about 675 µg) of the compound of Formula (I) or (II), or a stereoisomer thereof, or a pharmaceutically acceptable salt thereof, the powder composition or method of use thereof provides a maximum treprostinil plasma concentration (Cₘₐₓ) ranging from about 10 pg/mL to about 2000 pg/mL, for example, about 10 pg/mL, about 15 pg/mL, about 20 pg/mL, about 25 pg/mL about 30 pg/mL, about 35 pg/mL, about 40 pg/mL, about 45 pg/mL, about 50 pg/mL, about 55 pg/mL, about 60 pg/mL, about 65 pg/mL, about 70 pg/mL, about 75 pg/mL, about 80 pg/mL, about 85 pg/mL, about 90 pg/mL, about 95 pg/mL, about 100 pg/mL, about 110 pg/mL, about 120 pg/mL, about 130 pg/mL, about 140 pg/mL, about 150 pg/mL, about 160 pg/mL, about 170 pg/mL, about 180 pg/mL, about 190 pg/mL, about 200 pg/mL, about 210 pg/mL, about 220 pg/mL, about 230 pg/mL, about 240 pg/mL, about 250 pg/mL, about 260 pg/mL, about 270 pg/mL, about 280 pg/mL, about 290 pg/mL, about 300 pg/mL, about 310 pg/mL, about 320 pg/mL, about 330 pg/mL, about 340 pg/mL, about 350 pg/mL, about 360 pg/mL, about 370 pg/mL, about 380 pg/mL, about 390 pg/mL, about 400 pg/mL, about 410 pg/mL, about 420 pg/mL, about 430 pg/mL, about 440 pg/mL, about 450 pg/mL, about 460 pg/mL, about 470 pg/mL, about 480 pg/mL, about 490 pg/mL, about 500 pg/mL, about 510 pg/mL, about 520 pg/mL, about 530 pg/mL, about 540 pg/mL, about 550 pg/mL, about 560 pg/mL, about 570 pg/mL, about 580 pg/mL, about 590 pg/mL, about 600 pg/mL, about 610 pg/mL, about 620 pg/mL, about 630 pg/mL, about 640 pg/mL, about 650 pg/mL, about 660 pg/mL, about 670 pg/mL, about 680 pg/mL, about 690 pg/mL, about 700 pg/mL, about 750 pg/mL, about 800 pg/mL, about 850 pg/mL, about 900 pg/mL, about 950 pg/mL, about 1000 pg/mL, about 1050 pg/mL, about 1100 pg/mL, about 1150 pg/mL, about 1200 pg/mL, about 1250 pg/mL, about 1300 pg/mL, about 1350 pg/mL, about 1400 pg/mL, about 1450 pg/mL, about 1500 pg/mL, about 1550 pg/mL, about 1600 pg/mL, about 1650 pg/mL, about 1700 pg/mL, about 1750 pg/mL, about 1800 pg/mL, about 1850 pg/mL, about 1900 pg/mL, or about 2000 pg/mL, including all values and ranges therein. The powder composition, in one embodiment, is a spray-dried powder composition.

In some embodiments, following once daily administration of about 80 µg to about 1280 µg µg (e.g., about 80 µg to about 640 µg, or about 360 µg to about 1280 µg) of the compound of Formula (I) or (II), or a stereoisomer thereof or a pharmaceutically acceptable salt thereof, the powder composition or method of use thereof provides an area under the plasma concentration curve (AUC) ranging from about 300 pg*h/mL to about 20000 pg*h/mL, for example, about 300 pg*h/mL, about 400 pg*h/mL, about 500 pg*h/mL, about 600 pg*h/mL, about 700 pg*h/mL, about 800 pg*h/mL, about 900 pg*h/mL, about 1000 pg*h/mL, about 1100 pg*h/mL, about 1200 pg*h/mL, about 1300 pg*h/mL, about 1400 pg*h/mL, about 1500 pg*h/mL, about 1600 pg*h/mL, about 1700 pg*h/mL, about 1800 pg*h/mL, about 1900 pg*h/mL, about 2000 pg*h/mL, about 2100 pg*h/mL, about 2200 pg*h/mL, about 2300 pg*h/mL, about 2400 pg*h/mL, about 2500 pg*h/mL, about 2600 pg*h/mL, about 2700 ng*h/mL, about 2800 ng*h/mL, about 2900 pg*h/mL, about 3000 pg*h/mL, about 3100 pg*h/mL, about 3200 pg*h/mL, about 3300 pg*h/mL, about 3400 pg*h/mL, about 3500 pg*h/mL, about 3600 pg*h/mL, about 3700 pg*h/mL, about 3800 pg*h/mL, about 3900 pg*h/mL, about 4000 pg*h/mL, about 4100 pg*h/mL, about 4200 pg*h/mL, about 4300 pg*h/mL, about 4400 pg*h/mL, about 4500 pg*h/mL, about 4600 pg*h/mL, about 4700 pg*h/mL, about 4800 pg*h/mL, about 4900 pg*h/mL, about 5000 pg*h/mL, about 5100 pg*hr/mL, about 5200 pg*hr/mL, about 5300 pg*hr/mL, about 5400 pg*hr/mL, about 5500 pg*hr/mL, about 5600 pg*hr/mL, about 5700 pg*hr/mL, about 5800 pg*hr/mL, about 5900 pg*hr/mL, about 6000 pg*hr/mL, about 6100 pg*hr/mL, about 6200 pg*hr/mL, about 6300 pg*h/mL*hr/mL, about 6400 pg*h/mL, about 6500 pg*h/mL*hr/mL, about 6600 pg*hr/mL, about 6700 pg*hr/mL, about 6800 pg*hr/mL, about 6900 pg*hr/mL, about 7000 pg*hr/mL, about 7100 pg*hr/mL, about 7200 pg*hr/mL, about 7300 pg*hr/mL, about 7400 pg*hr/mL, about 7500 pg*hr/mL, about 7600 pg*hr/mL, about 7700 pg*hr/mL, about 7800 pg*hr/mL, about 7900 pg*hr/mL, about 8000 pg*hr/mL, about 8100 pg*hr/mL, about 8200 pg*hr/mL, about 8300 pg*hr/mL, about 8400 pg*hr/mL, about 8500 pg*hr/mL, about 8600 pg*hr/mL, about 8700 pg*hr/mL, about 8800 pg*hr/mL, about 8900 pg*hr/mL, about 9000 pg*hr/mL, about 9100 pg*hr/mL, about 9200 pg*hr/mL, about 9300 pg*hr/mL, about 9400 pg*hr/mL, about 9500 pg*hr/mL, about 9600 pg*hr/mL, about 9700 pg*hr/mL, about 9800 pg*hr/mL, about 9900 pg*hr/mL, about 10000 pg*hr/mL, about 10100 pg*hr/mL, about 10200 pg*hr/mL, about 10300 pg*hr/mL, about 10400 pg*hr/mL, about 10500 pg*hr/mL, about 10600 pg*hr/mL, about 10700 pg*hr/mL, about 10800 pg*hr/mL, about 10900 pg*hr/mL, about 11000 pg*hr/mL, about 11100 pg*hr/mL, about 11200 pg*hr/mL, about 11300 pg*hr/mL, about 11400 pg*hr/mL, about 11500 pg*hr/mL, about 11600 pg*hr/mL, about 11700 pg*hr/mL, about 11800 pg*hr/mL, about 11900 pg*hr/mL, about 12000 pg*hr/mL, about 12100 pg*hr/mL, about 12200 pg*hr/mL, about 12300 pg*hr/mL, about 12400 pg*hr/mL, about 12500 pg*hr/mL, about 12600 pg*hr/mL, about 12700 pg*hr/mL, about 12800 pg*hr/mL, about 12900 pg*hr/mL, about 13000 pg*hr/mL, about 13100 pg*hr/mL, about 13200 pg*hr/mL, about 13300 pg*hr/mL, about 13400 pg*hr/mL, about 13500 pg*hr/mL, about 13600 pg*hr/mL, about 13700 pg*hr/mL, about 13800 pg*hr/mL, about 13900 pg*hr/mL, about 14000 pg*hr/mL, about 14100 pg*hr/mL, about 14200 pg*hr/mL, about 14300 pg*hr/mL, about 14400 pg*hr/mL, about 14500 pg*hr/mL, about 14600 pg*hr/mL, about 14700 pg*hr/mL, about 14800 pg*hr/mL, about 14900 pg*hr/mL, about 15000 pg*hr/mL, about 15100 pg*hr/mL, about 15200 pg*hr/mL, about 15300 pg*hr/mL, about 15400 pg*hr/mL, about 15500 pg*hr/mL, about 15600 pg*hr/mL, about 15700 pg*hr/mL, about 15800 pg*hr/mL, about 15900 pg*hr/mL, about 16000 pg*hr/mL, about 16100 pg*hr/mL, about 16200 pg*hr/mL, about 16300 pg*hr/mL, about 16400 pg*hr/mL, about 16500 pg*hr/mL, about 16600 pg*hr/mL, about 16700 pg*hr/mL, about 16800 pg*hr/mL, about 16900 pg*hr/mL, about 17000 pg*hr/mL, about 17100 pg*hr/mL, about 17200 pg*hr/mL, about 17300 pg*hr/mL, about 17400 pg*hr/mL, about 17500 pg*hr/mL, about 17600 pg*hr/mL, about 17700 pg*hr/mL, about 17800 pg*hr/mL, about 17900 pg*hr/mL, about 18000 pg*hr/mL, about 18100 pg*hr/mL, about 18200 pg*hr/mL, about 18300 pg*hr/mL, about 18400 pg*hr/mL, about 18500 pg*hr/mL, about 18600 pg*hr/mL, about 18700 pg*hr/mL, about 18800 pg*hr/mL, about 18900 pg*hr/mL, about 19000 pg*hr/mL, about 19100 pg*hr/mL, about 19200 pg*hr/mL, about 19300 pg*hr/mL, about 19400 pg*hr/mL, about 19500 pg*hr/mL, about 19600 pg*hr/mL, about 19700 pg*hr/mL, about 19800 pg*hr/mL, about 19900 pg*hr/mL, or about 20000 pg*hr/mL, including all values and ranges therein. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, following once daily administration of about 80 µg to about 675 µg (e.g., about 80 µg to about 640 µg, or about 112.5 µg to about 675 µg) of the compound of Formula (I) or (II), or a stereoisomer thereof or a pharmaceutically acceptable salt thereof, the powder composition or method of use thereof provides an area under the plasma concentration curve (AUC) ranging from about 300 pg*h/mL to about 11000 pg*h/mL, for example, about 300 pg*h/mL, about 400 pg*h/mL, about 500 pg*h/mL, about 600 pg*h/mL, about 700 pg*h/mL, about 800 pg*h/mL, about 900 pg*h/mL, about 1000 pg*h/mL, about 1100 pg*h/mL, about 1200 pg*h/mL, about 1300 pg*h/mL, about 1400 pg*h/mL, about 1500 pg*h/mL, about 1600 pg*h/mL, about 1700 pg*h/mL, about 1800 pg*h/mL, about 1900 pg*h/mL, about 2000 pg*h/mL, about 2100 pg*h/mL, about 2200 pg*h/mL, about 2300 pg*h/mL, about 2400 pg*h/mL, about 2500 pg*h/mL, about 2600 pg*h/mL, about 2700 ng*h/mL, about 2800 ng*h/mL, about 2900 pg*h/mL, about 3000 pg*h/mL, about 3100 pg*h/mL, about 3200 pg*h/mL, about 3300 pg*h/mL, about 3400 pg*h/mL, about 3500 pg*h/mL, about 3600 pg*h/mL, about 3700 pg*h/mL, about 3800 pg*h/mL, about 3900 pg*h/mL, about 4000 pg*h/mL, about 4100 pg*h/mL, about 4200 pg*h/mL, about 4300 pg*h/mL, about 4400 pg*h/mL, about 4500 pg*h/mL, about 4600 pg*h/mL, about 4700 pg*h/mL, about 4800 pg*h/mL, about 4900 pg*h/mL, about 5000 pg*h/mL, about 5100 pg*hr/mL, about 5200 pg*hr/mL, about 5300 pg*hr/mL, about 5400 pg*hr/mL, about 5500 pg*hr/mL, about 5600 pg*hr/mL, about 5700 pg*hr/mL, about 5800 pg*hr/mL, about 5900 pg*hr/mL, about 6000 pg*hr/mL, about 6100 pg*hr/mL, about 6200 pg*hr/mL, about 6300 pg*h/mLg*hr/mL, about 6400 pg*h/mL, about 6500 pg*h/mLg*hr/mL, about 6600 pg*hr/mL, about 6700 pg*hr/mL, about 6800 pg*hr/mL, about 6900 pg*hr/mL, about 7000 pg*hr/mL, about 7100 pg*hr/mL, about 7200 pg*hr/mL, about 7300 pg*hr/mL, about 7400 pg*hr/mL, about 7500 pg*hr/mL, about 7600 pg*hr/mL, about 7700 pg*hr/mL, about 7800 pg*hr/mL, about 7900 pg*hr/mL, about 8000 pg*hr/mL, about 8100 pg*hr/mL, about 8200 pg*hr/mL, about 8300 pg*hr/mL, about 8400 pg*hr/mL, about 8500 pg*hr/mL, about 8600 pg*hr/mL, about 8700 pg*hr/mL, about 8800 pg*hr/mL, about 8900 pg*hr/mL, about 9000 pg*hr/mL, about 9100 pg*hr/mL, about 9200 pg*hr/mL, about 9300 pg*hr/mL, about 9400 pg*hr/mL, about 9500 pg*hr/mL, about 9600 pg*hr/mL, about 9700 pg*hr/mL, about 9800 pg*hr/mL, about 9900 pg*hr/mL, about 10000 pg*hr/mL, about 10100 pg*hr/mL, about 10200 pg*hr/mL, about 10300 pg*hr/mL, about 10400 pg*hr/mL, about 10500 pg*hr/mL, about 10600 pg*hr/mL, about 10700 pg*hr/mL, about 10800 pg*hr/mL, about 10900 pg*hr/mL, or about 11000 pg*hr/mL including all values and ranges therein. The powder composition, in one embodiment, is a spray-dried powder composition.

In some embodiments, the powder composition or method of use thereof achieves treprostinil plasma trough concentration during an administration period of the powder composition. In some embodiments, the plasma trough levels are sufficient to provide a sustained therapeutic response during the administration period.

In some embodiments, the powder composition or method of use comprises from about 80 µg to about 1280 µg of the compound of Formula (I) or (II), or a stereoisomer thereof or a pharmaceutically acceptable salt thereof, and following once daily administration, the treprostinil plasma trough concentration ranges from about 3 pg/mL to about 250 mg/mL, for example about 4 pg/mL, about 4 pg/mL, about 5 pg/mL, about 10 pg/mL, about 15 pg/mL, about 20 pg/mL, about 25 pg/mL about 30 pg/mL, about 35 pg/mL, about 40 pg/mL, about 45 pg/mL, about 50 pg/mL, about 55 pg/mL, about 60 pg/mL, about 65 pg/mL, about 70 pg/mL, about 75 pg/mL, about 80 pg/mL, about 85 pg/mL, about 90 pg/mL, about 95 pg/mL, about 100 pg/mL, about 100 pg/mL, about 105 pg/mL, about 110 pg/mL, about 115 pg/mL, about 120 pg/mL, about 125 pg/mL about 130 pg/mL, about 135 pg/mL, about 140 pg/mL, about 145 pg/mL, about 150 pg/mL, about 155 pg/mL, about 160 pg/mL, about 165 pg/mL, about 170 pg/mL, about 175 pg/mL, about 180 pg/mL, about 185 pg/mL, about 190 pg/mL, about 195 pg/mL, about 200 pg/mL, about 200 pg/mL, about 205 pg/mL, about 210 pg/mL, about 215 pg/mL, about 220 pg/mL, about 225 pg/mL about 230 pg/mL, about 235 pg/mL, about 240 pg/mL, about 245 pg/mL, or about 250 pg/mL, including all values and ranges therein. In some embodiments the powder composition is a spray-dried powder. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the powder composition or method of use thereof comprises from about 80 µg to about 675 µg of the compound of Formula (I) or a stereoisomer thereof (e.g., where R¹ is hexadecyl, e.g., linear hexadecyl), and following once daily administration, the powder composition provides or the subject (e.g., patient) has a treprostinil plasma trough concentration of at least about 1 pg/mL, about 2 pg/mL, about 3 pg/mL, about 4 pg/mL, about 5 pg/mL, about 10 pg/mL, about 15 pg/mL, about 20 pg/mL, about 25 pg/mL about 30 pg/mL, about 35 pg/mL, about 40 pg/mL, about 45 pg/mL, about 50 pg/mL, about 55 pg/mL, about 60 pg/mL, about 65 pg/mL, about 70 pg/mL, about 75 pg/mL, about 80 pg/mL, about 85 pg/mL, about 90 pg/mL, about 95 pg/mL, about 100 pg/mL, about 100 pg/mL, about 110 pg/mL, about 120 pg/mL, about 130 pg/mL, about 140 pg/mL, about 150 pg/mL, about 160 pg/mL, about 170 pg/mL, about 180 pg/mL, about 190 pg/mL, about 200 pg/mL, including all values and ranges therein.

In some embodiments, the powder composition or method of use comprises from about 80 µg to about 640 µg of the compound of Formula (I) or (II), a stereoisomer thereof, or a pharmaceutically acceptable salt thereof, and following once daily administration, the treprostinil plasma trough concentration ranges from about 3 pg/mL to about 125 pg/mL, for example about 3 pg/mL, about 4 pg/mL, about 5 pg/mL, about 10 pg/mL, about 15 pg/mL, about 20 pg/mL, about 25 pg/mL about 30 pg/mL, about 35 pg/mL, about 40 pg/mL, about 45 pg/mL, about 50 pg/mL, about 55 pg/mL, about 60 pg/mL, about 65 pg/mL, about 70 pg/mL, about 75 pg/mL, about 80 pg/mL, about 85 pg/mL, about 90 pg/mL, about 95 pg/mL, about 100 pg/mL, about 100 pg/mL, about 110 pg/mL, about 120 pg/mL, including all values and ranges therein. In some embodiments, the powder composition comprises from about 80 µg to about 640 µg of the compound of Formula (II), and the treprostinil plasma trough concentration ranges from about 10 pg/mL to about 100 pg/mL.

In some embodiments, following once daily administration of a powder composition comprising from about 80 µg to about 675 µg of the compound of Formula (I) or (II), a stereoisomer thereof, a pharmaceutically acceptable salt thereof, the powder composition or method of use thereof provides or the subject (e.g., patient) has at least one of the following characteristics:
(a) a maximum treprostinil plasma concentration (Cₘₐₓ) within about 80% to about 125% of the range of from about 17 pg/mL to about 1150 pg/mL, for example, about 13 pg/mL, about 14 pg/mL, about 15 pg/mL, about 20 pg/mL, about 25 pg/mL, about 30 pg/mL, about 35 pg/mL, about 40 pg/mL, about 45 pg/mL, about 50 pg/mL, about 55 pg/mL, about 60 pg/mL, about 65 pg/mL, about 70 pg/mL, about 75 pg/mL, about 80 pg/mL, about 85 pg/mL, about 90 pg/mL, about 95 pg/mL, about 100 pg/mL, about 110 pg/mL, about 120 pg/mL, about 130 pg/mL, about 140 pg/mL, about 150 pg/mL, about 160 pg/mL, about 170 pg/mL, about 180 pg/mL, about 190 pg/mL, about 200 pg/mL, about 210 pg/mL, about 220 pg/mL, about 230 pg/mL, about 240 pg/mL, about 250 pg/mL, about 260 pg/mL, about 270 pg/mL, about 280 pg/mL, about 290 pg/mL, about 300 pg/mL, about 310 pg/mL, about 320 pg/mL, about 330 pg/mL, about 340 pg/mL, about 350 pg/mL, about 360 pg/mL, about 370 pg/mL, about 380 pg/mL, about 390 pg/mL, about 400 pg/mL, about 410 pg/mL, about 420 pg/mL, about 430 pg/mL, about 440 pg/mL, about 450 pg/mL, about 460 pg/mL, about 470 pg/mL, about 480 pg/mL, about 490 pg/mL, about 500 pg/mL, about 510 pg/mL, about 520 pg/mL, about 530 pg/mL, about 540 pg/mL, about 550 pg/mL, about 560 pg/mL, about 570 pg/mL, about 580 pg/mL, about 590 pg/mL, about 600 pg/mL, about 610 pg/mL, about 620 pg/mL, about 630 pg/mL, about 640 pg/mL, about 650 pg/mL, about 660 pg/mL, about 670 pg/mL, about 680 pg/mL, about 690 pg/mL, about 700 pg/mL, about 750 pg/mL, about 800 pg/mL, about 850 pg/mL, about 900 pg/mL, about 950 pg/mL, about 1000 pg/mL, about 1050 pg/mL, about 1100 pg/mL, about 1150 pg/mL, about 1200 pg/mL, about 1250 pg/mL, about 1300 pg/mL, about 1350 pg/mL, about 1400 pg/mL, or about 1430 pg/mL, including all values and ranges therein; or
(b) a treprostinil area under the plasma concentration curve (AUC_{0-inf}) within about 80% to about 125% of the range of from about 475 pg*h/mL to about 8000 pg*h/mL, for example, about 370 pg*h/mL, about 400 pg*h/mL, about 450 pg*h/mL, about 500 pg*h/mL, about 550 pg*h/mL, about 600 pg*h/mL, about 650 pg*h/mL, about 700 pg*h/mL, about 800 pg*h/mL, about 900 pg*h/mL, about 1000 pg*h/mL, about 1100 pg*h/mL, about 1200 pg*h/mL, about 1300 pg*h/mL, about 1400 pg*h/mL, about 1500 pg*h/mL, about 1600 pg*h/mL, about 1700 pg*h/mL, about 1800 pg*h/mL, about 1900 pg*h/mL, about 2000 pg*h/mL, about 2100 pg*h/mL, about 2200 pg*h/mL, about 2300 pg*h/mL, about 2400 pg*h/mL, about 2500 pg*h/mL, about 2600 pg*h/mL, about 2700 ng*h/mL, about 2800 ng*h/mL, about 2900 pg*h/mL, about 3000 pg*h/mL, about 3100 pg*h/mL, about 3200 pg*h/mL, about 3300 pg*h/mL, about 3400 pg*h/mL, about 3500 pg*h/mL, about 3600 pg*h/mL, about 3700 pg*h/mL, about 3800 pg*h/mL, about 3900 pg*h/mL, about 4000 pg*h/mL, about 4100 pg*h/mL, about 4200 pg*h/mL, about 4300 pg*h/mL, about 4400 pg*h/mL, about 4500 pg*h/mL, about 4600 pg*h/mL, about 4700 pg*h/mL, about 4800 pg*h/mL, about 4900 pg*h/mL, about 5000 pg*h/mL, about 5100 pg*hr/mL, about 5200 pg*hr/mL, about 5300 pg*hr/mL, about 5400 pg*hr/mL, about 5500 pg*hr/mL, about 5600 pg*hr/mL, about 5700 pg*hr/mL, about 5800 pg*hr/mL, about 5900 pg*hr/mL, about 6000 pg*hr/mL, about 6100 pg*hr/mL, about 6200 pg*hr/mL, about 6300 pg*h/mL, about 6400 pg*h/mL, about 6500 pg*h/mL, about 6600 pg*hr/mL, about 6700 pg*hr/mL, about 6800 pg*hr/mL, about 6900 pg*hr/mL, about 7000 pg*hr/mL, about 7100 pg*hr/mL, about 7200 pg*hr/mL, about 7300 pg*hr/mL, about 7400 pg*hr/mL, about 7500 pg*hr/mL, about 7600 pg*hr/mL, about 7700 pg*hr/mL, about 7800 pg*hr/mL, about 7900 pg*hr/mL, about 8000 pg*hr/mL, about 8100 pg*hr/mL, about 8200 pg*hr/mL, about 8300 pg*h/mL, about 8400 pg*h/mL, about 8500 pg*h/mL, about 8600 pg*hr/mL, about 8700 pg*hr/mL, about 8800 pg*hr/mL, about 8900 pg*hr/mL, about 9000 pg*hr/mL, about 9100 pg*hr/mL, about 9200 pg*hr/mL, about 9300 pg*hr/mL, about 9400 pg*hr/mL, about 9500 pg*hr/mL, about 9600 pg*hr/mL, about 9700 pg*hr/mL, about 9800 pg*hr/mL, about 9900 pg*hr/mL, or about 10000 pg*hr/mL, including all values and ranges therein.

In some embodiments, the powder composition comprises about 80 µg of the compound of Formula (I) or (II), a stereoisomer thereof, or a pharmaceutically acceptable salt thereof, is administered once daily, and provides a treprostinil Cₘₐₓ ranging from about 14 pg/mL to about 155 pg/mL, for example, about 14pg/mL, about 15 pg/mL, about 20 pg/mL, about 25 pg/mL about 30 pg/mL, about 35 pg/mL, about 40 pg/mL, about 45 pg/mL, about 50 pg/mL, about 55 pg/mL, about 60 pg/mL, about 65 pg/mL, about 70 pg/mL, about 75 pg/mL, about 80 pg/mL, about 85 pg/mL, about 90 pg/mL, about 95 pg/mL, about 100 pg/mL, about 105 pg/mL about 110 pg/mL, about 115 pg/mL, about 120 pg/mL, about 125 pg/mL, about 130 pg/mL, about 135 pg/mL, about 140 pg/mL, about 145 pg/mL, about 150 pg/mL, and about 155 pg/mL, including all values and ranges therein. In some embodiments, about 80 µg of the compound of Formula (I) or (II), a stereoisomer thereof, or a pharmaceutically acceptable salt thereof is administered once daily and provides a treprostinil Cₘₐₓ of about 80%-125% of a range from about 17 pg/mL to about 125 pg/mL. In some embodiments, about 80 µg of the compound of Formula (I) or (II), a stereoisomer thereof, or pharmaceutically acceptable salt thereof is administered once daily and provides a treprostinil Cₘₐₓ of about 80%-125% of a range from about 35 pg/mL to about 105 pg/mL. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the powder composition comprises about 112.5 µg of the compound of Formula (I) or (II), a stereoisomer thereof, or a pharmaceutically acceptable salt thereof, and provides a treprostinil Cₘₐₓ (CV%) ranging from about 80% to about 125% of about 78.4 (72.9) pg/mL. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the powder composition comprises about 160 µg of the compound of Formula (I) or (II), a stereoisomer thereof, or a pharmaceutically acceptable salt thereof, is administered once daily, and provides a treprostinil Cₘₐₓ ranging from about 30 pg/mL to about 335 pg/mL, for example, about 30 pg/mL, about 35 pg/mL, about 40 pg/mL, about 45 pg/mL, about 50 pg/mL, about 55 pg/mL, about 60 pg/mL, about 65 pg/mL, about 70 pg/mL, about 75 pg/mL, about 80 pg/mL, about 85 pg/mL, about 90 pg/mL, about 95 pg/mL, about 100 pg/mL, about 105 pg/mL about 110 pg/mL, about 115 pg/mL, about 120 pg/mL, about 125 pg/mL, about 130 pg/mL, about 135 pg/mL, about 140 pg/mL, about 145 pg/mL, about 150 pg/mL, about 155 pg/mL, about 160 pg/mL, about 165 pg/mL, about 170 pg/mL, about 175 pg/mL, about 180 pg/mL, about 1850 pg/mL, about 190 pg/mL, about 195 pg/mL, about 200 pg/mL, about 205 pg/mL, about 210 pg/mL, about 215 pg/mL, about 220 pg/mL, about 225 pg/mL, about 230 pg/mL, about 235 pg/mL, about 240 pg/mL, about 245 pg/mL, about 250 pg/mL, about 255 pg/mL, about 260 pg/mL, about 265 pg/mL, about 270 pg/mL, about 275 pg/mL, about 280 pg/mL, about 285 pg/mL, about 290 pg/mL, about 295 pg/mL, about 300 pg/mL, about 305 pg/mL, about 310 pg/mL, about 315 pg/mL, about 320 pg/mL, about 325 pg/mL, about 330 pg/mL, about 335 pg/mL, about 340 pg/mL, about 345 pg/mL, or about 350 pg/mL, including all values and ranges therein. In some embodiments, about 160 µg of the compound of Formula (I) or (II), a stereoisomer thereof, or a pharmaceutically acceptable salt thereof is administered once daily and provides a treprostinil Cₘₐₓ from about 80%-125% of a range from about 35 pg/mL to about 270 pg/mL. In some embodiments, about 160 µg of the compound of Formula (I) or (II), a stereoisomer thereof, or a pharmaceutically acceptable salt thereof is administered once daily and provides a treprostinil Cₘₐₓ from about 80%-125% of a range from about 76 pg/mL to about 230 pg/mL. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the powder composition comprises about 225 µg of the compound of Formula (I) or (II), a stereoisomer thereof, or a pharmaceutically acceptable salt thereof, is administered once daily, and provides a treprostinil Cₘₐₓ ranging from about 80% to about 125% of about 287 (46.6) pg/mL. In some embodiments, the powder composition comprises about 225 µg of the compound of Formula (I) or (II), a stereoisomer thereof, or a pharmaceutically acceptable salt thereof, and provides a steady state treprostinil Cₘₐₓ ranging from about 80% to about 125% of about 193 (32.9) pg/mL. In some embodiments, the powder composition comprises about 225 µg of the compound of Formula (I) or (II), a stereoisomer thereof, or a pharmaceutically acceptable salt thereof, is administered once daily, and provides a steady state treprostinil Cₘₐₓ (CV%) ranging from about 80% to about 125% of about 228 (46.4) pg/mL. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the powder composition comprises about 240 µg of the compound of Formula (I) or (II), a stereoisomer thereof, or a pharmaceutically acceptable salt thereof is administered once daily, and provides a treprostinil Cₘₐₓ ranging from about 45 pg/mL to about 520 pg/mL, for example, about 45 pg/mL, about 50 pg/mL, about 60 pg/mL, about 70 pg/mL, about 80 pg/mL, about 90 pg/mL, about 100 pg/mL, about 110 pg/mL, about 120 pg/mL, about 130 pg/mL, about 140 pg/mL, about 150 pg/mL, about 160 pg/mL, about 170 pg/mL, about 180 pg/mL, about 190 pg/mL, about 200 pg/mL, about 210 pg/mL, about 220 pg/mL, about 230 pg/mL, about 240 pg/mL, about 250 pg/mL, about 260 pg/mL, about 270 pg/mL, about 280 pg/mL, about 290 pg/mL, about 300 pg/mL, about 310 pg/mL, about 320 pg/mL, about 330 pg/mL, about 340 pg/mL, about 350 pg/mL, about 360 pg/mL, about 370 pg/mL, about 380 pg/mL, about 390 pg/mL, about 400 pg/mL, about 410 pg/mL, about 420 pg/mL, about 430 pg/mL, about 440 pg/mL, about 450 pg/mL, about 460 pg/mL, about 470 pg/mL, about 480 pg/mL, about 490 pg/mL, about 500 pg/mL, about 510 pg/mL, or about 520 pg/mL, including all values and ranges therein. In some embodiments, about 240 µg of the compound of Formula (I) or (II), a stereoisomer thereof, or a pharmaceutically acceptable salt thereof is administered once daily and provides a treprostinil Cₘₐₓ from about 80%-125% of a range from about 55 pg/mL to about 415 pg/mL. In some embodiments, about 240 µg of the compound of Formula (I) or (II), a stereoisomer thereof, or a pharmaceutically acceptable salt thereof is administered once daily and provides a treprostinil Cₘₐₓ from about 80%-125% of a range from about 115 pg/mL to about 355 pg/mL. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the powder composition comprises about 320 µg of the compound of Formula (I) or (II), a stereoisomer thereof, or a pharmaceutically acceptable salt thereof, is administered once daily, and provides a treprostinil Cₘₐₓ ranging from about 60 pg/mL to about 700 pg/mL, for example, about 60 pg/mL, about 70 pg/mL, about 80 pg/mL, about 90 pg/mL, about 100 pg/mL, about 110 pg/mL, about 120 pg/mL, about 130 pg/mL, about 135 pg/mL, about 140 pg/mL, about 140 pg/mL, about 150 pg/mL, about 160 pg/mL, about 170 pg/mL, about 180 pg/mL, about 190 pg/mL, about 200 pg/mL, about 210 pg/mL, about 220 pg/mL, about 230 pg/mL, about 240 pg/mL, about 250 pg/mL, about 260 pg/mL, about 270 pg/mL, about 280 pg/mL, about 290 pg/mL, about 300 pg/mL, about 310 pg/mL, about 320 pg/mL, about 330 pg/mL, about 340 pg/mL, about 350 pg/mL, about 360 pg/mL, about 370 pg/mL, about 380 pg/mL, about 390 pg/mL, about 400 pg/mL, about 410 pg/mL, about 420 pg/mL, about 430 pg/mL, about 440 pg/mL, about 450 pg/mL, about 460 pg/mL, about 470 pg/mL, about 480 pg/mL, about 490 pg/mL, about 500 pg/mL, about 510 pg/mL, about 520 pg/mL, about 530 pg/mL, about 540 pg/mL, about 550 pg/mL, about 560 pg/mL, about 570 pg/mL, about 580 pg/mL, about 590 pg/mL, about 600 pg/mL, about 610 pg/mL, about 620 pg/mL, about 630 pg/mL, about 640 pg/mL, about 650 pg/mL, about 660 pg/mL, about 670 pg/mL, about 680 pg/mL, about 690 pg/mL, or about 700 pg/mL, including all values and ranges therein. In some embodiments, about 320 µg of the compound of Formula (I) or (II), a stereoisomer thereof, or a pharmaceutically acceptable salt thereof is administered once daily and provides a treprostinil Cₘₐₓ from about 80%-125% of a range from about 80 pg/mL to about 560 pg/mL. In some embodiments, about 320 µg of the compound of Formula (I) or (II), a stereoisomer thereof, or a pharmaceutically acceptable salt thereof is administered once daily and provides a treprostinil Cₘₐₓ from about 80%-125% of a range from about 160 pg/mL to about 480 pg/mL. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the powder composition comprises about 400 µg of the compound of Formula (I) or (II), a stereoisomer thereof, or a pharmaceutically acceptable salt thereof, is administered once daily, and provides a treprostinil Cₘₐₓ ranging from about 80 pg/mL to about 885 pg/mL, for example, about 80 pg/mL, about 90 pg/mL, about 100 pg/mL, about 110 pg/mL, about 120 pg/mL, about 130 pg/mL, about 135 pg/mL, about 140 pg/mL, about 140 pg/mL, about 150 pg/mL, about 160 pg/mL, about 170 pg/mL, about 180 pg/mL, about 190 pg/mL, about 200 pg/mL, about 210 pg/mL, about 220 pg/mL, about 230 pg/mL, about 240 pg/mL, about 250 pg/mL, about 260 pg/mL, about 270 pg/mL, about 280 pg/mL, about 290 pg/mL, about 300 pg/mL, about 310 pg/mL, about 320 pg/mL, about 330 pg/mL, about 340 pg/mL, about 350 pg/mL, about 360 pg/mL, about 370 pg/mL, about 380 pg/mL, about 390 pg/mL, about 400 pg/mL, about 410 pg/mL, about 420 pg/mL, about 430 pg/mL, about 440 pg/mL, about 450 pg/mL, about 460 pg/mL, about 470 pg/mL, about 480 pg/mL, about 490 pg/mL, about 500 pg/mL, about 510 pg/mL, about 520 pg/mL, about 530 pg/mL, about 540 pg/mL, about 550 pg/mL, about 560 pg/mL, about 570 pg/mL, about 580 pg/mL, about 590 pg/mL, about 600 pg/mL, about 610 pg/mL, about 620 pg/mL, about 630 pg/mL, about 640 pg/mL, about 650 pg/mL, about 660 pg/mL, about 670 pg/mL, about 680 pg/mL, about 690 pg/mL, and about 700 pg/mL, about 710 pg/mL, about 720 pg/mL, about 730 pg/mL, about 740 pg/mL, about 750 pg/mL, about 760 pg/mL, about 770 pg/mL, about 780 pg/mL, about 790 pg/mL, about 800 pg/mL, about 810 pg/mL, about 820 pg/mL, about 830 pg/mL, about 840 pg/mL, about 850 pg/mL, about 860 pg/mL, about 870 pg/mL, or about 880 pg/mL, including all values and ranges therein. In some embodiments, about 400 µg of the compound of Formula (I) or(II), a stereoisomer thereof, or a pharmaceutically acceptable salt thereof, is administered once daily and provides a treprostinil Cₘₐₓ from about 80%-125% of a range of about 100 pg/mL to about 705 pg/mL. In some embodiments, about 400 µg of the compound of Formula (I), (II), a stereoisomer thereof, or a pharmaceutically acceptable salt thereof is administered once daily and provides a treprostinil Cₘₐₓ from about 80%-125% of a range from about 200 pg/mL to about 605 pg/mL. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the powder composition comprises about 450 µg of the compound of Formula (I) or (II), a stereoisomer thereof, or a pharmaceutically acceptable salt thereof, is administered once daily, and provides a treprostinil Cₘₐₓ ranging from about 80% to about 125% of about 387 (38.6) pg/mL. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the powder composition comprises about 480 µg of the compound of Formula (I) or (II), a stereoisomer thereof, or a pharmaceutically acceptable salt thereof, is administered once daily, and provides a treprostinil Cₘₐₓ ranging from about 95 pg/mL to about 1065 pg/mL, for example, about 95 pg/mL, about 100 pg/mL, about 110 pg/mL, about 120 pg/mL, about 130 pg/mL, about 135 pg/mL, about 140 pg/mL, about 140 pg/mL, about 150 pg/mL, about 160 pg/mL, about 170 pg/mL, about 180 pg/mL, about 190 pg/mL, about 200 pg/mL, about 210 pg/mL, about 220 pg/mL, about 230 pg/mL, about 240 pg/mL, about 250 pg/mL, about 260 pg/mL, about 270 pg/mL, about 280 pg/mL, about 290 pg/mL, about 300 pg/mL, about 310 pg/mL, about 320 pg/mL, about 330 pg/mL, about 340 pg/mL, about 350 pg/mL, about 360 pg/mL, about 370 pg/mL, about 380 pg/mL, about 390 pg/mL, about 400 pg/mL, about 410 pg/mL, about 420 pg/mL, about 430 pg/mL, about 440 pg/mL, about 450 pg/mL, about 460 pg/mL, about 470 pg/mL, about 480 pg/mL, about 490 pg/mL, about 500 pg/mL, about 510 pg/mL, about 520 pg/mL, about 530 pg/mL, about 540 pg/mL, about 550 pg/mL, about 560 pg/mL, about 570 pg/mL, about 580 pg/mL, about 590 pg/mL, about 600 pg/mL, about 610 pg/mL, about 620 pg/mL, about 630 pg/mL, about 640 pg/mL, about 650 pg/mL, about 660 pg/mL, about 670 pg/mL, about 680 pg/mL, about 690 pg/mL, and about 700 pg/mL, about 710 pg/mL, about 720 pg/mL, about 730 pg/mL, about 740 pg/mL, about 750 pg/mL, about 760 pg/mL, about 770 pg/mL, about 780 pg/mL, about 790 pg/mL, about 800 pg/mL, about 810 pg/mL, about 820 pg/mL, about 830 pg/mL, about 840 pg/mL, about 850 pg/mL, about 860 pg/mL, about 870 pg/mL, about 880 pg/mL, about 890 pg/mL, about 900 pg/mL, about 910 pg/mL, about 920 pg/mL, about 930 pg/mL, about 940 pg/mL, about 950 pg/mL, about 960 pg/mL, about 970 pg/mL, about 980 pg/mL, about 1000 pg/mL, about 1010 pg/mL, about 1020 pg/mL, about 1030 pg/mL, about 1040 pg/mL, about 1050 pg/mL, about 1060 pg/mL, or about 1065 pg/mL including all values and ranges therein. In some embodiments, about 480 µg of the compound of Formula (I) or (II), a stereoisomer thereof, or a pharmaceutically acceptable salt thereof is administered once daily and provides a treprostinil Cₘₐₓ from about 80%-125% of about 120 pg/mL to about 855 pg/mL. In some embodiments, about 480 µg of the compound of Formula (I) or (II), a stereoisomer thereof, or a pharmaceutically acceptable salt thereof is administered once daily and provides a treprostinil Cₘₐₓ from about 80%-125% of a range from about 240 pg/mL to about 730 pg/mL. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the powder composition comprises about 640 µg of the compound of Formula (I) or (II), a stereoisomer thereof, or a pharmaceutically acceptable salt thereof, is administered once daily, and provides a treprostinil Cₘₐₓ ranging from about 130 pg/mL to about 1430 pg/mL, for example, about 130 pg/mL, about 135 pg/mL, about 140 pg/mL, about 140 pg/mL, about 150 pg/mL, about 160 pg/mL, about 170 pg/mL, about 180 pg/mL, about 190 pg/mL, about 200 pg/mL, about 210 pg/mL, about 220 pg/mL, about 230 pg/mL, about 240 pg/mL, about 250 pg/mL, about 260 pg/mL, about 270 pg/mL, about 280 pg/mL, about 290 pg/mL, about 300 pg/mL, about 310 pg/mL, about 320 pg/mL, about 330 pg/mL, about 340 pg/mL, about 350 pg/mL, about 360 pg/mL, about 370 pg/mL, about 380 pg/mL, about 390 pg/mL, about 400 pg/mL, about 410 pg/mL, about 420 pg/mL, about 430 pg/mL, about 440 pg/mL, about 450 pg/mL, about 460 pg/mL, about 470 pg/mL, about 480 pg/mL, about 490 pg/mL, about 500 pg/mL, about 510 pg/mL, about 520 pg/mL, about 530 pg/mL, about 540 pg/mL, about 550 pg/mL, about 560 pg/mL, about 570 pg/mL, about 580 pg/mL, about 590 pg/mL, about 600 pg/mL, about 610 pg/mL, about 620 pg/mL, about 630 pg/mL, about 640 pg/mL, about 650 pg/mL, about 660 pg/mL, about 670 pg/mL, about 680 pg/mL, about 690 pg/mL, and about 700 pg/mL, about 710 pg/mL, about 720 pg/mL, about 730 pg/mL, about 740 pg/mL, about 750 pg/mL, about 760 pg/mL, about 770 pg/mL, about 780 pg/mL, about 790 pg/mL, about 800 pg/mL, about 810 pg/mL, about 820 pg/mL, about 830 pg/mL, about 840 pg/mL, about 850 pg/mL, about 860 pg/mL, about 870 pg/mL, about 880 pg/mL, about 890 pg/mL, about 900 pg/mL, about 910 pg/mL, about 920 pg/mL, about 930 pg/mL, about 940 pg/mL, about 950 pg/mL, about 960 pg/mL, about 970 pg/mL, about 980 pg/mL, about 1000 pg/mL, about 1010 pg/mL, about 1020 pg/mL, about 1030 pg/mL, about 1040 pg/mL, about 1050 pg/mL, about 1060 pg/mL, about 1070 pg/mL, about 1080 pg/mL, about 1090 pg/mL, about 1100 pg/mL, about 1110 pg/mL, about 1120 pg/mL, about 1130 pg/mL, about 1140 pg/mL, about 1150 pg/mL, about 1160 pg/mL, about 1170 pg/mL, about 1180 pg/mL, about 1190 pg/mL, about 1200 pg/mL, about 1210 pg/mL, about 1220 pg/mL, about 1230 pg/mL, about 1240 pg/mL, about 1250 pg/mL, about 1260 pg/mL, about 1270 pg/mL, about 1280 pg/mL, about 1290 pg/mL, about 1300 pg/mL, about 1310 pg/mL, about 1320 pg/mL, about 1330 pg/mL, about 1340 pg/mL, about 1350 pg/mL, about 1360 pg/mL, about 1370 pg/mL, about 1380 pg/mL, about 1390 pg/mL, about 1400 pg/mL, about 1410 pg/mL, about 1420 pg/mL, or about 1430 pg/mL, including all values and ranges therein. In some embodiments, about 640 µg of the compound of Formula (I) or (II), a stereoisomer thereof, or a pharmaceutically acceptable salt thereof is administered once daily and provides a treprostinil Cₘₐₓ from about 80%-125% of a range of about 160 pg/mL to about 1140 pg/mL. In some embodiments, about 640 µg of the compound of Formula (I) or (II), a stereoisomer thereof, or a pharmaceutically acceptable salt thereof is administered once daily and provides a treprostinil Cₘₐₓ ranging from about 80%-125% of about 325 pg/mL to about 980 pg/mL. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the powder composition comprises about 675 µg of the compound of Formula (I) or (II), a stereoisomer thereof, or a pharmaceutically acceptable salt thereof, is administered once daily, and provides a treprostinil Cₘₐₓ ranging from about 80% to about 125% of about 717 (52.8) pg/mL. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the powder composition comprises about 720 µg of the compound of Formula (I) or (II), a stereoisomer thereof, or a pharmaceutically acceptable salt thereof, is administered once daily, and provides a treprostinil Cₘₐₓ ranging from about 80% to about 125% of about 185 pg/mL to about 1300 pg/mL, including all values and ranges therein. In some embodiments, the powder composition comprises about 720 µg of the compound of Formula (I) or (II), a stereoisomer thereof, or a pharmaceutically acceptable salt thereof, is administered once daily, and provides a treprostinil Cₘₐₓ ranging from about 80% to about 125% of about 368 pg/mL to about 1105 pg/mL, including all values and ranges therein. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the powder composition comprises about 800 µg of the compound of Formula (I) or (II), a stereoisomer thereof, or a pharmaceutically acceptable salt thereof, is administered once daily, and provides a treprostinil Cₘₐₓ ranging from about 80% to about 125% of about 200 pg/mL to about 1430 pg/mL, including all values and ranges therein. In some embodiments, the powder composition comprises about 800 µg of the compound of Formula (I) or (II), a stereoisomer thereof, or a pharmaceutically acceptable salt thereof, is administered once daily, and provides a treprostinil Cₘₐₓ ranging from about 80% to about 125% of about 400 pg/mL to about 1230 pg/mL, including all values and ranges therein. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the powder composition comprises about 880 µg of the compound of Formula (I) or (II), a stereoisomer thereof, or a pharmaceutically acceptable salt thereof, is administered once daily, and provides a treprostinil Cₘₐₓ ranging from about 80% to about 125% of about 225 pg/mL to about 1580 pg/mL, including all values and ranges therein. In some embodiments, the powder composition comprises about 880 µg of the compound of Formula (I) or (II), a stereoisomer thereof, or a pharmaceutically acceptable salt thereof, is administered once daily, and provides a treprostinil Cₘₐₓ ranging from about 80% to about 125% of about 450 pg/mL to about 1360 pg/mL, including all values and ranges therein. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the powder composition comprises about 960 µg of the compound of Formula (I) or (II), a stereoisomer thereof, or a pharmaceutically acceptable salt thereof, is administered once daily, and provides a treprostinil Cₘₐₓ ranging from about 80% to about 125% of about 240 pg/mL to about 1720 pg/mL, including all values and ranges therein. In some embodiments, the powder composition comprises about 960 µg of the compound of Formula (I) or (II), a stereoisomer thereof, or a pharmaceutically acceptable salt thereof, is administered once daily, and provides a treprostinil Cₘₐₓ ranging from about 80% to about 125% of about 490 pg/mL to about 1480 pg/mL, including all values and ranges therein. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the powder composition comprises about 1040 µg of the compound of Formula (I) or (II), a stereoisomer thereof, or a pharmaceutically acceptable salt thereof, is administered once daily, and provides a treprostinil Cₘₐₓ ranging from about 80% to about 125% of about 530 pg/mL to about 1605 pg/mL, including all values and ranges therein. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the powder composition comprises about 1120 µg of the compound of Formula (I) or (II), a stereoisomer thereof, or a pharmaceutically acceptable salt thereof, is administered once daily, and provides a treprostinil Cₘₐₓ ranging from about 80% to about 125% of about 280 pg/mL to about 2020 pg/mL, including all values and ranges therein. In some embodiments, the powder composition comprises about 1120 µg of the compound of Formula (I) or (II), a stereoisomer thereof, or a pharmaceutically acceptable salt thereof, is administered once daily, and provides a treprostinil Cₘₐₓ ranging from about 80% to about 125% of about 575 pg/mL to about 1730 pg/mL, including all values and ranges therein. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the powder composition comprises about 1200 µg of the compound of Formula (I) or (II), a stereoisomer thereof, or a pharmaceutically acceptable salt thereof, is administered once daily, and provides a treprostinil Cₘₐₓ ranging from about 80% to about 125% of about 300 pg/mL to about 2165 pg/mL, including all values and ranges therein. In some embodiments, the powder composition comprises about 1200 µg of the compound of Formula (I) or (II), a stereoisomer thereof, or a pharmaceutically acceptable salt thereof, is administered once daily, and provides a treprostinil Cₘₐₓ ranging from about 80% to about 125% of about 615 pg/mL to about 1855 pg/mL, including all values and ranges therein. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the powder composition comprises about 1280 µg of the compound of Formula (I) or (II), a stereoisomer thereof, or a pharmaceutically acceptable salt thereof, is administered once daily, and provides a treprostinil Cₘₐₓ ranging from about 80% to about 125% of about 325 pg/mL to about 2310 pg/mL, including all values and ranges therein. In some embodiments, the powder composition comprises about 1280 µg of the compound of Formula (I) or (II), a stereoisomer thereof, or a pharmaceutically acceptable salt thereof, is administered once daily, and provides a treprostinil Cₘₐₓ ranging from about 80% to about 125% of about 660 pg/mL to about 1980 pg/mL, including all values and ranges therein. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the powder composition comprises about 80 µg of the compound of Formula (I) or (II), a stereoisomer thereof, or a pharmaceutically acceptable salt thereof, is administered once daily, and provides a treprostinil AUC_{0-inf} ranging from about 375 pg*h/mL to about 1800 pg*h/mL, for example, 375 pg*h/mL, 400 pg*h/mL, 500 pg*h/mL, 600 pg*h/mL, about 700 pg*h/mL, about 800 pg*h/mL, about 900 pg*h/mL, about 1000 pg*h/mL, about 1100 pg*h/mL, about 1200 pg*h/mL, about 1300 pg*h/mL, about 1400 pg*h/mL, about 1500 pg*h/mL, about 1600 pg*h/mL, about 1700 pg*h/mL, or about 1800 pg*h/mL, including all values and ranges therein. In some embodiments, about 80 µg of the compound of Formula (I) or (II), a stereoisomer thereof, or a pharmaceutically acceptable salt thereof, is administered once daily, and provides a treprostinil AUC_{0-inf} from about 80%-125% of a range of about 475 pg*h/mL to about 1430 pg*h/mL. In some embodiments, the powder composition comprises about 80 µg of the compound of Formula (I) or (II), a stereoisomer thereof, or a pharmaceutically acceptable salt thereof, and upon administration, the powder composition or method of use thereof provides a treprostinil AUC_{0-inf} from about 80%-125% of about 660 pg*h/mL to about 1240 pg*h/mL. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the powder composition comprises about 112.5 µg of the compound of Formula (I) or (II), a stereoisomer thereof, or a pharmaceutically acceptable salt thereof, is administered once daily, and provides a treprostinil AUC_{0-inf} ranging from about 80% to about 125% of about 1090 (91.8) pg*h/mL. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the powder composition comprises about 160 µg of the compound of Formula (I) or (II), a stereoisomer thereof, or a pharmaceutically acceptable salt thereof, is administered once daily, and provides a treprostinil AUC_{0-inf} ranging from about 630 pg*h/mL to about 3000 pg*h/mL, for example, 630 pg*h/mL, about 700 pg*h/mL, about 800 pg*h/mL, about 900 pg*h/mL, about 1000 pg*h/mL, about 1100 pg*h/mL, about 1200 pg*h/mL, about 1300 pg*h/mL, about 1400 pg*h/mL, about 1500 pg*h/mL, about 1600 pg*h/mL, about 1700 pg*h/mL, about 1800 pg*h/mL, about 1900 pg*h/mL, about 2000 pg*h/mL, about 2100 pg*h/mL, about 2200 pg*h/mL, about 2300 pg*h/mL, about 2400 pg*h/mL, about 2500 pg*h/mL, about 2600 pg*h/mL, about 2700 pg*h/mL, about 2800 pg*h/mL, about 2900 pg*h/mL, or about 3000 pg*h/mL, including all values and ranges therein. In some embodiments, the powder composition comprises about 160 µg of the compound of Formula (I) or (II), a stereoisomer thereof, or a pharmaceutically acceptable salt thereof, is administered once daily, and provides a treprostinil AUC_{0-inf} from about 80%-125% of a range from about 785 pg*h/mL to about 2370 pg*h/mL. In some embodiments, the powder composition comprises about 160 µg of the compound of Formula (I) or (II), a stereoisomer thereof, or a pharmaceutically acceptable salt thereof, is administered once daily, and provides a treprostinil AUC_{0-inf} from about 80%-125% of a range from about 1100 pg*h/mL to about 2050 pg*h/mL. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the powder composition comprises about 225 µg of a compound of Formula (I) or (II), a stereoisomer thereof, or a pharmaceutically acceptable salt thereof, is administered once daily, and provides an AUC_{0-inf} ranging from about 80% to about 125% of about 2130 (30.0) ng*h/mL. In some embodiments, the powder composition comprises about 225 µg of the compound of Formula (I) or (II), a stereoisomer thereof, or a pharmaceutically acceptable salt thereof, is administered once daily, and provides a steady state treprostinil AUC₀₋₂₄ (CV%) ranging from about 80% to about 125% of about 1680 (28.7) ng*h/mL. In some embodiments, the powder composition comprises about 225 µg of the compound of Formula (I) or (II), a stereoisomer thereof, or a pharmaceutically acceptable salt thereof, is administered once daily, and provides a steady state treprostinil AUC₀₋₂₄ (CV%) ranging from about 80% to about 125% of about 1790 (39.6) ng*h/mL. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the powder composition comprises about 450 µg of the compound of Formula (I) or (II), a stereoisomer thereof, or a pharmaceutically acceptable salt thereof, is administered once daily, and provides a treprostinil AUC_{0-inf} ranging from about 80% to about 125% of about 4040 (27.4) pg*h/mL. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the powder composition comprises about 240 µg of the compound of Formula (I) or (II), a stereoisomer thereof, or a pharmaceutically acceptable salt thereof, is administered once daily, and provides a treprostinil AUC_{0-inf} ranging from about 880 pg*h/mL to about 4130 pg*h/mL, for example, about 800 pg*h/mL, about 900 pg*h/mL, about 950 pg*h/mL, about 1000 pg*h/mL, about 1050 pg*h/mL, about 1100 pg*h/mL, about 1150 pg*h/mL, about 1200 pg*h/mL, about 1250 pg*h/mL, about 1300 pg*h/mL, about 1350 pg*h/mL, about 1400 pg*h/mL, about 1450 pg*h/mL, about 1500 pg*h/mL, about 1550 pg*h/mL, about 1600 pg*h/mL, about 1650 pg*h/mL, about 1700 pg*h/mL, about 1750 pg*h/mL, about 1800 pg*h/mL, about 1850 pg*h/mL, about 1950 pg*h/mL, about 2000 pg*h/mL, about 2050 pg*h/mL, about 2100 pg*h/mL, about 2150 pg*h/mL, about 2200 pg*h/mL, about 2250 pg*h/mL, about 2300 pg*h/mL, about 2350 pg*h/mL, about 2400 pg*h/mL, about 2450 pg*h/mL, about 2500 pg*h/mL, about 2550 pg*h/mL, about 2600 pg*h/mL, about 2650 pg*h/mL, about 2700 pg*h/mL, about 2750 pg*h/mL, about 2800 pg*h/mL, about 2850 pg*h/mL, about 2950 pg*h/mL, about 3000 pg*h/mL, about 3050 pg*h/mL, about 3100 pg*h/mL, about 3150 pg*h/mL, about 3200 pg*h/mL, about 3250 pg*h/mL, about 3300 pg*h/mL, about 3350 pg*h/mL, about 3400 pg*h/mL, about 3450 pg*h/mL, about 3500 pg*h/mL, about 3550 pg*h/mL, about 3600 pg*h/mL, about 3650 pg*h/mL, about 3700 pg*h/mL, about 3750 pg*h/mL, about 3800 pg*h/mL, about 3850 pg*h/mL, about 3950 pg*h/mL, about 4000 pg*h/mL, about 4050 pg*h/mL, about 4100 pg*h/mL, about 4130 pg*h/mL, including all values and ranges therein. In some embodiments, the powder composition comprises about 240 µg of the compound of Formula (I) or (II), a stereoisomer thereof, or a pharmaceutically acceptable salt thereof, is administered once daily, and provides a treprostinil AUC_{0-inf} from about 80%-125% of a range from about 1100 pg*h/mL to about 3305 pg*h/mL. In some embodiments, the powder composition comprises about 240 µg of the compound of Formula (I) or (II), a stereoisomer thereof, or a pharmaceutically acceptable salt thereof, is administered once daily, and provides a treprostinil AUC_{0-inf} from about 80%-125% of a range from about 1540 pg*h/mL to about 2865 pg*h/mL. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the powder composition comprises about 320 µg of the compound of Formula (I) or (II), a stereoisomer thereof, or a pharmaceutically acceptable salt thereof, is administered once daily, and provides a treprostinil AUC_{0-inf} ranging from about 1130 pg*h/mL to about 5310 pg*h/mL, for example, about 1130 pg*h/mL, about 1200 pg*h/mL, about 1300 pg*h/mL, about 1400 pg*h/mL, about 1450 pg*h/mL, about 1500 pg*h/mL, about 1550 pg*h/mL, about 1600 pg*h/mL, about 1700 pg*h/mL, about 1800 pg*h/mL, about 1900 pg*h/mL, about 2000 pg*h/mL, about 2100 pg*h/mL, about 2200 pg*h/mL, about 2300 pg*h/mL, about 2400 pg*h/mL, about 2500 pg*h/mL, about 2600 pg*h/mL, about 2700 pg*h/mL, about 2800 pg*h/mL, about 2900 pg*h/mL, about 3000 pg*h/mL, about 3100 pg*h/mL, about 3200 pg*h/mL, about 3300 pg*h/mL, about 3400 pg*h/mL, about 3500 pg*h/mL, about 3600 pg*h/mL, about 3700 pg*h/mL, about 3800 pg*h/mL, about 3900 pg*h/mL, about 4000 pg*h/mL, about 4100 pg*h/mL, about 4200 pg*h/mL, about 4300 pg*h/mL, about 4400 pg*h/mL, about 4500 pg*h/mL, about 4600 pg*h/mL, about 4700 pg*h/mL, about 4800 pg*h/mL, about 4900 pg*h/mL, about 5000 pg*h/mL, about 5100 pg*h/mL, about 5200 pg*h/mL, about 5300 pg*h/mL, about 5300 pg*h/mL, or about 5310 pg*h/mL, including all values and ranges therein. In some embodiments, the powder composition comprises about 320 µg of the compound of Formula (I) or (II), a stereoisomer thereof, or a pharmaceutically acceptable salt thereof, is administered once daily, and provides a treprostinil AUC_{0-inf} from about 80%-125% of a range from about 1400 pg*h/mL to about 4250 pg*h/mL. In some embodiments, about 320 µg of the compound of Formula (I) or (II), a stereoisomer thereof, or a pharmaceutically acceptable salt thereof, is administered once daily, and provides a treprostinil AUC_{0-inf} from about 80%-125% of a range from about 1975 pg*h/mL to about 3680 pg*h/mL. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the powder composition comprises about 400 µg of the compound of Formula (I) or (II), a stereoisomer thereof, or a pharmaceutically acceptable salt thereof, is administered once daily, and provides a treprostinil AUC_{0-inf} ranging from about 1380 pg*h/mL to about 6480 pg*h/mL, for example, about 1380 pg*h/mL, about 1400 pg*h/mL, about 1450 pg*h/mL, about 1500 pg*h/mL, about 1550 pg*h/mL, about 1600 pg*h/mL, about 1700 pg*h/mL, about 1800 pg*h/mL, about 1900 pg*h/mL, about 2000 pg*h/mL, about 2100 pg*h/mL, about 2200 pg*h/mL, about 2300 pg*h/mL, about 2400 pg*h/mL, about 2500 pg*h/mL, about 2600 pg*h/mL, about 2700 pg*h/mL, about 2800 pg*h/mL, about 2900 pg*h/mL, about 3000 pg*h/mL, about 3100 pg*h/mL, about 3200 pg*h/mL, about 3300 pg*h/mL, about 3400 pg*h/mL, about 3500 pg*h/mL, about 3600 pg*h/mL, about 3700 pg*h/mL, about 3800 pg*h/mL, about 3900 pg*h/mL, about 4000 pg*h/mL, about 4100 pg*h/mL, about 4200 pg*h/mL, about 4300 pg*h/mL, about 4400 pg*h/mL, about 4500 pg*h/mL, about 4600 pg*h/mL, about 4700 pg*h/mL, about 4800 pg*h/mL, about 4900 pg*h/mL, about 5000 pg*h/mL, about 5100 pg*h/mL, about 5200 pg*h/mL, about 5300 pg*h/mL, about 5400 pg*h/mL, about 5500 pg*h/mL, about 5600 pg*h/mL, about 5700 pg*h/mL, about 5800 pg*h/mL, about 5900 pg*h/mL, about 6000 pg*h/mL, about 6100 pg*h/mL, about 6200 pg*h/mL, about 6300 pg*h/mL, about 6400 pg*h/mL, or about 6480 pg*h/mL, including all values and ranges therein. In some embodiments, the powder composition comprises about 400 µg of the compound of Formula (I) or (II), a stereoisomer thereof, or a pharmaceutically acceptable salt thereof, is administered once daily, and provides a treprostinil AUC_{0-inf} from about 80%-125% of a range from about 1725 pg*h/mL to about 5180 pg*h/mL. In some embodiments, the powder composition comprises about 400 µg of the compound of Formula (I) or (II), a stereoisomer thereof, or a pharmaceutically acceptable salt thereof, is administered once daily, and provides a treprostinil AUC_{0-inf} from about 80%-125% of a range from about 2415pg*h/mL to about 4490 pg*h/mL. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the powder composition comprises about 480 µg of the compound of Formula (I) or (II), a stereoisomer thereof, or a pharmaceutically acceptable salt thereof, is administered once daily, and provides a treprostinil AUC_{0-inf} ranging from about 1630 pg*h/mL to about 7650 pg*h/mL, for example, about 1630 pg*h/mL, about 1700 pg*h/mL, about 1800 pg*h/mL, about 1900 pg*h/mL, about 2000 pg*h/mL, about 2100 pg*h/mL, about 2200 pg*h/mL, about 2300 pg*h/mL, about 2400 pg*h/mL, about 2500 pg*h/mL, about 2600 pg*h/mL, about 2700 pg*h/mL, about 2800 pg*h/mL, about 2900 pg*h/mL, about 3000 pg*h/mL, about 3100 pg*h/mL, about 3200 pg*h/mL, about 3300 pg*h/mL, about 3400 pg*h/mL, about 3500 pg*h/mL, about 3600 pg*h/mL, about 3700 pg*h/mL, about 3800 pg*h/mL, about 3900 pg*h/mL, about 4000 pg*h/mL, about 4100 pg*h/mL, about 4200 pg*h/mL, about 4300 pg*h/mL, about 4400 pg*h/mL, about 4500 pg*h/mL, about 4600 pg*h/mL, about 4700 pg*h/mL, about 4800 pg*h/mL, about 4900 pg*h/mL, about 5000 pg*h/mL, about 5100 pg*h/mL, about 5200 pg*h/mL, about 5300 pg*h/mL, about 5400 pg*h/mL, about 5500 pg*h/mL, about 5600 pg*h/mL, about 5700 pg*h/mL, about 5800 pg*h/mL, about 5900 pg*h/mL, about 6000 pg*h/mL, about 6100 pg*h/mL, about 6200 pg*h/mL, about 6300 pg*h/mL, about 6400 pg*h/mL, about 6500 pg*h/mL, about 6600 pg*h/mL, about 6700 pg*h/mL, about 6800 pg*h/mL, about 6900 pg*h/mL, about 7000 pg*h/mL, about 7100 pg*h/mL, about 7200 pg*h/mL, about 7300 pg*h/mL, about 7400 pg*h/mL, about 7500 pg*h/mL, or about 7650 pg*h/mL, including all values and ranges therein. In some embodiments, the powder composition comprises about 480 µg of the compound of Formula (I) or (II), a stereoisomer thereof, or a pharmaceutically acceptable salt thereof, is administered once daily, and provides a treprostinil AUC_{0-inf} from about 80%-125% of a range from about 2040 pg*h/mL to about 6120 pg*h/mL. In some embodiments, the powder composition comprises about 480 µg of the compound of Formula (I) or (II), a stereoisomer thereof, or a pharmaceutically acceptable salt thereof, is administered once daily, and provides a treprostinil AUC_{0-inf} from about 80%-125% of a range from about 2855 pg*h/mL to about 5310 pg*h/mL. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the powder composition comprises about 640 µg of the compound of Formula (I) or (II), a stereoisomer thereof, or a pharmaceutically acceptable salt thereof, is administered once daily, and provides a treprostinil AUC_{0-inf} ranging from about 2130 pg*h/mL to about 10000 pg*h/mL, for example, about 2130 pg*h/mL, about 2200 pg*h/mL, about 2300 pg*h/mL, about 2400 pg*h/mL, about 2500 pg*h/mL, about 2600 pg*h/mL, about 2700 pg*h/mL, about 2800 pg*h/mL, about 2900 pg*h/mL, about 3000 pg*h/mL, about 3100 pg*h/mL, about 3200 pg*h/mL, about 3300 pg*h/mL, about 3400 pg*h/mL, about 3500 pg*h/mL, about 3600 pg*h/mL, about 3700 pg*h/mL, about 3800 pg*h/mL, about 3900 pg*h/mL, about 4000 pg*h/mL, about 4100 pg*h/mL, about 4200 pg*h/mL, about 4300 pg*h/mL, about 4400 pg*h/mL, about 4500 pg*h/mL, about 4600 pg*h/mL, about 4700 pg*h/mL, about 4800 pg*h/mL, about 4900 pg*h/mL, about 5000 pg*h/mL, about 5100 pg*h/mL, about 5200 pg*h/mL, about 5300 pg*h/mL, about 5400 pg*h/mL, about 5500 pg*h/mL, about 5600 pg*h/mL, about 5700 pg*h/mL, about 5800 pg*h/mL, about 5900 pg*h/mL, about 6000 pg*h/mL, about 6100 pg*h/mL, about 6200 pg*h/mL, about 6300 pg*h/mL, about 6400 pg*h/mL, about 6500 pg*h/mL, about 6600 pg*h/mL, about 6700 pg*h/mL, about 6800 pg*h/mL, about 6900 pg*h/mL, about 7000 pg*h/mL, about 7100 pg*h/mL, about 7200 pg*h/mL, about 7300 pg*h/mL, about 7400 pg*h/mL, about 7500 pg*h/mL, about 7600 pg*h/mL, about 7700 pg*h/mL, about 7800 pg*h/mL, about 8000 pg*h/mL, about 8100 pg*h/mL, about 8200 pg*h/mL, about 8300 pg*h/mL, about 8400 pg*h/mL, about 8500 pg*h/mL, about 8600 pg*h/mL, about 8700 pg*h/mL, about 8800 pg*h/mL, about 8900 pg*h/mL, about 9000 pg*h/mL, about 9100 pg*h/mL, about 9200 pg*h/mL, about 9300 pg*h/mL, about 9350 pg*h/mL, about 9400 pg*h/mL, about 9450 pg*h/mL, about 9500 pg*h/mL, about 9600 pg*h/mL, about 9700 pg*h/mL, about 9800 pg*h/mL, about 9900 pg*h/mL, or about 10000 pg*h/mL, including all values and ranges therein. In some embodiments, the powder composition comprises about 640 µg of the compound of Formula (I) or (II), a stereoisomer thereof, or a pharmaceutically acceptable salt thereof, is administered once daily, and provides a treprostinil AUC_{0-inf} from about 80%-125% of a range from about 2650 pg*h/mL to about 8000 pg*h/mL. In some embodiments, the powder composition comprises about 640 µg of the compound of Formula (I) or (II), a stereoisomer thereof, or a pharmaceutically acceptable salt thereof, is administered once daily, and provides a treprostinil AUC_{0-inf} from about 80%-125% of a range from about 3730 to about 6935 pg*h/mL. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the powder composition comprises about 675 µg of the compound of Formula (I) or (II), a stereoisomer thereof, or a pharmaceutically acceptable salt thereof, is administered once daily, and provides a treprostinil AUC₀₋₂₄ ranging from about 80% to about 125% of about 5480 (13.8) pg*h/mL. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the powder composition comprises about 720 µg of the compound of Formula (I) or (II), a stereoisomer thereof, or a pharmaceutically acceptable salt thereof, is administered once daily, and provides a treprostinil AUC_{0-inf} from about 80%-125% of a range from about 2975 pg*h/mL to about 8940 pg*h/mL. In some embodiments, the powder composition comprises about 720 µg of the compound of Formula (I) or (II), a stereoisomer thereof, or a pharmaceutically acceptable salt thereof, is administered once daily, and provides a treprostinil AUC_{0-inf} from about 80%-125% of a range from about 4170 pg*h/mL to about 7750 pg*h/mL. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the powder composition comprises about 800 µg of the compound of Formula (I) or (II), a stereoisomer thereof, or a pharmaceutically acceptable salt thereof, is administered once daily, and provides a treprostinil AUC_{0-inf} from about 80%-125% of a range from about 3290 pg*h/mL to about 9900 pg*h/mL. In some embodiments, the powder composition comprises about 800 µg of the compound of Formula (I) or (II), a stereoisomer thereof, or a pharmaceutically acceptable salt thereof, is administered once daily, and provides a treprostinil AUC_{0-inf} from about 80%-125% of a range from about 4600 pg*h/mL to about 8560 pg*h/mL. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the powder composition comprises about 880 µg of the compound of Formula (I) or (II), a stereoisomer thereof, or a pharmaceutically acceptable salt thereof, is administered once daily, and provides a treprostinil AUC_{0-inf} from about 80%-125% of a range from about 3605 pg*h/mL to about 10815 pg*h/mL. In some embodiments, the powder composition comprises about 880 µg of the compound of Formula (I) or (II), a stereoisomer thereof, or a pharmaceutically acceptable salt thereof, is administered once daily, and provides a treprostinil AUC_{0-inf} from about 80%-125% of a range from about 5045 pg*h/mL to about 9380 pg*h/mL. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the powder composition comprises about 960 µg of the compound of Formula (I) or (II), a stereoisomer thereof, or a pharmaceutically acceptable salt thereof, and upon administration, the powder composition or method of use thereof provides a treprostinil AUC_{0-inf} from about 80%-125% of a range from about 3900 pg*h/mL to about 11800 pg*h/mL. In some embodiments, the powder composition comprises about 960 µg of the compound of Formula (I) or (II), a stereoisomer thereof, or a pharmaceutically acceptable salt thereof, is administered once daily, and provides a treprostinil AUC_{0-inf} from about 80%-125% of a range from about 5485 pg*h/mL to about 10190 pg*h/mL. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the powder composition comprises about 1040 µg of the compound of Formula (I) or (II), a stereoisomer thereof, or a pharmaceutically acceptable salt thereof, is administered once daily, and provides a treprostinil AUC_{0-inf} from about 80%-125% of a range from about 4230 pg*h/mL to about 12700 pg*h/mL. In some embodiments, the powder composition comprises about 1040 µg of the compound of Formula (I) or (II), a stereoisomer thereof, or a pharmaceutically acceptable salt thereof, is administered once daily, and provides a treprostinil AUC_{0-inf} from about 80%-125% of a range from about 5920 pg*h/mL to about 11000 pg*h/mL. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the powder composition comprises about 1120 µg of the compound of Formula (I) or (II), a stereoisomer thereof, or a pharmaceutically acceptable salt thereof, is administered once daily, and provides a treprostinil AUC_{0-inf} from about 80%-125% of a range from about 4500 pg*h/mL to about 13650 pg*h/mL. In some embodiments, the powder composition comprises about 1120 µg of the compound of Formula (I) or (II), a stereoisomer thereof, or a pharmaceutically acceptable salt thereof, is administered once daily, and provides a treprostinil AUC_{0-inf} from about 80%-125% of a range from about 6360 pg*h/mL to about 11815 pg*h/mL. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the powder composition comprises about 1200 µg of the compound of Formula (I) or (II), a stereoisomer thereof, or a pharmaceutically acceptable salt thereof, is administered once daily, and provides a treprostinil AUC_{0-inf} from about 80%-125% of a range from about 4850 pg*h/mL to about 14600 pg*h/mL. In some embodiments, the powder composition comprises about 1200 µg of the compound of Formula (I) or (II), a stereoisomer thereof, or a pharmaceutically acceptable salt thereof, is administered once daily, and provides a treprostinil AUC_{0-inf} from about 80%-125% of a range from about 6795 pg*h/mL to about 12630 pg*h/mL. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the powder composition comprises about 1280 µg of the compound of Formula (I) or (II), a stereoisomer thereof, or a pharmaceutically acceptable salt thereof, is administered once daily, and provides a treprostinil AUC_{0-inf} from about 80%-125% of a range from about 5150 pg*h/mL to about 15550 pg*h/mL. In some embodiments, the powder composition comprises about 1280 µg of the compound of Formula (I) or (II), a stereoisomer thereof, or a pharmaceutically acceptable salt thereof, is administered once daily, and provides a treprostinil AUC_{0-inf} from about 80%-125% of a range from about 7235 pg*h/mL to about 13445 pg*h/mL. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the powder composition comprises from about 80 µg to about 1280 µg of the compound of Formula (I) or (II), a stereoisomer thereof, or a pharmaceutically acceptable salt thereof, and the powder composition provides or following once daily administration the subject (e.g., patient) has a treprostinil plasma trough concentration ranging from about 3 pg/mL to about 250 mg/mL, for example about 4 pg/mL, about 4 pg/mL, about 5 pg/mL, about 10 pg/mL, about 15 pg/mL, about 20 pg/mL, about 25 pg/mL about 30 pg/mL, about 35 pg/mL, about 40 pg/mL, about 45 pg/mL, about 50 pg/mL, about 55 pg/mL, about 60 pg/mL, about 65 pg/mL, about 70 pg/mL, about 75 pg/mL, about 80 pg/mL, about 85 pg/mL, about 90 pg/mL, about 95 pg/mL, about 100 pg/mL, about 100 pg/mL, about 105 pg/mL, about 110 pg/mL, about 115 pg/mL, about 120 pg/mL, about 125 pg/mL about 130 pg/mL, about 135 pg/mL, about 140 pg/mL, about 145 pg/mL, about 150 pg/mL, about 155 pg/mL, about 160 pg/mL, about 165 pg/mL, about 170 pg/mL, about 175 pg/mL, about 180 pg/mL, about 185 pg/mL, about 190 pg/mL, about 195 pg/mL, about 200 pg/mL, about 200 pg/mL, about 205 pg/mL, about 210 pg/mL, about 215 pg/mL, about 220 pg/mL, about 225 pg/mL about 230 pg/mL, about 235 pg/mL, about 240 pg/mL, about 245 pg/mL, or about 250 pg/mL, including all values and ranges therein. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the powder composition comprises from about 80 µg to about 675 µg of the compound of Formula (I) or (II), a stereoisomer thereof, or a pharmaceutically acceptable salt thereof, and the powder composition provides or following once daily administration the subject (e.g., patient) has a treprostinil plasma trough concentration ranging from about 3 pg/mL to about 150 mg/mL, for example about 4 pg/mL, about 4 pg/mL, about 5 pg/mL, about 10 pg/mL, about 15 pg/mL, about 20 pg/mL, about 25 pg/mL about 30 pg/mL, about 35 pg/mL, about 40 pg/mL, about 45 pg/mL, about 50 pg/mL, about 55 pg/mL, about 60 pg/mL, about 65 pg/mL, about 70 pg/mL, about 75 pg/mL, about 80 pg/mL, about 85 pg/mL, about 90 pg/mL, about 95 pg/mL, about 100 pg/mL, about 100 pg/mL, about 105 pg/mL, about 110 pg/mL, about 115 pg/mL, about 120 pg/mL, about 125 pg/mL about 130 pg/mL, about 135 pg/mL, about 140 pg/mL, about 145 pg/mL, or about 150 pg/mL, including all values and ranges therein. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the powder composition comprises about 80 µg of the compound of Formula (I) or (II), a stereoisomer thereof, or a pharmaceutically acceptable salt thereof, and the powder composition provides or following once daily administration the subject (e.g., patient) has a treprostinil plasma trough concentration ranging from about 3 pg/mL to about 25 mg/mL, for example, about 3 pg/mL, about 4 pg/mL pg/mL, about 5 pg/mL, about 10 pg/mL, about 15 pg/mL, about 20 pg/mL, or about 25 pg/mL, including all values and ranges therein. In a further embodiment, the treprostinil plasma trough concentration ranges from about 6 pg/mL to about 18 mg/mL. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the powder composition comprises about 112.5 µg of the compound of Formula (I) or (II), a stereoisomer thereof, or a pharmaceutically acceptable salt thereof, and the powder composition provides or following once daily administration the subject (e.g., patient) has a treprostinil plasma trough concentration ranging from about 4 pg/mL to about 30 mg/mL, for example about 4 pg/mL, about 5 pg/mL, about 10 pg/mL, about 15 pg/mL, about 20 pg/mL, about 25 pg/mL, or about 30 pg/mL, including all values and ranges therein. The powder composition, in one embodiment, is a spray-dried powder composition. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the powder composition comprises about 160 µg of the compound of Formula (I) or (II), a stereoisomer thereof, or a pharmaceutically acceptable salt thereof, and the powder composition provides or following once daily administration the subject (e.g., patient) has a treprostinil plasma trough concentration ranging from about 5 pg/mL to about 35 mg/mL, for example about 5 pg/mL, about 10 pg/mL, about 15 pg/mL, about 20 pg/mL, about 25 pg/mL, about 30 pg/mL, or about 35 pg/mL, including all values and ranges therein. In a further embodiment, the treprostinil plasma trough concentration ranges from about 10 pg/mL to about 30 mg/mL, or from 15 pg/mL to about 25 pg/mL. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the powder composition comprises from about 225 µg of the compound of Formula (I) or (II), a stereoisomer thereof, or a pharmaceutically acceptable salt thereof, and the powder composition provides or following once daily administration the subject (e.g., patient) has a treprostinil plasma trough concentration ranging from about 15 pg/mL to about 45 mg/mL, for example about 15 pg/mL, about 20 pg/mL, about 25 pg/mL, about 30 pg/mL, about 35 pg/mL, about 40 pg/mL, or about 45 pg/mL, including all values and ranges therein. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the powder composition comprises from about 240 µg of the compound of Formula (I) or (II), a stereoisomer thereof, or a pharmaceutically acceptable salt thereof, and the powder composition provides or following once daily administration the subject (e.g., patient) has a treprostinil plasma trough concentration ranging from about 7 pg/mL to about 50 mg/mL, for example about 7 pg/mL, about 10 pg/mL, about 15 pg/mL, about 20 pg/mL, about 25 pg/mL, about 30 pg/mL, about 35 pg/mL, about 40 pg/mL, about 45 pg/mL, or about 50 pg/mL, including all values and ranges therein. In some embodiments, the treprostinil plasma trough concentration ranges from about 15 pg/mL to about 50 mg/mL, or from 20 pg/mL to about 45 pg/mL. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the powder composition comprises about 320 µg of the compound of Formula (I) or (II), a stereoisomer thereof, or a pharmaceutically acceptable salt thereof and the powder composition provides or following once daily administration the subject (e.g., patient) has a treprostinil plasma trough concentration ranging from about 9 pg/mL to about 65 mg/mL, for example about 9 pg/mL, about 10 pg/mL, about 15 pg/mL, about 20 pg/mL, about 25 pg/mL, about 30 pg/mL, about 35 pg/mL, about 40 pg/mL, about 45 pg/mL, about 50 pg/mL, about 55 pg/mL, about 60 pg/mL, or about 65 pg/mL, including all values and ranges therein. In some embodiments, the treprostinil plasma trough concentration ranges from about 15 pg/mL to about 50 mg/mL, or from 20 pg/mL to about 45 pg/mL. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the powder composition comprises about 400 µg of the compound of Formula (I) or (II), a stereoisomer thereof, or a pharmaceutically acceptable salt thereof, and the powder composition provides or following once daily administration the subject (e.g., patient) has a treprostinil plasma trough concentration ranging from about 10 pg/mL to about 80 mg/mL, for example about 10 pg/mL, about 15 pg/mL, about 20 pg/mL, about 25 pg/mL, about 30 pg/mL, about 35 pg/mL, about 40 pg/mL, about 45 pg/mL, about 50 pg/mL, about 55 pg/mL, about 60 pg/mL, about 65 pg/mL, about 70 pg/mL, about 75 pg/mL, or about 80 pg/mL including all values and ranges therein. In some embodiments, the treprostinil plasma trough concentration ranging from about 35 pg/mL to about 70 mg/mL, or from 40 pg/mL to about 65 pg/mL. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the powder composition comprises about 450 µg of the compound of Formula (I) or (II), a stereoisomer thereof, or a pharmaceutically acceptable salt thereof, and the powder composition provides or following once daily administration the subject (e.g., patient) has a treprostinil plasma trough concentration ranging from about 30 pg/mL to about 75 mg/mL, for example about 30 pg/mL, about 35 pg/mL, about 40 pg/mL, about 45 pg/mL, about 50 pg/mL, about 55 pg/mL, about 60 pg/mL, about 65 pg/mL, about 70 pg/mL, and about 75 pg/mL, including all values and ranges therein. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the powder composition comprises about 480 µg of the compound of Formula (I) or (II), a stereoisomer thereof, or a pharmaceutically acceptable salt thereof, and the powder composition provides or following once daily administration the subject (e.g., patient) has a treprostinil plasma trough concentration ranging from about 13 pg/mL to about 95 mg/mL, for example about 13 pg/mL, about 15 pg/mL, about 20 pg/mL, about 25 pg/mL, about 30 pg/mL, about 35 pg/mL, about 40 pg/mL, about 45 pg/mL, about 50 pg/mL, about 55 pg/mL, about 60 pg/mL, about 65 pg/mL, about 70 pg/mL, about 75 pg/mL, about 80 pg/mL, about 85 pg/mL, about 90 pg/mL, about 95 pg/mL, including all values and ranges therein. In some embodiments, the treprostinil plasma trough concentration ranging from about 25 pg/mL to about 75 mg/mL, or from 30 pg/mL to about 70 pg/mL. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the powder composition comprises about 640 µg of the compound of Formula (I) or (II), a stereoisomer thereof, or a pharmaceutically acceptable salt thereof, and the powder composition provides or following once daily administration the subject (e.g., patient) has a treprostinil plasma trough concentration ranging from about 15 pg/mL to about 125 mg/mL, for example about 15 pg/mL, about 20 pg/mL, about 25 pg/mL, about 30 pg/mL, about 35 pg/mL, about 40 pg/mL, about 45 pg/mL, about 50 pg/mL, about 55 pg/mL, about 60 pg/mL, about 65 pg/mL, about 70 pg/mL, about 75 pg/mL, about 80 pg/mL, about 85 pg/mL, about 90 pg/mL, about 95 pg/mL, about 100 pg/mL, about 105 pg/mL, about 110 pg/mL, about 115 pg/mL, about 120 pg/mL, or about 125 pg/mL, including all values and ranges therein. In some embodiments, the treprostinil plasma trough concentration ranging from about 35 pg/mL to about 100 mg/mL, or from 50 pg/mL to about 90 pg/mL. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the powder composition comprises about 675 µg of the compound of Formula (I) or (II), a stereoisomer thereof, or a pharmaceutically acceptable salt thereof and the powder composition provides or following once daily administration the subject (e.g., patient) has a treprostinil plasma trough concentration ranging from about 50 pg/mL to about 100 mg/mL, for example about 50 pg/mL, about 55 pg/mL, about 60 pg/mL, about 65 pg/mL, about 70 pg/mL, about 75 pg/mL, about 80 pg/mL, about 85 pg/mL, about 90 pg/mL, about 95 pg/mL, about 100 pg/mL, and about 100 pg/mL, including all values and ranges therein. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the powder composition comprises about 720 µg of the compound of Formula (I) or (II), a stereoisomer thereof, or a pharmaceutically acceptable salt thereof, and the powder composition provides or following once daily administration the subject (e.g., patient) has a treprostinil plasma trough concentration ranging from about 20 pg/mL to about 135 mg/mL, including all values and ranges therein. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the powder composition comprises about 800 µg of the compound of Formula (I) or (II), a stereoisomer thereof, or a pharmaceutically acceptable salt thereof, and the powder composition provides or following once daily administration the subject (e.g., patient) has a treprostinil plasma trough concentration ranging from about 21 pg/mL to about 150 mg/mL, including all values and ranges therein. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the powder composition comprises about 960 µg of the compound of Formula (I) or (II), a stereoisomer thereof, or a pharmaceutically acceptable salt thereof, and the powder composition provides or following once daily administration the subject (e.g., patient) has a treprostinil plasma trough concentration ranging from about 25 pg/mL to about 180 mg/mL, including all values and ranges therein. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the powder composition comprises about 1040 µg of the compound of Formula (I) or (II), a stereoisomer thereof, or a pharmaceutically acceptable salt thereof, and the powder composition provides or following once daily administration the subject (e.g., patient) has a treprostinil plasma trough concentration ranging from about 28 pg/mL to about 195 mg/mL, including all values and ranges therein. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the powder composition comprises about 1120 µg of the compound of Formula (I) or (II), a stereoisomer thereof, or a pharmaceutically acceptable salt thereof, and the powder composition provides or following once daily administration the subject (e.g., patient) has a treprostinil plasma trough concentration ranging from about 30 pg/mL to about 209 mg/mL, including all values and ranges therein. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the powder composition comprises about 1200 µg of the compound of Formula (I) or (II), a stereoisomer thereof, or a pharmaceutically acceptable salt thereof, and the powder composition provides or following once daily administration the subject (e.g., patient) has a treprostinil plasma trough concentration ranging from about 32 pg/mL to about 224 mg/mL, including all values and ranges therein. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the powder composition comprises about 1280 µg of the compound of Formula (I) or (II), a stereoisomer thereof, or a pharmaceutically acceptable salt thereof, and the powder composition provides or following once daily administration the subject (e.g., patient) has a treprostinil plasma trough concentration ranging from about 34 pg/mL to about 238 mg/mL, including all values and ranges therein. The powder composition, in one embodiment, is a spray-dried powder composition. In some embodiments, the spray-dried powder composition comprises particles. In some embodiments, particles have an average diameter ranging from about 1.0 µm to about 2.0 µm. In some embodiments, the particles have a D10 of about 0.3 µm, a D50 of about 1.7 µm, and/or a D90 of about 3.8 µm. In some embodiments, when the spray-dried powder composition is administered via a dry powder inhaler, the dry powder inhaler provides an aerosol comprising a fine particle fraction (FPF) greater than or equal to about 68% (e.g., about 68% to about 80%, about 68% to about 73%, or about 78% to about 80%), and one or more of (i) a fine particle dose (FPD) greater than or equal to 50% of a loaded dose of the powder composition (e.g. about 53% to about 77% or from about 60% to about 65%), (ii) an aerosol comprising an emitted dose (ED) greater than or equal to 72%, e.g., about 79% to about 89%, or (iii) a mass mean aerodynamic diameter (MMAD) less than 5 µm (e.g., from about 0.75 µm to about 3.0 µm or from about 0.8 µm to about 2.0 µm) when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the one or more pharmaceutically acceptable excipients which are suitable for use in a dry powder inhaler comprise sugar, amino acid, and optionally distearoyl phosphoethanoamine-polyethylene glycol 2000 (DPSE-PEG2000). In some embodiments of the powder compositions or methods described herein, the powder composition comprises from about 25 wt% to about 61 wt% of leucine, with the balance being one or more sugars. In some embodiments, the one or more sugars are selected from trehalose and mannitol. In some embodiments of the powder compositions or methods described herein, the powder composition does not include distearoylphosphoethanoamine-polyethylene glycol 2000 (DPSE-PEG2000).

### Aerosolized compositions

The powder compositions described herein are in some embodiments, aerosolized via a DPI to provide an aerosolized composition. The powder composition, in one embodiment, is a spray-dried powder composition.

The aerosolized composition is administered to a patient in need of treatment of PH, e.g., PAH or PH associated with interstitial lung disease (PH-ILD). In one embodiment, the aerosolized composition is administered to a patient in need of treatment of pulmonary fibrosis (e.g., PH-ILD where the ILD is pulmonary fibrosis). The aerosolized composition can be characterized by certain parameters known to those of skill in the art, such as fine particle fraction (FPF), fine particle dose (FPD), emitted dose (ED), mass median aerodynamic diameter (MMAD) and/or particle size and distribution. In embodiments, these parameters are measured by NGI. In embodiments, the NGI uses a flow rate corresponding to a 3kPa to 4.5 kPa air pressure drop relative the resistance of the DPI. In embodiments, the pressure drop is 4 kPa. For example, the performance of an ultra high resistance (UHR), high resistance (HR) and medium resistance (MR) devices may be evaluated by drawing 4 L of air through the DPI at a flow rate of 40, 60 and 85 LPM, respectively, corresponding to the 4 kPa air pressure drop. Aerosol characterization data for the above-noted parameters can be compared across different DPI devices (with different resistances). In embodiments, the mean inhaler resistance of a high resistance DPI used to achieve the parameters described herein ranges from 0.025 to 0.040 kPA^{1/2} (L/min⁻¹)⁻¹. In embodiments, the mean inhaler resistance of a high resistance DPI used to achieve the parameters described herein ranges from 0.030 to 0.040 kPA^{1/2} (L/min⁻¹)⁻¹. In embodiments, the mean inhaler resistance of a high resistance DPI used to achieve the parameters described herein ranges from about 0.034 to about 0.036 kPA^{1/2} (L/min⁻¹)⁻¹.

### (i) Fine particle fraction (FPF)

"Fine particle fraction" or "FPF" refers to the fraction of an aerosol having a particle size less than 5 µm in diameter, as measured by cascade impaction, such as NGI. FPF is usually expressed as a percentage. FPF has been demonstrated to correlate to the fraction of the powder that is deposited in the lungs of the subject (e.g., patient).

In some embodiments, the powder composition is in the form of an aerosol comprising particles with an FPF of at least 50%, at least 55%, at least 60%, at least 65%, at least 68%, at least 70%, at least 74%, at least 75%, at least 80%, at least 85%, or at least 90%. In some embodiments, the powder composition is in the form of an aerosol comprising particles with a FPF of about 50%, about 55%, about 60%, about 65%, about 70%, about 75%, about 80%, about 85%, about 90%, about 95%, or about 99%, or any value or range therein. In one embodiment, the aerosolized powder composition comprises particles with an FPF of from about 50% to about 100%, from about 60% to about 90%, from about 65% to about 85%, from about 68% to about 80%, from about 68% to about 73%, or from about 78% to about 80%, as measured by NGI.

In some embodiments, the aerosolized powder composition has an FPF at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89% or, at least 90%, or any range therein, when measured using a Next Generation Impactor (NGI), with a inspiratory flow rate of 30-60 liter per minute (L/min), inspiratory volume of 1-4 L, and inhalation acceleration rate of 0.5-20 L/sec².

In one embodiment, the powder composition exhibiting one of the FPF profiles provided above comprises mannitol. In another embodiment, the powder composition exhibiting the FPF profile provided above comprises trehalose.

In some embodiments, a powder composition comprises (a) about 0.5 wt% to about 5 wt% of a compound of Formula (I), or stereoisomer or a pharmaceutically acceptable salt thereof, wherein R1 is tetradecyl, pentadecyl, hexadecyl, heptadecyl, or octadecyl, (b) about 10 wt% to about 61wt% leucine, and (c) trehalose, mannitol or a combination thereof, wherein the sum of (a), (b), and (c) is 100%. When the powder composition is administered via a dry powder inhaler, the dry powder inhaler provides an aerosol comprising a fine particle fraction (FPF) greater than or equal to about 68%, when measured using a Next Generation Impactor (NGI), with a inspiratory flow rate of 60 liter per minute (L/min), inspiratory volume of 4 L, and inhalation acceleration rate of 0.5-20 L/sec².

In some embodiments, the aerosolized powder composition has FPF ranges from 78% to about 80% for 16 mg TPIP capsule (containing 160 µg TP) at 4L of inspiratory volume and 60 LPM of inspiratory flow rate. In some embodiments, the powder composition has FPF ranges from 76% to about 80% for 16 mg TPIP capsule (containing 160 µg TP) at 1L or 2L of inspiratory volume and 60 LPM of inspiratory flow rate. In some embodiments, the powder composition has FPF ranges from 74% to about 76% for 16 mg TPIP capsule (containing 160 µg TP) at 1L or 2L of inspiratory volume and 30 LPM of inspiratory flow rate.

In some embodiments, the aerosolized powder composition has FPF ranges from 68% to about 73% for 32 mg TPIP capsule (containing 320 µg TP) at 4L of inspiratory volume and 60 LPM of inspiratory flow rate. In some embodiments, the powder composition has FPF ranges from 63% to about 68% for 32 mg TPIP capsule at 1L or 2L of inspiratory volume and 60 LPM of inspiratory flow rate.

### (ii) Fine particle dose (FPD)

The term "fine particle dose" or "FPD", as used herein, refers to the mass of drug in an aerosol having a particle size less than 5 µm in diameter, as measured by cascade impaction. FPD may be expressed as percentage or mass. In the present disclosure, FPD is expressed as a percentage.

In some embodiments, The FPD of the aerosolized powder composition is at least 50%, at least 51%, at least 52%, at least 53%, at least 54%, at least 55%, at least 56%, at least 57%, at least 58%, at least 59%, at least 60%, at least 61%, at least 62%, at least 63%, at least 64%, at least 65%, at least 66%, at least 67%, at least 68%, at least 69%, at least 70%, at least 75%, at least 80%, at least 85%, or at least 90% of a loaded dose of the powder composition, when measured using a Next Generation Impactor (NGI), with a inspiratory flow rate of 30-60 liter per minute (L/min), inspiratory volume of 1-4 L, and inhalation acceleration rate of 0.5-20 L/sec². In some embodiments, The FPD of the aerosolized powder composition of about 50%, about 51%, about 52%, about 53%, about 54%, about 55%, about 56%, about 57%, about 58%, about 59%, about 60%, about 61%, about 62%, about 63%, about 64%, about 65%, about 66%, about 67%, about 68%, about 69%, about 70%, about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90%, or more, or any ranges therein, of a loaded dose of the powder composition, when measured using a Next Generation Impactor (NGI), with a inspiratory flow rate of 30-60 liter per minute (L/min), inspiratory volume of 1-4 L, and inhalation acceleration rate of 0.5-20 L/sec².

In some embodiments, the aerosolized powder composition has FPD ranges from 53% to about 77% for 16 or 32 mg TPIP capsule in different inspiratory flow rates, different acceleration rate and inspiratory volumes taught herein.

In some embodiments, the aerosolized powder composition has FPD ranges from 53% to about 65% for 32 mg TPIP capsule (containing 320 µg TP) and from 60% to about 65% for 16 mg TPIP capsule (containing 160 µg TP), when measured using a Next Generation Impactor (NGI), with a inspiratory flow rate of 30-60 liter per minute (L/min), inspiratory volume of 1-4 L, and inhalation acceleration rate of 0.5-20 L/sec².

### (iii) Emitted Dose (ED)

The term "emitted dose" or "ED", as used herein, refers to the percentage of drug recovered in the NGI relative to the total recovery of drug. The ED is an indication of the delivery of a drug from a suitable inhaler device after a firing or dispersion event. For powder compositions, the ED is a measure of the percentage of powder that is drawn out of a unit dose package. The ED is defined as the ratio of the dose delivered by an inhaler device to the nominal dose (i.e. the mass of powder per unit dose placed into the DPI prior to firing).

In some embodiments, the aerosolized powder composition has an ED greater than or equal to at least 70%, at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, or at least 90%, when measured using a Next Generation Impactor (NGI), with a inspiratory flow rate of 30-60 liter per minute (L/min), inspiratory volume of 1-4 L, and inhalation acceleration rate of 0.5-20 L/sec².

In some embodiments, the aerosolized powder composition has an ED of about 70%, about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90% or more, including any ranges therein, when measured using a Next Generation Impactor (NGI), with a inspiratory flow rate of 30-60 liter per minute (L/min), inspiratory volume of 1-4 L, and inhalation acceleration rate of 0.5-20 L/sec².

In some embodiments, the aerosolized powder composition has ED greater than or equal to about 79% for 16 or 32 mg TPIP capsule at 60 LPM and about 72% for 16 mg TPIP capsule at 30 LPM.

In some embodiments, the aerosolized powder composition has ED ranges from about 79% to about 89% for 16 or 32 mg TPIP capsule at 60 LPM. In some embodiments, the aerosolized powder composition has ED ranges from about 72% to about 76% for 16 mg TPIP capsule at 30 LPM.

### (iv) Mass Median Diameter (MMD) and Mass Median Aerodynamic Diameter (MMAD)

The term "mass median diameter" or "MMD", as used herein, refers to the average particle diameter in an aerosol based on the mass of the particles in the aerosol. MMD is determined by cascade impaction.

The term "mass median aerodynamic diameter" or "MMAD" is the MMD normalized with respect to the aerodynamic separation of aerosol droplets. MMAD is determined by cascade impaction. Mass median aerodynamic diameter (MMAD) is the value of aerodynamic diameter for which 50% of the mass in a given aerosol is associated with particles smaller than the median aerodynamic diameter (MAD), and 50% of the mass is associated with particles larger than the MAD.

For dry powder inhalers, oral cavity deposition is dominated by inertial impaction and so characterized by the aerosol's Stokes number (DeHaan et al. Journal of Aerosol Science, 35 (3), 309-331, 2003). For equivalent inhaler geometry, breathing pattern and oral cavity geometry, the Stokes number, and so the oral cavity deposition, is primarily affected by the aerodynamic size of the inhaled powder. Hence, factors that contribute to oral deposition of a powder include the size distribution of the individual particles and the dispersibility of the powder. If the MMAD of the individual particles is too large, e.g. above 5 µm, then an increasing percentage of powder will deposit in the oral cavity. Likewise, if a powder has poor dispersibility, it is an indication that the particles will leave the dry powder inhaler and enter the oral cavity as agglomerates. Agglomerated powder will perform aerodynamically like an individual particle as large as the agglomerate, therefore even if the individual particles are small (e.g., MMAD of about 5 µm or less), the size distribution of the inhaled powder may have an MMAD of greater than about 5 µm, leading to enhanced oral cavity deposition. As discussed herein, the present disclosure is based, in part, on the surprising discovery the spray dried composition described herein does not agglomerate and produces a high percentage of small particles that can be delivered to the deep lung regions such as the alveolar spaces.

Powder particles with an MMAD of about 1 µm to about 3 µm can be effectively delivered to the deep lung regions such as the alveolar spaces. Larger aerodynamic diameters, for example, from about 3 µm to about 5 µm can be delivered to the central and upper airways.

In some embodiments, the aerosolized powder composition comprises particles with an MMAD of from about 1 µm to about 10 µm, from about 1 µm to about 7 µm, from about 1 µm to about 5 µm, or from about 1 µm to about 4 µm, or from about 1.5 µm to about 3.5 µm, or from about 2 µm to about 3 µm, as measured by NGI. In some embodiments, the present disclosure relates to a powder composition with MMAD of 5 µm or less, e.g. between about 1 µm to 5 µm (e.g., about 1 µm, about 2 µm, about 3 µm, about 4 µm, or about 5 µm).

In other embodiments, the aerosolized powder composition comprises a mass mean aerodynamic diameter (MMAD) less than 5 µm. In other embodiments, the aerosolized powder composition has the MMAD ranges from about 0.75 µm to about 3.0 µm (e.g., about 0.75 µm, about 1.0 µm, about 1.25 µm, about 1.5 µm, about 1.75 µm, about 2.0 µm, about 2.25 µm, about 2.5 µm, about 2.75 µm, or about 3.0 µm). In other embodiments, the aerosolized powder composition has the MMAD ranges from about 0.8 µm to about 2.0 µm.

In one embodiment, the powder composition exhibiting one of the MMAD profiles provided above comprises mannitol. In another embodiment, the powder composition exhibiting the MMAD profile provided above comprises trehalose.

### (v) Particle size and distribution

In some embodiments, the powder composition comprises particles that have an average diameter ranging from about 1.0 µm to about 2.0 µm. In some embodiments, the particles have a D10 of about 0.3 µm. In some embodiments, the particles have a D50 of about 1.7 µm. In some embodiments, the particles have a D90 of about 3.8 µm. In some embodiments, these particle size distribution values distinguish the disclosed spray-dried powder compositions from similar compositions prepared by blending.

The present disclosure teaches the powder composition taught herein comprises from about 80 µg to about 1280 µg, from about 80 µg to about 640 µg, or from about 160 µg to about 1280 µg, or about 160 µg to about 640 µg of the compound of Formula (I).

In some embodiments, the powder composition taught herein comprises from about 80 µg to about 360 µg, from about 80 µg to about 320 µg, or from about 160 µg to about 320 µg of the compound of Formula (I).

In some embodiments, the powder composition taught herein comprises about 80 µg, 160 µg, 240 µg, 320 µg, 400 µg, 480 µg, 640 µg, 675 µg, 720 µg, 800 µg, 880 µg, 960 µg, 1040 µg , 1120 µg, 1200 µg, or 1280 µg of the compound of Formula (I), which is treprostinil palmitil. In other embodiments, the powder composition taught herein comprises about 80 µg, 160 µg, 240 µg, or 320 µg of the compound of Formula (I), which is treprostinil palmitil.

The present disclosure provides a powder composition comprising from about 80 µg to about 1280 µg of a compound of Formula (I), wherein R1 is linear hexadecyl, and wherein upon once daily inhalation administration via a dry powder inhaler, wherein the dry powder inhaler provides an aerosol comprising a fine particle fraction (FPF) greater than or equal to about 68%, as measured using a Next Generation Impactor (NGI).

The present disclosure also provides a powder composition comprising from about 160 µg to about 320 µg of a compound of Formula (I), wherein R1 is linear hexadecyl, and wherein upon once daily inhalation administration via a dry powder inhaler, wherein the dry powder inhaler provides an aerosol comprising a fine particle fraction (FPF) greater than or equal to about 68%, as measured using a Next Generation Impactor (NGI).

In some embodiments, the powder composition comprises about 160 µg of a compound of Formula (I), wherein R1 is linear hexadecyl, and upon once daily inhalation administration via a dry powder inhaler, the dry powder inhaler provides an aerosol comprising the FPF that ranges from 78% to about 80%.

In some embodiments, the powder composition comprises about 320 µg of a compound of Formula (I), wherein R1 is linear hexadecyl, and upon once daily inhalation administration via a dry powder inhaler, the dry powder inhaler provides an aerosol comprising the FPF that ranges from 68% to about 73%.

In some embodiments, the powder composition comprises from about 160 µg to about 320 µg of a compound of Formula (I), wherein R1 is linear hexadecyl, and upon once daily inhalation administration via a dry powder inhaler, the dry powder inhaler provides an aerosol comprising a fine particle dose (FPD) greater than or equal to 50% of a loaded dose of the powder composition.

In some embodiments, the powder composition comprises about 160 µg of a compound of Formula (I), wherein R1 is linear hexadecyl, and upon once daily inhalation administration via a dry powder inhaler, the dry powder inhaler provides an aerosol comprising the FPD that ranges from 60% to about 65%.

In some embodiments, the powder composition comprises about 320 µg of a compound of Formula (I), wherein R1 is linear hexadecyl, and upon once daily inhalation administration via a dry powder inhaler, the dry powder inhaler provides an aerosol comprising the FPF that ranges from 53% to about 65%.

In some embodiments, the powder composition comprises from about 160 µg to about 320 µg of a compound of Formula (I), wherein R1 is linear hexadecyl, and upon once daily inhalation administration via a dry powder inhaler, the dry powder inhaler provides an aerosol comprising an emitted dose (ED) greater than or equal to 72%.

In some embodiments, the powder composition comprises from about 160 µg to about 320 µg of a compound of Formula (I), wherein R1 is linear hexadecyl, and upon once daily inhalation administration via a dry powder inhaler, the dry powder inhaler provides an aerosol comprising the ED that ranges from about 79% to about 89%.

In some embodiments, the powder composition comprises from about 160 µg to about 320 µg of a compound of Formula (I), wherein R1 is linear hexadecyl, and upon once daily inhalation administration via a dry powder inhaler, the dry powder inhaler provides an aerosol comprising a mass mean aerodynamic diameter (MMAD) less than 5 µm.

In some embodiments, the powder composition comprises about 160 µg of a compound of Formula (I), wherein R1 is linear hexadecyl, and upon once daily inhalation administration via a dry powder inhaler, the dry powder inhaler provides an aerosol comprising the MMAD that ranges from about 1.0 µm to about 1.3 µm.

In some embodiments, the powder composition comprises about 320 µg of a compound of Formula (I), wherein R1 is linear hexadecyl, and upon once daily inhalation administration via a dry powder inhaler, the dry powder inhaler provides an aerosol comprising the MMAD that ranges from about 1.3 µm to about 1.7 µm.

### Production of Powder compositions

The powder compositions of the present disclosure may be produced from liquid compositions using lyophilization or spray-drying techniques. In one embodiment, spray-drying methods are performed to arrive at the powder compositions described herein. When lyophilization is used, the lyophilized composition may be milled to obtain the finely divided powder containing particles within the desired size range described above. When spray-drying is used, the process is carried out under conditions that result in a finely divided powder containing particles within the desired size range described above. Exemplary methods of preparing dry powder forms of pharmaceutical compositions are disclosed in WO 96/32149, WO 97/41833, WO 98/29096, and U.S. Patent Nos. 5,976,574, 5,985,248, and 6,001,336, the disclosure of each of which is incorporated herein by reference in their entireties. Exemplary spray drying methods are described in U.S. Application Publication No. 2020/0338005, and U.S. Patent Nos. 6,848,197 and 8,197,845, the disclosure of each of which is incorporated herein by reference in their entireties.

In some embodiments, the powder composition of the present disclosure is prepared by the following process. A stock solution of a compound of Formula (I) or (II), a stereoisomer thereof, or a pharmaceutically acceptable salt thereof is prepared using an organic solvent, such as an alcohol (e.g., 1-propanol). Aqueous stock solutions of a sugar (e.g., mannitol or trehalose) and leucine are also prepared. Afterwards required amounts of the above stock solutions are added to a mixture of water and the organic solvent to form a spray drying feed solution. In the spray drying feed solution, the volume ratio of water to the organic solvent may be from about 3:2 to about 1:1.

Spray drying is initiated by starting the drying gas flow and heating up the drying gas by setting the desired inlet temperature at, for example, from about 120 °C to about 180 °C, or from about 135 °C to about 150 °C. After the spray dryer outlet temperature reaches a suitable temperature, for example, at from about 55 °C to about 65 °C, the liquid skid inlet is set to allow blank solvents to be atomized with the aid of nitrogen into the spray dryer, and the system is allowed to cool and stabilize. Product filter pulsing is initiated, and product filter purge flow is set, for example, to 10 to 20 scfh. After the system stabilizes, the liquid skid inlet is switched to the feed solution prepared above and the process is continued till the feed solution runs out. At the point when the feed solution runs out, the liquid skid inlet is switched back to blank solvents, which are allowed to spray for from about 5 to about 20 minutes. At this point, powder is collected at the bottom of the product filter. After spraying the blank solvent for from about 5 to about 20 minutes, the system is shut down by shutting down the liquid lines, atomization gas, drying gas heater, drying gas inlet and finally the exhaust.

### Dry powder inhaler (DPI)

The powder compositions of the present disclosure are delivered to the lungs of a subject (e.g., patient) via inhalation using a dry powder inhaler (DPI). In one embodiment, the dry powder inhaler is a single dose dry powder inhaler. A propellant-free device, a DPI delivers powder to the lungs of a subject (e.g., patient) using the subject (e.g., patient) inspiration. The unit dose of a powder composition used in a DPI device is often a dry powder blister disc of hard capsule. Exemplary DPI devices suitable for delivering the powder compositions of the present disclosure include the devices described in the following paragraphs, as well as the DPIs described in U.S. Patent Nos. 6,766,799, 7,278,425 and 8,496,002, the disclosure of each of which is herein incorporated by reference in their entireties.

The DPI in one embodiment, is a capsule based DPI. In a further embodiment, the capsule based DPI is manufactured by Plastiape. In even a further embodiment, the capsule based DPI is a RS01 monodose dry powder inhaler developed by Plastiape, which features a compact size and a simple and effective perforation system and is suited for both gelatin and HMPC capsules.

Four Plastiape RS01 Model 7 DPIs (Plastiape S. p.A., Osnago, Italy) differ with respect to their device resistance: Ultra-high Resistance (UHR, code 239700005AA), High Resistance (HR, code 239700002AA), Medium Resistance (MR, code 239700006AA) and Low Resistance (LR, code 239700001AB). The pressure drop across each device containing an empty capsule was measured as a function of flow rate between 30 and 120 L/min (LPM). The present disclosure teaches that the high resistance DPI device is selected for evaluation and use. A peak inspiratory flow rate of 80 LPM, 60 LPM, 40 LPM, or 30 LPM corresponds to a pressure drop of 5.4 kPa, 4 kPa , 1.4 kPa, or 0.89 kPa, respectively, for the high resistance DPI.

Other dry powder inhalers are also available to the public and known to those skilled in the art, which may be used for the powder compositions of the present disclosure. A number of DPIs are available, such as, the inhalers disclosed is U.S. Pat. Nos. 4,995,385 and 4,069,819.

The AIR^{®} inhaler (Alkermes) includes a small, breath-activated system that delivers porous powder from a capsule. The porous particles have an aerodynamic diameter of 1-5 µm. *See* International Patent Application Publication Nos. WO 99/66903 and WO 00/10541, the disclosure of each of which is incorporated herein by reference in their entireties.

Aerolizer^{™} (Novartis) is a single dose dry powder inhaler. In this device, dry powder medicament is stored in a capsule and released by piercing the capsule wall with TEFLON-coated steel pins. *See* U.S. Patent Nos. 6,488,027 and 3,991,761, the disclosure of each of which is incorporated herein by reference in their entireties.

Bang Olufsen provides a breath actuated inhaler using blister strips with up to sixty doses. The dose is made available only during the inhalation by a novel trigger mechanism. The device is equipped with a dose counter and can be disposed of after all doses have been used. *See* EP 1522325, the disclosure of which is incorporated herein by reference in its entirety.

Clickhaler^{®} (Innovata PLC) is a large reservoir breath-activated multidose device. S*ee* U.S. Pat. 5,437,270, the disclosure of which is incorporated herein by reference in its entirety.

DirectHaler^{™} (Direct-Haler A/S) is a single dose, pre-metered, pre-filled, disposable DPI device made from polypropylene. S*ee* U.S. Patent No. 5,797,392, the disclosure of which is incorporated herein by reference in its entirety.

Diskus^{™} (GlaxoSmithKline) is a disposable small DPI device that holds up to 60 doses contained in double foil blister strips to provide moisture protection. *See* GB2242134, the disclosure of which is incorporated herein by reference in its entirety.

Eclipse^{™} (Aventis) is a breath actuated re-usable capsule device capable of delivering up to 20 mg of a powder composition. The powder is sucked from the capsule into a vortex chamber where a rotating ball assists in powder disaggregation as a subject (e.g., patient) inhales. S*ee* U.S. Pat. 6,230,707 and WO 9503846, the disclosure of each of which is incorporated herein by reference in their entireties.

Flexhaler^{®} is a plastic breath-activated dry powder inhaler and is amenable for use with the powder compositions provided herein.

FlowCaps^{®} (Hovione) is a capsule-based, re-fillable, re-usable passive dry-powder inhaler that holds up to 14 capsules. The inhaler itself is moisture-proof. S*ee* U.S. Pat. 5,673,686, the disclosure of which is incorporated herein by reference in its entirety.

Gyrohaler^{®} (Vectura) is a passive disposable DPI containing a strip of blisters. *See* GB2407042, the disclosure of which is incorporated herein by reference in its entirety.

The HandiHaler^{®} (Boehringer Ingelheim GmbH) is a single dose DPI device. It can deliver up to 30 mg of a powder composition in capsules. *See* International Patent Application Publication No. WO 04/024156, the disclosure of which is incorporated herein by reference in its entirety.

MicroDose DPI (Microdose Technologies) is a small electronic DPI device. It uses piezoelectric vibrator (ultrasonic frequencies) to deaggragate the drug powder in an aluminum blister (single or multiple dose). *See* U.S. Patent No. 6,026,809, the disclosure of which is incorporated herein by reference in its entirety.

Nektar Dry Powder Inhaler^{®} (Nektar) is a palm-sized and easy-to-use device. It provides convenient dosing from standard capsules and flow-rate-independent lung deposition.

Nektar Pulmonary Inhaler^{®} (Nektar) efficiently removes powders from the packaging, breaks up the particles and creates an aerosol cloud suitable for deep lung delivery. It enables the aerosolized particles to be transported from the device to the deep lung during a subject's (e.g., patient's) breath, reducing losses in the throat and upper airways. Compressed gas is used to aerosolize the powder. *See* AU4090599 and U.S. Patent No. 5,740,794, the disclosure of each of which is incorporated herein by reference in their entireties.

NEXT DPI^{™} is a device featuring multidose capabilities, moisture protection, and dose counting. The device can be used regardless of orientation (upside down) and doses only when proper aspiratory flow is reached. *See* EP 1196146, U.S. Patent No. 6,528,096, WO0178693, and WO0053158, the disclosure of each of which is incorporated herein by reference in their entireties.

Neohaler^{®} is a capsule-based plastic breath-activated dry powder inhaler.

Oriel^{™} DPI is an active DPI that utilizes a piezoelectric membrane and nonlinear vibrations to aerosolize powder formulations. *See* International Patent Application Publication No. WO 01/68169, the disclosure of which is incorporated herein by reference in its entirety.

Pressair^{™} is a plastic breath-activated dry powder inhaler.

Pulvinal^{®} inhaler (Chiesi) is a breath-actuated multi-dose (100 doses) dry powder inhaler. The dry powder is stored in a reservoir which is transparent and clearly marked to indicate when the 100th dose has been delivered. *See* U.S. Patent No. 5,351,683, the disclosure of which is incorporated herein by reference in its entirety.

The Rotohaler^{®} (GlaxoSmithKline) is a single use device that utilizes capsules. *See* U.S. Patent Nos. 5,673,686 and 5,881,721, the disclosure of each of which is incorporated herein by reference in their entireties.

Rexam DPI (Rexam Pharma) is a single dose, reusable device designed for use with capsules. *See* U.S. Patent No. 5,651,359 and EP 0707862, the disclosure of each of which is incorporated herein by reference in their entireties.

S2 (Innovata PLC) is a re-useable or disposable single-dose DPI for the delivery of a powder composition in high concentrations. Its dispersion mechanism requires minimal effort to achieve excellent drug delivery to the subject's (e.g., patient's) lungs. S2 is easy to use and has a passive engine so no battery or power source is required. *See* AU3320101, the disclosure of which is incorporated herein by reference in its entirety.

SkyeHaler^{®} DPI (SkyePharma) is a multidose device containing up to 300 individual doses in a single-use, or replaceable cartridge. The device is powered by breath and requires no coordination between breathing and actuation. *See* U.S. Patent No. 6,182,655 and WO97/20589, the disclosure of each of which is incorporated herein by reference in their entireties.

Taifun^{®} DPI (LAB International) is a multiple-dose (up to 200) DPI device. It is breath actuated and flow rate independent. The device includes a unique moisture-balancing drug reservoir coupled with a volumetric dose metering system for consistent dosing. *See* U.S. Patent No. 6,132,394, the disclosure of which is incorporated herein by reference in its entirety.

The TurboHaler^{®} (AstraZeneca) is described in U.S. Patent No. 5,983,893, the disclosure of which is incorporated herein by reference in its entirety. This DPI device is an inspiratory flow-driven, multi-dose dry-powder inhaler with a multi-dose reservoir that provides up to 200 doses of a powder composition and a dose range from a few micrograms to 0.5 mg.

The Twisthaler^{®} (Schering-Plough) is a multiple dose device with a dose counting feature and is capable of 14-200 actuations. A powder composition is packaged in a cartridge that contains a desiccant. *See* U.S. Patent No. 5,829,434, the disclosure of which is incorporated herein by reference in its entirety.

Ultrahaler^{®} (Aventis) combines accurate dose metering and good dispersion. It is an easy-to-use, discrete, pocket-sized device with a numerical dose counter, dose taken indicator and a lock-out mechanism. The device is capable of delivering up to 20 mg of a powder composition. Ultrahaler^{®} is described in U.S. Patent No. 5,678,538 and WO2004026380, the disclosure of each of which is incorporated herein by reference in their entireties.

Xcelovair^{™} (Meridica/Pfizer) holds 60 pre-metered, hermetically sealed doses in the range of 5-20 mg. The device provides moisture protection under accelerated conditions of 40°C/75% RH. The dispersion system maximizes the fine particle fraction, delivering up to 50% fine particle mass.

The respirable dry particles and dry powders can be administered to the respiratory tract of a subject in need thereof using any suitable method, such as instillation techniques, and/or an inhalation device, such as a dry powder inhaler (DPI). Generally, inhalation devices (e.g., DPIs) are able to deliver a maximum amount of dry powder or dry particles in a single inhalation, which is related to the capacity of the blisters, capsules with respective volumetric capacities or other means that contain the dry particles or dry powders within the inhaler. Accordingly, delivery of a desired dose or effective amount may require at least one inhalation. Each dose that is administered to a subject in need thereof contains an effective amount of dry particles or dry powder. Each dose of dry particles or dry powder can be administered in a single inhalation or 2, 3, 4, 5 or 6 inhalations. The dry particles and dry powders are administered in a single, breath-activated step using a breath-activated DPI. When this type of device is used, the energy of the subject's inhalation both disperses the dry particles and draws them into the respiratory tract.

The time between multiple inhalations may be about 15 seconds, about 30 seconds, about 45 seconds, about 1 minute, about 2 minutes, about 3 minutes, about 5 minutes, about 10 minutes, about 15 minutes, or about 30 minutes. The time is between about 15 seconds and 5 minutes, between about 30 seconds and 4 minutes, or between about 15 seconds and 4 minutes. The time between multiple inhalations may be up to about 15 seconds, up to about 30 seconds, up to about 45 seconds, up to about 1 minute, up to about 2 minutes, up to about 3 minutes, up to about 4 minutes, up to about 5 minutes, up to about between about 15 seconds and 5 minutes, up to about between about 30 seconds and 4 minutes, or up to about between about 15 seconds and 4 minutes.

In another aspect, a system is provided comprising (i) one of the powder compositions described herein and (ii) a dry powder inhaler (DPI) for administration of the powder composition. The DPI includes (a) a reservoir comprising the powder composition disclosed herein, and (b) a means for introducing the powder composition into the subject's lungs via inhalation. The reservoir in one embodiment, comprises the powder composition of the present disclosure in a capsule or in a blister pack. The material for the shell of a capsule can be gelatin, cellulose derivatives, starch, starch derivatives, chitosan, or synthetic plastics. The DPI may be a single dose or a multidose inhaler. In addition, the DPI may be pre-metered or device-metered. In one embodiment, the dry powder inhaler is a single dose dry powder inhaler.

The system, in one embodiment, is used for treating pulmonary hypertension (e.g., group 1 or group 3 PH), portopulmonary hypertension, or pulmonary fibrosis as described in further detail below. The system includes the powder composition disclosed herein, i.e., a powder composition comprising a compound of Formula (I) or (II), a stereoisomer thereof, or a pharmaceutically acceptable salt thereof, and a DPI. In one embodiment, the powder composition comprises a compound of Formula (I) or (II), or a pharmaceutically acceptable salt thereof. In another embodiment, the powder composition comprises a compound of Formula (I) or (II). The dry powder inhaler may be one described above, may be a single dose or a multidose inhaler, and/or may be pre-metered or device-metered. In one embodiment, the dry powder inhaler is a single dose dry powder inhaler.

The present disclosure provides a system for treating pulmonary hypertension, comprising (i) a powder composition and (ii) a dry powder inhaler, the powder comprising: (a) from about 0.5 wt% to about 5 wt% of a compound of Formula (I), or stereoisomer or a pharmaceutically acceptable salt thereof, wherein R1 is tetradecyl, pentadecyl, hexadecyl, heptadecyl, or octadecyl, (b) from about 10 wt% to about 61wt% leucine, and (c) trehalose, mannitol or a combination thereof, wherein the sum of (a), (b), and (c) is 100%. In embodiments, the dry powder inhaler, which is a ultra-high, high, medium, or low resistance dry powder inhaler, provides an aerosol comprising fine particle fraction (FPF) greater than or equal to about 68%, as measured using a Next Generation Impactor (NGI), with a inspiratory flow rate of 30-60 liter per minute (L/min), inspiratory volume of 1-4 L, and inhalation acceleration rate of 0.5-20 L/sec². In some embodiments, the dry powder inhaler is a high resistance dry powder inhaler. In further embodiments, the high resistance dry powder inhaler is one of RS01 Models manufactured by Plastiape.

In some embodiments, the dry powder inhaler (DPI) is a capsule-based DPI, and the composition is present in a single DPI capsule. In further embodiments, the composition is split between two DPI capsules.

### Methods of Treatment

The term "treating" includes: (1) preventing or delaying the appearance of clinical symptoms of the state, disorder or condition developing in the patient that may be afflicted with or predisposed to the state, disorder or condition but does not yet experience or display clinical or subclinical symptoms of the state, disorder or condition; (2) inhibiting the state, disorder or condition (e.g., arresting, reducing or delaying the development of the disease, or a relapse thereof in case of maintenance treatment, of at least one clinical or subclinical symptom thereof); and/or (3) relieving the condition (e.g., causing regression of the state, disorder or condition or at least one of its clinical or subclinical symptoms). In one embodiment, "treating" refers to inhibiting the state, disorder or condition (e.g., arresting, reducing or delaying the development of the disease, or a relapse thereof in case of maintenance treatment, of at least one clinical or subclinical symptom thereof). In another embodiment, "treating" refers to relieving the condition (for example, by causing regression of the state, disorder or condition or at least one of its clinical or subclinical symptoms). The benefit to a patient to be treated is either statistically significant as compared to the state or condition of the same patient before the treatment, or as compared to the state or condition of an untreated control patient, or the benefit is at least perceptible to the patient or to the physician.

"Effective amount" means an amount of a powder composition of the present disclosure that is sufficient to result in the desired therapeutic response. The "effective amount" is the amount of the compound of Formula (I) or (II), a stereoisomer thereof, or a pharmaceutically acceptable salt thereof that is administered in a single dosing session.

In one aspect of the disclosure, a method for treating pulmonary hypertension (PH) in a patient in need thereof is provided. The method includes administering an effective amount of one of the powder compositions disclosed herein to the lungs of the patient via a dry powder inhaler (DPI), once daily during an administration period. The powder composition comprises a compound of Formula (I) or (II), a stereoisomer thereof, or a pharmaceutically acceptable salt thereof. The administering comprises (i) aerosolizing the powder composition via a DPI to provide an aerosolized powder composition, and (ii) administering the aerosolized powder composition to the lungs of the patient via inhalation by the DPI.

In some embodiments, the effective amount of one of the powder compositions disclosed herein comprises about 80 µg, about 160 µg, about 240 µg, about 320 µg, about 400 µg, about 480 µg, about 560 µg, about 640 µg, about 720 µg, about 800 µg, about 880 µg, about 960 µg, about 1040 µg, about 1120 µg, about 1200 µg, or about 1280 µg of a compound of Formula (I) or (II), a stereoisomer thereof, or a pharmaceutically acceptable salt thereof, and may also be referred to as the amount administered once daily. The World Health Organization (WHO) has classified PH into five groups. Group 1 PH includes pulmonary arterial hypertension (PAH), idiopathic pulmonary arterial hypertension (IPAH), familial pulmonary arterial hypertension (FPAH), and pulmonary arterial hypertension associated with other diseases (APAH). For example, pulmonary arterial hypertension associated with collagen vascular disease (e.g., scleroderma), congenital shunts between the systemic and pulmonary circulation, portal hypertension and/or HIV infection are included in group 1 PH. Group 2 PH includes pulmonary hypertension associated with left heart disease, e.g., atrial or ventricular disease, or valvular disease (e.g., mitral stenosis). WHO group 3 pulmonary hypertension is characterized as pulmonary hypertension associated with lung diseases, e.g., chronic obstructive pulmonary disease (COPD), interstitial lung disease (ILD), and/or hypoxemia. Group 4 pulmonary hypertension is pulmonary hypertension due to chronic thrombotic and/or embolic disease. Group 4 PH is also referred to as chronic thromboembolic pulmonary hypertension. Group 4 PH patients experience blocked or narrowed blood vessels due to blood clots. Group 5 PH is the "miscellaneous" category, and includes PH caused by blood disorders (e.g., polycythemia vera, essential thrombocythemia), systemic disorders (e.g., sarcoidosis, vasculitis) and/or metabolic disorders (e.g., thyroid disease, glycogen storage disease).

The methods provided herein can be used to treat group 1, group 2, group 3, group 4 or group 5 PH patients, as characterized by the WHO.

In one embodiment of the methods, the pulmonary hypertension treated is chronic thromboembolic pulmonary hypertension.

In one preferred embodiment, the pulmonary hypertension is group 1 PH, as characterized by the WHO. In a further embodiment, the method provided herein is a method for treating treated is pulmonary arterial hypertension (PAH). In a further embodiment, the PAH is class I PAH, class II PAH, class III PAH, or class IV PAH, as characterized by the New York Heart Association (NYHA).

In one embodiment, the PAH is class I PAH, as characterized by the NYHA.

In another embodiment, the PAH is class II PAH, as characterized by the NYHA.

In yet another embodiment, the PAH is class III PAH, as characterized by the NYHA.

In still another embodiment, the PAH is class IV PAH, as characterized by the NYHA.

In one embodiment, the pulmonary hypertension (PH) is portopulmonary hypertension (PPH). PPH is defined by the coexistence of portal and pulmonary hypertension. The diagnosis of portopulmonary hypertension is based on hemodynamic criteria: (1) portal hypertension and/or liver disease (clinical diagnosis-ascites/varices/splenomegaly), (2) mean pulmonary artery pressure > 25 mmHg at rest, (3) pulmonary vascular resistance > 240 dynes s/cm⁵, (4) pulmonary artery occlusion pressure < 15mmHg or transpulmonary gradient > 12 mmHg. PPH is a serious complication of liver disease, and is present in 0.25 to 4% of patients suffering from cirrhosis. PPH is comorbid in an estimated 4-6% of those referred for a liver transplant.

In one preferred embodiment, the pulmonary hypertension is group 3 PH, as characterized by the WHO. In a further embodiment, the method provided herein is a method for treating PH associated with interstitial lung disease (PH-ILD).

In the methods for treating PH-ILD provided herein, the ILD may include one or more lung conditions. The one or more lung conditions comprise, in one embodiment, idiopathic pulmonary fibrosis (IPF), cryptogenic organizing pneumonia (COP), desquamative interstitial pneumonitis, nonspecific interstitial pneumonitis, hypersensitivity pneumonitis, acute interstitial pneumonitis, interstitial pneumonia (e.g., idiopathic interstitial pneumonia), connective tissue disease, sarcoidosis or asbestosis. In one embodiment, the ILD is connective tissue disease-associated interstitial lung disease (CTD-ILD). In another embodiment, the ILD is sarcoidosis. In yet another embodiment, the ILD is IPF. In even another embodiment, the ILD is an idiopathic interstitial peneumonia (IIP).

In one embodiment for treating PH-ILD provided herein, the ILD includes pulmonary fibrosis, e.g., idiopathic pulmonary fibrosis (IPF). Pulmonary fibrosis is a respiratory disease in which scars are formed in the lung tissues, leading to serious breathing problems. Scar formation, i.e., the accumulation of excess fibrous connective tissue, leads to thickening of the walls, and causes reduced oxygen supply in the blood. As a result, pulmonary fibrosis patients suffer from perpetual shortness of breath. In some patients the specific cause of the disease can be diagnosed, but in others the probable cause cannot be determined, a condition called IPF.

The length of the administration period in any given case may depend on the nature and severity of the PH being treated and how well a patient tolerates and responds to the therapy. The treatment methods provided herein are provided as a chronic therapy, and as such, a patient is on-therapy as long as the therapy is safe and effective. Accordingly, the administration period in one embodiment, continues until a patient dies. In another embodiment, the administration period is the length of time the treatment is effective.

In one embodiment, if a patient experiences an adverse reaction to the therapy, they are provided a decreased dose during the administration period. Similarly, a patient may be titrated to a higher dose should they show a lower dose be shown to be well tolerated. In one embodiment, the uptitration takes place only after the patient has shown to tolerate a lower dose for two or more days, e.g., two days, three days, four days, five days, six days or seven days.

In some embodiments, the administration period is about 6 months, about 7 months, about 8 months, about 9 months, about 10 months, about 11 months, about 1 year, about 2 years, about 3 years, about 4 years, about 5 years, about 6 years, about 7 years, about 8 years, about 9 years, about 10 years, about 15 years, about 20 years or about 30 years.

In another embodiment, the administration period for the methods provided herein is at least about 6 months, at least about 7 months, at least about 8 months, at least about 9 months, at least about 10 months, at least about 11 months, at least about 1 year, at least about 2 years, at least about 3 years, at least about 4 years, at least about 5 years, at least about 6 years, at least about 7 years, at least about 8 years, at least about 9 years or at least about 10 years or at least about 20 years. The administration period, in another embodiment, is from about 30 days to about 2 years. In another embodiment, the administration period is from about 6 months to about 3 years, or from 6 months to about 4 years, or from about 6 months to about 5 years, or from about 6 months to about 6 years, or from about 6 months to about 7 years, or from about 6 months to about 8 years, or from about 1 year to about 10 years, or from about 2 years to about 10 years, or from about 6 months to about 20 years, or from about 5 years to about 20 years, or from about 10 years to about 30 years.

In one embodiment, the administration period is at least about 1 year.

In one embodiment, the administration period is at least about 5 years.

In even another embodiment, the administration period is from about 1 year to about 30 years. In one embodiment, the administration period is from about 1 year to about 25 years. In another embodiment, the administration period is from about 5 years to about 30 years. In yet another embodiment, the administration period is from about 1 years to about 20 years. In even another embodiment, the administration period is from about 1 year to about 15 years. In one embodiment, the administration period is from about 1 year to about 10 years. In one embodiment, the administration period is from about 1 year to about 5 years. In another embodiment, the administration period is from about 5 years to about 15 years. In yet another embodiment, the administration period is from about 10 years to about 20 years.

In one embodiment of the disclosed methods, a patient is administered the powder composition once daily in a single dosing session during an administration period. The administration period, in one embodiment, comprises a titration period. In embodiments, during the titration period, the patient is administered a starting dose of a compound of Formula (I), a stereoisomer thereof, or a pharmaceutically acceptable salt thereof, and the starting dose is titrated (e.g., incrementally increased) to an effective amount, e.g., a highest tolerated dose. In one embodiment, the administration period comprises a maintenance period. In embodiments, during the maintenance period, the patient is administered the highest tolerated dose once daily during a single dosing session. In another embodiment, the patient is administered the powder composition twice daily, i.e., in two separate dosing sessions. In one embodiment, the administration is with food. In one embodiment, each dosing session comprises 1 to 5 inhalations (puffs) from a DPI, for example 1 inhalation (1 puff), 2 inhalations (2 puffs), 3 inhalations (3 puffs), 4 inhalations (4 puffs) or 5 inhalations (5 puffs). As used herein, a "dosing session" refers to 1 to 5 inhalations (puffs) from a DPI as required to administer from about 80 µg to about 700 µg of the compound of Formula (I) or (II), a stereoisomer thereof, or a pharmaceutically acceptable salt thereof. The DPI, in one embodiment, is small and transportable by the patient. In one embodiment, the DPI is a single dose DPI.

In order to achieve a particular dose, in one embodiment, more than one DPI capsule comprising the composition can be employed. For example, in the case of a 1280 µg dose, four 320 µg DPI and in the case of a 640 µg dose, two 320 µg DPI capsules can be used. Each capsule can be administered via 1 or 2 inhalations, for example.

The effective amount of the compound of Formula (I) or (II), a stereoisomer thereof, or a pharmaceutically acceptable salt thereof, may include a fixed dose of a compound of Formula (I) or (II), a stereoisomer thereof, or a pharmaceutically acceptable salt thereof. The fixed dose, in one embodiment, is present in one or multiple DPI capsules. The fixed dose, in one embodiment, is a dose that is titrated (either up or down) from a prior dose. In another embodiment, the fixed dose is the same dose or substantially the same dose as a prior dose. The effective amount, in one embodiment, is the amount of the compound of Formula (I) or (II), a stereoisomer thereof, or a pharmaceutically acceptable salt thereof, administered during each dosing session. In some embodiments, the amount "administered" refers to the amount of the compound of Formula (I) or (II), a stereoisomer thereof, or a pharmaceutically acceptable salt thereof, in the capsule, or multiple capsules in the DPI, administered in a single dosing session. In some embodiments, the fixed dose ranges from about 80 µg to about 1280 µg of a compound of Formula (I) or (II), a stereoisomer thereof, or a pharmaceutically acceptable salt thereof, e.g., about 80 µg, about 112.5 µg, about 160 µg, about 225 µg, about 240 µg, about 320 µg, about 400 µg, about 450 µg, about 480 µg, about 640 µg, about 675 µg, about 720 µg, about 800 µg, about 880 µg, about 960 µg, about 1040 µg , about 1120 µg, about 1200 µg, or about 1280 µg of the compound of Formula (I) or Formula (II), a stereoisomer thereof, or a pharmaceutically acceptable salt thereof. In some embodiments, the fixed dose ranges from about 80 µg to about 700 µg of a compound of Formula (I) or (II), a stereoisomer thereof, or a pharmaceutically acceptable salt thereof, e.g., about 80 µg, about 112.5 µg, about 160 µg, about 225 µg, about 240 µg, about 320 µg, about 400 µg, about 450 µg, about 480 µg, about 640 µg, or 675 µg of the compound of Formula (I) or Formula (II), a stereoisomer thereof, or a pharmaceutically acceptable salt thereof. For example, if the powder composition is administered once daily in a single dosing session, the effective amount can be considered to be the amount of the compound of Formula (I) or (II), a stereoisomer thereof, or a pharmaceutically acceptable salt thereof, in the capsule or multiple capsules that is administered during the single dosing session. For example, in one embodiment, one or more capsules may be formulated with the powder composition wherein the one or more capsules have a total dose of about 80 µg, about 112.5 µg, about 160 µg, about 225 µg, about 240 µg, about 320 µg, about 400 µg, about 450 µg, about 480 µg, about 640 µg, 675 µg, about 720 µg, about 800 µg, about 880 µg, about 960 µg, about 1040 µg , about 1120 µg, about 1200 µg, or about 1280 µg of a compound of Formula (I) or (II), a stereoisomer thereof, or a pharmaceutically acceptable salt thereof, and each of the aforementioned dosages may be an effective amount, and may also be referred to as the amount administered once daily in a single dosing session, during the administration period. In another exemplary embodiment, one or more capsules may be formulated with the powder composition wherein the one or more capsules have a total dose of about 80 µg, about 112.5 µg, about 160 µg, about 225 µg, about 240 µg, about 320 µg, about 400 µg, about 450 µg, about 480 µg, about 640 µg, or 675 µg of a compound of Formula (I) or (II), a stereoisomer thereof, or a pharmaceutically acceptable salt thereof, and each of the aforementioned dosages may be an effective amount, and may also be referred to as the amount administered once daily in a single dosing session, during the administration period. As a further example, in one embodiment, the capsule comprises a powder composition comprising about 320 µg of a compound of Formula (I) or (II), a stereoisomer thereof, or a pharmaceutically acceptable salt thereof, and, for purposes of this disclosure, the amount administered is 640 µg, even if it takes 2 or more puffs from two capsules to administer the 640 µg. Similarly, in this example, the amount administered is 640 µg even if a residual amount of compound of Formula (I) or (II), a stereoisomer thereof, or a pharmaceutically acceptable salt thereof remains in the DPI (e.g., if about 5%, 10%, 20%, 30%, 40%, or 50% remains in the DPI.)

The dose "administered" in a single dosing session also encompasses situations where the DPI is refiled or reloaded 1 or more times (e.g., by changing the capsules) in order to achieve the desired effective amount. In such situations, "administration" refers to the total dosage in the capsules which are administered in the dosing session. For example, to administer a dosage of 240 µg of a compound of Formula (I) or (II), a stereoisomer thereof, or a pharmaceutically acceptable salt thereof, one 80 µg capsule and one 160 µg capsule may be used. The DPI may be filed with a first 80 µg capsule, and after emptying the cartridge in 1 or more puffs, a 160 µg capsule may be loaded in the DPI and emptied in 1 or more puffs. Both capsules are used in the same dosing session, and therefore the dose administered is 240 µg.

In another embodiment, the effective amount comprises an escalating dose during the administration period. In a further embodiment, the effective amount is based upon an upwards titration, based on the highest tolerated dose for the patient. In some embodiments, during the administration period, the patient is initially administered 80 µg or 160 µg of the compound of Formula (I), Formula (II), a stereoisomer thereof, or a pharmaceutically acceptable salt thereof. In one embodiment, during the administration period, the patient is initially administered 80 µg of the compound of Formula (I), Formula (II), a stereoisomer thereof, or a pharmaceutically acceptable salt thereof. In another embodiment, during the administration period, the patient is initially administered 160 µg of the compound of Formula (I), Formula (II), a stereoisomer thereof, or a pharmaceutically acceptable salt thereof. If this dose is well tolerated, the dose is uptitrated until reaching the patient's highest tolerable dose. In embodiments, the maximum dose administered to patients according to the methods of the disclosure is 1280 µg. Therefore, if a patient tolerates a dose of 1280 µg, then 1280 µg is considered the highest tolerated dose. However, an individual's highest tolerated dose may be less than 1280 µg. Accordingly, in embodiments, a patient's highest tolerated dose is selected from the group consisting of 160 µg, 240 µg, 320 µg, 400 µg, 480 µg, 560 µg, 640 µg, 675 µg, 720 µg, 800 µg, 880 µg, 960 µg, 1040 µg, 1120 µg, 1200 µg and 1280 µg. In some embodiments, the highest tolerated dose is 160 µg. In some embodiments, the highest tolerated dose is 240 µg. In some embodiments, the highest tolerated dose is 320 µg. In some embodiments, the highest tolerated dose is 400 µg. In some embodiments, the highest tolerated dose is 480 µg. In some embodiments, the highest tolerated dose is 640 µg. In some embodiments, the highest tolerated dose is 675 µg. In some embodiments, the highest tolerated dose is 720 µg. In some embodiments, the highest tolerated dose is 800 µg. In some embodiments, the highest tolerated dose is 880 µg. In some embodiments, the highest tolerated dose is 960 µg. In some embodiments, the highest tolerated dose is 1040 µg. In some embodiments, the highest tolerated dose is 1120 µg. In some embodiments, the highest tolerated dose is 1200 µg. In some embodiments, the highest tolerated dose is 1280 µg. During the titration period, the patient stays on the same dose for a minimum number of cumulative days, e.g., 2 days, 3 days or 4 days, 5 days, 6 days, or 7 days, prior to titrating to the next higher dose. If a dose is not tolerated, the dose may be decreased to the previous dose level.

During a titration period, each patient's dose can be uptitrated to the highest tolerated dose for that patient. As an example, a patient, in one embodiment, starts the method of the disclosure with a single 80 µg DPI capsule, once-daily. If this dose is well tolerated, the dose is uptitrated until reaching the patient's highest tolerable dose. In some embodiments, the dose is titrated to a higher dose in increments of 80 until the patient's highest tolerated dose is achieved. During the Titration Period, patients stay on study drug for the minimum number of cumulative days (e.g., 2 days at 80 µg, 160 µg, or 240 µg, 3 days at 320 µg, or 4 days at 400 µg or 480 µg) prior to starting the next higher dose. Study drug titration may occur slower than the above example, but not faster. If a dose is not tolerated, the dose may be decreased to the previous dose level.

In embodiments of the disclosed methods, during the titration period, a dose X of the compound of Formula (I) or (II), a stereoisomer thereof, a pharmaceutically acceptable salt thereof is increased by about 80 µg to about 200 µg to a dose Y (e.g., by about 80 µg to about 160 µg or by about 80 µg), if the patient is shown to tolerate dose X. In this example, dose X may be the starting dose or any dose between the starting dose and the highest tolerated dose, and dose Y may be the next sequential dose (e.g., a dose that is 80-160 µg higher than dose X), or dose Y may be the patient's highest tolerated dose. In a further embodiment, the patient is administered the dose X for about one to about seven days prior to increasing the dose to dose Y. In a further embodiment, the patient is administered a dose X for about one to about five days prior to increasing the dose to dose Y. In even a further embodiment, the patient is administered a dose X for about one to about three days prior to increasing the dose to dose Y. In embodiments when dose Y is less than the highest tolerated dose, the highest tolerated dose may be achieved by increasing dose X by 80-160 µg to achieve dose Y and administering dose Y for a period of time (e.g., 2-7 days), then increasing dose Y by 80-160 µg, and repeating until reaching the highest tolerated dose.

The upwards titration, in one embodiment, comprises increasing the dose of the compound of Formula (I) or (II), a stereoisomer thereof, or a pharmaceutically acceptable salt thereof by increments of about 80-160 µg. In a further embodiment, the upwards dose titration, comprises increasing the dose of the compound of Formula (I) or (II), a stereoisomer thereof, or a pharmaceutically acceptable salt thereof, by about 80 µg. In another embodiment, the upwards dose titration, comprises increasing the dose of the compound of Formula (I) or (II), a stereoisomer thereof, or a pharmaceutically acceptable salt thereof, by about 160 µg. During an upwards dose titration, in one embodiment, a patient remains on a dose of the compound of Formula (I) or (II), a stereoisomer thereof, or a pharmaceutically acceptable salt thereof, for about one to seven days, for example, one to six days prior to further incrementally increasing the dose. Accordingly, in one embodiment uptitration comprises incrementally increasing the patient's dose of the compound of Formula (I) or (II) by about 80 or 160 µg after a patient is shown to tolerate a prior dose for about 2-7 days. If a patient experiences an adverse event, then the patient is downtitrated to the prior dose, which can then be considered the maximally tolerated dose. In a preferred embodiment, the compound of Formula (I) is treprostinil palmitil.

In some embodiments, the patient treated by the disclosed methods manifests one or more of the following therapeutic responses during the administration period as compared to prior to the administration period: (1) a reduction in the pulmonary vascular resistance index (PVRI), (2) a reduction in mean pulmonary artery pressure, (3) an increase in the hypoxemia score, (4) a decrease in the oxygenation index, (5) improved right heart function, and (6) improved exercise capacity (e.g., as measured by the six-minute walk test).

PVRI relates the absolute value of PVR to the patient's body surface area to account for effect of body size on blood flow. In some embodiments, the PVRI is reduced by about 5%, by about 6%, by about 7%, by about 8%, by about 9%, by about 10%, by about 11%, by about 12%, by about 13%, by about 14%, by about 15%, by about 16%, by about 17%, by about 18%, by about 19%, by about 20%, by about 25%, by about 30%, by about 35%, by about 40%, by about 45%, by about 50%, by about 55%, by about 60%, by about 65%, by about 70%, by about 75%, by about 80%, by about 85%, or by about 90% compared to prior to the administration period. In some embodiments, the PVRI is reduced at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, or at least about 75% compared to prior to the administration period. In some embodiments, PVRI is reduced after treating for at least about 4 weeks, at least about 6 weeks, at least about 8 weeks, at least about 12 weeks, at least about 16 weeks, at least about 20 weeks, or at least about 24 weeks.

In some embodiments, right heart function is improved by about 1%, by about 2%, by about 3%, by about 4%, by about 5%, by about 6%, by about 7%, by about 8%, by about 9%, by about 10%, by about 11%, by about 12%, by about 13%, by about 14%, by about 15%, by about 16%, by about 17%, by about 18%, by about 19%, by about 20%, by about 25%, by about 30%, by about 35%, by about 40%, by about 45%, by about 50%, by about 55%, by about 60%, by about 65%, by about 70%, by about 75%, by about 80%, by about 85%, or by about 90% compared to prior to the administration period. In some embodiments, right heart function is improved at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, or at least about 75% compared to prior to the administration period.

In some embodiments, the patient treated by the disclosed methods exhibits an improvement in exercise capacity compared to prior to the administration period, e.g., as measure by changes in the 6-minute walk test (6MWT).

6MWT is a validated method for measuring exercise capacity and assessment of pulmonary function, and performed according to the American Thoracic Society (ATS) guidelines. See American Thoracic Society. ATS Statement: Guidelines for the six minute walk test. Am J Respir Crit Care Med. 2002;166(1):111-17, incorporated herein by reference in its entirety for all purposes. In one embodiment, the 6MWT is performed at approximately the same time on a day during the administration period as on a day prior to the administration period. In a further embodiment, the same equipment is used to perform the 6MWT. In still a further embodiment, the same person administers the 6MWT.

In one embodiment, the patient's distance walked in the 6MWT is increased during the administration period, as compared to prior to the administration period, by at least about 5 meters, at least about 10 meters, at least about 20 meters, at least about 30 meters, at least about 40 meters, at least about 50 meters, at least about 60 meters or at least about 70 meters. In another embodiment, the patient's distance walked in the 6MWT is increased during the administration period, as compared to prior to the administration period, by from about 5 meters to about 75 meters, by from about 5 meters to about 60 meters, by from about 5 meters to about 50 meters, by from about 10 meters to about 50 meters, by from about 15 meters to about 50 meters, by from about 25 meters to about 75 meters, by from about 20 meters to about 50 meters, or by from about 20 meters to about 40 meters. In yet another embodiment, the patient's distance walked in the 6MWT is increased by at least about 30 meters, during the administration period, compared to prior to the administration period.

In one embodiment, the patient's distance walked in the 6MWT is increased during the administration period, as compared to prior to the administration period, by about 1%, by about 2%, by about 3%, by about 4%, by about 5%, by about 6%, by about 7%, by about 8%, by about 9%, by about 10%, by about 11%, by about 12%, by about 13%, by about 14%, by about 15%, by about 16%, by about 17%, by about 18%, by about 19%, by about 20%, by about 25%, by about 30%, by about 35%, by about 40%, by about 45%, by about 50%, by about 55%, by about 60%, by about 65%, by about 70%, by about 75%, by about 80%, by about 85%, or by about 90%. In another embodiment, the patient's distance walked in the 6MWT is increased during the administration period, as compared to prior to the administration period, by at least about 5%, by at least about 10%, by at least about 15%, by at least about 20%, by at least about 25%, by at least about 30%, by at least about 35%, by at least about 40%, by at least about 45%, or by at least about 50%. In another embodiment, the patient's distance walked in the 6MWT is increased during the administration period, as compared to prior to the administration period, by about 5% to about 50%, by about 5% to about 40%, by about 5% to about 30%, by about 5% to about 20%, by about 10% to about 50%, by about 15% to about 50%, by about 20% to about 50%, or by about 25% to about 50%.

Pressure overload of the right heart due to PH activates the natriuretic peptide system. In PAH, for example, serum NT-proBNP levels correlate with right hear dysfunction and provide prognostic information. Thus, measurement of natriuretic peptides (*e.g.*, BNP or NT-proBNP) is useful in the evaluation of patients with PH. In some embodiments, the present methods of treatment provide a decrease of N-terminal pro b-type natriuretic peptide (NT-proBNP) levels in the blood of the patient of about 5% to about 50%, e.g., about 5%, about 10%, about 15%, about 20%, about 25%, about 30%, about 35%, about 40%, about 45%, or about 50%.

In one embodiment for treating PH, treating comprises improving the quality of life of the patient during the administration period, compared to the quality of life of the patient prior to the administration period. The quality of life, in one embodiment, is measured by the Cambridge Pulmonary Hypertension Outcome Review (CAMPHOR) Questionnaire. McCabe et al. (2013). Chest. 2013;144(2):522-30, incorporated by reference herein in its entirety for all purposes. The CAMPHOR Questionnaire is a pulmonary hypertension specific measure of health-related quality of life (QOL) consisting of 3 sections that evaluate a total of 65 items (25 relating to symptoms, 15 relating to activities, and 25 relating to QOL). The CAMPHOR scoring is negatively weighted therefore, a higher score indicates worse QOL and greater functional limitation. Symptom and QOL items are both scored out of 25 and activity items have 3 possible responses (score 0-2), giving a score out of 30. Each CAMPHOR assessment takes an average of 10 minutes. In one embodiment for treating PH, treating comprises decreasing the patient's CAMPHOR Questionnaire score during the administration period, compared to the CAMPHOR Questionnaire score prior to the administration period. The decrease, in one embodiment, is by from 1 to about 10, from 1 to about 9, from 1 to 8, from 1 to 7, from 1 to 6, from 1 to 5, from 1 to 4, from 1 to 3 or from 1 to 2.

In one embodiment of a method for treating PH, the method comprises increasing the patient's saturation of peripheral capillary oxygenation (SpO₂) at rest assessed by pulse oximetry during the administration period, compared to the patient's SpO₂ at rest prior to the administration period.

Oxygen saturation is an indication of how much hemoglobin in the blood is bound to oxygen, and is typically provided as a percentage of oxyhemoglobin to the total hemoglobin. SpO₂ is an indication of oxygen saturation in the peripheral capillaries. Exemplary methods to measure SpO₂ include, but are not limited to, pulse oximetry using a pulse oximeter. In one embodiment of a method for treating PH provided herein, the method comprises increasing the patient's SpO₂ at rest during the administration period, as compared to prior to the administration period, by at least about 1%, at least about 2%, at least about 3%, at least about 4%, at least about 5%, at least about 6%, at least about 7%, at least about 8%, at least about 9%, at least about 10%, at least about 11%, at least about 12%, at least about 13%, at least about 14%, at least about 15%, at least about 16%, at least about 17%, at least about 18%, at least about 19%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, or by at least about 90%. In another embodiment, the method for treating PH comprises increasing the patient's SpO₂ at rest during the administration period, as compared to prior to the administration period, by about 5% to about 50%, by about 5% to about 40%, by about 5% to about 30%, by about 5% to about 20%, by about 10% to about 50%, by about 15% to about 50%, by about 20% to about 50%, or by about 25% to about 50%.

In one embodiment, the method for treating PH provided herein comprises improving the lung function of the patient during the administration period, as compared to the lung function of the patient prior to the administration period. The improvement in lung function in one embodiment, is measured by spirometry.

Improving the lung function of the patient, in one embodiment, comprises increasing the patient's forced vital capacity (FVC), increasing the patient's percent predicted forced vital capacity (ppFVC), increasing the patient's forced expiratory volume in 1 second (FEV₁), increasing the patient's percent predicted forced expiratory volume in one second (ppFEV₁), increasing the patient's forced expiratory flow between 25% and 75% of FVC (FEF_{(25-75%)}), increasing the patient's total lung capacity (TLC), or increasing the patient's lung diffusion capacity for carbon monoxide (DLCO), during the administration period, as compared to the respective value prior to the administration period.

The assessment of lung function, e.g., via FVC, ppFVC, FEV₁, ppFEV₁, FEF(_{25-75%)}, TLC, or DLCO measurement, in one embodiment, comprises comparing the lung function in the patient prior to the administration period, e.g., immediately prior to treatment, to a time point during the administration period the administration period, or to an average of measurements taken during the administration period.

As provided herein, in one embodiment, the method for treating PH comprises improving the lung function in the patient during the administration period, as compared to the respective value prior to the administration period, wherein the lung function is measured by spirometry. Spirometry is a physiological test that measures how an individual inhales or exhales volumes of air. The primary signal measured in spirometry may be volume or flow. For the methods described herein, pulmonary function test (PFT) by spirometry (e.g., FEV₁, FVC, FEF_{(25-75%)}, and TLC) is performed per the American Thorasic Society (ATS) / European Respiratory Society (ERS) criteria, e.g., as set forth by Miller et al. (Miller et al., "Standardization of Spirometry," Eur. Respir. J. 26:319-38 (2005), incorporated by reference herein in its entirety for all purposes). DLCO can be measured using techniques described by Modi P, Cascella M, "Diffusing Capacity Of The Lungs For Carbon Monoxide," [Updated 2021 Mar 24]. In: StatPearls [Internet]. Treasure Island (FL): StatPearls Publishing; 2021 Jan-. Available from: ncbi.nlm.nih.gov/books/NBK556149/; Graham et al., "2017 ERS/ATS standards for single-breath carbon monoxide uptake in the lung," European Respiratory Journal 49:1600016 (2017); each of which is incorporated herein by reference in its entirety for all purposes.

In one embodiment, the spirometer is capable of accumulating volume for greater than or equal to 15 seconds, e.g., ≥ 20 seconds, ≥ 25 seconds, ≥ 30 seconds, ≥ 35 seconds. The spirometer in one embodiment can measure volumes of ≥ 8 L (BTPS) with an accuracy of at least ± 3% of reading or ± 0.050 L, whichever is greater, with flows between 0 and 14 L•s⁻¹. In one embodiment, the total resistance to airflow of the spirometer at 14 L•s⁻¹ is < 1.5 cmH₂O•L⁻¹•s⁻¹ (0.15 kPa? L⁻¹•s⁻¹). In one embodiment, the total resistance of the spirometer is measured with any tubing, valves, pre-filter, etc. included that may be inserted between the patient and the spirometer. With respect to devices that exhibit changes in resistance due to water vapor condensation, in one embodiment, spirometer accuracy requirements are met under BTPS (body temperature, ambient pressure, saturated with water vapor) conditions for up to eight successive FVC maneuvers performed in a 10-min period without inspiration from the instrument.

With respect to the forced expiratory maneuvers described herein, in one embodiment, the range and accuracy recommendations as set forth in Table 6 of Miller et al., are met (Miller et al., "Standardization of Spirometry," Eur. Respir. J. 26:319-38 (2005), incorporated by reference herein in its entirety for all purposes).

In one embodiment, improving lung function comprises improving the forced vital capacity (FVC) of the patient, i.e., the maximal volume of air exhaled with maximally forced effort from a maximal inspiration, during the administration period, as compared to the FVC prior to the administration period. The FVC is expressed in liters at body temperature and ambient pressure saturated with water vapor (BTPS). In another embodiment, the improvement in lung function is an improvement in the percent predicted forced vital capacity (ppFVC).

"Forced vital capacity" (FVC) denotes the volume of gas which is exhaled during a forced expiration starting from a position of full inspiration and ending at complete expiration and is one measure of treatment efficacy. FVC may be expressed as a percentage of the predicted FVC (i.e., ppFVC) obtained from a normal population, based on the patient's age, height, gender, and sometimes weight and race. In one embodiment of a method for treating PH, improving the patient's lung function comprises increasing the patient's FVC or ppFVC during the administration period, compared to the patient's corresponding FVC or ppFVC prior to the administration period. The increase in FVC or ppFVC, in one embodiment, is an increase of at least about 5%, at least about 10%, at least about 15%, at least about 20%, at least about 25%, at least about 30%, at least about 35%, at least about 40%, at least about 45%, or at least about 50%. In another embodiment, the increase in FVC or ppFVC is an increase of from about 1% to about 20%, from about 1% to about 15%, from about 1% to about 10%, from about 1% to about 5%, from about 5% to about 50%, from about 5% to about 40%, from about 5% to about 30%, from about 5% to about 20%, from about 10% to about 50%, from about 15% to about 50%, from about 20% to about 50%, or from about 25% to about 50%. In one embodiment, increasing FVC or ppFVC is increasing pre-bronchodilator FVC or ppFVC. In another embodiment, increasing FVC or ppFVC is increasing post-bronchodilator FVC or ppFVC.

In one embodiment, the patient's ppFVC is 80% or less prior to the administration period. In a further embodiment, the patient's ppFVC is 70% or less prior to the administration period. In a further embodiment, the patient's ppFVC is 60% or less prior to the administration period. In a further embodiment, the patient's ppFVC is 50% or less prior to the administration period. In another embodiment, the patient's ppFVC is from 30% to 80%, from 40% to 70%, or from 50% to 60%, prior to the administration period.

FVC maneuvers can be performed according to the procedures known to those of ordinary skill in the art. Briefly, the three distinct phases to the FVC maneuver are (1) maximal inspiration; (2) a "blast" of exhalation and (3) continued complete exhalation to the end of test (EOT). The maneuver can be carried out via the closed circuit method or open circuit method. In either instance, the patient inhales rapidly and completely with a pause of less than 1 second at total lung capacity (TLC). The patient then exhales maximally until no more air can be expelled while maintaining an upright posture. The exhalation begins with a "blast" of air from the lungs and then is encouraged to fully exhale. Enthusiastic coaching of the patient may continue for a minimum of three maneuvers.

FEV is the volume of gas exhaled in a specified time (typically 1 second, i.e., FEV₁) from the start of the forced vital capacity maneuver (Quanjer et al. (1993). Eur. Respir. J. 6, Suppl. 16, pp. 5-40, incorporated by reference herein in its entirety for all purposes). FEV₁ may also be expressed as a percentage of the predicted FEV₁ (i.e., ppFEV₁) obtained from a normal population, based on the patient's gender, height, and age, and sometimes race and weight.

In one embodiment, improving the lung function of the patient comprises increasing the patient's FEV₁ or ppFEV₁ during the administration period, compared to the patient's corresponding FEV₁ or ppFEV₁ prior to the administration period. The increase in FEV₁ or ppFEV₁, in one embodiment, is an increase of about 1%, about 2%, about 3%, about 4%, about 5%, about 6%, about 7%, about 8%, about 9%, about 10%, about 11%, about 12%, about 13%, about 14%, about 15%, about 16%, about 17%, about 18%, about 19%, about 20%, about 25%, about 30%, about 35%, about 40%, about 45%, about 50%, about 55%, about 60%, about 65%, about 70%, about 75%, about 80%, about 85%, or about 90%. In another embodiment, the increase in FEV₁ or ppFEV₁ is an increase of about 5%, about 10%, about 15%, about 20%, about 25%, about 30%, about 35%, about 40%, about 45%, or about 50%. In another embodiment, increasing the FEV₁ or ppFEV₁ comprises increasing by at least about 5%, at least about 10%, at least about 15%, at least about 20%, at least about 25%, at least about 30%, at least about 35%, at least about 40%, at least about 45%, or at least about 50%. In another embodiment, increasing FEV₁ or ppFEV₁ is increasing of about 5% to about 50%, about 5% to about 40%, about 5% to about 30%, about 5% to about 20%, about 10% to about 50%, about 15% to about 50%, about 20% to about 50%, or about 25% to about 50%.

In one embodiment, increasing FEV₁ or ppFEV₁ is increasing in pre-bronchodilator FEV₁ or ppFEV₁. In another embodiment, increasing FEV₁ or ppFEV₁ is increasing post-bronchodilator FEV₁ or ppFEV₁.

In one embodiment, the patient's ppFEV₁ is 80% or less prior to the administration period. In a further embodiment, the patient's ppFEV₁ is 70% or less prior to the administration period. In a further embodiment, the patient's ppFEV₁ is 60% or less prior to the administration period. In a further embodiment, the patient's ppFEV₁ is 50% or less prior to the administration period. In another embodiment, the patient's pp FEV₁ is from 30% to 80%, from 40% to 70%, or from 50% to 60%, prior to the administration period.

In another embodiment, improving the lung function of the patient comprises increasing the patient's FEV₁ during the administration period, compared to prior to the administration period, by from about 25 mL to about 500 mL, from about 25 mL to about 400 mL, from about 25 mL to about 300 mL, from about 25 mL to about 250 mL, from about 25 mL to about 200 mL, or from about 50 mL to about 200 mL, as compared to the patient's FEV₁ prior to the administration period. In one embodiment, increasing FEV₁ is increasing pre-bronchodilator FEV₁. In another embodiment, increasing FEV₁ is increasing post-bronchodilator FEV₁.

In one embodiment, improving the lung function of the patient comprises increasing the mean forced expiratory flow between 25% and 75% of FVC (FEF_{(25-75%)}) (also referred to as the maximum mid-expiratory flow) of the patient during the administration period, as compared to the patient's FEF_{(25-75%)} prior to the administration period. The FEF_{(25-75%)} measurement is dependent on the validity of the FVC measurement and the level of expiratory effort. The FEF_{(25-75%)} index is taken from the blow with the largest sum of FEV₁ and FVC.

In one embodiment, increasing the patient's FEF_{(25-75%)} during the administration period comprises increasing by at least about 1%, by at least about 5%, by at least about 10%, by at least about 15%, by at least about 20%, by at least about 25%, by at least about 30%, by at least about 35%, by at least about 40%, by at least about 45%, or by at least about 50%. In another embodiment, increasing the patient's FEF_{(25-75%)} during the administration period comprises increasing by about 5% to about 50%, by about 5% to about 40%, by about 5% to about 30%, by about 5% to about 20%, by about 10% to about 50%, by about 15% to about 50%, by about 20% to about 50%, or by about 25% to about 50%. In one embodiment, increasing FEF_{(25-75%)} is increasing pre-bronchodilator FEF_{(25-75%)}. In another embodiment, increasing FEF_{(25-75%)} is increasing post-bronchodilator FEF_{(25-75%)}.

Total lung capacity (TLC) is the sum of the vital capacity and residual volume that represents the total volume of air that can be contained in the lung. The total lung capacity (TLC) is divided into four volumes. The tidal volume (V_{T}) is the volume inhaled or exhaled in normal quiet breathing. The inspiratory reserve volume (IRV) is the maximum volume that can be inhaled following a normal quiet inhalation. The expiratory reserve volume (ERV) is the maximum volume that can be exhaled following a normal quiet exhalation. The residual volume (RV) is the volume remaining in the lungs following a maximal exhalation. The vital capacity (VC) is the maximum volume that can be exhaled following a maximal inhalation; VC=IRV+V_{T}+ERV. In one embodiment, improving the lung function of the patient comprises increasing the patient's total lung capacity (TLC) during the administration period, compared to the patient's TLC prior to the administration period. In one embodiment, increasing is by at least about 1%, at least about 2%, at least about 5%, at least about 10%, at least about 15%, at least about 20%, at least about 25%, at least about 30%, at least about 35%, at least about 40%, at least about 45%, or by at least about 50%. In another embodiment, increasing is by from about 1% to about 50%, by from about 5% to about 50%, by from about 5% to about 40%, by from about 5% to about 30%, by from about 5% to about 20%, by from about 10% to about 50%, by from about 15% to about 50%, by from about 20% to about 50%, or by from about 25% to about 50%.

Also known as the transfer factor, lung diffusion capacity for carbon monoxide (DLCO) is a measurement to assess the lungs' ability to transfer gas from inspired air to the bloodstream. Carbon monoxide (CO) has a high affinity for hemoglobin, and it follows the same pathway as that of oxygen to finally bind with hemoglobin. Inhaled CO is used for this test due to its high affinity for hemoglobin (200 to 250 times that of oxygen). As anemia can reduce DLCO, DLCO may be adjusted for hemoglobin values. DLCO may also need to be adjusted for several other factors, such as carboxyhemoglobin, FiO. See Modi P, Cascella M, "Diffusing Capacity Of The Lungs For Carbon Monoxide," [Updated 2021 Mar 24]. In: StatPearls [Internet]. Treasure Island (FL): StatPearls Publishing; 2021 Jan, incorporated herein by reference in its entirety for all purposes. In one embodiment, improving the lung function of the patient comprises increasing the patient's DLCO during the administration period, compared to the patient's DLCO prior to the administration period. In one embodiment, DLCO is adjusted for hemoglobin level, i.e., improving the lung function of the patient comprises increasing the patient's DLCO adjusted for hemoglobin during the administration period compared to the patient's DLCO adjusted for hemoglobin prior to the administration period. In another embodiment, improving the lung function of the patient comprises increasing the patient's DLCO percent (DLCO %) predicted during the administration period compared to the patient's DLCO % predicted prior to the administration period. Predicted normal DLCO values may be calculated according to the equation established by Crapo et al., Am Rev Respir Dis. 123(2):185-9 (1981), or according to the equation established by Miller et al., Am Rev Respir Dis. 127(3):270-7 (1983), each of which is incorporated by reference in its entirety for all purposes. In a further embodiment, the patient's DLCO % predicted is adjusted for hemoglobin.

In one embodiment, improving lung function comprises increasing the patient's DLCO or DLCO % predicted by at least about 1%, at least about 5%, at least about 10%, at least about 15%, at least about 20%, at least about 25%, at least about 30%, at least about 35%, at least about 40%, at least about 45%, or by at least about 50%. In another embodiment, improving lung function comprises increasing the patient's DLCO or DLCO % predicted by from about 5% to about 50%, by from about 5% to about 40%, by from about 5% to about 30%, by from about 5% to about 20%, by from about 10% to about 50%, by from about 15% to about 50%, by from about 20% to about 50%, or by from about 25% to about 50%. In a further embodiment, the patient's DLCO or DLCO % predicted is adjusted for hemoglobin.

In one embodiment, the patient's DLCO % predicted is 80% or less, 70% or less, 60% or less, or 50% or less, prior to the administration period. In a further embodiment, the patient's DLCO % predicted is adjusted for hemoglobin. In another embodiment, the patient's DLCO % predicted is from 30% to 80%, from 40% to 70%, or from 50% to 60%, prior to the administration period. In a further embodiment, the patient's DLCO % predicted is adjusted for hemoglobin.

In one embodiment of a method for treating PH provided herein, the method comprises increasing the length of time to clinical worsening, as compared to an untreated PH patient, or a PH patient not treated with a compound of Formula (I) or (II), wherein the clinical worsening is one selected from the group consisting of death, hospitalization due to a respiratory indication (e.g., dyspnea, and/or deterioration of lung function indicated by reductions in FVC, DLCO, and/or SpO₂), 10% or greater decline in percent predicted FVC (ppFVC) relative to the patient's ppFVC prior to the administration period on two consecutive occasions 4-14 weeks apart, lung transplantation, and 15% or greater decrease in distance walked in a 6-minute walk test (6MWT) relative to the patient's distance walked in a 6MWT prior to the administration period on two consecutive occasions at least 24 hours apart.

In one embodiment, the length of time to clinical worsening is increased by about 1 day, about 3 days, about 1 week, about 2 weeks, about 3 weeks, about 4 weeks, about 5 weeks, or about 6 weeks. In another embodiment, the length of time to clinical worsening is increased by at least about 1 day, at least about 3 days, at least about 1 week, at least about 2 weeks, at least about 3 weeks, at least about 4 weeks, at least about 5 weeks, or at least about 6 weeks. In another embodiment, the length of time to clinical worsening is increased about 20 days to about 100 days, about 30 days to about 100 days, about 20 days to about 75 days, about 20 days to about 50 days, or about 20 days to about 40 days. In another embodiment, the length of time to clinical worsening is increased at least 1 month, e.g., about 1 month to about 6 months, about 1 month to about 4 months, or about 1 month to about 3 months.

In one embodiment, a method for treating PH provided herein comprises increasing the patient's lung lobar volume and/or airway volume assessed by computerized tomography (CT) during the administration period, compared to the patient's lung lobar volume and/or airway volume prior to the administration period. CT may be performed via chest CT scan during a breathing cycle to generate CT images at functional residual capacity (FRC) and/or total lung capacity (TLC). In one embodiment, the lung lobar volume is the volume of the lung lobar structure of the patient's respiratory system at TLC or FRC, and the airway volume is the volume of the airway structure of the patient's respiratory system at TLC or FRC.

In one embodiment, increasing the patient's lung lobar volume and/or airway volume comprises increasing by at least about 1%, at least about 5%, at least about 10%, at least about 15%, at least about 20%, at least about 25%, at least about 30%, at least about 35%, at least about 40%, at least about 45%, or by at least about 50%. In another embodiment, the patient's lung lobar volume and/or airway volume is increased by from about 5% to about 50%, by from about 5% to about 40%, by from about 5% to about 30%, by from about 5% to about 20%, by from about 10% to about 50%, by from about 15% to about 50%, by from about 20% to about 50%, or by from about 25% to about 50%.

### EXAMPLES

The present disclosure is further illustrated by reference to the following Examples. However, it should be noted that the Examples, like the embodiments described above, are illustrative and are not to be construed as restricting the scope of the disclosure in any way.

### Example 1. Evaluation of the DPIs having Four Different Resistances with 8, 16, and 32mg TPIP Capsule Doses

In this Example, Applicant investigated the aerosol performance of TPIP in response to changes in capsule fill, device resistance, and inspiratory flow rate to enable selection of an inhaler for clinical use. Capsules containing 8, 16 or 32mg of TPIP (80, 160, or 320 µg TP, respectively) were evaluated using four breath-actuated RS01 devices with low, medium, high or ultra-high inspiratory resistances, creating 12 different capsule and device configurations for evaluation.

The TP aerosol particle size distribution profiles for the UHR, HR, MR, and LR DPI devices are shown in **FIGs. 7A-7C** for the 8, 16 and 32 mg TPIP capsules, respectively. The TP aerosol particle size distribution profiles are polydisperse for the LR, MR and HR devices with the UHR device showing a trend towards a less polydisperse profile. The values for the total mass recoveries, emitted dose, MMAD, GSD, FPF, and FPD for the UHR, HR, MR, and LR devices loaded with 8, 16 and 32 mg TPIP capsules are shown in Table 5. The mean total recovery of TP ranged between 90 and 100% for the 12 conditions indicating that the experimental procedure was well executed.

The FPF decreased with capsule load ranging from 80 to 85% for the 8 mg capsule and decreasing to 71-81% for the 16 mg capsule and 58-76% for the 32mg capsule (**FIG. 8A**). The FPF was lower for the MR and LR devices at each capsule dose compared to the UHR and HR devices, and this was especially evident for the 32 mg capsule dose with the FPF decreasing to 62 and 58%, respectively. The FPD was uniform for all four devices for the 8 and 16 mg capsule doses, ranging between 51 and 56 µg TP for the 8mg dose and 98-104 µg TP for the 16 mg dose (**FIG. 8B**). The FPD was dose proportional for the UHR and HR devices with an FPD of 197-200 µg for the 32 mg capsule dose; however, for the MR and LR devices the FPD decreased to 168 and 155 µg, respectively (**FIG. 8C**).

Based on the totality of the aerosol characterization data (including superior FPF and polydisperse profile) for the four devices, the HR device was selected for further analysis at flow rates that deviated from the compendial target of 4 kPa. The TP aerosol particle size distribution profile for the device operated at 1.4, 4 and 5.4 kPa (corresponding to 40, 60 and 80 LPM, respectively) is shown in **FIGs. 9A-9C** for the 8, 16 and 32 mg TPIP capsules, respectively.

Applicant surprisingly discovered that the high resistance (HR) DPI (e.g., RS01 device) provided unexpectedly superior FPF with low variability along with dose proportionality from three different capsule doses (8, 16, and 32 mg TPIP) compared to devices with different resistances. See Table 5. Applicant also surprisingly discovered that the HR DPI provided unexpectedly superior ED. See Table 5.

### Example 2. Materials and Methods for Evaluating HR DPI

All the experiments in the following examples of present disclosure were performed with the RS01 Model 7 Plastiape (Plastiape S.p.A., Osnago, Italy) high resistance (HR) (code 239700002AA) DPI device for evaluation. The following examples relate to in vitro aerosol performance of the capsules containing 16 and 32 mg TPIP utilizing the HR RS01 Plastiape device in response to more clinically relevant inhalation profiles deviating from the rectangular wave profile. The aerosol performance were evaluated at different inhalation volumes of 4 L, 2L or 1 L and at a different peak inspiratory flow rate of 60 LPM corresponding to a pressure drop of 4 kPa or 30 LPM corresponding to a pressure drop of 0.89 kPa across the device to reflect a diverse set of user scenarios.

An RS01 Model 7 Plastiape (Plastiape S.p.A., Osnago, Italy) high resistance (HR) (code 239700002AA) DPI device was used for evaluation. The pressure drop across the HR device containing an empty capsule was measured as a function of flow rate between 30 to 100 L/min (LPM).

TPIP is composed of 1.0% TP, ~69.7% mannitol and ~29.3% leucine and was manufactured by spray drying at GLP production scale. TPIP powder has a white physical appearance composed of collapsed spheres by scanning electron microscope, contains ~0.38% moisture, and has a tap density of ~0.52 g/cm³ and bulk density of ~0.25 g/cm³. The particle size distribution of TPIP powder was characterized by laser diffraction (Malvern Mastersizer 3,000 with the Aero S Dry Powder Disperser Unit, Malvern, PA) and was described by a D10 of 0.3 µm, D50 of 1.7 µm, D90 of 3.8 µm, and a Span represented by ((D90-D10)/D50) of 2.0. TPIP powder was filled into size #3 Harro-filled Vcaps^{®} Plus Hypromellose capsules targeting 16 or 32 mg of TPIP, which represents a loaded dose of 160 or 320 µg TP, respectively.

To evaluate the differences in aerosol performance in response to different breathing profiles, TPIP aerosol emitted from the HR Plastiape device was delivered to the NGI (MSP Corp., Shoreview, MN) at ambient temperatures and low humidity (< 45% RH) under various scenarios generated by the breathing simulator (BRS 3100, Copley), as presented in **FIG. 1****.** A mixing inlet (Copley) was attached in between the pre-separator and the induction port to allow a constant flow rate of 30 or 60 LPM to be achieved through the NGI, while varying the change in inhalation profiles from the HR Plastiape device via BRS system. The outlet port of the NGI was connected to a vacuum pump drawing 30 or 60 LPM airflow (X) via a critical flow controller (TPK 2100, Copley), and an airflow of 30 or 60 LPM (Y) was drawn into the mixing inlet to ensure that initially no airflow (X-Y=0) was drawn through the inhaler. Aerosolization of the powder into the NGI was triggered by the BRS system via a diversion valve that redirects the airflow (Y) into the BRS system. For example, if 30 LPM airflow of Y is redirected to the BRS system, this would cause 30 LPM airflow to be drawn through the inhaler and into the mixing inlet. Therefore, the simulated inhalation profile generated by the BRS system is identical to the inhalation profile through the inhaler.

The BRS software was manually programmed through a Word Pad script to simulate inhalation acceleration rates of 20, 5.88, 2, 1, and 0.5 L/sec² corresponding to 50, 170, 500, 1000, and 2000 millisecond (ms) ramp times for a total inhaled volume of 4 L, and for 2 and 1 L inhalation volumes at both extreme acceleration rates to achieve the 60 LPM flow rate. Additionally, the breathing profile scripts for the 1, 2, and 4 L inhalation volumes at 20 and 0.5 L/s² (corresponding to 50 and 2000 ms ramp times) to reach the 30 LPM flow rate are included. A trapezoidal waveform was used to maintain the 4 L total inhalation volume as well as to keep the maximum inhalation flow rate at 60 LPM corresponding to the 4 kPa pressure drop across the device (**Fig. 2A**). Additionally, the breathing profiles with reduced inhalation volumes of 1 and 2 L were evaluated at the two extreme acceleration rates of 20 and 0.5 L/s² (corresponding to 50 and 2000 ms ramp times) for the 60 LPM inspiratory flow rate (**Fig. 2B**), and 25 and 1000 ms ramp times for the 30 LPM inspiratory flow rate (**Fig. 2C**).

The aerosol performance of each inhalation profile was characterized for TP with respect to its emitted dose (ED), mass median aerodynamic diameter (MMAD), geometric standard deviation (GSD), fine particle fraction < 5 µm (FPF) and fine particle dose < 5 µm (FPD) as described below. The total recovery of TP was also measured to provide assurance that each experiment was conducted acceptably.

TP was quantified by an Agilent 1290 Infinity II HPLC with a mass spectrometry detector using an 8-point internal standard log-log calibration range of 4-500 ng/mL TP using TPD4 as an internal standard at a concentration of 70 ng/mL. The sample injection volume was 10 µL with separation conducted at an isocratic flow rate of 1.0 ml/min using a mobile phase of 85:15 acetonitrile: water containing 1% formic acid. The C8 HPLC column was equilibrated at 35°C.

An Agilent LC/MSD XT single quad mass spectrometer was used as follows: The spray chamber was operated at a drying gas flow of 10 L/min, a nebulizer pressure of 35 psig, a drying gas temperature of 225°C, and a capillary voltage of 2,000 V. The TP and TPD4 internal standard mass spec (single quad) conditions were set to positive selected ion monitor (SIM) at 579.6 m/z and 583.6 m/z, respectively, using the electrospray ionization (ESI) and fragmentation energy, both at 250 V.

The aerosol performance of each inhalation profile was characterized for TP with respect to its emitted dose (ED), mass median aerodynamic diameter (MMAD), geometric standard deviation (GSD), fine particle fraction < 5 µm (FPF) and fine particle dose < 5 µm (FPD). The total recovery of TP was also measured to provide assurance that each experiment was conducted acceptably.

To determine ED, MMAD, GSD, FPF and FPD, total recovery of TP was expressed relative to the nominal loaded dose of TP in the capsule which included quantitation of the recovered mass of TP from all the components of the NGI, the residual powder in the capsule, and the DPI device. The emitted dose was expressed as the percentage of TP recovered only from the NGI components relative to the nominal loaded dose of TP in the capsule. The aerosol MMAD, GSD, FPF and FPD were calculated in Microsoft Excel following the USP and Ph. Eur. compendial methods. See (1) United States Pharmacopeia and National Formulary (USP 43-NF 38), General Chapters <601> Inhalation and Nasal Drug Products: Aerosols, Sprays, and Powders-Performance Quality Tests and (2) European Pharmacopeia (Ph. Eur.), 7th Edition, 2.9.18. Preparations for Inhalation: Aerodynamic Assessment of Fine Particles, Monograph 20918. (2008).

A linear regression analysis was performed based on the normal probability *z-*score of the cumulative TP mass recovered (inverse of standard normal cumulative distribution) versus the log cut-off diameters associated with NGI stages 1 to 7 at 60 or 30 LPM flow rates. The cut-off diameters for NGI stages 1 to 7 were 8.06, 4.46, 2.82, 1.66, 0.94, 0.55 and 0.34 µm at the 60 LPM flow rate, respectively, and 11.72, 6.40, 3.99, 2.30, 1.36, 0.83, and 0.54 µm at the 30 LPM flow rate, respectively. The MMAD was calculated based on the normal probability *z*-score corresponding to the cumulative TP mass percentage of 50%, and from the slope and the intercept of the linear regression analysis from the NGI stages. The GSD was calculated as the square root of the ratio based on the normal probability *z*-score corresponding the aerodynamic diameter associated with 84.1% of the cumulative TP mass to that for 15.9% of the cumulative TP mass. The FPF, expressed as the percentage of TP emitted dose with an aerodynamic diameter of less than 5 µm, was obtained from a normal probability *z*-score based on the log aerodynamic diameter of 5 µm, and the slope and the intercept of the linear regression analysis from the NGI stages. The FPD was calculated from the FPF as the mass of TP emitted dose with an aerodynamic diameter of less than 5 µm.

Mean values of ED, FPF, and FPD were calculated for each of the inhalation profiles for TPIP capsule doses of 16 mg or 32 mg, containing 160 or 320 µg TP, respectively. A 2-tailed t-test analysis was used to compare individual results within the same group and an ANOVA single-factor analysis was used for an overall comparison of results within a group in Microsoft Excel. Statistical significance was noted for p-values < 0.05, while non-significance was noted for p-values > 0.05. Additionally, the best fit linear regression analysis was applied for the FPD trend response as a function of inhalation acceleration rate for each capsule tested.

### Example 3. Evaluation of the Inhalation Acceleration Rates for the 16 and 32 mg TPIP Capsule Doses at 60 LPM Inspiratory Flow Rate and 4 L Inhalation Volume

The deposition profiles based on TP recovery from the NGI is shown in **FIGs. 3A** and **3B** for the 16 mg and 32 mg TPIP capsules, respectively, using the 50, 170, 500, 1000, and 2000 ms ramp times to achieve the 60 LPM inspiratory flow rate with a 4 L inhalation volume. The TP aerosol particle size distributions for both capsule doses were polydisperse, but there was a small shift in distribution as a function of inhalation ramp times. As the inhalation ramp time increased, there was slightly greater deposition on stages 6 to the filter and slightly less on stages 2 to 5 with the greatest change for stage 3 and the filter. The values for the total mass recoveries, ED, MMAD, GSD, FPF, and FPD for the 50, 170, 500, 1000, and 2000 ms ramp times for the 16 and 32 mg TPIP capsules are shown in Table 1 and **FIGs. 3A** and **3B**. The mean total recovery of TP ranged between 90 and 99% (including 91% to 95% for 16 mg TPIP, 60 LPM, 4L in **FIG. 3A**) for the five different ramp conditions covering both TPIP capsule doses. This indicates that the experimental procedure was well executed.

The ED was independent of the capsule dose, ranging from 80 to 82% for the 160 µg TP in the 16 mg TPIP capsule and 81 to 89% for the 320 µg TP in the 32 mg TPIP capsule. Variations in inhalation ramp times did not alter the near complete release of TPIP from the capsule compared to the compendial rectangular waveform condition.

The MMAD values increased with capsule load but remained within the respirable range (< 5 µm), varying from 1.0 µm to 1.3 µm for the 16 mg TPIP capsule and increasing to 1.3 to 1.7 µm for the 32 mg TPIP capsule. The MMAD values were slightly higher for the shortest ramp times (50 ms and 170 ms) by 0.1 to 0.2 µm. This result suggests that a slower acceleration (i.e., longer ramp time) to the 60 LPM flow rate was more effective at fully dispersing the TPIP powder. The GSDs were all above 3.5 for both doses indicating significant polydispersity in aerosol particle size.

The FPF and FPD values were uniform across all five ramp times for the 16 mg TPIP capsule ranging from 78 to 80% and 100 to 104 µg TP, respectively (Table 1 and **FIG. 3A**). There were no statistical differences (p > 0.05) among the individual FPD results or overall for this group (Table 2 and **FIG. 4**). For the 32 mg TPIP capsule, the FPF and FPD values ranged from 68 to 73% and 176 to 209 µg TP, respectively, with the longer ramp times (i.e., slower acceleration rates) improving powder dispersion for the higher capsule load (**FIG. 3B**). This led to a statistical difference (p < 0.05) in FPD for the 1000 ms ramp time compared to the 50 ms ramp time, and for the 2000 ms ramp time compared to the 50, 170 and 500 ms ramp times, as well as for the overall FPD results for this group (p=0.005) (Table 2). While there were statistical differences in FPD for some of the conditions, the plot of FPD as a function of inhalation acceleration rate for both capsule doses shows that there were no meaningful differences in FPD at all conditions (**FIG. 4**).

### Example 4. Evaluation of the Inhalation Volume for the 16 and 32 mg TPIP Capsule Doses at 60 LPM Inspiratory Flow Rate

Reduced inhalation volumes of 1 and 2 L for the 16 and 32 mg dose were evaluated at both the minimum (20 L/s²) and maximum (0.5 L/s²) inhalation acceleration rates to achieve the 60 LPM target. The deposition profiles based on TP recovery from the NGI as a function of inhalation volume for the 50 ms and 2000 ms ramp times are shown in **FIG. 5A** for the 16 mg TPIP capsule and in **FIG. 5B** for the 32 mg TPIP capsule. The TP aerosol particle size distribution profiles remained polydisperse across all inhalation volumes for the 16 mg and 32 mg TPIP capsules at both extremes of inhalation flow rate ramp times to achieve the 60 LPM inspiratory flow rate, similar to what was observed for the acceleration study. The values for the total recovery, ED, MMAD, GSD, FPF, and FPD are shown in Table 3. The mean total recovery of TP ranged between 90 and 99% (including between 91 and 97% for 16 mg TPIP 60 LPM) for the different inhalation volume conditions indicating that the experimental procedure was well executed. Reductions in inhalation volume down to 1 L did not impair the near complete release of TPIP from the capsule.

The ED was independent of the dose, ranging from 79 to 83% for the 160 µg TP dose and 81 to 89% for the 320 µg TP dose. Reductions in inhalation volume down to 1 L did not impair the near complete release of TPIP from the capsule.

The MMAD values increased slightly with capsule load but remained well within the respirable range (< 5 µm), increasing from 1.1 µm to 1.3 µm for the 16 mg TPIP capsule to 1.3 to 1.7 µm for the 32 mg TPIP capsule. Decreasing the inhalation volume at both extremes of ramp times did not significantly affect the median aerosol particle size of TPIP. However, the MMAD values for the 16 mg and 32 mg TPIP capsules were highest for the fastest ramp time of 50 ms, similar to what was observed in the acceleration study suggesting that the slower acceleration (i.e., longer ramp time) was more effective at deagglomerating the TPIP powder. The GSDs were all above 3.5 for both doses indicating significant polydispersity in aerosol particle size.

The FPF and FPD values were uniform across all inhalation volumes (1, 2, and 4 L) at both extremes of ramp time for the 16 mg TPIP capsule ranging from 76 to 80% and 97 to 104 µg TP, respectively (Table 3 and **FIG. 6A**). There were no significant differences among the individual FPD values and for the overall FPD results (Table 4) at the same acceleration rate, but individual differences were observed when comparing two extreme ramp times at the same inhalation volume for 50 and 2000 ms at 2 L, and when comparing two extreme ramp times with different inhalation volumes for 50 ms at 1 L and 2000 ms at 4 L, and for 50 ms at 2 L and 2000 ms at 1 L and 4 L. For the 32 mg TPIP capsule, the FPF and FPD values ranged from 63 to 68% and 169 to 178 µg TP for the 50 ms ramp time, respectively, and from 72 to 73% and 187 to 209 µg TP for the 2000 ms ramp time, respectively (Table 3 and **FIG. 6B**). Significant differences between individual values were also observed when comparing the two extreme ramp times at the same inhalation volume but these observations were consistent with that noted in the acceleration study for this capsule dose, and when comparing two extreme ramp times with different inhalation volumes. The longer ramp time resulted in a greater amount of TP being delivered in the respirable range (i.e., increased FPD) (Table 4).

### Example 5. Evaluation of the Inhalation Volume for the 16 mg TPIP Capsule Dose at 30 LPM Inspiratory Flow Rate

Reduced inhalation volumes of 1 L and 2 L were evaluated for the 16 mg TPIP capsule dose at both the minimum (20 L/s²) and maximum (0.5 L/s²) inhalation acceleration rates to achieve the 30 LPM target. The deposition profiles based on TP recovery from the NGI as a function of inhalation volume for the 25 ms and 1000 ms ramp times are shown in **FIG. 5C**. The TP aerosol particle size distribution profiles remained polydisperse across all inhalation volumes for the 16 mg TPIP capsule at both extremes of inhalation flow rate ramp times to achieve the 30 LPM inspiratory flow rate. The values for the total recovery, ED, MMAD, GSD, FPF, and FPD are shown in Table 3. The mean total recovery of TP ranged between 95 and 101% for the different inhalation volume conditions indicating that the experimental procedure was well executed.

The ED range was reduced to 72 to 76% for the 30 LPM flow rate as compared to the 79 to 83% range for the 60 LPM flow rate. Reductions in inhalation volume down to 1 L did not impair the release of TPIP from the capsule, but there was increased deposition in the RS01 device at the 30 LPM flow rate (17 to 25% TP) as shown in **FIG. 5C** compared to the 60 LPM flow rate (10 to 12%) as shown in **FIG. 5A**, indicating that the 0.89 kPa pressure drop generated at 30 LPM had insufficient energy to completely minimize deposition in the device.

The MMAD values remained well within the respirable range (< 5 µm) from 1.9 µm to 2.3 µm but were higher than those observed for the same 16 mg TPIP capsule dose at the 60 LPM flow rate. Decreasing the inhalation volume at both extremes of ramp times did not significantly affect the median aerosol particle size of TPIP. However, the MMAD values were highest for the fastest ramp time of 25 ms, similar to what was observed in the inhalation volume study at the 60 LPM flow rate, suggesting that the slower acceleration rate (i.e., longer ramp time) may be more effective at deagglomerating the TPIP powder even at the very low flow rate of 30 LPM. The GSDs were all above 2.3 indicating significant polydispersity in aerosol particle size.

The FPF and FPD values were uniform across all inhalation volumes (1, 2, and 4 L) at both extremes of ramp times for the 16 mg TPIP capsule ranging from 74 to 76% and 86 to 93 µg TP, respectively (Table 3 and **FIG. 6C**). There were no significant differences among the individual FPD values and for the overall FPD results (Table 4) at the same acceleration rate, but individual differences were observed when comparing the two extreme ramp times at the same inhalation volume (i.e., for the 25 and 1000 ms ramp times at the 1 and 2 L volumes), and when comparing the two extreme ramp times at different inhalation volumes, consistent with that observed in the 60 LPM volume study for the same 16 mg TPIP capsule dose. However, for this study at 30 LPM, the longer ramp time resulted in less TP being delivered in the respirable range (i.e., decreased FPD) (Table 4), perhaps due to the lower energy to disperse the powder at the reduced flow rate. While these differences in FPF and FPD were statistically significant, the absolute differences are not likely to be meaningful.

These results confirm that the performance of TPIP in the HR RS01 device is relatively insensitive to changes in inhalation profiles with varied ramp rates to achieve the 60 LPM flow rate for the compendial volume of 4 L down to a reduced volume of 1 L. Minimal changes in aerosol performance were observed in response to the inhalation profiles generated from either slow or fast ramp rates, a reduction in target flow from 60 to 30 LPM flow rate, or reductions in inhalation volumes down to 1 L. These in vitro results suggest that subjects with significant respiratory muscle dysfunction or compromised lung capacity should be able to utilize the TPIP device without a meaningful deterioration in device performance or reduction in lung dose of TP.

Based on these in vitro findings, the TPIP delivery system does not appear to be affected by changes in inspiratory flow profiles, as well as inspiratory volumes that might be expected to occur in the target patient populations of PAH and PH-ILD.

### Example 6. Summary of Drug Aerosol Performance for Powder Formulations

Effective drug aerosol performance for powder formulations is dependent on the combination of the device characteristics and formulation properties. Passive DPIs require an inspiratory flow rate through the device with adequate energy to deagglomerate and disperse the powder to reach the lungs, and this minimum force will vary depending upon the specific characteristics of that powder in that device.

Applicant surprisingly discovered that the high resistance (HR) DPI (e.g., RS01 device) using a spray-dried powder composition described herein provided unexpectedly superior FPF with low variability along with dose proportionality from three different capsule doses (8, 16, and 32 mg TPIP) compared to devices with different resistances as described in Table 5. See also Example 1.

Subsequent in vitro studies were performed to evaluate the effect of variations in inspiratory pressure on device performance with high resistance. The results of the studies in the prior Examples demonstrated consistent aerosol performance independent of the TPIP capsule dose for inspiratory device flow rates both below (40 LPM flow rate corresponding to a 1.4 kPa pressure drop) and above (80 LPM corresponding to a 5.4 kPa pressure drop) the compendial 4 kPa pressure drop equivalent to a 60 LPM inspiratory flow rate.

As described in Examples of the present disclosure, various inhalation profiles were generated in vitro using the breathing simulator to model differences in the rate of ramp from 0 to 60 LPM inspiratory flow, keeping the total inspired volume constant at 4 L. The NGI was integrated into the breathing simulator to assess the impact on aerosol performance for inhalation profiles that differed from compendial NGI operation utilizing a rectangular wave profile utilizing a forceful and rapid, almost-instantaneous rate of acceleration to the target 60 LPM inspiration flow. Applicant observed that variations in the inhalation acceleration rates using the HR RS01 device, ranging from a short ramp time of 50 ms to a long ramp time of 2000 ms (Table 1), did not compromise aerosol performance, with FPDs ranging between 100 to 104 µg TP for the 16 mg TPIP capsule load and 176 to 209 µg TP for the 32 mg TPIP capsule load, consistent with the previous in vitro HR device evaluation studies with TPIP under compendial NGI conditions: 104 and 197 µg TP for the 16 and 32 mg capsule loads, respectively. A slight increase in the FPDs and slight decrease in the MMADs were observed as the ramp time was lengthened, especially for the 32 mg TPIP capsule (Table 1). Thus, these results suggest that a more clinically relevant inhalation profile with a slower rate of acceleration in inhalation flow rate to the 60 LPM target, with its concomitant longer ramp time, may lead to slightly more favorable powder dispersibility and greater FPD than that observed under the compendial testing conditions.

The present disclosure investigated the effect of in vitro device performance under conditions replicating inspiration scenarios for subjects with reduced lung volumes and with respiratory muscle weakness. A reduction in the volume and internal pressure drop of inhalation air passing through the DPI may lead to inability to disperse or deagglomerate all of the powder. As described above in Examples 3 and 4, inhalation volumes of both 2 and 1 L were evaluated for the dispersion of the 16 and 32 mg TPIP capsules in the HR RS01 device with both fast (20 L/s²) and slow (0.5 L/s²) acceleration rates to achieve the 60 LPM compendial target flow rate. Additionally, evaluation at these inhalation volume parameters at the reduced flow rate of 30 LPM was extended for the 16 mg TPIP capsule to assess the effects of a more reduced pressure drop generated during inhalation.

The device emitted doses (> 79%) for the 1 and 2 L inhalation volumes were comparable to that for the 4 L volume (Table 3) for the 60 LPM flow rate for both capsule doses, suggesting that the TPIP powder is nearly completely dispersed and exits the capsule within the first 1 L of inhaled volume. The MMAD, FPF, and FPD values were also similar to those for the 4 L volume under the same acceleration conditions (50 and 2000 ms ramp times). While statistically significant differences in FPD results were observed for some conditions with the 32 mg TPIP capsule, they do not appear to be clinically meaningful differences. The slower inhalation acceleration rate (2000 ms ramp time) resulted in improved FPDs over the inhalation volume range.

For the 30 LPM flow rate at the 1, 2, and 4 L inhalation volumes, emitted doses were reduced slightly to 72-75% at the slowest acceleration rate (1000 ms ramp time) compared to the fastest acceleration rate (25 ms ramp time) with an ED of 76% (Table 3). The 1 L inhalation volume did not impair the powder dispersion at either the fastest acceleration rate, with an FPD of 93 µg compared to 92 µg for the 4 L volume, or at the slowest acceleration rate, with an FPD of 87 µg compared to 90 µg for the 4 L volume. The MMAD and FPF values were also similar at the lower inhaled volume to that at 4 L but were modestly different than what was observed for the 60 LPM conditions. The increase in MMAD values (1.9 to 2.3 µm) and decrease in FPD values (86 to 93 µg TP) compared to the 60 LPM MMAD values (1.1 to 1.3 µm) and FPD values (97 to 104 µg TP) suggests slightly lower deagglomeration capabilities under extremely low DPI device pressure drop and inhalation rate conditions, but again these differences do not appear to be clinically meaningful.

| Table 1. Aerosol performance characteristics for the 16 and 32 mg TPIP capsules as a function of inhalation flow rate ramp times: 60 LPM peak inspiratory flow rate and 4 L inhalation volume. | | | | | | | | | | |
|---|---|---|---|---|---|---|---|---|---|---|
| Inhalation Ramp Time | n | Total Recovery | | Emitted Dose | | MMAD | GSD | FPF (< 5 µm) | FPD (< 5 µm) | |
| | | % | µg, TP | % | µg, TP | µm | | % | % of Loaded Dose | µg, TP |
| 16 mg capsule containing 160 µg TP | | | | | | | | | | |
| 50 ms | 3 | 90.9 ± 0.6 | 145.42 ± 1.01 | 79.8 ± 1.1 | 127.68 ± 1.70 | 1.27 ± 0.08 | 3.54 ± 0.11 | 78.1 ± 1.1 | 62.3 ± 1.7 | 99.71 ± 2.76 |
| 170 ms | 3 | 93.1 ± 0.8 | 148.99 ± 1.26 | 81.3 ± 1.2 | 130.11 ± 1.95 | 1.18 ± 0.03 | 3.71 ± 0.05 | 77.6 ± 1.1 | 63.1 ± 0.8 | 100.89 ± 1.30 |
| 500 ms | 3 | 92.4 ± 1.2 | 147.88 ± 1.88 | 79.8 ± 0.9 | 127.69 ± 1.46 | 1.04 ± 0.03 | 3.83. ± 0.05 | 80.0 ± 0.4 | 63.8 ± 1.0 | 102.11 ± 1.64 |
| 1000 ms | 3 | 92.7 ± 1.6 | 148.33 ± 2.51 | 80.3 ± 2.7 | 128.47 ± 4.24 | 1.05 ± 0.05 | 3.93 ± 0.10 | 80.4 ± 0.3 | 64.5 ± 2.4 | 103.23 ± 3.80 |
| 2000 ms | 3 | 94.9 ± 0.9 | 151.77 ± 1.39 | 81.8 ± 0.6 | 130.88 ± 1.00 | 1.06 ± 0.02 | 3.97 ± 0.10 | 79.6 ± 1.4 | 65.1 ± 1.6 | 104.23 ± 2.57 |

| 32 mg capsule containing 320 µg TP | | | | | | | | | | |
|---|---|---|---|---|---|---|---|---|---|---|
| 50 ms | 3 | 90.0 ± 1.6 | 288.06 ± 5.23 | 81.3 ± 1.7 | 260.10 ± 5.55 | 1.66 ± 0.07 | 4.30 ± 0.21 | 67.6 ± 1.3 | 54.9 ± 1.3 | 175.81 ± 4.14 |
| 170 ms | 3 | 92.5 ± 2.0 | 296.11 ± 6.40 | 82.7 ± 1.3 | 264.76 ± 4.01 | 1.51 ± 0.02 | 4.35 ± 0.10 | 68.6 ± 3.3 | 56.8 ± 3.5 | 181.59 ± 11.15 |
| 500 ms | 3 | 90.9 ± 1.0 | 290.81 ± 3.13 | 81.3 ± 0.3 | 260.04 ± 1.07 | 1.38 ± 0.10 | 4.59 ± 0.26 | 70.7 ± 1.6 | 57.5 ± 1.1 | 183.93 ± 3.48 |
| 1000 ms | 3 | 92.6 ± 0.4 | 296.15 ± 1.38 | 82.9 ± 0.4 | 265.20 ± 1.12 | 1.40 ± 0.13 | 4.57 10.23 | 71.3 ± 2.4 | 59.1 ± 2.3 | 189.18 ± 7.19 |
| 2000 ms | 3 | 98.7 ± 1.9 | 315.89 ± 6.16 | 89.4 ± 2.2 | 286.14 ± 7.08 | 1.33 ± 0.10 | 4.32 ± 0.09 | 72.9 ± 2.0 | 65.2 ± 3.3 | 208.75 ± 10.62 |
| MMAD=Mass Median Aerodynamic Diameter; | | | | | | | | | | |
| GSD=Geometric Standard Deviation; | | | | | | | | | | |
| FPF=Fine Particle | | | | | | | | | | |

| Table 1. Aerosol performance characteristics for the 16 and 32 mg TPIP capsules as a function of inhalation flow rate ramp times: 60 LPM peak inspiratory flow rate and 4 L inhalation volume. | | | | | | | | | | |
|---|---|---|---|---|---|---|---|---|---|---|
| Inhalation Ramp Time | n | Total Recovery | | Emitted Dose | | MMAD | GSD | FPF (< 5 µm) | FPD (< 5 µm) | |
| | | % | µg, TP | % | µg, TP | µm | | % | % of Loaded Dose | µg, TP |
| Fraction; | | | | | | | | | | |
| FPD=Fine Particle Dose; | | | | | | | | | | |
| TP= Treprostinil Palmitil | | | | | | | | | | |

| Table 2. Comparison of the FPDs between the various combinations of inhalation acceleration rates (50 to 2000 ms flow rate ramp) for the 16 mg and 32 mg TPIP capsules: 60 LPM peak inspiratory flow rate and 4 L inhalation volume. | | | | | |
|---|---|---|---|---|---|
| Ramp Time | FPD (< 5 µm) t-test 2-tailed p-values | | | | |
| | vs. 50 ms | vs. 170 ms | vs. 500 ms | vs. 1000 ms | vs. 2000 ms |
| 16 mg capsule containing 160 µg TP | | | | | |
| 50 ms | - | 0.540 | 0.265 | 0.265 | 0.106 |
| 170 ms | 0.540 | - | 0.370 | 0.371 | 0.115 |
| 500 ms | 0.265 | 0.370 | - | 0.664 | 0.294 |
| 1000 ms | 0.265 | 0.371 | 0.664 | - | 0.724 |
| 2000 ms | 0.106 | 0.115 | 0.294 | 0.724 | - |
| Overall p-value (Anova: single factor) | 0.282 | | | | |

| 32 mg capsule containing 320 µg TP | | | | | |
|---|---|---|---|---|---|
| 50 ms | - | 0.447 | 0.060 | 0.049 | 0.007 |
| 170 ms | 0.447 | - | 0.747 | 0.378 | 0.038 |
| 500 ms | 0.060 | 0.747 | - | 0.318 | 0.018 |
| 1000 ms | 0.049 | 0.378 | 0.318 | - | 0.057 |
| 2000 ms | 0.007 | 0.038 | 0.018 | 0.057 | - |
| Overall p-value (Anova: single factor) | 0.005 | | | | |
| FPD=Fine Particle Dose | | | | | |

| Table 3. Aerosol performance characteristics for the 16 and 32 mg TPIP capsules as a function of inhalation volume (1, 2 and 4 L) and acceleration rate (50 and 2000 ms flow rate ramps for the 60 LPM, and 25 and 1000 ms flow rate ramps for the 30 LPM peak inspiratory flow rates). | | | | | | | | | | |
|---|---|---|---|---|---|---|---|---|---|---|
| Inhalation Parameters: ramp time, inhaled volume | n | Total Recovery | | Emitted Dose | | MMAD | GSD | FPF (< 5 µm) | FPD (< 5 µm) | |
| | | % | µg, TP | % | µg, TP | µm | | % | % of Loaded Dose | µg, TP |
| 16 mg capsule containing 160 µg TP at 60 LPM (50 and 2000 ramp times corresponding to 20 and 0.5 L/s² acceleration rates, respectively) | | | | | | | | | | |
| 50 ms, 1 L | 3 | 93.8 ± 1.0 | 150.08 ± 1.66 | 81.1 ± 0.6 | 129.81 ± 0.96 | 1.24 ± 0.02 | 3.80 ± 0.07 | 76.0 ± 1.5 | 61.7 ± 0.7 | 98.70 ± 1.19 |
| 50 ms, 2 L | 3 | 92.0 ± 1.2 | 147.18 ± 1.88 | 79.1 ± 1.9 | 126.53 ± 3.07 | 1.28 ± 0.01 | 3.68 ± 0.13 | 76.9 ± 1.0 | 60.8 ± 0.7 | 97.28 ± 1.12 |
| *50 ms, 4 L | 3 | 90.9 ± 0.6 | 145.42 ± 1.01 | 79.8 ± 1.1 | 127.68 ± 1.70 | 1.27 ± 0.08 | 3.54 ± 0.11 | 78.1 ± 1.1 | 62.3 ± 1.7 | 99.71 ± 2.76 |
| | | | | | | | | | | |
| 2000 ms, 1 L | 3 | 96.5 ± 1.4 | 154.42 ± 2.31 | 82.3 ± 1.2 | 131.71 ± 1.92 | 1.19 ± 0.05 | 4.16 ± 0.05 | 76.8 ± 1.0 | 63.2 ± 1.0 | 101.13 ± 1.61 |
| 2000 ms, 2 L | 3 | 95.6 ± 1.9 | 152.97 ± 3.11 | 82.5 ± 2.3 | 131.94 ± 3.63 | 1.06 ± 0.03 | 3.95 ± 0.05 | 78.6 ± 0.7 | 64.8 ± 2.4 | 103.68 ± 3.81 |
| *2000 ms, 4 L | 3 | 94.9 ± 0.9 | 151.77 ± 1.39 | 81.8 ± 0.6 | 130.88 ± 1.00 | 1.06 ± 0.02 | 3.97 ± 0.10 | 79.6 ± 1.4 | 65.1 ± 1.6 | 104.23 ± 2.57 |

| 32 mg capsule containing 320 µg TP at 60 LPM (50 and 2000 ramp times corresponding to 20 and 0.5 L/s² acceleration rates, respectively) | | | | | | | | | | |
|---|---|---|---|---|---|---|---|---|---|---|
| 50 ms, 1 L | 3 | 94.8 ± 1.0 | 303.26 ± 3.28 | 84.1 ± 0.5 | 269.01 ± 1.62 | 1.68 ± 0.03 | 4.51 ± 0.07 | 63.0 ± 1.0 | 53.0 ± 0.8 | 169.43 ± 2.45 |
| 50 ms, 2 L | 3 | 94.8 ± 1.2 | 303.31 ± 3.97 | 84.7 ± 1.3 | 271.12 ± 3.99 | 1.65 ± 0.02 | 4.30 ± 0.14 | 65.6 ± 1.0 | 55.6 ± 0.9 | 177.94 ± 2.73 |
| *50 ms, 4 L | 3 | 90.0 ± 1.6 | 288.06 ± 5.23 | 81.3 ± 1.7 | 260.10 ± 5.55 | 1.66 ± 0.07 | 4.30 ± 0.21 | 67.6 ± 1.3 | 54.9 ± 1.3 | 175.81 ± 4.14 |
| 50 ms, 4 L | 3 | 92.7 ± 0.2 | 296.56 ± 0.76 | 83.9 ± 0.5 | 268.37 ± 1.53 | 1.69 ± 0.07 | 4.35 ± 0.08 | 64.6 ± 1.5 | 54.2 ± 1.3 | 173.38 ± 4.28 |
| | | | | | | | | | | |
| 2000 ms, 1 L | 3 | 93.7 ± 1.5 | 299.82 ± 4.88 | 81.2 ± 1.3 | 259.99 ± 4.08 | 1.27 ± 0.08 | 4.37 ± 0.16 | 72.1 ± 1.4 | 58.6 ± 0.3 | 187.40 ± 0.88 |
| 2000 ms, 2 L | 3 | 95.0 ± 0.6 | 303.95 ± 2.02 | 86.8 ± 0.6 | 277.59 ± 2.00 | 1.34 ± 0.03 | 4.25 ± 0.10 | 72.6 ± 1.4 | 63.0 ± 1.1 | 201.61 ± 3.49 |
| *2000 ms, 4 L | 3 | 98.7 ± 1.9 | 315.89 ± 6.16 | 89.4 ± 2.2 | 286.14 ± 7.08 | 1.33 ± 0.10 | 4.32 ± 0.09 | 72.9 ± 2.0 | 65.2 ± 3.3 | 208.75 ± 10.62 |
| 2000 ms, 4 L | 3 | 96.5 ± 0.4 | 308.87 ± 1.32 | 87.6 ± 0.9 | 280.22 ± 2.81 | 1.35 ± 0.03 | 4.33 ± 0.01 | 72.2 ± 0.2 | 63.2 ± 0.6 | 202.20 ± 1.96 |

| 16 mg capsule containing 160 µg TP at 30 LPM (25 and 1000 ramp times corresponding to 20 and 0.5 L/s² acceleration rates, respectively) | | | | | | | | | | |
|---|---|---|---|---|---|---|---|---|---|---|
| 25 ms, 1 L | 3 | 101.0 ± 0.4 | 161.59 ± 0.60 | 76.4 ± 0.7 | 122.30 ± 1.04 | 2.19 ± 0.11 | 2.34 ± 0.05 | 76.2 ± 1.7 | 58.2 ± 0.9 | 93.13 ± 1.45 |
| 25 ms, 2 L | 3 | 95.4 ± 0.3 | 152.57 ± 0.56 | 76.1 ± 2.3 | 121.79 ± 3.61 | 2.25 ± 0.08 | 2.31 ± 0.10 | 75.6 ± 1.4 | 57.5 ± 0.9 | 92.08 ± 1.40 |
| 25 ms, 4 L | 3 | 95.6 ± 1.1 | 152.90 ± 1.74 | 76.1 ± 1.8 | 121.83 ± 2.95 | 2.16 ± 0.08 | 2.40 ± 0.06 | 75.9 ± 1.8 | 57.7 ± 1.0 | 92.39 ± 1.60 |
| | | | | | | | | | | |
| 1000 ms, 1 L | 3 | 100.9 ± 0.9 | 161.38 ± 1.49 | 71.7 ± 1.8 | 114.73 ± 2.83 | 1.90 ± 0.11 | 2.58 ± 0.07 | 76.0 ± 1.3 | 54.5 ± 0.8 | 87.16 ± 1.27 |

| Table 3. Aerosol performance characteristics for the 16 and 32 mg TPIP capsules as a function of inhalation volume (1, 2 and 4 L) and acceleration rate (50 and 2000 ms flow rate ramps for the 60 LPM, and 25 and 1000 ms flow rate ramps for the 30 LPM peak inspiratory flow rates). | | | | | | | | | | |
|---|---|---|---|---|---|---|---|---|---|---|
| Inhalation Parameters: ramp time, inhaled volume | n | Total Recovery | | Emitted Dose | | MMAD | GSD | FPF (< 5 µm) | FPD (< 5 µm) | |
| | | % | µg, TP | % | µg, TP | µm | | % | % of Loaded Dose | µg, TP |
| 1000 ms, 2 L | 3 | 101.3 ± 0.9 | 162.13 ± 1.43 | 72.7 ± 1.1 | 116.30 ± 1.71 | 1.88 ± 0.08 | 2.67 ± 0.03 | 74.2 ± 1.1 | 53.9 ± 0.9 | 86.26 ± 1.44 |
| 1000 ms, 4 L | 3 | 97.5 ± 2.6 | 155.93 ± 4.13 | 74.8 ± 2.3 | 119.67 ± 3.75 | 1.95 ± 0.11 | 2.54 ± 0.09 | 74.9 ± 0.6 | 56.0 ± 2.1 | 89.61 ± 3.29 |

| | | | | | | | | | | |
|---|---|---|---|---|---|---|---|---|---|---|
| * Original data from acceleration rate study for the 16 mg and 32 mg capsule. No significant difference (p > 0.05) between original and repeat run for emitted dose, MMAD, FPF, & FPD. MMAD=Mass Median Aerodynamic Diameter; GSD=Geometric Standard Deviation; FPF=Fine Particle Fraction; FPD=Fine Particle Dose; TP= Treprostinil Palmitil. | | | | | | | | | | |

| Table 4. Comparison of the FPDs between various combinations of inhalation volumes (1, 2 and 4 L) at the longest and shortest flow rate ramp time for the 16 mg and 32 mg TPIP capsules: 30 and 60 LPM peak inspiratory flow rates. | | | | | | |
|---|---|---|---|---|---|---|
| Ramp Time, Volume | FPD (< 5 µm) t-test 2-tailed p-values | | | | | |
| | vs. 50 ms 1 L | vs. 50 ms 2 L | vs. 50 ms 4 L | vs. 2000 ms 1 L | vs. 2000 ms 2 L | vs. 2000 ms 4 L |
| 16 mg capsule containing 160 µg TP at 60 LPM (50 and 2000 ramp times corresponding to 20 and 0.5 L/s2 acceleration rates, respectively) | | | | | | |
| 50 ms, 1 L | - | 0.206 | 0.588 | 0.103 | 0.097 | 0.028 |
| 50 ms, 2 L | 0.206 | - | 0.229 | 0.027 | 0.049 | 0.013 |
| 50 ms, 4 L | 0.588 | 0.229 | - | 0.485 | 0.218 | 0.106 |
| 2000 ms, 1 L | 0.103 | 0.027 | 0.485 | - | 0.345 | 0.151 |
| 2000 ms, 2 L | 0.097 | 0.049 | 0.218 | 0.345 | - | 0.846 |
| 2000 ms, 4 L | 0.028 | 0.013 | 0.106 | 0.151 | 0.846 | - |
| (Anova: single factor) | 0.336 | | | 0.409 | | |
| Overall p-value (Anova: single factor) | 0.022 | | | | | |

| 32 mg capsule containing 320 µg TP at 60 LPM (50 and 2000 ramp times corresponding to 20 and 0.5 L/s2 acceleration rates, respectively) | | | | | | |
|---|---|---|---|---|---|---|
| 50 ms, 1 L | - | 0.016 | 0.237 | 0.000 | 0.000 | 0.000 |
| 50 ms, 2 L | 0.016 | - | 0.194 | 0.005 | 0.001 | 0.000 |
| 50 ms, 4 L | 0.237 | 0.194 | - | 0.005 | 0.001 | 0.000 |
| 2000 ms, 1 L | 0.000 | 0.005 | 0.005 | - | 0.002 | 0.000 |
| 2000 ms, 2 L | 0.000 | 0.001 | 0.001 | 0.002 | - | 0.811 |
| 2000 ms, 4 L | 0.000 | 0.000 | 0.000 | 0.000 | 0.811 | - |
| p-value (Anova: single factor) | 0.050 | | | 0.004 | | |
| Overall p-value (Anova: single factor) | 0.000 | | | | | |
| | | | | | | |
| Ramp Time, Volume | vs. 25 ms 1 L | vs. 25 ms 2 L | vs. 25 ms 4 L | vs. 1000 ms 1 L | vs. 1000 ms 2 L | vs. 1000 ms 4 L |

| 16 mg capsule containing 160 µg TP at 30 LPM (25 and 1000 ramp times corresponding to 20 and 0.5 L/s² acceleration rates, respectively) | | | | | | |
|---|---|---|---|---|---|---|
| 25 ms, 1 L | - | 0.415 | 0.581 | 0.006 | 0.004 | 0.165 |
| 25 ms, 2 L | 0.415 | - | 0.813 | 0.011 | 0.007 | 0.298 |
| 25 ms, 4 L | 0.581 | 0.813 | - | 0.011 | 0.008 | 0.259 |
| 1000 ms, 1 L | 0.006 | 0.011 | 0.011 | - | 0.463 | 0.296 |
| 1000 ms, 2 L | 0.004 | 0.007 | 0.008 | 0.463 | - | 0.182 |
| 1000 ms, 4 L | 0.165 | 0.298 | 0.259 | 0.296 | 0.182 | - |
| (Anova: single factor) | 0.686 | | | 0.235 | | |
| Overall p-value (Anova: single factor) | 0.003 | | | | | |
| FPD=Fine Particle Dose | | | | | | |

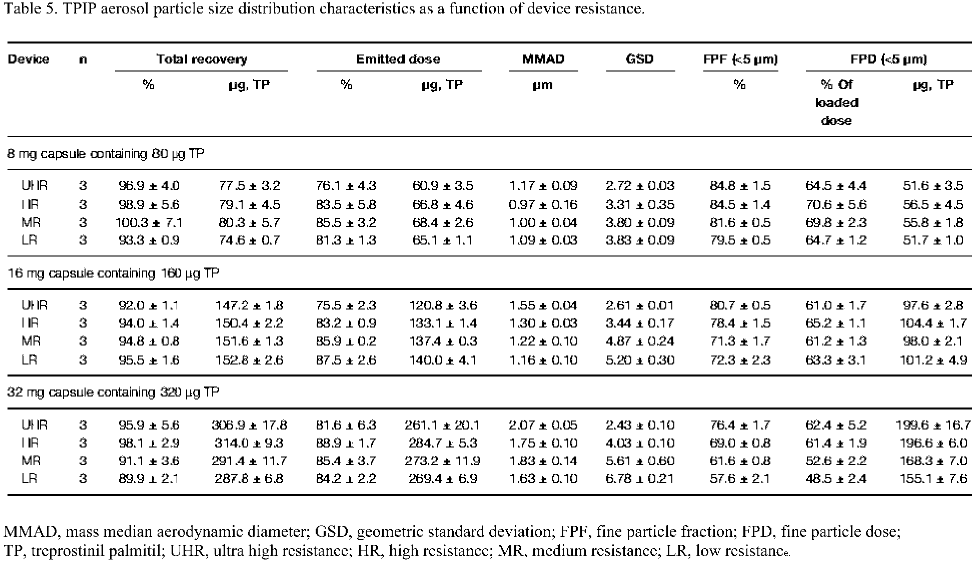

### NUMBERED EMBODIMENTS

Other subject matter contemplated by the present disclosure is set out in the following numbered embodiments:
1. A powder composition comprising:
(a) about 0.5 wt% to about 5 wt% of a compound of Formula (I) 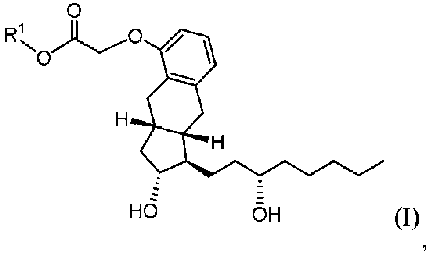 or stereoisomer or a pharmaceutically acceptable salt thereof, wherein R1 is tetradecyl, pentadecyl, hexadecyl, heptadecyl, or octadecyl,
(b) about 10 wt% to about 61wt% leucine, and
(c) trehalose, mannitol or a combination thereof,

wherein the sum of (a), (b), and (c) is 100%; and
when administered via a dry powder inhaler, the dry powder inhaler provides an aerosol comprising a fine particle fraction (FPF) greater than or equal to about 60%, when measured using a Next Generation Impactor (NGI), with a inspiratory flow rate of 30-60 liter per minute (L/min), inspiratory volume of 1-4 L, and inhalation acceleration rate of 0.5-20 L/sec².
2. The powder composition of embodiment 1, wherein the FPF ranges from 63% to about 73%.
3. The powder composition of embodiment 1, wherein the FPF ranges from 73% to about 80%.
4. The powder composition of any one of embodiments 1-3, wherein the aerosol comprises a fine particle dose (FPD) greater than or equal to 50% of a loaded dose of the powder composition.
5. The powder composition of any one of embodiments 1-4, wherein the FPD ranging from about 50% to about 80%.
6. The powder composition of any one of embodiments 1-4, wherein the FPD ranging from about 55% to about 70%.
7. The powder composition of any one of embodiments 1-6, wherein the aerosol comprises an emitted dose (ED) greater than or equal to 70%.
8. The powder composition of embodiment 7, wherein the ED ranges from about 75% to about 90%.
9. The powder composition of any one of embodiments 1-8, wherein the aerosol comprises a mass mean aerodynamic diameter (MMAD) less than 5 µm.
10. The powder composition of embodiment 9, wherein the MMAD ranges from about 0.75 µm to about 3.0 µm.
11. The powder composition of embodiment 9, wherein the MMAD ranges from about 0.8 µm to about 2.0 µm.
12. The powder composition of any one of embodiments 1-11, comprising particles, at least a portion of the particles comprising:
(i) a compound of Formula (I) or stereoisomer or a pharmaceutically acceptable salt,
(ii) leucine, and
(iii) trehalose, mannitol or a combination thereof,
wherein the particles have an average diameter ranging from about 1.0 µm to about 2.0 µm.
13. The powder composition of embodiment 12, wherein the particles have a D10 of about 0.3 µm.
14. The powder composition of embodiment 12, wherein the particles have a D50 of about 1.7 µm.
15. The powder composition of embodiment 12, wherein the particles have a D90 of about 3.8 µm.
16. The powder composition of embodiment 1, wherein R1 is hexadecyl.
17. The powder composition of embodiment 16, wherein R1 is linear hexadecyl.
18. The powder composition of embodiment 1, wherein R1 is tetradecyl.
19. The powder composition of embodiment 18, wherein R1 is linear tetradecyl.
20. The powder composition of embodiment 1, wherein R1 is pentadecyl.
21. The powder composition of embodiment 20, wherein R1 is linear pentadecyl.
22. The powder composition of embodiment 1, wherein R1 is hexadecyl.
23. The powder composition of embodiment 22, wherein R1 is linear hexadecyl.
24. The powder composition of embodiment 1, wherein R1 is heptadecyl.
25. The powder composition of embodiment 24, wherein R1 is linear heptadecyl.
26. The powder composition of embodiment 1, wherein R1 is octadecyl.
27. The powder composition of embodiment 26, wherein R1 is linear octadecyl.
28. The powder composition of any one of embodiments 1-27, wherein the compound of Formula (I), or a pharmaceutically acceptable salt thereof, is present at from about 0.5 wt% to about 4.5 wt% of the total weight of the powder composition.
29. The powder composition of embodiment 28, wherein the compound of Formula (I), or a pharmaceutically acceptable salt thereof, is present at from about 1 wt% to about 4.5 wt% of the total weight of the powder composition.
30. The powder composition of embodiment 28, wherein the compound of Formula (I), or a pharmaceutically acceptable salt thereof, is present at from about 1 wt% to about 4 wt% of the total weight of the powder composition.
31. The powder composition of embodiment 28, wherein the compound of Formula (I), or a pharmaceutically acceptable salt thereof, is present at from about 1 wt% to about 3.5 wt% of the total weight of the powder composition.
32. The powder composition of embodiment 28, wherein the compound of Formula (I), or a pharmaceutically acceptable salt thereof, is present at from about 1 wt% to about 3 wt% of the total weight of the powder composition.
33. The powder composition of embodiment 28, wherein the compound of Formula (I), or a pharmaceutically acceptable salt thereof, is present at from about 1 wt% to about 2.5 wt% of the total weight of the powder composition.
34. The powder composition of embodiment 28, wherein the compound of Formula (I), or a pharmaceutically acceptable salt thereof, is present at from about 1 wt% to about 2 wt% of the total weight of the powder composition.
35. The powder composition of embodiment 28, wherein the compound of Formula (I), or a pharmaceutically acceptable salt thereof, is present at from about 1 wt% to about 1.5 wt% of the total weight of the powder composition.
36. The powder composition of embodiment 28, wherein the compound of Formula (I), or a pharmaceutically acceptable salt thereof is present at from about 2 wt% to about 4 wt% of the total weight of the powder composition.
37. The powder composition of embodiment 28, wherein the compound of Formula (I), or a pharmaceutically acceptable salt thereof, is present at from about 2 wt% to about 3.5 wt% of the total weight of the powder composition.
38. The powder composition of embodiment 28, wherein the compound of Formula (I), or a pharmaceutically acceptable salt thereof, is present at from about 2 wt% to about 3 wt% of the total weight of the powder composition.
39. The powder composition of embodiment 28, wherein the compound of Formula (I), or a pharmaceutically acceptable salt thereof, is present at from about 2 wt% to about 2.5 wt% of the total weight of the powder composition.
40. The powder composition of embodiment 28, wherein the compound of Formula (I), or a pharmaceutically acceptable salt thereof, is present at from about 3 wt% to about 4.5 wt% of the total weight of the powder composition.
41. The powder composition of embodiment 28, wherein the compound of Formula (I), or a pharmaceutically acceptable salt thereof, is present at from about 3 wt% to about 4 wt% of the total weight of the powder composition.
42. The powder composition of embodiment 28, wherein the compound of Formula (I), or a pharmaceutically acceptable salt thereof, is present at from about 3 wt% to about 3.5 wt% of the total weight of the powder composition.
43. The powder composition of embodiment 28, wherein the compound of Formula (I), or a pharmaceutically acceptable salt thereof, is present at from about 0.5 wt% to about 2 wt% of the total weight of the powder composition.
44. The powder composition of embodiment 28, wherein the compound of Formula (I), or a pharmaceutically acceptable salt thereof, is present at from about 0.5 wt% to about 1.5 wt% of the total weight of the powder composition.
45. The powder composition of embodiment 28, wherein the compound of Formula (I), or a pharmaceutically acceptable salt thereof, is present at from about 0.5 wt% to about 1 wt% of the total weight of the powder composition.
46. The powder composition of embodiment 28, wherein the compound of Formula (I), or a pharmaceutically acceptable salt thereof, is present at from about 3.5 wt% to about 4.5 wt% of the total weight of the powder composition.
47. The powder composition of embodiment 28, wherein the compound of Formula (I), or a pharmaceutically acceptable salt thereof, is present at from about 4 wt% to about 4.5 wt% of the total weight of the powder composition.
48. The powder composition of embodiment 28, wherein the compound of Formula (I), or a pharmaceutically acceptable salt thereof is present at from about 0.5 wt% of the total weight of the powder composition.
49. The powder composition of embodiment 28, wherein the compound of Formula (I), or a pharmaceutically acceptable salt thereof, is present at about 1 wt% of the total weight of the powder composition.
50. The powder composition of embodiment 28, wherein the compound of Formula (I), or a pharmaceutically acceptable salt thereof, is present at about 1.5 wt% of the total weight of the powder composition.
51. The powder composition of embodiment 28, wherein the compound of Formula (I), or a pharmaceutically acceptable salt thereof, is present at about 2 wt% of the total weight of the powder composition.
52. The powder composition of embodiment 28, wherein the compound of Formula (I), or a pharmaceutically acceptable salt thereof, is present at about 2.5 wt% of the total weight of the powder composition.
53. The powder composition of embodiment 28, wherein the compound of Formula (I), or a pharmaceutically acceptable salt thereof, is present at about 3 wt% of the total weight of the powder composition.
54. The powder composition of embodiment 28, wherein the compound of Formula (I), or a pharmaceutically acceptable salt thereof, is present at about 3.5 wt% of the total weight of the powder composition.
55. The powder composition of embodiment 28, wherein the compound of Formula (I), or a pharmaceutically acceptable salt thereof, is present at about 4 wt% of the total weight of the powder composition.
56. The powder composition of any one of embodiments 1-55, wherein the compound of Formula (I), or a pharmaceutically acceptable salt thereof, is a compound of Formula (II).
57. The powder composition of any one of embodiments 1-56, wherein the leucine is present at from about 25 wt% to about 61 wt% of the total weight of the powder composition.
58. The powder composition of any one of embodiments 1-56, wherein the leucine is present at from about 40 wt% to about 61 wt% of the total weight of the powder composition.
59. The powder composition of any one of embodiments 1-56, wherein the leucine is present at from about 50 wt% to about 61 wt% of the total weight of the powder composition.
60. The powder composition of any one of embodiments 1-56, wherein the leucine is present at from about 55 wt% to about 61 wt% of the total weight of the powder composition.
61. The powder composition of any one of embodiments 1-56, wherein the leucine is present at from about 58 wt% to about 61 wt% of the total weight of the powder composition.
62. The powder composition of any one of embodiments 1-56, wherein the leucine is present at from about 40 wt% to about 45 wt% of the total weight of the powder composition.
63. The powder composition of any one of embodiments 1-56, wherein the leucine is present at from about 44 wt% to about 51 wt% of the total weight of the powder composition.
64. The powder composition of any one of embodiments 1-56, wherein the leucine is present at from about 43 wt% to about 48 wt% of the total weight of the powder composition.
65. The powder composition of any one of embodiments 1-56, wherein the leucine is present at from about 25 wt% to about 30 wt% of the total weight of the powder composition.
66. The powder composition of any one of embodiments 1-56, wherein the leucine is present at from about 28 wt% to about 30 wt% of the total weight of the powder composition.
67. The powder composition of any one of embodiments 1-56, wherein the leucine is present at from about 25 wt% to about 33 wt% of the total weight of the powder composition.
68. The powder composition of any one of embodiments 1-56, wherein the leucine is present at from about 27 wt% to about 33 wt% of the total weight of the powder composition.
69. The powder composition of any one of embodiments 1-56, wherein the leucine is present at from about 27 wt% to about 31 wt% of the total weight of the powder composition.
70. The powder composition of any one of embodiments 1-56, wherein the leucine is present at from about 27 wt% to about 30 wt% of the total weight of the powder composition.
71. The powder composition of any one of embodiments 1-56, wherein the leucine is present at from about 28 wt% to about 30 wt% of the total weight of the powder composition.
72. The powder composition of any one of embodiments 1-56, wherein the leucine is present at about 30 wt% of the total weight of the powder composition.
73. The powder composition of any one of embodiments 1-56, wherein the leucine is present at about 45 wt% of the total weight of the powder composition.
74. The powder composition of any one of embodiments 1-56, wherein the leucine is present at about 60 wt% of the total weight of the powder composition.
75. The powder composition of any one of embodiments 1-74, wherein the sugar is trehalose.
76. The powder composition of any one of embodiments 1-74, wherein the sugar is mannitol.
77. The powder composition of embodiment 1, selected from one of the following compositions:

| **TPIP composition #** | **Treprostinil palmitil (%w)** | **Leucine (%w)** | **Mannitol (%w)** | **Total composition (%w)** |
|---|---|---|---|---|
| **1** | 0.5 | 60.0 | 39.5 | 100 |
| **2** | 2.0 | 61.2 | 36.8 | 100 |
| **3** | 3.0 | 60.7 | 36.3 | 100 |
| **4** | 4.0 | 60.0 | 36.0 | 100 |
| **5** | 0.4 | 45.0 | 54.6 | 100 |
| **6** | 1.5 | 44.4 | 54.1 | 100 |
| **7** | 2.0 | 45.0 | 53.0 | 100 |
| **8** | 3.0 | 44.5 | 52.5 | 100 |
| **9** | 4.0 | 45.0 | 51.0 | 100 |
| **10** | 0.5 | 30.0 | 69.5 | 100 |
| **11** | 1.0 | 29.3 | 69.7 | 100 |
| **12** | 1.5 | 29.6 | 68.9 | 100 |
| **13** | 1.5 | 29.3 | 69.2 | 100 |
| **14** | 2.0 | 28.8 | 69.2 | 100 |
| **15** | 3.0 | 28.6 | 68.4 | 100 |
| **16** | 4.0 | 30.0 | 66.0 | 100 |

78. The powder composition of embodiment 1, having a leucine-to-mannitol weight ratio of about 0.40-to-1 (leucine-to-mannitol) to about 0.50-to-1 (leucine-to-mannitol).
79. The powder composition of embodiment 78, having a leucine-to-mannitol weight ratio of about 0.40-to-1 (leucine-to-mannitol) to about 0.45-to-1 (leucine-to-mannitol).
80. The powder composition of embodiment 1, having a leucine-to-mannitol weight ratio of about 0.75-to-1 (leucine-to-mannitol) to about 0.90-to-1 (leucine-to-mannitol).
81. The powder composition of embodiment 80, having a leucine-to-mannitol weight ratio of about 0.80-to-1 (leucine-to-mannitol) to about 0.90-to-1 (leucine-to-mannitol).
82. The powder composition of embodiment 1, having a leucine-to-mannitol weight ratio of about 1.5-to-1 (leucine-to-mannitol) to about 1.7-to-1 (leucine-to-mannitol).
83. The powder composition of embodiment 82, having a leucine-to-mannitol weight ratio of about 1.65-to-1 (leucine-to-mannitol) to about 1.7-to-1 (leucine-to-mannitol).
84. The powder composition of any one of embodiments 78-83, comprising the compound of Formula (I) at from about 0.5 wt% to about 4 wt% of the total weight of the powder composition.
85. The powder composition of any one of embodiments 78-83, comprising the compound of Formula (I) at from about 0.5 wt% to about 3 wt% of the total weight of the powder composition.
86. The powder composition of any one of embodiments 78-83, comprising the compound of Formula (I) at from about 0.5 wt% to about 2 wt% of the total weight of the powder composition.
87. The powder composition of any one of embodiments 78-83, comprising the compound of Formula (I) at from about 0.5 wt% to about 1.5 wt% of the total weight of the powder composition.
88. The powder composition of any one of embodiments 78-87, comprising the compound of Formula (I) at from about 1 wt% to about 1.5 wt% of the total weight of the powder composition.
89. The powder composition of any one of embodiments 78-87, comprising the compound of Formula (I) at from about 2 wt% to about 4 wt% of the total weight of the powder composition.
90. The powder composition of any one of embodiments 78-87, comprising the compound of Formula (I) at from about 3 wt% to about 4 wt% of the total weight of the powder composition.
91. The powder composition of any one of embodiments 78-90, wherein R1 is linear hexadecyl.
92. The powder composition of embodiment 1, comprising (a) about 0.5 wt% of the compound of Formula (I), wherein R1 is linear hexadecyl, (b) about 29 wt% to about 30 wt% leucine, and the balance being (c) mannitol.
93. The powder composition of embodiment 1, comprising (a) about 1 wt% of the compound of Formula (I), wherein R1 is linear hexadecyl, (b) about 29 wt% to about 30 wt% leucine, and the balance being (c) mannitol.
94. The powder composition of embodiment 1, comprising (a) about 1.5 wt% of the compound of Formula (I), wherein R1 is linear hexadecyl, (b) about 29 wt% to about 30 wt% leucine, and the balance being (c) mannitol.
95. The powder composition of embodiment 1, comprising (a) about 2 wt% of the compound of Formula (I), wherein R1 is linear hexadecyl, (b) about 29 wt% to about 30 wt% leucine, and the balance being (c) mannitol.
96. The powder composition of embodiment 1, comprising (a) about 3 wt% of the compound of Formula (I), wherein R1 is linear hexadecyl, (b) about 29 wt% to about 30 wt% leucine, and the balance being (c) mannitol.
97. The powder composition of embodiment 1, comprising (a) about 4 wt% of the compound of Formula (I), wherein R1 is linear hexadecyl, (b) about 29 wt% to about 30 wt% leucine, and the balance being (c) mannitol.
98. The powder composition of embodiment 1, comprising (a) about 0.5 wt% of the compound of Formula (I), wherein R1 is linear hexadecyl, (b) about 60 wt% to about 61 wt% leucine, and the balance being (c) mannitol.
99. The powder composition of embodiment 1, comprising (a) about 1 wt% of the compound of Formula (I), wherein R1 is linear hexadecyl, (b) about 60 wt% to about 61 wt% leucine, and the balance being (c) mannitol.
100. The powder composition of embodiment 1, comprising (a) about 1.5 wt% of the compound of Formula (I), wherein R1 is linear hexadecyl, (b) about 60 wt% to about 61 wt% leucine, and the balance being (c) mannitol.
101. The powder composition of embodiment 1, comprising (a) about 2 wt% of the compound of Formula (I), wherein R1 is linear hexadecyl, (b) about 60 wt% to about 61 wt% leucine, and the balance being (c) mannitol.
102. The powder composition of embodiment 1, comprising (a) about 3 wt% of the compound of Formula (I), wherein R1 is linear hexadecyl, (b) about 60 wt% to about 61 wt% leucine, and the balance being (c) mannitol.
103. The powder composition of embodiment 1, comprising (a) about 4 wt% of the compound of Formula (I), wherein R1 is linear hexadecyl, (b) about 60 wt% to about 61 wt% leucine, and the balance being (c) mannitol.
104. The powder composition of embodiment 1, comprising (a) about 0.5 wt% of the compound of Formula (I), wherein R1 is linear hexadecyl, (b) about 43 wt% to about 45 wt% leucine, and the balance being (c) mannitol.
105. The powder composition of embodiment 1, comprising (a) about 1 wt% of the compound of Formula (I), wherein R1 is linear hexadecyl, (b) about 43 wt% to about 45 wt% leucine, and the balance being (c) mannitol.
106. The powder composition of embodiment 1, comprising (a) about 1.5 wt% of the compound of Formula (I), wherein R1 is linear hexadecyl, (b) about 43 wt% to about 45 wt% leucine, and the balance being (c) mannitol.
107. The powder composition of embodiment 1, comprising (a) about 2 wt% of the compound of Formula (I), wherein R1 is linear hexadecyl, (b) about 43 wt% to about 45 wt% leucine, and the balance being (c) mannitol.
108. The powder composition of embodiment 1, comprising (a) about 3 wt% of the compound of Formula (I), wherein R1 is linear hexadecyl, (b) about 43 wt% to about 45 wt% leucine, and the balance being (c) mannitol.
109. The powder composition of embodiment 1, comprising (a) about 4 wt% of the compound of Formula (I), wherein R1 is linear hexadecyl, (b) about 43 wt% to about 45 wt% leucine, and the balance being (c) mannitol.
110. The powder composition of any one of embodiments 1-109, wherein the leucine is L- leucine.
111. The powder composition of any one of embodiments 1-110, comprising from about 80 µg to about 1280 µg of the compound of Formula (I).
112. The powder composition of any one of embodiments 1-110, comprising from about 80 µg to about 640 µg of the compound of Formula (I).
113. The powder composition of any one of embodiments 1-110, comprising from about 80 µg to about 360 µg of the compound of Formula (I).
114. The powder composition of any one of embodiments 111-113, comprising from about 80 µg to about 320 µg of the compound of Formula (I).
115. The powder composition of any one of embodiments 111-113, comprising from about 160 µg to about 320 µg of the compound of Formula (I).
116. The powder composition of any one of embodiments 111-113, comprising about 80 µg of the compound of Formula (I).
117. The powder composition of any one of embodiments 111-113, comprising about 160 µg of the compound of Formula (I).
118. The powder composition of any one of embodiments 111-113, comprising about 240 µg of the compound of Formula (I).
119. The powder composition of any one of embodiments 111-113, comprising about 320 µg of the compound of Formula (I).
120. The powder composition of embodiment 111 or 112, comprising about 400 µg of the compound of Formula (I).
121. The powder composition of embodiment 111 or 112, comprising about 480 µg of the compound of Formula (I).
122. The powder composition of embodiment 111 or 112, comprising about 640 µg of the compound of Formula (I).
123. The powder composition of embodiment 111 or 112, comprising about 720 µg of the compound of Formula (I).
124. The powder composition of embodiment 111 or 112, comprising about 800 µg of the compound of Formula (I).
125. The powder composition of embodiment 111 or 112, comprising about 880 µg of the compound of Formula (I).
126. The powder composition of embodiment 111 or 112, comprising about 960 µg of the compound of Formula (I).
127. The powder composition of embodiment 111 or 112, comprising about 1040 µg of the compound of Formula (I).
128. The powder composition of embodiment 111 or 112, comprising about 1120 µg of the compound of Formula (I).
129. The powder composition of embodiment 111 or 112, comprising about 1200 µg of the compound of Formula (I).
130. The powder composition of embodiment 111 or 112, comprising about 1280 µg of the compound of Formula (I).
131. The powder composition of any one of embodiments 1-130, wherein the composition comprises from about 160 µg to about 320 µg of a compound of Formula (I), wherein R1 is linear hexadecyl, and upon once daily inhalation administration via a dry powder inhaler, the dry powder inhaler provides an aerosol comprising a fine particle fraction (FPF) greater than or equal to about 60%, as measured using a Next Generation Impactor (NGI).
132. The powder composition of any one of embodiments 1-131, wherein the composition comprises about 160 µg of a compound of Formula (I), wherein R1 is linear hexadecyl, and upon once daily inhalation administration via a dry powder inhaler, the dry powder inhaler provides an aerosol comprising the FPF that ranges from 73% to about 80%.
133. The powder composition of any one of embodiments 1-131, wherein the composition comprises about 320 µg of a compound of Formula (I), wherein R1 is linear hexadecyl, and upon once daily inhalation administration via a dry powder inhaler, the dry powder inhaler provides an aerosol comprising the FPF that ranges from 63% to about 73%.
134. The powder composition of any one of embodiments 1-133, wherein the composition comprises from about 160 µg to about 320 µg of a compound of Formula (I), wherein R1 is linear hexadecyl, and upon once daily inhalation administration via a dry powder inhaler, the dry powder inhaler provides an aerosol comprising a fine particle dose (FPD) greater than or equal to 50% of a loaded dose of the powder composition.
135. The powder composition of any one of embodiments 1-134, wherein the composition comprises about 160 µg of a compound of Formula (I), wherein R1 is linear hexadecyl, and upon once daily inhalation administration via a dry powder inhaler, the dry powder inhaler provides an aerosol comprising the FPD that ranges from 55% to about 70%.
136. The powder composition of any one of embodiments 1-134, wherein the composition comprises about 320 µg of a compound of Formula (I), wherein R1 is linear hexadecyl, and upon once daily inhalation administration via a dry powder inhaler, the dry powder inhaler provides an aerosol comprising the FPD that ranges from 50% to about 70%.
137. The powder composition of any one of embodiments 1-136, wherein the composition comprises from about 160 µg to about 320 µg of a compound of Formula (I), wherein R1 is linear hexadecyl, and wherein upon once daily inhalation administration via a dry powder inhaler, the dry powder inhaler provides an aerosol comprising an emitted dose (ED) greater than or equal to 70%.
138. The powder composition of any one of embodiments 1-137, wherein the composition comprises from about 160 µg to about 320 µg of a compound of Formula (I), wherein R1 is linear hexadecyl, and wherein upon once daily inhalation administration via a dry powder inhaler, the dry powder inhaler provides an aerosol comprising the ED that ranges from about 75% to about 90%.
139. The powder composition of any one of embodiments 1-138, wherein the composition comprises from about 160 µg to about 320 µg of a compound of Formula (I), wherein R1 is linear hexadecyl, and wherein upon once daily inhalation administration via a dry powder inhaler, the dry powder inhaler provides an aerosol comprising a mass mean aerodynamic diameter (MMAD) less than 5 µm.
140. The powder composition of any one of embodiments 1-139, wherein the composition comprises about 160 µg of a compound of Formula (I), wherein R1 is linear hexadecyl, and upon once daily inhalation administration via a dry powder inhaler, the dry powder inhaler provides an aerosol comprising the MMAD that ranges from about 1.0 µm to about 1.3 µm.
141. The powder composition of any one of embodiments 1-139, wherein the composition comprises about 320 µg of a compound of Formula (I), wherein R1 is linear hexadecyl, and upon once daily inhalation administration via a dry powder inhaler, the dry powder inhaler provides an aerosol comprising the MMAD that ranges from about 1.3 µm to about 1.7 µm.
142. A method of treating pulmonary hypertension (PH) in a patient in need thereof, comprising administering once daily to the patient an effective amount of the powder composition of any one of claims 1-141 to the lungs of the patient via inhalation with a dry powder inhaler (DPI), once daily during an administration period.
143. The method of embodiment 142, wherein administering comprises (i) aerosolizing the powder composition via the DPI to provide an aerosolized powder composition, and (ii) administering the aerosolized powder composition to the lungs of the patient via inhalation with the DPI.
144. The method of embodiment 142 or 143, wherein the effective amount of the powder composition comprises about 80 µg to 1280 µg of the compound of Formula (I).
145. The method of embodiment 142 or 143, wherein the effective amount of the powder composition is 80 µg, 160 µg, 240 µg, 320 µg, 400 µg, 480 µg, 640 µg, 720 µg, 800 µg, 880 µg, 960 µg, 1040 µg, 1120 µg, 1200 µg, or 1280 µg of the compound of Formula (I).
146. The method of embodiment 142 or 143, wherein the effective amount of the powder composition comprises 80 µg to 640 µg of the compound of Formula (I).
147. The method of claims 142 or 143, wherein the effective amount of the powder composition comprises 640 µg to 1280 µg of the compound of Formula (I).
148. The method of embodiment 142 or 143, wherein the effective amount of the powder composition comprises about 80 µg to about 320 µg of the compound of Formula (I).
149. The method of claims 142 or 143, wherein the effective amount of the powder composition comprises 400 µg, 480 µg, 640 µg, 720 µg, 800 µg, 880 µg, 960 µg, 1040 µg, 1120 µg, 1200 µg, or 1280 µg of the compound of Formula (I).
150. The method of any one of embodiments 142-149, wherein the patient is administered two or more different dosages of the compound of Formula (I) during the administration period.
151. The method of embodiment 150, wherein the patient is administered two different dosages of the compound of Formula (I) during the administration period.
152. The method of embodiment 150, wherein the patient is administered three different dosages of the compound of Formula (I) during the administration period.
153. The method of embodiment 150, wherein the patient is administered four different dosages of the compound of Formula (I) during the administration period.
154. The method of embodiment 150, wherein the patient is administered five different dosages of the compound of Formula (I) during the administration period.
155. The method of any one of embodiments 150-154, wherein prior to receiving a higher dosage of the compound of Formula (I), or a pharmaceutically acceptable salt thereof, the patient is administered a lower dosage for two or more consecutive days.
156. The method of any one of embodiments 150-154, wherein prior to receiving a higher dosage of the compound of Formula (I), or a pharmaceutically acceptable salt thereof, the patient is administered a lower dosage for three or more consecutive days.
157. The method of any one of embodiments 142-156, wherein the PH is group 1 PH, as classified by the World Health Organization (WHO).
158. The method of any one of embodiments 142-156, wherein the PH is group 2 PH, as classified by the WHO.
159. The method of any one of embodiments 142-156, wherein the PH is group 3 PH, as classified by the WHO.
160. The method of any one of embodiments 142-156, wherein the PH is group 4 PH, as classified by the WHO.
161. The method of any one of embodiments 142-156, wherein the PH is group 5 PH, as classified by the WHO.
162. The method of any one of embodiments 142-156, wherein the PH is pulmonary arterial hypertension (PAH).
163. The method of embodiment 162, wherein the pulmonary arterial hypertension is class I pulmonary arterial hypertension, as characterized by the New York Heart Association (NYHA).
164. The method of embodiment 162, wherein the pulmonary arterial hypertension is class II pulmonary arterial hypertension, as characterized by the NYHA.
165. The method of embodiment 162, wherein the pulmonary arterial hypertension is class III pulmonary arterial hypertension, as characterized by the NYHA.
166. The method of embodiment 162, wherein the pulmonary arterial hypertension is class IV pulmonary arterial hypertension, as characterized by the NYHA.
167. The method of any one of embodiments 142-161, wherein the PH is portopulmonary hypertension (PPH).
168. The method of any one of embodiments 142-161, wherein the PH is PH associated with interstitial lung disease (ILD).
169. The method of embodiment 168, wherein the ILD comprises one or more lung conditions selected from the group consisting of idiopathic pulmonary fibrosis (IPF), cryptogenic organizing pneumonia (COP), desquamative interstitial pneumonitis, nonspecific interstitial pneumonitis, hypersensitivity pneumonitis, acute interstitial pneumonitis, interstitial pneumonia, connective tissue disease, sarcoidosis or asbestosis.
170. The method of embodiment 168, wherein the ILD is idiopathic interstitial pneumonia (IIP).
171. The method of embodiment 168, wherein the ILD is sarcoidosis.
172. The method of embodiment 168, wherein the ILD is connective tissue disease-associated interstitial lung disease (CTD-ILD).
173. The method of embodiment 168, wherein the ILD is idiopathic pulmonary fibrosis (IPF).
174. The method of any one of embodiments 142-173, wherein treating comprises reducing the pulmonary vascular index (PVRI) of the patient during the administration period, compared to the patient's PVRI prior to the administration period.
175. The method of any one of embodiments 142-174, wherein treating comprises reducing the mean pulmonary artery pressure of the patient during the administration period, compared to the mean pulmonary artery pressure of the patient prior to the administration period.
176. The method of any one of embodiments 142-175, wherein treating comprises increasing the hypoxemia score of the patient during the hypoxemia score of the patient prior to the administration period.
177. The method of any one of embodiments 142-176, wherein decreasing the oxygenation index of the patient during the administration period, compared to the oxygenation index of the patient prior to the administration period.
178. The method of any one of embodiments 142-177, wherein treating comprises improving the right heart function of the patient during the administration period, compared to the right heart function of the patient prior to the administration period.
179. The method of any one of embodiments 142-178, wherein treating comprises improving exercise capacity of the patient during the administration period, compared to the exercise capacity of the patient prior to the administration period.
180. The method of embodiment 179, wherein exercise capacity is measured by the six-minute walk test (6MWT).
181. The method of embodiment 180, wherein improving exercise capacity comprises increasing the patient's distance walked in the 6MWT by at least about 5 meters, at least about 10 meters, at least about 20 meters, at least about 30 meters, at least about 40 meters, or at least about 50 meters during the administration period, compared to the patient's distance walked in the 6MWT prior to the administration period.
182. The method of embodiment 180, wherein improving exercise capacity comprises increasing the patient's distance walked in the 6MWT by from about 5 meters to about 60 meters, by from about 5 meters to about 50 meters, by from about 10 meters to about 50 meters, by from about 15 meters to about 50 meters, or by from about 20 meters to about 40 meters, during the administration period, compared to the patient's distance walked in the 6MWT prior to the administration period.
183. The method of any one of embodiments 142-182, wherein treating comprises improving the quality of life of the patient during the administration period, compared to the quality of life of the patient prior to the administration period.
184. The method of embodiment 183, wherein the quality of life of the patient is measured by the Cambridge Pulmonary Hypertension Outcome Review (CAMPHOR) Questionnaire.
185. The method of embodiment 184, wherein treating comprises decreasing the patient's CAMPHOR Questionnaire score during the administration period by from 1 to about 10, from 1 to about 9, from 1 to 8, from 1 to 7, from 1 to 6, from 1 to 5, from 1 to 4, from 1 to 3 or from 1 to 2, compared to the patient's CAMPHOR Questionnaire score prior to the administration period.
186. The method of any one of embodiments 142-185, wherein treating comprises increasing the patient's saturation of peripheral capillary oxygenation (SpCh) at rest assessed by pulse oximetry, during the administration period, compared to the patient's SpCh at rest assessed by pulse oximetry prior to the administration period.
187. The method of any one of embodiments 142-185, wherein treating comprises improving the patient's lung function during the administration period, as compared to the patient's lung function prior to the administration period.
188. The method of embodiment 187, wherein improving the patient's lung function comprises increasing the patient's forced vital capacity (FVC) during the administration period, compared to the patient's PVC prior to the administration period.
189. The method of embodiment 187, wherein improving the patient's lung function comprises increasing the patient's percent predicted forced vital capacity (ppFVC) during the administration period, compared to the patient's ppFVC prior to the administration period.
190. The method of embodiment 187, wherein improving the patient's lung function comprises increasing the patient's the patient's forced expiratory volume in 1 second (FEV₁) during the administration period, compared to the patient's FEV₁ prior to the administration period.
191. The method of embodiment 190, wherein increasing the patient's the patient's forced expiratory volume in 1 second (FEV₁) during the administration period comprises increasing the patient's FEVI by from about 5% to about 50%, from about 5% to about 40% or from about 5% to about 30%, compared to the patient's FEV₁ prior to the administration period.
192. The method of embodiment 190, wherein increasing the FEV₁ comprises increasing by about 5%, about 10%, about 15%, by about 20%, by about 25%, by about 30%, by about 35%, by about 40%, by about 45% or by about 50%.
193. The method of embodiment 190, wherein increasing the FEV₁ comprises increasing by at least about 5%, at least about 10%, at least about 15%, by at least about 20%, by at least about 25%, by at least about 30%, by at least about 35%, by at least about 40%, by at least about 45% or by at least about 50%.
194. The method of embodiment 190, wherein increasing the FEV₁ comprises increasing by about 5% to about 50%, by about 5% to about 40%, by about 5% to about 30%, by about 5% to about 20%, by about 10% to about 50%, by about 15% to about 50%, by about 20% to about 50% or by about 25% to about 50%.
195. The method of embodiment 190, wherein increasing the FEV₁ comprises increasing by of at least about 5%.
196. The method of embodiment 190, wherein increasing the FEV₁ comprises increasing by from about 5% to about 50%, or from about 10% to about 50%, or from about 15% to about 50%.
197. The method of embodiment 190, wherein increasing the FEV₁ comprises increasing by about 25 mL to about 500 mL.
198. The method of embodiment 190, wherein increasing the FEV₁ comprises increasing by about 25 mL to about 250 mL.
199. The method of embodiment 188, wherein increasing the patient's forced vital capacity (FVC) during the administration period comprises increasing the patient's FVC by about 1%, by about 2%, by about 3%, by about 4%, by about 5%, by about 6%, by about 7%, by about 8%, by about 9%, by about 10%, by about 11%, by about 12%, by about 13%, by about 14%, by about 15%, by about 16%, by about 17%, by about 18%, by about 19%, by about 20%, by about 25%, by about 30%, by about 35%, by about 40%, by about 45%, by about 50%, by about 55%, by about 60%, by about 65%, by about 70%, by about 75%, by about 80%, by about 85% or by about 90%, as compared to a FVC of the patient prior to the administration period.
200. The method of embodiment 188, wherein increasing the patient's forced vital capacity (FVC) during the administration period comprises increasing the patient's FVC by from about 1% to about 20%, from about 1% to about 15%, from about 1% to about 10%, from about 1% to about 5%, from about 5% to about 50%, from about 5% to about 40%, from about 5% to about 30%, from about 5% to about 20%, from about 10% to about 50%, from about 15% to about 50%, from about 20% to about 50%, or from about 25% to about 50%, as compared to a FVC of the patient prior to the administration period.
201. The method of embodiment 187, wherein improving the patient's lung function comprises increasing the patient's total lung capacity (TLC) during the administration period, compared to the patient's TLC prior to the administration period.
202. The method of embodiment 201, wherein increasing the patient's total lung capacity (TLC) comprises increasing the patient's TLC by at least about 1%, at least about 2%, at least about 5%, at least about 10%, at least about 15%, at least about 20%, at least about 25%, at least about 30%, at least about 35%, at least about 40%, at least about 45%, or by at least about 50% during the administration period, compared to the patient's TLC prior to the administration period.
203. The method of embodiment 201, wherein increasing the patient's total lung capacity (TLC) comprises increasing the patient's TLC by from about 1% to about 50%, by from about 5% to about 50%, by from about 5% to about 40%, by from about 5% to about 30%, by from about 5% to about 20%, by from about 10% to about 50%, by from about 15% to about 50%, by from about 20% to about 50%, or by from about 25% to about 50%.during the administration period, compared to the patient's TLC prior to the administration period.
204. The method of any one of embodiments 142-203, wherein the administration period is from about 1 year to about 30 years.
205. The method of embodiment 204, wherein the administration period is from about 1 year to about 25 years.
206. The method of embodiment 204, wherein the administration period is from about 5 years to about 30 years.
207. The method of embodiment 204, wherein the administration period is from about 1 year to about 20 years.
208. The method of embodiment 204, wherein the administration period is from about 1 year to about 15 years.
209. The method of embodiment 204, wherein the administration period is from about 1 year to about 10 years.
210. The method of embodiment 204, wherein the administration period is from about 1 year to about 5 years.
211. The method of any one of embodiments 142-210, wherein the dry powder inhaler (DPI) is a capsule-based DPI, and the composition is present in a single DPI capsule.
212. The method of any one of embodiments 142-210, wherein the dry powder inhaler (DPI) is a capsule-based DPI, and the composition is split between two DPI capsules.
213. A system for treating pulmonary hypertension, comprising (i) a powder composition and (ii) a dry powder inhaler,
the powder comprising:
(a) from about 0.5 wt% to about 5 wt% of a compound of Formula (I) 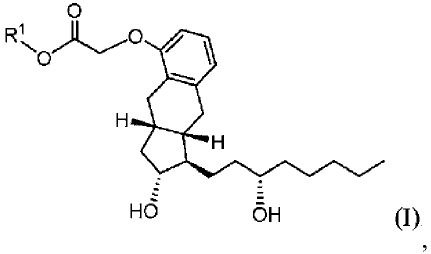 or stereoisomer or a pharmaceutically acceptable salt thereof, wherein R1 is tetradecyl, pentadecyl, hexadecyl, heptadecyl, or octadecyl,
(b) from about 10 wt% to about 61wt% leucine, and
(c) trehalose, mannitol or a combination thereof,

wherein the sum of (a), (b), and (c) is 100%; and
wherein the dry powder inhaler provides an aerosol comprising fine particle fraction (FPF) greater than or equal to about 60%, as measured using a Next Generation Impactor (NGI), with a inspiratory flow rate of 30-60 liter per minute (L/min), inspiratory volume of 1-4 L, and inhalation acceleration rate of 0.5-20 L/sec².
214. The system of embodiment 213, wherein the dry powder inhaler is a high resistance dry powder inhaler.
215. A powder of embodiment 1, wherein the powder composition is a spray-dried powder composition.
216. A method of embodiment 142, wherein the powder composition is a spray-dried powder composition.
217. A system of embodiment 213, wherein the powder composition is a spray-dried powder composition.
218. The powder, method or system of any one of embodiments 1-217, wherein the inspiratory flow rate is 30 liter per minute (L/min).
219. The powder, method or system of any one of embodiments 1-217, wherein the inspiratory flow rate is 40 liter per minute (L/min).
220. The powder, method or system of any one of embodiments 1-217, wherein the inspiratory flow rate is 60 liter per minute (L/min).
221. The powder, method or system of any one of embodiments 1-220, wherein the dry powder inhaler has a resistance ranging from about 0.030 to about 0.040 kPA^{1/2} (L/min⁻¹)⁻¹.
222. The powder, method or system of any one of embodiments 1-220, wherein the dry powder inhaler has a resistance ranging from about 0.034 to about 0.036 kPA^{1/2} (L/min⁻¹)⁻¹.

While the described disclosure has been described with reference to the specific embodiments thereof it should be understood by those skilled in the art that various changes may be made and equivalents may be substituted without departing from the true spirit and scope of the disclosure. In addition, many modifications may be made to adopt a particular situation, material, composition of matter, process, process step or steps, to the objective spirit and scope of the described disclosure. All such modifications are intended to be within the scope of the claims appended hereto.

Patents, patent applications, patent application publications, journal articles and protocols referenced herein are incorporated by reference in their entireties, for all purposes. However, mention of any reference, article, publication, patent, patent publication, and patent application cited herein is not, and should not be taken as acknowledgment or any form of suggestion that they constitute valid prior art or form part of the common general knowledge in any country in the world.
The invention is further defined in the following numbered clauses:
1. A powder composition comprising:
(a) about 0.5 wt% to about 5 wt% of a compound of Formula (I) 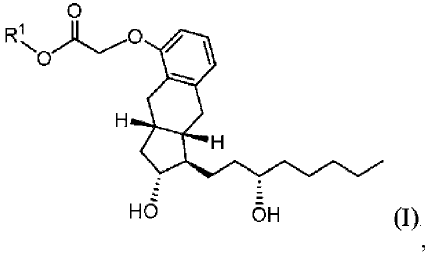 , or stereoisomer or a pharmaceutically acceptable salt thereof, wherein R1 is tetradecyl, pentadecyl, hexadecyl, heptadecyl, or octadecyl,
(b) about 10 wt% to about 61wt% leucine, and
(c) trehalose, mannitol or a combination thereof,

wherein the sum of (a), (b), and (c) is 100%; and
when administered via a dry powder inhaler, the dry powder inhaler provides an aerosol comprising a fine particle fraction (FPF) greater than or equal to about 68%, when measured using a Next Generation Impactor (NGI), with a inspiratory flow rate of 60 liter per minute (L/min), inspiratory volume of 4 L, and inhalation acceleration rate of 0.5-20 L/sec².
2. The powder composition of clause 1, wherein the FPF ranges from 68% to about 73%.
3. The powder composition of clause 1, wherein the FPF ranges from 78% to about 80%.
4. The powder composition of any one of clauses 1-3, wherein the aerosol comprises a fine particle dose (FPD) greater than or equal to 50% of a loaded dose of the powder composition.
5. The powder composition of any one of clauses 1-4, wherein the FPD ranging from about 53% to about 77%.
6. The powder composition of any one of clauses 1-4, wherein the FPD ranging from about 60% to about 65%.
7. The powder composition of any one of clauses 1-6, wherein the aerosol comprises an emitted dose (ED) greater than or equal to 72%.
8. The powder composition of clause 7, wherein the ED ranges from about 79% to about 89%.
9. The powder composition of any one of clauses 1-8, wherein the aerosol comprises a mass mean aerodynamic diameter (MMAD) less than 5 µm.
10. The powder composition of clause 9, wherein the MMAD ranges from about 0.75 µm to about 3.0 µm.
11. The powder composition of clause 9, wherein the MMAD ranges from about 0.8 µm to about 2.0 µm.
12. The powder composition of any one of clauses 1-11, comprising particles, at least a portion of the particles comprising:
(i) a compound of Formula (I) or stereoisomer or a pharmaceutically acceptable salt,
(ii) leucine, and
(iii) trehalose, mannitol or a combination thereof,
wherein the particles have an average diameter ranging from about 1.0 µm to about 2.0 µm.
13. The powder composition of clause 12, wherein the particles have a D10 of about 0.3 µm.
14. The powder composition of clause 12, wherein the particles have a D50 of about 1.7 µm.
15. The powder composition of clause 12, wherein the particles have a D90 of about 3.8 µm.
16. The powder composition of clause 1, wherein R1 is hexadecyl.
17. The powder composition of clause 16, wherein R1 is linear hexadecyl.
18. The powder composition of clause 1, wherein R1 is tetradecyl.
19. The powder composition of clause 18, wherein R1 is linear tetradecyl.
20. The powder composition of clause 1, wherein R1 is pentadecyl.
21. The powder composition of clause 20, wherein R1 is linear pentadecyl.
22. The powder composition of clause 1, wherein R1 is hexadecyl.
23. The powder composition of clause 22, wherein R1 is linear hexadecyl.
24. The powder composition of clause 1, wherein R1 is heptadecyl.
25. The powder composition of clause 24, wherein R1 is linear heptadecyl.
26. The powder composition of clause 1, wherein R1 is octadecyl.
27. The powder composition of clause 26, wherein R1 is linear octadecyl.
28. The powder composition of any one of clauses 1-27, wherein the compound of Formula (I), or a pharmaceutically acceptable salt thereof, is present at from about 0.5 wt% to about 4.5 wt% of the total weight of the powder composition.
29. The powder composition of clause 28, wherein the compound of Formula (I), or a pharmaceutically acceptable salt thereof, is present at from about 1 wt% to about 4.5 wt% of the total weight of the powder composition.
30. The powder composition of clause 28, wherein the compound of Formula (I), or a pharmaceutically acceptable salt thereof, is present at from about 1 wt% to about 4 wt% of the total weight of the powder composition.
31. The powder composition of clause 28, wherein the compound of Formula (I), or a pharmaceutically acceptable salt thereof, is present at from about 1 wt% to about 3.5 wt% of the total weight of the powder composition.
32. The powder composition of clause 28, wherein the compound of Formula (I), or a pharmaceutically acceptable salt thereof, is present at from about 1 wt% to about 3 wt% of the total weight of the powder composition.
33. The powder composition of clause 28, wherein the compound of Formula (I), or a pharmaceutically acceptable salt thereof, is present at from about 1 wt% to about 2.5 wt% of the total weight of the powder composition.
34. The powder composition of clause 28, wherein the compound of Formula (I), or a pharmaceutically acceptable salt thereof, is present at from about 1 wt% to about 2 wt% of the total weight of the powder composition.
35. The powder composition of clause 28, wherein the compound of Formula (I), or a pharmaceutically acceptable salt thereof, is present at from about 1 wt% to about 1.5 wt% of the total weight of the powder composition.
36. The powder composition of clause 28, wherein the compound of Formula (I), or a pharmaceutically acceptable salt thereof is present at from about 2 wt% to about 4 wt% of the total weight of the powder composition.
37. The powder composition of clause 28, wherein the compound of Formula (I), or a pharmaceutically acceptable salt thereof, is present at from about 2 wt% to about 3.5 wt% of the total weight of the powder composition.
38. The powder composition of clause 28, wherein the compound of Formula (I), or a pharmaceutically acceptable salt thereof, is present at from about 2 wt% to about 3 wt% of the total weight of the powder composition.
39. The powder composition of clause 28, wherein the compound of Formula (I), or a pharmaceutically acceptable salt thereof, is present at from about 2 wt% to about 2.5 wt% of the total weight of the powder composition.
40. The powder composition of clause 28, wherein the compound of Formula (I), or a pharmaceutically acceptable salt thereof, is present at from about 3 wt% to about 4.5 wt% of the total weight of the powder composition.
41. The powder composition of clause 28, wherein the compound of Formula (I), or a pharmaceutically acceptable salt thereof, is present at from about 3 wt% to about 4 wt% of the total weight of the powder composition.
42. The powder composition of clause 28, wherein the compound of Formula (I), or a pharmaceutically acceptable salt thereof, is present at from about 3 wt% to about 3.5 wt% of the total weight of the powder composition.
43. The powder composition of clause 28, wherein the compound of Formula (I), or a pharmaceutically acceptable salt thereof, is present at from about 0.5 wt% to about 2 wt% of the total weight of the powder composition.
44. The powder composition of clause 28, wherein the compound of Formula (I), or a pharmaceutically acceptable salt thereof, is present at from about 0.5 wt% to about 1.5 wt% of the total weight of the powder composition.
45. The powder composition of clause 28, wherein the compound of Formula (I), or a pharmaceutically acceptable salt thereof, is present at from about 0.5 wt% to about 1 wt% of the total weight of the powder composition.
46. The powder composition of clause 28, wherein the compound of Formula (I), or a pharmaceutically acceptable salt thereof, is present at from about 3.5 wt% to about 4.5 wt% of the total weight of the powder composition.
47. The powder composition of clause 28, wherein the compound of Formula (I), or a pharmaceutically acceptable salt thereof, is present at from about 4 wt% to about 4.5 wt% of the total weight of the powder composition.
48. The powder composition of clause 28, wherein the compound of Formula (I), or a pharmaceutically acceptable salt thereof is present at from about 0.5 wt% of the total weight of the powder composition.
49. The powder composition of clause 28, wherein the compound of Formula (I), or a pharmaceutically acceptable salt thereof, is present at about 1 wt% of the total weight of the powder composition.
50. The powder composition of clause 28, wherein the compound of Formula (I), or a pharmaceutically acceptable salt thereof, is present at about 1.5 wt% of the total weight of the powder composition.
51. The powder composition of clause 28, wherein the compound of Formula (I), or a pharmaceutically acceptable salt thereof, is present at about 2 wt% of the total weight of the powder composition.
52. The powder composition of clause 28, wherein the compound of Formula (I), or a pharmaceutically acceptable salt thereof, is present at about 2.5 wt% of the total weight of the powder composition.
53. The powder composition of clause 28, wherein the compound of Formula (I), or a pharmaceutically acceptable salt thereof, is present at about 3 wt% of the total weight of the powder composition.
54. The powder composition of clause 28, wherein the compound of Formula (I), or a pharmaceutically acceptable salt thereof, is present at about 3.5 wt% of the total weight of the powder composition.
55. The powder composition of clause 28, wherein the compound of Formula (I), or a pharmaceutically acceptable salt thereof, is present at about 4 wt% of the total weight of the powder composition.
56. The powder composition of any one of clauses 1-55, wherein the compound of Formula (I), or a pharmaceutically acceptable salt thereof, is a compound of Formula (II).
57. The powder composition of any one of clauses 1-56, wherein the leucine is present at from about 25 wt% to about 61 wt% of the total weight of the powder composition.
58. The powder composition of any one of clauses 1-56, wherein the leucine is present at from about 40 wt% to about 61 wt% of the total weight of the powder composition.
59. The powder composition of any one of clauses 1-56, wherein the leucine is present at from about 50 wt% to about 61 wt% of the total weight of the powder composition.
60. The powder composition of any one of clauses 1-56, wherein the leucine is present at from about 55 wt% to about 61 wt% of the total weight of the powder composition.
61. The powder composition of any one of clauses 1-56, wherein the leucine is present at from about 58 wt% to about 61 wt% of the total weight of the powder composition.
62. The powder composition of any one of clauses 1-56, wherein the leucine is present at from about 40 wt% to about 45 wt% of the total weight of the powder composition.
63. The powder composition of any one of clauses 1-56, wherein the leucine is present at from about 44 wt% to about 51 wt% of the total weight of the powder composition.
64. The powder composition of any one of clauses 1-56, wherein the leucine is present at from about 43 wt% to about 48 wt% of the total weight of the powder composition.
65. The powder composition of any one of clauses 1-56, wherein the leucine is present at from about 25 wt% to about 30 wt% of the total weight of the powder composition.
66. The powder composition of any one of clauses 1-56, wherein the leucine is present at from about 28 wt% to about 30 wt% of the total weight of the powder composition.
67. The powder composition of any one of clauses 1-56, wherein the leucine is present at from about 25 wt% to about 33 wt% of the total weight of the powder composition.
68. The powder composition of any one of clauses 1-56, wherein the leucine is present at from about 27 wt% to about 33 wt% of the total weight of the powder composition.
69. The powder composition of any one of clauses 1-56, wherein the leucine is present at from about 27 wt% to about 31 wt% of the total weight of the powder composition.
70. The powder composition of any one of clauses 1-56, wherein the leucine is present at from about 27 wt% to about 30 wt% of the total weight of the powder composition.
71. The powder composition of any one of clauses 1-56, wherein the leucine is present at from about 28 wt% to about 30 wt% of the total weight of the powder composition.
72. The powder composition of any one of clauses 1-56, wherein the leucine is present at about 30 wt% of the total weight of the powder composition.
73. The powder composition of any one of clauses 1-56, wherein the leucine is present at about 45 wt% of the total weight of the powder composition.
74. The powder composition of any one of clauses 1-56, wherein the leucine is present at about 60 wt% of the total weight of the powder composition.
75. The powder composition of any one of clauses 1-74, wherein the sugar is trehalose.
76. The powder composition of any one of clauses 1-74, wherein the sugar is mannitol.
77. The powder composition of clause 1, selected from one of the following compositions:

| **TPIP composition #** | **Treprostinil palmitil (%w)** | **Leucine (%w)** | **Mannitol (%w)** | **Total composition (%w)** |
|---|---|---|---|---|
| **1** | 0.5 | 60.0 | 39.5 | 100 |
| **2** | 2.0 | 61.2 | 36.8 | 100 |
| **3** | 3.0 | 60.7 | 36.3 | 100 |
| **4** | 4.0 | 60.0 | 36.0 | 100 |
| **5** | 0.4 | 45.0 | 54.6 | 100 |
| **6** | 1.5 | 44.4 | 54.1 | 100 |
| **7** | 2.0 | 45.0 | 53.0 | 100 |
| **8** | 3.0 | 44.5 | 52.5 | 100 |
| **9** | 4.0 | 45.0 | 51.0 | 100 |
| **10** | 0.5 | 30.0 | 69.5 | 100 |
| **11** | 1.0 | 29.3 | 69.7 | 100 |
| **12** | 1.5 | 29.6 | 68.9 | 100 |
| **13** | 1.5 | 29.3 | 69.2 | 100 |
| **14** | 2.0 | 28.8 | 69.2 | 100 |
| **15** | 3.0 | 28.6 | 68.4 | 100 |
| **16** | 4.0 | 30.0 | 66.0 | 100 |

78. The powder composition of clause 1, having a leucine-to-mannitol weight ratio of about 0.40-to-1 (leucine-to-mannitol) to about 0.50-to-1 (leucine-to-mannitol).
79. The powder composition of clause 78, having a leucine-to-mannitol weight ratio of about 0.40-to-1 (leucine-to-mannitol) to about 0.45-to-1 (leucine-to-mannitol).
80. The powder composition of clause 1, having a leucine-to-mannitol weight ratio of about 0.75-to-1 (leucine-to-mannitol) to about 0.90-to-1 (leucine-to-mannitol).
81. The powder composition of clause 80, having a leucine-to-mannitol weight ratio of about 0.80-to-1 (leucine-to-mannitol) to about 0.90-to-1 (leucine-to-mannitol).
82. The powder composition of clause 1, having a leucine-to-mannitol weight ratio of about 1.5-to-1 (leucine-to-mannitol) to about 1.7-to-1 (leucine-to-mannitol).
83. The powder composition of clause 82, having a leucine-to-mannitol weight ratio of about 1.65-to-1 (leucine-to-mannitol) to about 1.7-to-1 (leucine-to-mannitol).
84. The powder composition of any one of clauses 78-83, comprising the compound of Formula (I) at from about 0.5 wt% to about 4 wt% of the total weight of the powder composition.
85. The powder composition of any one of clauses 78-83, comprising the compound of Formula (I) at from about 0.5 wt% to about 3 wt% of the total weight of the powder composition.
86. The powder composition of any one of clauses 78-83, comprising the compound of Formula (I) at from about 0.5 wt% to about 2 wt% of the total weight of the powder composition.
87. The powder composition of any one of clauses 78-83, comprising the compound of Formula (I) at from about 0.5 wt% to about 1.5 wt% of the total weight of the powder composition.
88. The powder composition of any one of clauses 78-87, comprising the compound of Formula (I) at from about 1 wt% to about 1.5 wt% of the total weight of the powder composition.
89. The powder composition of any one of clauses 78-87, comprising the compound of Formula (I) at from about 2 wt% to about 4 wt% of the total weight of the powder composition.
90. The powder composition of any one of clauses 78-87, comprising the compound of Formula (I) at from about 3 wt% to about 4 wt% of the total weight of the powder composition.
91. The powder composition of any one of clauses 78-90, wherein R1 is linear hexadecyl.
92. The powder composition of clause 1, comprising (a) about 0.5 wt% of the compound of Formula (I), wherein R1 is linear hexadecyl, (b) about 29 wt% to about 30 wt% leucine, and the balance being (c) mannitol.
93. The powder composition of clause 1, comprising (a) about 1 wt% of the compound of Formula (I), wherein R1 is linear hexadecyl, (b) about 29 wt% to about 30 wt% leucine, and the balance being (c) mannitol.
94. The powder composition of clause 1, comprising (a) about 1.5 wt% of the compound of Formula (I), wherein R1 is linear hexadecyl, (b) about 29 wt% to about 30 wt% leucine, and the balance being (c) mannitol.
95. The powder composition of clause 1, comprising (a) about 2 wt% of the compound of Formula (I), wherein R1 is linear hexadecyl, (b) about 29 wt% to about 30 wt% leucine, and the balance being (c) mannitol.
96. The powder composition of clause 1, comprising (a) about 3 wt% of the compound of Formula (I), wherein R1 is linear hexadecyl, (b) about 29 wt% to about 30 wt% leucine, and the balance being (c) mannitol.
97. The powder composition of clause 1, comprising (a) about 4 wt% of the compound of Formula (I), wherein R1 is linear hexadecyl, (b) about 29 wt% to about 30 wt% leucine, and the balance being (c) mannitol.
98. The powder composition of clause 1, comprising (a) about 0.5 wt% of the compound of Formula (I), wherein R1 is linear hexadecyl, (b) about 60 wt% to about 61 wt% leucine, and the balance being (c) mannitol.
99. The powder composition of clause 1, comprising (a) about 1 wt% of the compound of Formula (I), wherein R1 is linear hexadecyl, (b) about 60 wt% to about 61 wt% leucine, and the balance being (c) mannitol.
100. The powder composition of clause 1, comprising (a) about 1.5 wt% of the compound of Formula (I), wherein R1 is linear hexadecyl, (b) about 60 wt% to about 61 wt% leucine, and the balance being (c) mannitol.
101. The powder composition of clause 1, comprising (a) about 2 wt% of the compound of Formula (I), wherein R1 is linear hexadecyl, (b) about 60 wt% to about 61 wt% leucine, and the balance being (c) mannitol.
102. The powder composition of clause 1, comprising (a) about 3 wt% of the compound of Formula (I), wherein R1 is linear hexadecyl, (b) about 60 wt% to about 61 wt% leucine, and the balance being (c) mannitol.
103. The powder composition of clause 1, comprising (a) about 4 wt% of the compound of Formula (I), wherein R1 is linear hexadecyl, (b) about 60 wt% to about 61 wt% leucine, and the balance being (c) mannitol.
104. The powder composition of clause 1, comprising (a) about 0.5 wt% of the compound of Formula (I), wherein R1 is linear hexadecyl, (b) about 43 wt% to about 45 wt% leucine, and the balance being (c) mannitol.
105. The powder composition of clause 1, comprising (a) about 1 wt% of the compound of Formula (I), wherein R1 is linear hexadecyl, (b) about 43 wt% to about 45 wt% leucine, and the balance being (c) mannitol.
106. The powder composition of clause 1, comprising (a) about 1.5 wt% of the compound of Formula (I), wherein R1 is linear hexadecyl, (b) about 43 wt% to about 45 wt% leucine, and the balance being (c) mannitol.
107. The powder composition of clause 1, comprising (a) about 2 wt% of the compound of Formula (I), wherein R1 is linear hexadecyl, (b) about 43 wt% to about 45 wt% leucine, and the balance being (c) mannitol.
108. The powder composition of clause 1, comprising (a) about 3 wt% of the compound of Formula (I), wherein R1 is linear hexadecyl, (b) about 43 wt% to about 45 wt% leucine, and the balance being (c) mannitol.
109. The powder composition of clause 1, comprising (a) about 4 wt% of the compound of Formula (I), wherein R1 is linear hexadecyl, (b) about 43 wt% to about 45 wt% leucine, and the balance being (c) mannitol.
110. The powder composition of any one of clauses 1-109, wherein the leucine is L- leucine.
111. The powder composition of any one of clauses 1-110, comprising from about 80 µg to about 360 µg of the compound of Formula (I).
112. The powder composition of clause 111, comprising from about 80 µg to about 320 µg of the compound of Formula (I).
113. The powder composition of clause 111, comprising from about 160 µg to about 320 µg of the compound of Formula (I).
114. The powder composition of clause 111, comprising about 80 µg of the compound of Formula (I).
115. The powder composition of clause 111, comprising about 160 µg of the compound of Formula (I).
116. The powder composition of clause 111, comprising about 240 µg of the compound of Formula (I).
117. The powder composition of clause 111, comprising about 320 µg of the compound of Formula (I).
118. The powder composition of any one of clauses 1-117, wherein the composition comprises from about 160 µg to about 320 µg of a compound of Formula (I), wherein R1 is linear hexadecyl, and upon once daily inhalation administration via a dry powder inhaler, the dry powder inhaler provides an aerosol comprising a fine particle fraction (FPF) greater than or equal to about 68%, as measured using a Next Generation Impactor (NGI).
119. The powder composition of any one of clauses 1-118, wherein the composition comprises about 160 µg of a compound of Formula (I), wherein R1 is linear hexadecyl, and upon once daily inhalation administration via a dry powder inhaler, wherein the dry powder inhaler provides an aerosol comprising the FPF that ranges from 78% to about 80%.
120. The powder composition of any one of clauses 1-118, wherein the composition comprises about 320 µg of a compound of Formula (I), wherein R1 is linear hexadecyl, and upon once daily inhalation administration via a dry powder inhaler, the dry powder inhaler provides an aerosol comprising the FPF that ranges from 68% to about 73%.
121. The powder composition of any one of clauses 1-120, wherein the composition comprises from about 160 µg to about 320 µg of a compound of Formula (I), wherein R1 is linear hexadecyl, and upon once daily inhalation administration via a dry powder inhaler, the dry powder inhaler provides an aerosol comprising a fine particle dose (FPD) greater than or equal to 50% of a loaded dose of the powder composition.
122. The powder composition of any one of clauses 1-121, wherein the composition comprises about 160 µg of a compound of Formula (I), wherein R1 is linear hexadecyl, and upon once daily inhalation administration via a dry powder inhaler, the dry powder inhaler provides an aerosol comprising the FPD that ranges from 60% to about 65%.
123. The powder composition of any one of clauses 1-121, wherein the composition comprises about 320 µg of a compound of Formula (I), wherein R1 is linear hexadecyl, and upon once daily inhalation administration via a dry powder inhaler, the dry powder inhaler provides an aerosol comprising the FPD that ranges from 53% to about 65%.
124. The powder composition of any one of clauses 1-123, wherein the composition comprises from about 160 µg to about 320 µg of a compound of Formula (I), wherein R1 is linear hexadecyl, and upon once daily inhalation administration via a dry powder inhaler, the dry powder inhaler provides an aerosol comprising an emitted dose (ED) greater than or equal to 72%.
125. The powder composition of any one of clauses 1-124, wherein the composition comprises from about 160 µg to about 320 µg of a compound of Formula (I), wherein R1 is linear hexadecyl, and upon once daily inhalation administration via a dry powder inhaler, the dry powder inhaler provides an aerosol comprising the ED that ranges from about 79% to about 89%.
126. The powder composition of any one of clauses 1-125, wherein the composition comprises from about 160 µg to about 320 µg of a compound of Formula (I), wherein R1 is linear hexadecyl, and upon once daily inhalation administration via a dry powder inhaler, the dry powder inhaler provides an aerosol comprising a mass mean aerodynamic diameter (MMAD) less than 5 µm.
127. The powder composition of any one of clauses 1-126, wherein the composition comprises about 160 µg of a compound of Formula (I), wherein R1 is linear hexadecyl, and upon once daily inhalation administration via a dry powder inhaler, the dry powder inhaler provides an aerosol comprising the MMAD that ranges from about 1.0 µm to about 1.3 µm.
128. The powder composition of any one of clauses 1-126, wherein the composition comprises about 320 µg of a compound of Formula (I), wherein R1 is linear hexadecyl, and upon once daily inhalation administration via a dry powder inhaler, the dry powder inhaler provides an aerosol comprising the MMAD that ranges from about 1.3 µm to about 1.7 µm.
129. A method of treating pulmonary hypertension (PH) in a patient in need thereof, comprising administering to the lungs of the patient, via inhalation with a dry powder inhaler (DPI), once daily during an administration period, an effective amount of the powder composition of any one of clauses 1-128.
130. The method of clause 129, wherein administering comprises (i) aerosolizing the powder composition via the DPI to provide an aerosolized powder composition, and (ii) administering the aerosolized powder composition to the lungs of the patient via inhalation with the DPI.
131. The method of clause 129 or 130, wherein the effective amount of the powder composition comprises about 80 µg to about 1280 µg of the compound of Formula (I).
132. The method of any one of clauses 129-131, wherein the patient is administered two or more different dosages of the compound of Formula (I) during the administration period.
133. The method of clause 132, wherein the patient is administered two different dosages of the compound of Formula (I) during the administration period.
134. The method of clause 132, wherein the patient is administered three different dosages of the compound of Formula (I) during the administration period.
135. The method of clause 132, wherein the patient is administered four different dosages of the compound of Formula (I) during the administration period.
136. The method of clause 132, wherein the patient is administered five different dosages of the compound of Formula (I) during the administration period.
137. The method of any one of clauses 132-136, wherein prior to receiving a higher dosage of the compound of Formula (I), or a pharmaceutically acceptable salt thereof, the patient is administered a lower dosage for two or more consecutive days.
138. The method of any one of clauses 132-136, wherein prior to receiving a higher dosage of the compound of Formula (I), or a pharmaceutically acceptable salt thereof, the patient is administered a lower dosage for three or more consecutive days.
139. The method of any one of clauses 129-138, wherein the PH is group 1 PH, as classified by the World Health Organization (WHO).
140. The method of any one of clauses 129-138, wherein the PH is group 2 PH, as classified by the WHO.
141. The method of any one of clauses 129-138, wherein the PH is group 3 PH, as classified by the WHO.
142. The method of any one of clauses 129-138, wherein the PH is group 4 PH, as classified by the WHO.
143. The method of any one of clauses 129-138, wherein the PH is group 5 PH, as classified by the WHO.
144. The method of any one of clauses 129-138, wherein the PH is pulmonary arterial hypertension (PAH).
145. The method of clause 144, wherein the pulmonary arterial hypertension is class I pulmonary arterial hypertension, as characterized by the New York Heart Association (NYHA).
146. The method of clause 144, wherein the pulmonary arterial hypertension is class II pulmonary arterial hypertension, as characterized by the NYHA.
147. The method of clause 144, wherein the pulmonary arterial hypertension is class III pulmonary arterial hypertension, as characterized by the NYHA.
148. The method of clause 144, wherein the pulmonary arterial hypertension is class IV pulmonary arterial hypertension, as characterized by the NYHA.
149. The method of clause 141, wherein the PH is portopulmonary hypertension (PPH).
150. The method of clause 141, wherein the PH is PH associated with interstitial lung disease (ILD).
151. The method of clause 150, wherein the ILD comprises one or more lung conditions selected from the group consisting of idiopathic pulmonary fibrosis (IPF), cryptogenic organizing pneumonia (COP), desquamative interstitial pneumonitis, nonspecific interstitial pneumonitis, hypersensitivity pneumonitis, acute interstitial pneumonitis, interstitial pneumonia, connective tissue disease, sarcoidosis or asbestosis.
152. The method of clause 150, wherein the ILD is idiopathic interstitial pneumonia (IIP).
153. The method of clause 150, wherein the ILD is sarcoidosis.
154. The method of clause 150, wherein the ILD is connective tissue disease-associated interstitial lung disease (CTD-ILD).
155. The method of clause 150, wherein the ILD is idiopathic pulmonary fibrosis (IPF).
156. The method of any one of clauses 129-155, wherein treating comprises reducing the pulmonary vascular index (PVRI) of the patient during the administration period, compared to the patient's PVRI prior to the administration period.
157. The method of any one of clauses 129-156, wherein treating comprises reducing the mean pulmonary artery pressure of the patient during the administration period, compared to the mean pulmonary artery pressure of the patient prior to the administration period.
158. The method of any one of clauses 129-157, wherein treating comprises increasing the hypoxemia score of the patient during the hypoxemia score of the patient prior to the administration period.
159. The method of any one of clauses 129-158, wherein decreasing the oxygenation index of the patient during the administration period, compared to the oxygenation index of the patient prior to the administration period.
160. The method of any one of clauses 129-159, wherein treating comprises improving the right heart function of the patient during the administration period, compared to the right heart function of the patient prior to the administration period.
161. The method of any one of clauses 129-160, wherein treating comprises improving exercise capacity of the patient during the administration period, compared to the exercise capacity of the patient prior to the administration period.
162. The method of clause 161, wherein exercise capacity is measured by the six-minute walk test (6MWT).
163. The method of clause 162, wherein improving exercise capacity comprises increasing the patient's distance walked in the 6MWT by at least about 5 meters, at least about 10 meters, at least about 20 meters, at least about 30 meters, at least about 40 meters, or at least about 50 meters during the administration period, compared to the patient's distance walked in the 6MWT prior to the administration period.
164. The method of clause 162, wherein improving exercise capacity comprises increasing the patient's distance walked in the 6MWT by from about 5 meters to about 60 meters, by from about 5 meters to about 50 meters, by from about 10 meters to about 50 meters, by from about 15 meters to about 50 meters, or by from about 20 meters to about 40 meters, during the administration period, compared to the patient's distance walked in the 6MWT prior to the administration period.
165. The method of any one of clauses 129-164, wherein treating comprises improving the quality of life of the patient during the administration period, compared to the quality of life of the patient prior to the administration period.
166. The method of clause 165, wherein the quality of life of the patient is measured by the Cambridge Pulmonary Hypertension Outcome Review (CAMPHOR) Questionnaire.
167. The method of clause 166, wherein treating comprises decreasing the patient's CAMPHOR Questionnaire score during the administration period by from 1 to about 10, from 1 to about 9, from 1 to 8, from 1 to 7, from 1 to 6, from 1 to 5, from 1 to 4, from 1 to 3 or from 1 to 2, compared to the patient's CAMPHOR Questionnaire score prior to the administration period.
168. The method of any one of clauses 129-167, wherein treating comprises increasing the patient's saturation of peripheral capillary oxygenation (SpCh) at rest assessed by pulse oximetry, during the administration period, compared to the patient's SpCh at rest assessed by pulse oximetry prior to the administration period.
169. The method of any one of clauses 129-167, wherein treating comprises improving the patient's lung function during the administration period, as compared to the patient's lung function prior to the administration period.
170. The method of clause 169, wherein improving the patient's lung function comprises increasing the patient's forced vital capacity (FVC) during the administration period, compared to the patient's PVC prior to the administration period.
171. The method of clause 169, wherein improving the patient's lung function comprises increasing the patient's percent predicted forced vital capacity (ppFVC) during the administration period, compared to the patient's ppFVC prior to the administration period.
172. The method of clause 169, wherein improving the patient's lung function comprises increasing the patient's the patient's forced expiratory volume in 1 second (FEV₁) during the administration period, compared to the patient's FEV₁ prior to the administration period.
173. The method of clause 172, wherein increasing the patient's the patient's forced expiratory volume in 1 second (FEV₁) during the administration period comprises increasing the patient's FEVI by from about 5% to about 50%, from about 5% to about 40% or from about 5% to about 30%, compared to the patient's FEV₁ prior to the administration period.
174. The method of clause 172, wherein increasing the FEV₁ comprises increasing by about 5%, about 10%, about 15%, by about 20%, by about 25%, by about 30%, by about 35%, by about 40%, by about 45% or by about 50%.
175. The method of clause 172, wherein increasing the FEV₁ comprises increasing by at least about 5%, at least about 10%, at least about 15%, by at least about 20%, by at least about 25%, by at least about 30%, by at least about 35%, by at least about 40%, by at least about 45% or by at least about 50%.
176. The method of clause 172, wherein increasing the FEV₁ comprises increasing by about 5% to about 50%, by about 5% to about 40%, by about 5% to about 30%, by about 5% to about 20%, by about 10% to about 50%, by about 15% to about 50%, by about 20% to about 50% or by about 25% to about 50%.
177. The method of clause 172, wherein increasing the FEV₁ comprises increasing by of at least about 5%.
178. The method of clause 172, wherein increasing the FEV₁ comprises increasing by from about 5% to about 50%, or from about 10% to about 50%, or from about 15% to about 50%.
179. The method of clause 172, wherein increasing the FEV₁ comprises increasing by about 25 mL to about 500 mL.
180. The method of clause 172, wherein increasing the FEV₁ comprises increasing by about 25 mL to about 250 mL.
181. The method of clause 170, wherein increasing the patient's forced vital capacity (FVC) during the administration period comprises increasing the patient's FVC by about 1%, by about 2%, by about 3%, by about 4%, by about 5%, by about 6%, by about 7%, by about 8%, by about 9%, by about 10%, by about 11%, by about 12%, by about 13%, by about 14%, by about 15%, by about 16%, by about 17%, by about 18%, by about 19%, by about 20%, by about 25%, by about 30%, by about 35%, by about 40%, by about 45%, by about 50%, by about 55%, by about 60%, by about 65%, by about 70%, by about 75%, by about 80%, by about 85% or by about 90%, as compared to a FVC of the patient prior to the administration period.
182. The method of clause 170, wherein increasing the patient's forced vital capacity (FVC) during the administration period comprises increasing the patient's FVC by from about 1% to about 20%, from about 1% to about 15%, from about 1% to about 10%, from about 1% to about 5%, from about 5% to about 50%, from about 5% to about 40%, from about 5% to about 30%, from about 5% to about 20%, from about 10% to about 50%, from about 15% to about 50%, from about 20% to about 50%, or from about 25% to about 50%, as compared to a FVC of the patient prior to the administration period.
183. The method of clause 169, wherein improving the patient's lung function comprises increasing the patient's total lung capacity (TLC) during the administration period, compared to the patient's TLC prior to the administration period.
184. The method of clause 183, wherein increasing the patient's total lung capacity (TLC) comprises increasing the patient's TLC by at least about 1%, at least about 2%, at least about 5%, at least about 10%, at least about 15%, at least about 20%, at least about 25%, at least about 30%, at least about 35%, at least about 40%, at least about 45%, or by at least about 50% during the administration period, compared to the patient's TLC prior to the administration period.
185. The method of clause 183, wherein increasing the patient's total lung capacity (TLC) comprises increasing the patient's TLC by from about 1% to about 50%, by from about 5% to about 50%, by from about 5% to about 40%, by from about 5% to about 30%, by from about 5% to about 20%, by from about 10% to about 50%, by from about 15% to about 50%, by from about 20% to about 50%, or by from about 25% to about 50%.during the administration period, compared to the patient's TLC prior to the administration period.
186. The method of any one of clauses 129-185, wherein the administration period is from about 1 year to about 30 years.
187. The method of clause 186, wherein the administration period is from about 1 year to about 25 years.
188. The method of clause 186, wherein the administration period is from about 5 years to about 30 years.
189. The method of clause 186, wherein the administration period is from about 1 year to about 20 years.
190. The method of clause 186, wherein the administration period is from about 1 year to about 15 years.
191. The method of clause 186, wherein the administration period is from about 1 year to about 10 years.
192. The method of clause 186, wherein the administration period is from about 1 year to about 5 years.
193. The method of any one of clauses 129-192, wherein the dry powder inhaler (DPI) is a capsule-based DPI, and the composition is present in a single DPI capsule.
194. The method of any one of clauses 129-192, wherein the dry powder inhaler (DPI) is a capsule-based DPI, and the composition is split between two DPI capsules.
195. A system for treating pulmonary hypertension, comprising (i) a powder composition and (ii) a dry powder inhaler,
the powder comprising:
(a) from about 0.5 wt% to about 5 wt% of a compound of Formula (I) 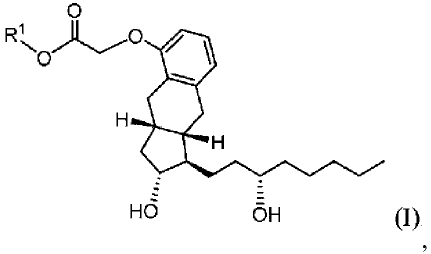 or stereoisomer or a pharmaceutically acceptable salt thereof, wherein R1 is tetradecyl, pentadecyl, hexadecyl, heptadecyl, or octadecyl,
(b) from about 10 wt% to about 61wt% leucine, and
(c) trehalose, mannitol or a combination thereof,

wherein the sum of (a), (b), and (c) is 100%; and
wherein the dry powder inhaler provides an aerosol comprising fine particle fraction (FPF) greater than or equal to about 68%, as measured using a Next Generation Impactor (NGI), with a inspiratory flow rate of 60 liter per minute (L/min), inspiratory volume of 4L, and inhalation acceleration rate of 0.5-20 L/sec².
196. The system of clause 195, wherein the dry powder inhaler is a high resistance dry powder inhaler.
197. The powder, method or system of any one of clauses 1-196, wherein the dry powder inhaler has a resistance ranging from about 0.030 to about 0.040 kPA^{1/2} (L/min⁻¹)⁻¹.
198. The powder, method or system of any one of embodiments 1-197, wherein the dry powder inhaler has a resistance ranging from about 0.034 to about 0.036 kPA^{1/2} (L/min⁻¹)⁻¹.

## Claims

1. A powder composition comprising treprostinil palmitil, or a pharmaceutically acceptable salt thereof, wherein the powder composition is formulated to administer once daily to the lungs of a patient by inhalation about 80 µg to about 1280 µg of treprostinil palmitil or a pharmaceutically acceptable salt thereof.

2. The powder composition of claim 1, wherein when the powder composition is administered via a dry powder inhaler, the dry powder inhaler provides an aerosol comprising a fine particle fraction (FPF) greater than or equal to about 68%.

3. The powder composition of claim 2, wherein when the powder composition is administered via a dry powder inhaler, the dry powder inhaler provides an FPF of about 68% to about 80%, about 68% to about 73%, or about 78% to about 80%

4. The powder composition of claim 2 or 3, wherein when the powder composition is administered via a dry powder inhaler, the dry powder inhaler provides one or more of:
(i) a fine particle dose (FPD) greater than or equal to 50% of a loaded dose of the powder composition when measured using a Next Generation Impactor (NGI),
(ii) an aerosol comprising an emitted dose (ED) greater than or equal to 72% when measured using an NGI, or
(iii) an aerosol comprising a mass mean aerodynamic diameter (MMAD) less than 5 µm (e.g., from about 0.75 µm to about 3.0 µm or from about 0.8 µm to about 2.0 µm) when measured using an NGI,
wherein the NGI has a inspiratory flow rate of 60 liter per minute (L/min), inspiratory volume of 4 L, and inhalation acceleration rate of 0.5-20 L/sec².

5. The powder composition of claim 4, wherein when the powder composition is administered via a dry powder inhaler, the dry powder inhaler provides an FPD of about 53% to about 77% when measured using an NGI.

6. The powder composition of claim 4 or 5, wherein when the powder composition is administered via a dry powder inhaler, the dry powder inhaler provides an emitted dose of about 79% to about 89% when measured using an NGI.

7. The powder composition of any of claims 4-6, wherein when the powder composition is administered via a dry powder inhaler, the dry powder inhaler provides an aerosol comprising an MMAD about 0.75 µm to about 3.0 µm when measured using an NGI.

8. The powder composition of any preceding claims, wherein the powder composition comprises particles.

9. The powder composition of claim 8, wherein the particles have a D10 of about 0.3 µm, a D50 of about 1.7 µm, and/or a D90 of about 3.8 µm.

10. The powder composition of any preceding claim, wherein the powder composition is formulated to administer once daily to the lungs of the patient by inhalation about 80 µg to about 675 µg of treprostinil palmitil or a pharmaceutically acceptable salt thereof.

11. The powder composition of any preceding claim, wherein the powder composition comprises about 80 µg, about 112.5 µg, about 160 µg, about 225 µg, about 240 µg, about 320 µg, about 400 µg, about 450 µg, about 480 µg, about 640 µg, about 675 µg, about 720 µg, about 800 µg, about 960 µg, about 1040 µg, about 1120 µg, about 1200 µg, or about 1280 µg of treprostinil palmitil or a pharmaceutically acceptable salt thereof.

12. The powder composition of any preceding claim for use in a method of treatment.

13. The powder composition for use of claim 12, wherein the powder composition is aerosolized via a dry powder inhaler (DPI) to provide an aerosolized composition.

14. The powder composition for use of claim 12, wherein the powder composition is delivered to the lungs of a patient via inhalation using a DPI.

15. The powder composition for use of claim 14, wherein the DPI is a capsule-based DPI, and the composition is present in a single DPI capsule or is split between two DPI capsules.

16. The powder composition for use of any of claims 13-15 wherein the DPI has a resistance ranging from 0.025 to 0.040 kPA^{1/2} (L/min⁻¹)⁻¹.
